# EUROPEAN PATENT APPLICATION

(11) **EP 4 092 026 A1**
(43) Date of publication of application: **23.11.2022**
(21) Application number: 21741983.7
(22) Date of filing: 12.01.2021
(51) Int. Cl.: C07D 471/04, C07D 487/04, A61K 31/519, A61P 35/00

(54) **ARYL OR HETEROARYL PYRIDONE OR PYRIMIDINE DERIVATIVE, PREPARATION METHOD AND USE THEREOF**

(30) Priority: 13.01.2020 CN 202010033277; 12.06.2020 CN 202010534664; 25.12.2020 CN 202011563650
(71) Applicant: Suzhou Zelgen Biopharmaceutical Co., Ltd., Jiangsu 215300 (CN); Shanghai Zelgen Pharma.Tech Co., Ltd., Shanghai 201203 (CN)
(72) Inventor: LV, Binhua, Shanghai 201203 (CN); CUI, Dawei, Shanghai 201203 (CN); LIU, Lianjun, Shanghai 201203 (CN)
(74) Representative: dompatent von Kreisler Selting Werner - Partnerschaft von Patent- und Rechtsanwälten mbB
(86) International application number: PCT/CN2021/071331
(87) International publication number: WO 2021/143693

(57) **Abstract**

The present invention relates to an aryl or heteroaryl pyridone or pyrimidine derivative, and a preparation method and use thereof. Specifically, the compounds of the present invention have a structure represented by formula (I). Further disclosed in the present invention are preparation methods for said compounds, and the use of said compounds as KRAS^{G12C} inhibitors. The compounds have an excellent ability to selectively inhibit KRAS^{G12C}, improved pharmacodynamic, pharmacokinetic performance, and reduced toxic side effects.

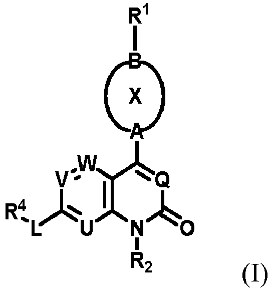

## Description

### TECHNICAL FIELD

The invention belongs to the field of pharmaceuticals, and specifically relates to an aryl or heteroaryl pyridone or pyrimidinone derivative and a preparation method and application thereof.

### BACKGROUND OF THE INVENTION

Lung cancer is one of the important causes of human cancer death. Lung cancer can be divided into small cell lung cancer (SCLC) and non-small cell lung cancer (NSCLC) according to cell type, of which NSCLC accounts for 85% of all lung cancer patients. According to statistics, the global NSCLC market was approximately $20.9 billion in 2016, of which the US market occupied half, followed by Japan, Germany and China. Based on current trends, the non-small cell lung cancer market continues to grow and is expected to reach $54 billion worldwide by 2023 ( Nature, 2018;553(7689):446-454).

At present, the main therapeutic drugs for NSCLC include chemotherapy drugs, molecular targeting drugs, and tumor immunotherapy, etc. Among them, chemotherapy drugs mainly include gemcitabine, paclitaxel, and platinum drugs, but these drugs generally have poor selectivity and high toxicity, leading to relatively strong toxic and side effects. In recent years, the molecular targeted drugs have gradually become a research hotspot due to their obvious advantages such as high selectivity, relatively small toxic and side effects, and the ability to achieve precision therapy. Existing NSCLC molecular targeted drugs include EGFR inhibitors (such as afatinib, gefitinib, erlotinib, lapatinib, dactinib, icotinib, pyrlotinib, Rociletinib, osimertinib, etc.), ALK inhibitors (such as seritinib, alitinib, brigatinib, lorlatinib, ocatinib, etc.), and VEGFR inhibitors (sorafenib, regorafenib, cabozantinib, sunitinib, donafenib, etc.).

In the patients with lung cancer, KRAS mutation is often detected, accounting for about 32% of all carcinogenic mutations. The KRAS^{G12C} mutation accounted for 44% of all carcinogenic mutations in NSCLC. So far, there is still no drug for KRAS ^{G12C} mutations on the market.

Since KRAS ^{G12C} target protein is related to a variety of diseases in pathology, novel KRAS ^{G12C} inhibitors are currently needed for clinical treatment. High selective and highly active KRAS ^{G12C} inhibitors have a more urgent clinical need for more effective treatment of KRAS^{G12C} mutation-induced cancers and other diseases, as well as the potential to reduce off-target effects.

### SUMMARY OF THE INVENTION

The object of the present invention is to provide a new class of compounds with selective inhibition of KRAS ^{G12C} and/or better pharmacodynamic properties and the use thereof.

In the first aspect of the present invention, it provides a compound of formula (I), or stereoisomer, tautomer, crystal form, pharmaceutically acceptable salt, hydrate, solvate or prodrug thereof: 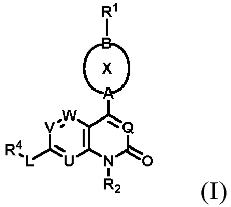 wherein:
A and B are the same or different, and are each independently selected from the group consisting of CH, CR⁵ and N;
X is selected from the group consisting of 4-14 membered saturated or unsaturated heterocyclyl, C₄-C₁₄ cycloalkyl, C₆-C₁₄ aryl and 5-14 membered heteroaryl, wherein the heterocyclyl, cycloalkyl, aryl or heteroaryl can optionally be substituted by one or more (e. g., 2, 3 or 4) R⁸;
U, V, W and Q are the same or different, and are each independently selected from the group consisting of CH, CR³ and N;
R¹ is selected from the group consisting of **-C(O)C(R*^{A}*)** 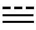 **C(R*^{B}*)*ₚ***, **-S(O)₂C(R*^{A}*)** 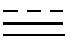 **C(R*^{B}*)*ₚ***, **-NR⁶C(O)C(R*^{A}*)** 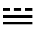 **C(R*^{B}*)*ₚ*** and **-NR⁶S(O₂C(R*^{A}*)** 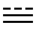 **C(R*^{B}*)*ₚ*;** wherein " 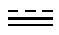 " represents double bond " 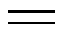 " or triple bond " 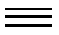 ";
R*^{A}* is absent, or is independently selected from the group consisting of hydrogen, deuterium, fluorine, cyano and C₁-C₃ alkyl; R*^{B}* is each independently selected from the group consisting of hydrogen, deuterium, cyano and C₁-C₃ alkyl; wherein, the alkyl can be substituted by one or more (e. g., 2, 3 or 4) substituents selected from the group consisting of deuterium, halogen, cyano, amino, C₃-C₇ cycloalkyl, 4-7-membered heterocyclyl, NHR⁹ and NR⁹R¹⁰; R⁹ and R¹⁰ are each independently C₁-C₃ alkyl; or R⁹ and R¹⁰ together with the N atom to which they are attached form a substituted or unsubstituted 4-8-membered heterocyclyl;
p is an integer of 1 or 2;
R² is selected from the substituted group consisting of C₆-C₁₄ aryl and 5-14-membered heteroaryl, wherein the substituted means being substituted by one or more groups selected from the group consisting of R', -SR', -SOR', -SO₂R', -SO₂NR'R", -NR'SO₂R" and -P(= O)R'R"; with the proviso that the C₆-C₁₄ aryl or 5-14-membered heteroaryl contains at least one substituent selected from -SR', -SOR', -SO₂R', -SO₂NR'R", -NR'SO₂R", or -P(= O)R'R"; R' and R" are the same or different, and are each independently selected from the substituted or unsubstituted group consisting of hydrogen, deuterium, halogen, nitro, hydroxyl, cyano, ester group, amino, amido, C₁-C₆ alkyl, C₃-C₈ cycloalkyl, C₄-C₁₀ cycloalkenyl, 4-8 membered heterocyclyl, C₆-C₁₄ aryl and 5-14 membered heteroaryl; or when R' and R" are attached to the same N atom, R' and R" together with the N atom to which they are attached form a substituted or unsubstituted 4-8-membered heterocyclyl;
R³ is selected from the substituted or unsubstituted group consisting of hydrogen, deuterium, C₁-C₁₈ alkyl, deuterated C₁-C₁₈ alkyl, halogenated C₁-C₁₈ alkyl, C₃-C₂₀ cycloalkyl, C₁-C₁₈ alkoxy, deuterated C₁-C₁₈ alkoxy, halogenated C₁-C₁₈ alkoxy, halogen, nitro, hydroxyl, cyano, ester group, amino, amido, sulfonamido, carbamido, 4-20-membered heterocyclyl, C₆-C₁₄ aryl and 5-14-membered heteroaryl;
L is selected from the group consisting of bond, -C(O)- and C₁-C₃ alkylene;
R⁴ is selected from the substituted or unsubstituted group consisting of hydrogen, deuterium, C₁-C₁₈ alkyl, deuterated C₁-C₁₈ alkyl, halogenated C₁-C₁₈ alkyl, C₃-C₂₀ cycloalkyl, C₁-C₁₈ alkoxy, deuterated C₁-C₁₈ alkoxy, halogenated C₁-C₁₈ alkoxy, amino, hydroxyl, 4-20-membered heterocyclyl, C₆-C₁₄ aryl and 5-14 heteroaryl;
R⁵ is selected from the substituted or unsubstituted group consisting of hydrogen, deuterium, C₁-C₁₈ alkyl, deuterated C₁-C₁₈ alkyl, halogenated C₁-C₁₈ alkyl, C₃-C₂₀ cycloalkyl, C₁-C₁₈ alkoxy, deuterated C₁-C₁₈ alkoxy, halogenated C₁-C₁₈ alkoxy, halogen, nitro, hydroxyl, cyano, ester group, amino, amido, sulfonamido, carbamido, 4-20-membered heterocyclyl, C₆-C₁₄ aryl and 5-14-membered heteroaryl;
R⁶ is selected from the substituted or unsubstituted group consisting of hydrogen, deuterium, C₁-C₁₈ alkyl, deuterated C₁-C₁₈ alkyl, halogenated C₁-C₁₈ alkyl, C₃-C₂₀ cycloalkyl, C₁-C₁₈ alkoxy, deuterated C₁-C₁₈ alkoxy, halogenated C₁-C₁₈ alkoxy, 4-20 membered heterocyclyl, C₆-C₁₄ aryl and 5-14 membered heteroaryl;
R⁸ is independently selected from the substituted or unsubstituted group consisting of hydrogen, deuterium, C₁-C₁₈ alkyl, deuterated C₁-C₁₈ alkyl, halogenated C₁-C₁₈ alkyl, C₃-C₂₀ cycloalkyl, C₁-C₁₈ alkoxy, deuterated C₁-C₁₈ alkoxy, halogenated C₁-C₁₈ alkoxy, amino, hydroxyl, 4-20 membered heterocyclyl, C₆-C₁₄ aryl and 5-14 membered heteroaryl;
wherein, unless otherwise stated, the "substituted" means being substituted by one or more substituents selected from the group consisting of hydrogen, deuterium, C₁-C₁₈ alkyl, deuterated C₁-C₁₈ alkyl, halogenated C₁-C₁₈ alkyl, C₃-C₂₀ cycloalkyl, C₁-C₁₈ alkoxy, deuterated C₁-C₁₈ alkoxy, halogenated C₁-C₁₈ alkoxy, C₆-C₁₄ aryl, 5-14-membered heteroaryl, 4-20-membered heterocyclyl, halogen, nitro, hydroxyl, cyano, ester group, amino, NR_{b}C(=O)ORₑ, OC(=O)Rₑ, OC(=O)NR_{b}Rₑ, amido, sulfonamido and carbamido; R_{b} and Rₑ can be independently hydrogen, deuterium, C1-C6 alkyl, C3-C8 cycloalkyl, 4-8-membered heterocyclyl, 5-14-membered heteroaryl or C6-C14 aryl, or R_{b} and Rₑ together with the N atom can form 4-8-membered heterocyclyl; Rₑ can be independently hydrogen, C1-C6 alkyl, C3-C8 cycloalkyl, C2-C6 alkenyl, C3-C6 cycloalkenyl, C2-C6 alkynyl, 4-8-membered heterocyclyl, 5-14-membered heteroaryl or C6-C14 aryl;
with the proviso that
when B is N, R¹ is selected from the group consisting of -**C(O)C(R*^{A}*)** 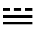 **C(R***^{B}***)*ₚ*** and **-S(O)₂C(R*^{A}*)** 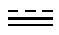 **C(R*^{B}*)*ₚ*,** wherein p is 2;
when B is CH or CR⁵, R¹ is selected from the group consisting of **-NR⁶C(O)C(R*^{A}*)** 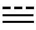 **C(R*^{B}*)*ₚ*** and **-NR⁶S(O)₂C(R*^{A}*)** 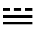 **C(R*^{B}*)*ₚ*,** wherein p is 2;
when V is C(Cl), R² is not selected from: 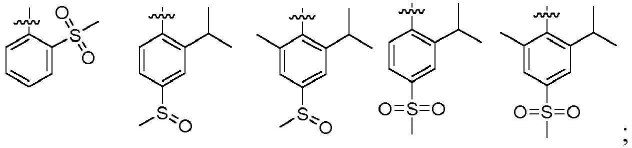 and L is not selected from bond; and R⁴ is not selected from:

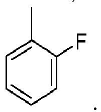

In another preferred embodiment, R⁸ is independently selected from the substituted or unsubstituted group consisting of hydrogen, deuterium, C₁-C₆ alkyl, deuterated C₁-C₆ alkyl, halogenated C₁-C₆ alkyl, C₃-C₈ cycloalkyl, C₁-C₆ alkoxy, deuterated C₁-C₆ alkoxy, halogenated C₁-C₆ alkoxy, amino, hydroxyl and 4-8-membered heterocyclyl; the substituted refers to be substituted by one or more substituents selected from the group consisting of hydrogen, deuterium, halogen, nitro, hydroxyl, cyano, ester group, amino, amido, sulfonamido and carbamido.

In another preferred embodiment, R⁸ is independently selected from the substituted or unsubstituted group consisting of hydrogen, deuterium, C₁-C₁₈ alkyl, deuterated C₁-C₁₈ alkyl and halogenated C₁-C₁₈ alkyl; wherein the substituted means being substituted by cyano.

In another preferred embodiment, R¹ is selected from the group consisting of **-C(O)C(R*^{A}*)=C(R*^{B}*)*₂*, -S(O)₂C(R*^{A}*)=C(R*^{B}*)*₂*, -NR⁶C(O)C(R*^{A}*)=C(R*^{B}*)*₂* and -NR⁶S(O)₂C(R*^{A}*)=C(R*^{B}*)*₂*;**
wherein, R*^{A}* is independently selected from the group consisting of hydrogen, deuterium, fluorine, cyano and C₁-C₃ alkyl; each R*^{B}* is the same or different, and is independently selected from the group consisting of hydrogen, deuterium, cyano and C₁-C₃ alkyl; wherein, the alkyl can be substituted by one or more substituents selected from the group consisting of deuterium, halogen, cyano, amino, C₃-C₇ cycloalkyl, 4-7-membered heterocyclyl, NHR⁹ and NR⁹R¹⁰; R⁹ and R¹⁰ are each independently C₁-C₃ alkyl; or R⁹ and R¹⁰ together with the N atom which they are attached to form a substituted or unsubstituted 4-8-membered heterocyclyl;
R⁶ is selected from the substituted or unsubstituted group consisting of hydrogen, deuterium, C₁-C₆ alkyl, deuterated C₁-C₆ alkyl, halogenated C₁-C₆ alkyl, C₃-C₈ cycloalkyl, C₁-C₆ alkoxy, deuterated C₁-C₆ alkoxy, halogenated C₁-C₆ alkoxy, 4-8 membered heterocyclyl, C₆-C₁₄ aryl and 5-14 membered heteroaryl;
wherein, unless otherwise stated, the "substituted" means being substituted by one or more substituents selected from the group consisting of hydrogen, deuterium, C₁-C₆ alkyl, deuterated C₁-C₆ alkyl, halogenated C₁-C₆ alkyl, C₃-C₈ cycloalkyl, C₁-C₆ alkoxy, deuterated C₁-C₆ alkoxy, halogenated C₁-C₆ alkoxy, C₆-C₁₀ aryl, 5-10 membered heteroaryl, 4-8 membered heterocyclyl, halogen, nitro, hydroxy, cyano, ester group, amino, amido, sulfonamido and carbamido.
with the proviso that
when B is N, R¹ is selected from the group consisting of **-C(O)C(R*^{A}*)=C(R*^{B}*)*₂*** and **-S(O)₂C(R*^{A}*)=C(R*^{B}*)*₂*;**
when B is CH or CR⁵, R¹ is selected from the group consisting of **-NR⁶C(O)C(R*^{A}*)=C(R*^{B}*)*₂*** and **-NR⁶S(O)₂C(R*^{A}*)=C(R*^{B}*)*₂*.**

In another preferred embodiment, R¹ is **-C(O)C(R*^{A}*)=C(R*^{B}*)*₂*,** wherein **R*^{A}*** is independently selected from the group consisting of hydrogen and fluorine; each **R*^{B}*** is the same or different, and is independently selected from the group consisting of hydrogen and C₁-C₃ alkyl, wherein the alkyl can be substituted by one or more substituents selected from the group consisting of deuterium, halogen, cyano, amino, C₃-C₇ cycloalkyl, 4-7 membered heterocyclyl, NHR⁹ and NR⁹R¹⁰; R⁹ and R¹⁰ are each independently C₁-C₃ alkyl; or R⁹ and R¹⁰ together with the N atom to which they are attached form a 4-8 membered heterocyclyl.

In another preferred embodiment, R² is selected from the substituted group consisting of phenyl and 5-6-membered heteroaryl, wherein the substituted in R² means being substituted by one or more substituents selected from the group consisting of R', -SO₂R', -SO₂NR'R", -NR'SO₂R", and -P(= O)R'R"; R' and R" are the same or different, and are each independently selected from the substituted or unsubstituted group consisting of hydrogen, deuterium, halogen, nitro, hydroxyl, cyano, ester group, amino, amido, C₁-C₆ alkyl, C₃-C₆ cycloalkyl, C₄-C₆ cycloalkenyl, 4-8 membered heterocyclyl, C₆-C₁₀ aryl and 5-10 membered heteroaryl; or when R' and R" are attached to the same N atom, R' and R" together with the N atom to which they are attached form a substituted or unsubstituted 4-6 membered heterocyclyl; the substituted in R' and R" means being substituted by one or more substituents selected from the group consisting of hydrogen, deuterium, C₁-C₆ alkyl, deuterated C₁-C₆ alkyl, halogenated C₁-C₆ alkyl, C₃-C₈ cycloalkyl, C₁-C₆ alkoxy, deuterated C₁-C₆ alkoxy, halogenated C₁-C₆ alkoxy, C₆-C₁₀ aryl, 5-10 membered heteroaryl, 4-8 membered heterocyclyl, halogen, nitro, hydroxyl, cyano, ester group, amino, amido, sulfonamido and carbamido.

In another preferred embodiment, R³ is halogen.

In another preferred embodiment, R⁴ is selected from the substituted or unsubstituted group consisting of phenyl and 5-6-membered heteroaryl, wherein the substituted means being substituted by one or more substituents selected from the group consisting of hydrogen, deuterium, halogen, ester group, NR_{b}C(=O)ORₑ, OC(= O)Rₑ, OC(= O)NR_{b}Rₑ, amino, halogenated C₁-C₁₈ alkyl (preferably halogenated C₁-C₆ alkyl, more preferably halogenated C₁-C₃ alkyl) and hydroxyl; R_{b} and R_{c} can be independently hydrogen, deuterium, C1-C6 alkyl, C3-C8 cycloalkyl, 4-8-membered heterocyclyl, 5-14-membered heteroaryl or C6-C14 aryl, or R_{b} and Rₑ together with the N atom can form 4-8-membered heterocyclyl; Rₑ can be independently hydrogen, C1-C6 alkyl, C3-C8 cycloalkyl, C2-C6 alkenyl, C3-C6 cycloalkenyl, C2-C6 alkynyl, 4-8-membered heterocyclyl, 5-14-membered heteroaryl, or C6-C14 aryl.

In another preferred embodiment, A and B are the same or different, and are each independently CH or N.

In another preferred embodiment, Q is N.

In another preferred embodiment, U is N.

In another preferred embodiment, V, W are each independently CR³, and R³ is H or halogen.

In another preferred embodiment, the compound of formula (I), or stereoisomer, tautomer, crystal form, pharmaceutically acceptable salt, hydrate, solvate or prodrug thereof, has the structure shown in formula (II-A) or (II-B): 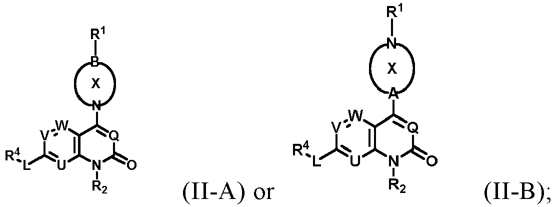 wherein:
R¹, R², R⁴, A, B, L, X, U, V, W and Q are defined as above.

In another preferred embodiment, the compound of formula (I), or stereoisomer, tautomer, crystal form, pharmaceutically acceptable salt, hydrate, solvate or prodrug thereof, has the structure shown in formula (III): 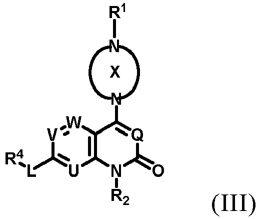 R¹, R², R⁴, X, L, U, V, W and Q are defined as above.

In another preferred embodiment, the compound of formula (I), or stereoisomer, tautomer, crystal form, pharmaceutically acceptable salt, hydrate, solvate or prodrug thereof, has the structure shown in formula (IV): 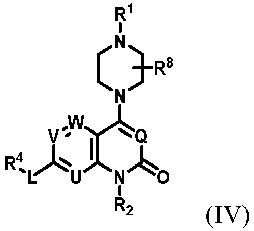 wherein:
R¹, R², R⁴, R⁸, L, U, V, W and Q are defined as above.

In another preferred embodiment, the compound of formula (I), or stereoisomer, tautomer, crystal form, pharmaceutically acceptable salt, hydrate, solvate or prodrug thereof, has the structure shown in formula (V): 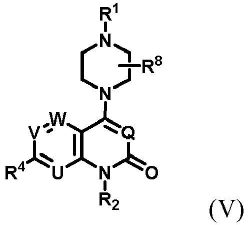 wherein:
R¹, R², R⁴, R⁸, U, V, W and Q are defined as above.

In another preferred embodiment, the compound of formula (I), or stereoisomer, tautomer, crystal form, pharmaceutically acceptable salt, hydrate, solvate or prodrug thereof, has the structure shown in formula (VI): 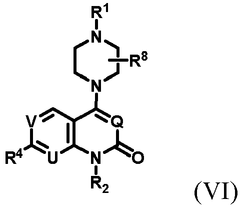 wherein:
R¹, R², R⁴, R⁸, U, V and Q are defined as above.

In another preferred embodiment, the compound of formula (I), or stereoisomer, tautomer, crystal form, pharmaceutically acceptable salt, hydrate, solvate or prodrug thereof, has the structure shown in formula (VII): 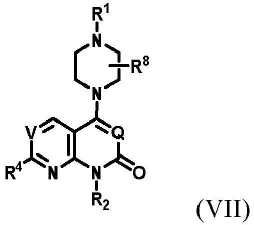 wherein:
R¹, R², R⁴, R⁸, V and Q are defined as above.

In another preferred embodiment, the compound of formula (I), or stereoisomer, tautomer, crystal form, pharmaceutically acceptable salt, hydrate, solvate or prodrug thereof, has the structure shown in formula (VIII): 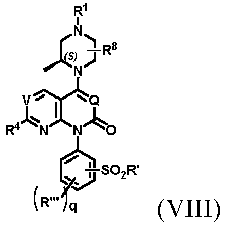 wherein,
R'" is selected from the substituted or unsubstituted group consisting of hydrogen, deuterium, halogen, nitro, hydroxyl, cyano, ester group, amino, amido, C₁-C₆ alkyl, C₃-C₈ cycloalkyl , C₄-C₁₀ cycloalkenyl, 4-8 membered heterocyclyl, C₆-C₁₄ aryl , 5-14 membered heteroaryl, wherein, the substituted means being substituted by one or more substituents selected from the group consisting of deuterium, halogen, nitro, hydroxyl, cyano, ester group, amino, amido, C₁-C₆ alkyl, C₃-C₈ cycloalkyl, C₄-C₁₀ cycloalkenyl, 4-8-membered heterocyclyl, C₆-C₁₄ aryl and 5-14 membered heteroaryl;
q is selected from 1, 2, 3 or 4;
R¹, R⁴, R⁸, R', V and Q are defined as above.

In another preferred embodiment, in 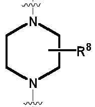 moiety, the number of R⁸ can be 1, 2, 3 or 4, or two adjacent R⁸ together with the C atom to which they are attached can form a C₃-C₆ cycloalkyl.

In another preferred embodiment the 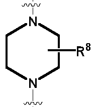 is selected from the group consisting of 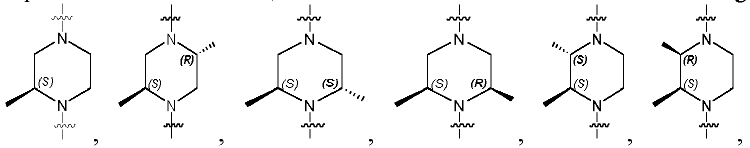 and

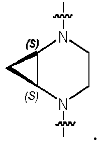

In another preferred embodiment, the 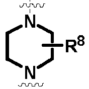 moiety is selected from 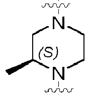 or

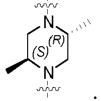

In another preferred embodiment, R¹ is selected from 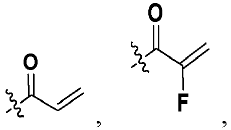 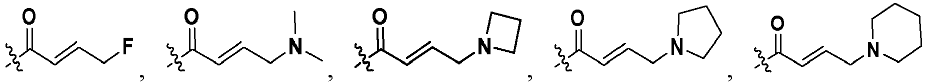 or

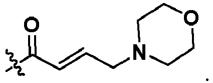

In another preferred embodiment, the compound of formula (I), or stereoisomer, tautomer, crystal form, pharmaceutically acceptable salt, hydrate, solvate or prodrug thereof, has the structure shown in formula (IX): 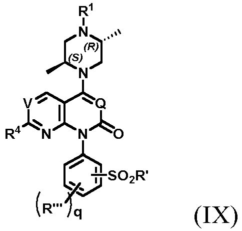 wherein,
R¹ is selected from 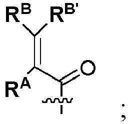 wherein, R^{A} is selected from H, D, halogen or cyano; R^{B} and R^{B'} are the same or different, and are each independently selected from H, D, halogen, cyano, or substituted or unsubstituted C₁-C₃ alkyl; wherein, the substituted means being substituted by one or more substituents selected from the group consisting of D, halogen, cyano, C₁-C₃ alkyl, C₃-C₆ cycloalkyl, 4-6-membered heterocyclyl and NR^{IV}R^{V}; R^{IV} and R^{V} are the same or different, and are each independently selected from H, C₁-C₃ alkyl, C₃-C₆ cycloalkyl or 4-6-membered heterocyclyl; or R^{IV}, R^{V} and adjacent N cyclize together to form 4-6-membered heterocyclyl;
R⁴, R', V, Q, R‴ and q are defined as above.

In another preferred embodiment, R'" is selected from the substituted or unsubstituted group consisting of C₁-C₆ alkyl, C₃-C₈ cycloalkyl, C₄-C₁₀ cycloalkenyl, 4-8 membered heterocyclyl, C₆-C₁₀ aryl and 5-10 membered heteroaryl, wherein the substituted means being substituted by one or more substituents selected from the group consisting of deuterium, halogen, nitro, hydroxyl, cyano, ester group, amino, amido, C₁-C₆ alkyl, C₃-C₈ cycloalkyl , 4-8-membered heterocyclyl.

In another preferred embodiment, R'" is selected from the substituted or unsubstituted group consisting of C₁-C₆ alkyl, C₃-C₆ cycloalkyl, C₄-C₆ cycloalkenyl and 4-6 membered heterocyclyl, wherein the substituted means being substituted by one or more substituents selected from the group consisting of deuterium, halogen, nitro, hydroxyl, cyano, ester group, amino, amido and C₁-C₃ alkyl.

In another preferred embodiment, R'" is selected from the substituted or unsubstituted group consisting of C₃-C₈ cycloalkyl and 4-8 membered heterocyclyl, wherein the substituted means being substituted by one or more substituents selected from the group consisting of deuterium, halogen, nitro, hydroxyl, cyano, ester group, amino, amido and C₁-C₃ alkyl.

In another preferred embodiment, R² is selected from: 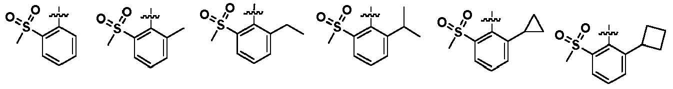 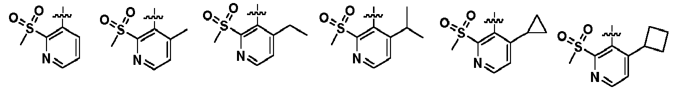 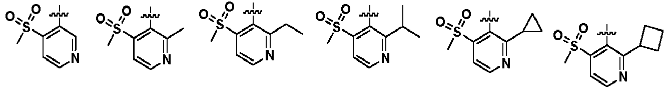 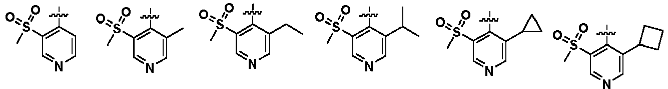 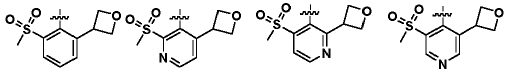 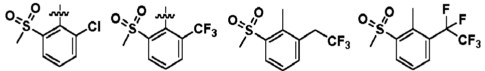 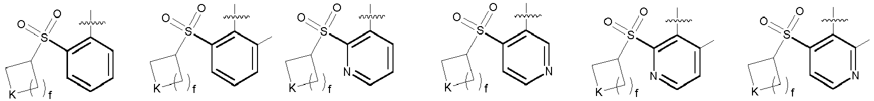 K=O, S,CH₂ or NH; f=0, 1 or 2

In another preferred embodiment, R² is selected from the group consisting of

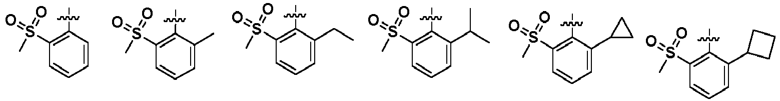

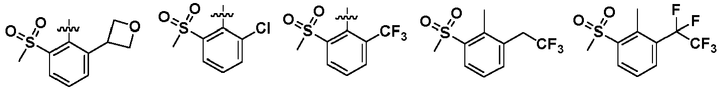

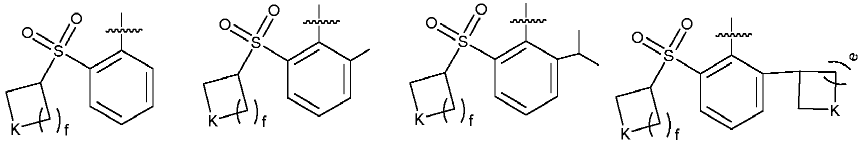

K is independently O, S, CH₂ or NH; e and f are each independently 0, 1 or 2, preferably H in the above-mentioned groups may be optionally substituted by deuterium, halogen, nitro, hydroxyl, cyano, ester group, amino, amido, or C₁-C₃ alkyl.

In another preferred embodiment, the 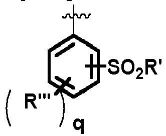 moiety is selected from the group consisting of

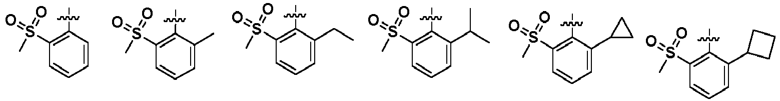

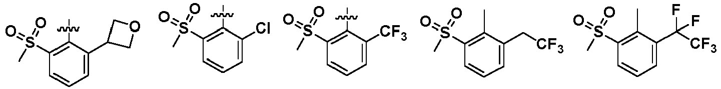

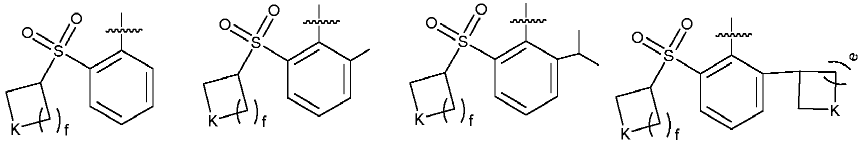

K is independently O, S, CH₂ or NH; e and f are each independently 0, 1 or 2, preferably H in the above-mentioned groups may be optionally substituted by deuterium, halogen, nitro, hydroxyl, cyano, ester group, amino, amido, or C₁-C₃ alkyl.

In another preferred embodiment, the compound of formula (I), or stereoisomer, tautomer, crystal form, pharmaceutically acceptable salt, hydrate, solvate or prodrug thereof, has the structure shown in formula (X) or (XI): 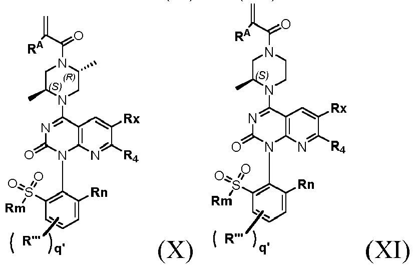 wherein,
R⁴ is selected from substituted or unsubstituted C₆-C₁₄ aryl or 5-10 membered heteroaryl, wherein the substituted means being substituted by one or more (such as 2, 3, 4 or 5) substituents selected from the group consisting of deuterium, halogen, ester group, cyano, NR_{b}C(=O)ORₑ, OC (= O)Rₑ, OC (= O)NR_{b}Rₑ, amino, C₁-C₆ alkyl, halogenated C₁-C₆ alkyl, and hydroxyl; R_{b} and R_{c} can be independently hydrogen, deuterium, C₁-C₆ alkyl, C₃-C₈ cycloalkyl, 4-8 membered heterocyclyl, 5-14 membered heteroaryl or C₆-C₁₄ aryl, or R_{b} and Rₑ together with the N atom can form 4-8 membered heterocyclyl; Rₑ can be independently hydrogen, C₁-C₆ alkyl, C₃-C₈ cycloalkyl, C₂-C₆ alkenyl, C₃-C₆ cycloalkenyl, C₂-C₆ alkynyl, 4-8 membered heterocyclyl, 5-14-membered heteroaryl or C₆-C₁₄ aryl;
Rm is selected from the substituted or unsubstituted group consisting of amino, C₁-C₆ alkyl, C₃-C₆ cycloalkyl and 4-6 membered heterocyclyl, wherein the substituted means being substituted by one or more (such as 2, 3, 4) substituents selected from the group consisting of deuterium, halogen, nitro, hydroxyl, cyano, ester group, amino, amido, C₁-C₃ alkyl, C3-C6 cycloalkyl and 4-6 membered heterocyclyl;
Rn is selected from the substituted or unsubstituted group consisting of amino, C₁-C₆ alkyl, C₁-C₆ alkoxy, C₃-C₆ cycloalkyl , -O-C₃-C₆ cycloalkyl, C₁-C₆ alkyl C₃-C₆ cycloalkyl, -O-C₁-C₆ alkyl C₃-C₆ cycloalkyl and 4-6 membered heterocyclyl, wherein the substituted means being substituted by one or more (such as 2, 3, 4) substituents selected from the group consisting of deuterium, halogen, nitro, hydroxyl, cyano, ester group, amino, amido, C₁-C₃ alkyl, halogenated C₁-C₃ alkyl, C3-C6 cycloalkyl and 4-6 membered heterocyclyl;
Rx is selected from F or Cl;
R^{A} is selected from H, D, or halogen, and preferably R^{A} is selected from H or F;
R'" is defined as above;
q' is selected from 0, 1, 2, or 3.

In another preferred embodiment, Rn is selected from the substituted or unsubstituted group consisting of ethyl, n-propyl, isopropyl, cyclopropyl, cyclobutyl, cyclopentyl, cyclohexyl, cyclopropylamino, azetidinyl, azacyclopentanyl, azacyclohexanyl, oxiranyl, oxetanyl, oxecyclopentanyl, oxacyclohexanyl, wherein the substituted means being substituted by one or more (such as 2, 3, 4) substituents selected from the group consisting of deuterium, halogen, nitro, hydroxyl, cyano, ester group, amino, amido, C₁-C₃ alkyl.

In another preferred embodiment, Rm is selected from the substituted or unsubstituted group consisting of methyl, ethyl, n-propyl, isopropyl, cyclopropyl, cyclobutyl, cyclopentyl, cyclohexyl, cyclopropylamino, azetidinyl, azacyclopentanyl, azacyclohexanyl, oxiranyl, oxetanyl, oxecyclopentanyl, oxacyclohexanyl, wherein the substituted means being substituted by one or more (such as 2, 3, 4) substituents selected from the group consisting of deuterium, halogen, nitro, hydroxyl, cyano, ester group, amino, amido, C₁-C₃ alkyl.

In another preferred embodiment, R¹, R², R⁴, L, U, V, W, Q, p, A, B, X, Rn, Rm, Rx, R^{A}, R"', q and q' are the corresponding specific groups of each specific compounds in the examples.

In another preferred embodiment, the compound of formula (I), the stereoisomer, tautomer, crystal form, pharmaceutically acceptable salt, hydrate, solvate or prodrug thereof is selected from the group consisting of

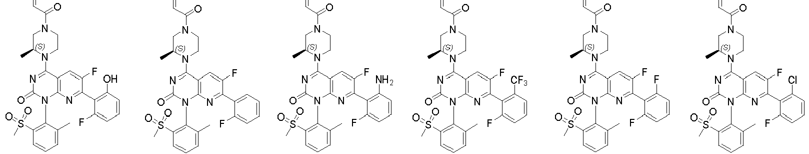

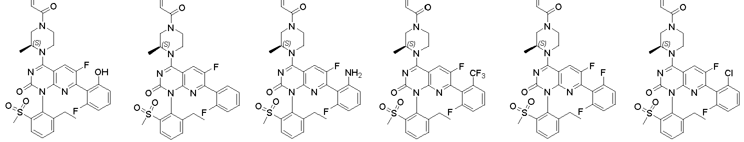

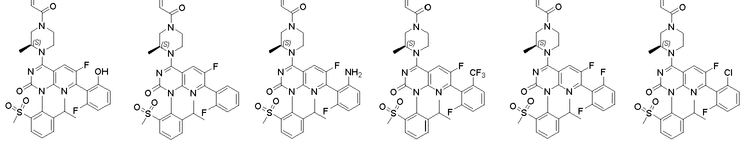

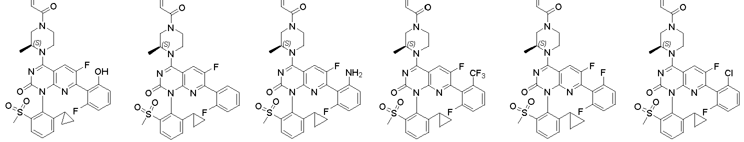

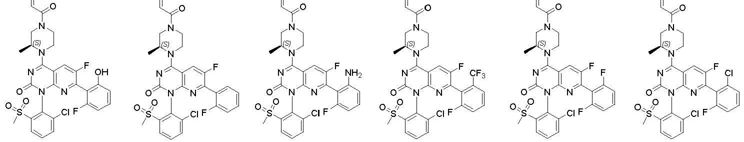

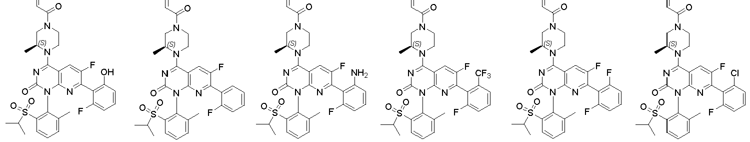

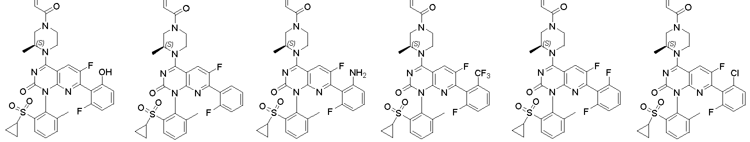

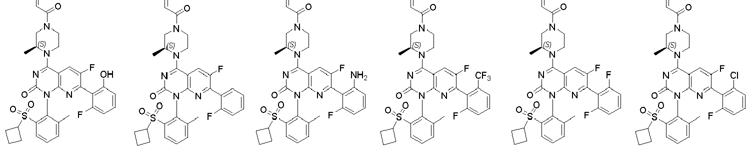

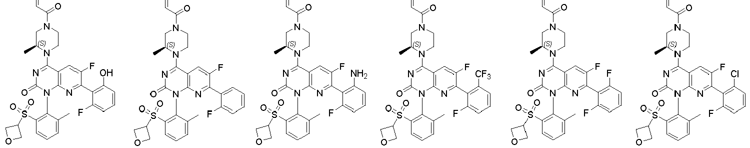

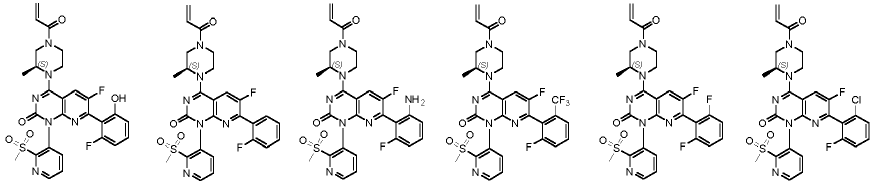

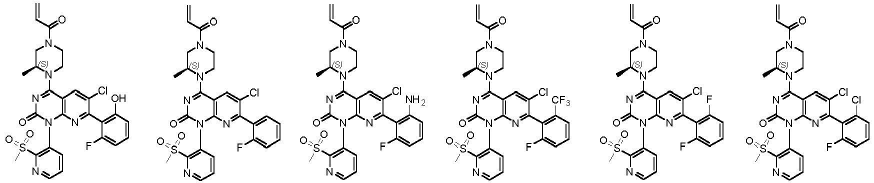

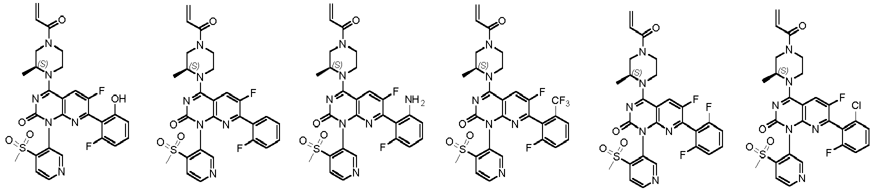

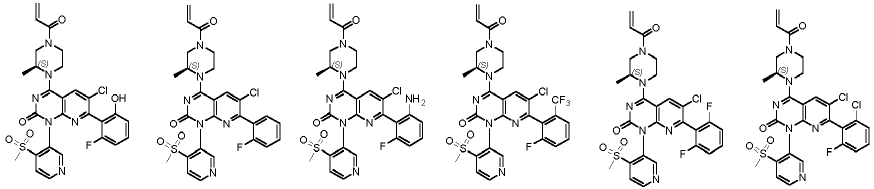

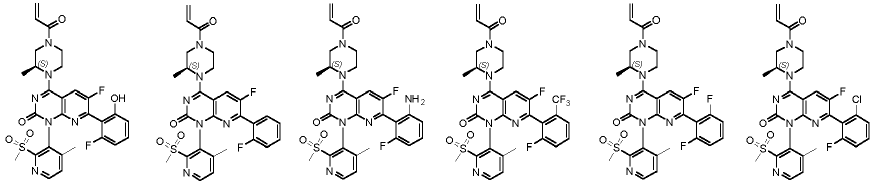

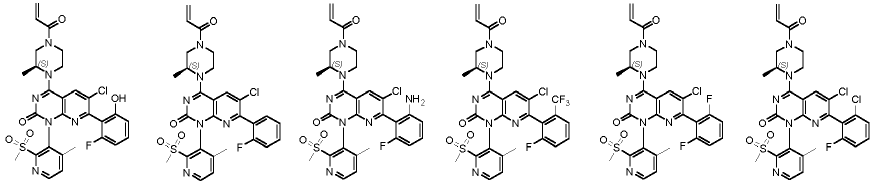

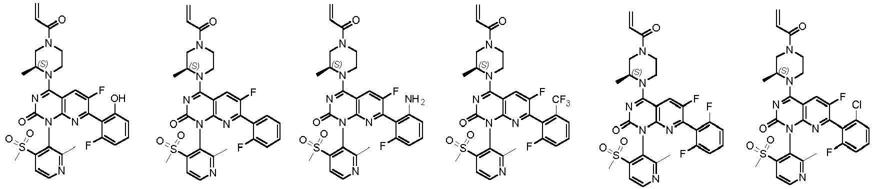

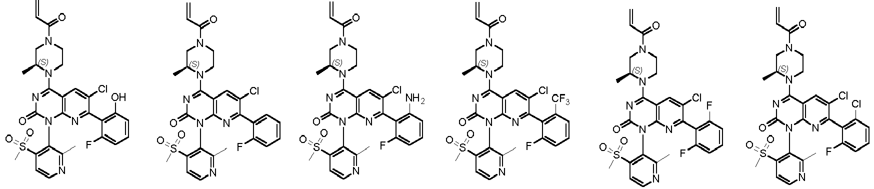

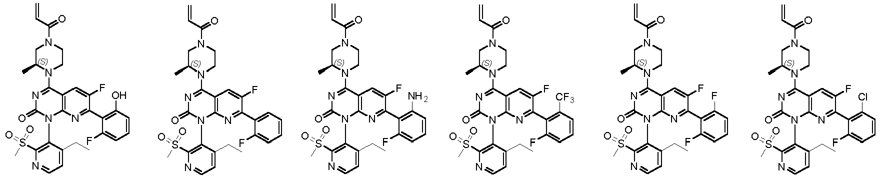

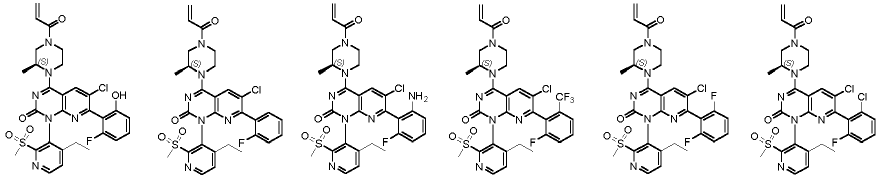

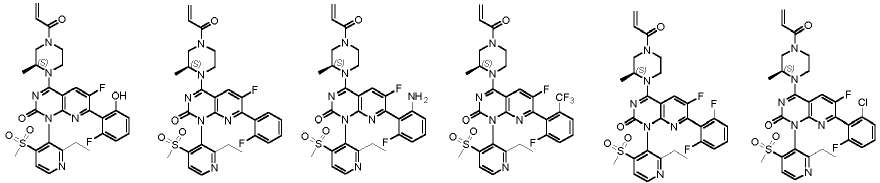

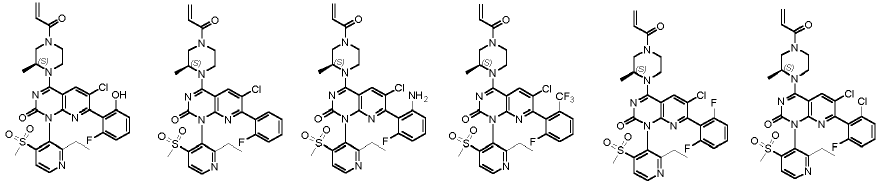

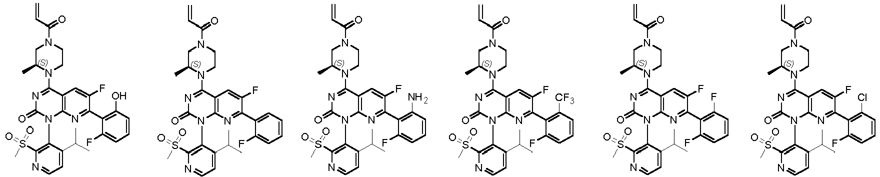

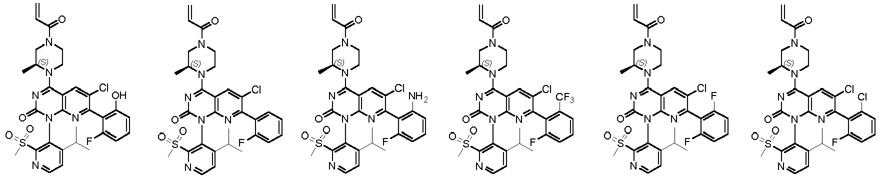

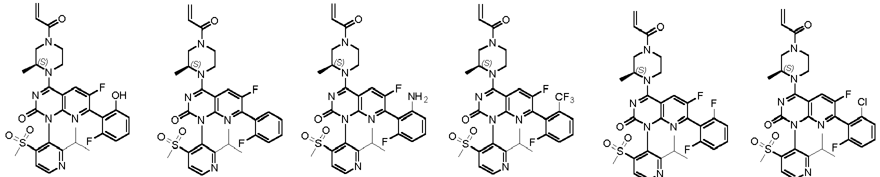

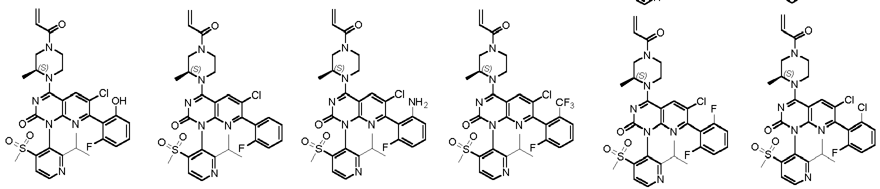

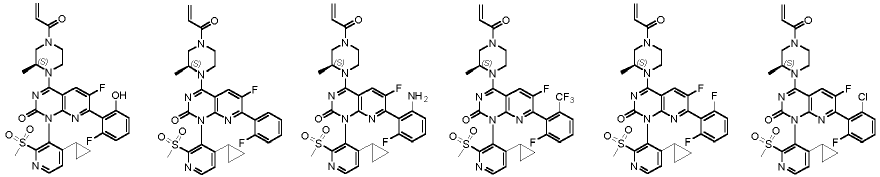

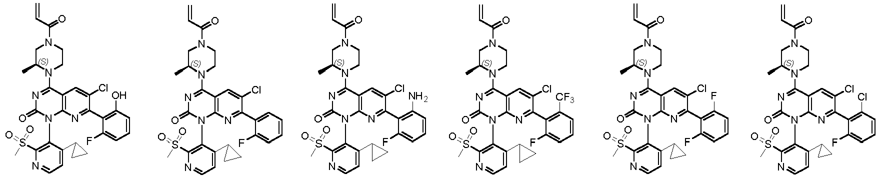

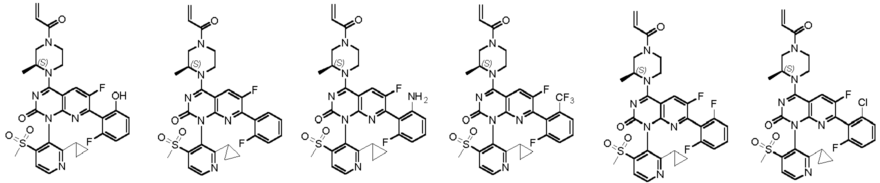

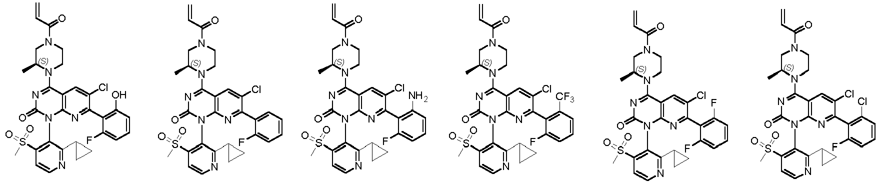

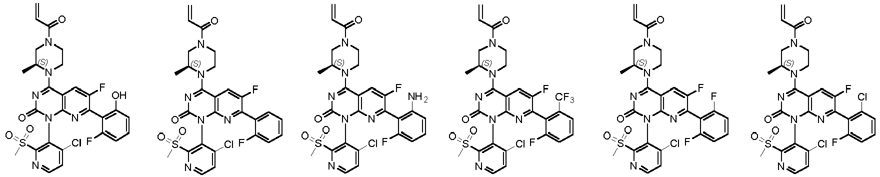

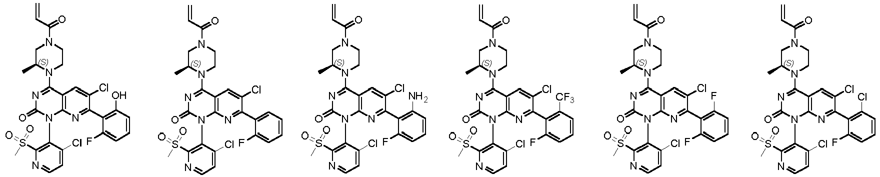

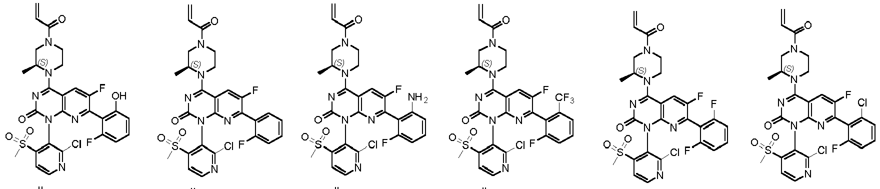

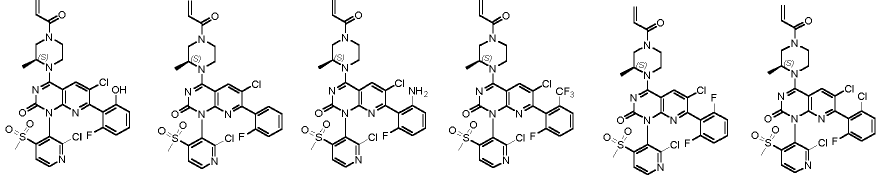

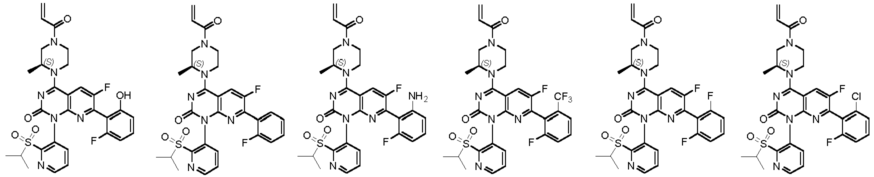

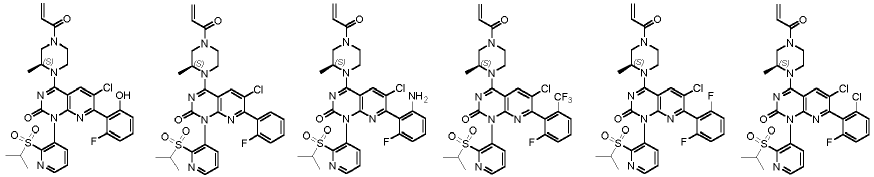

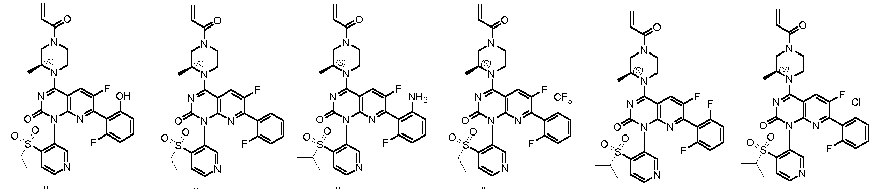

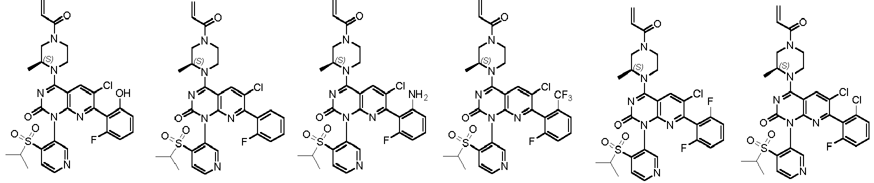

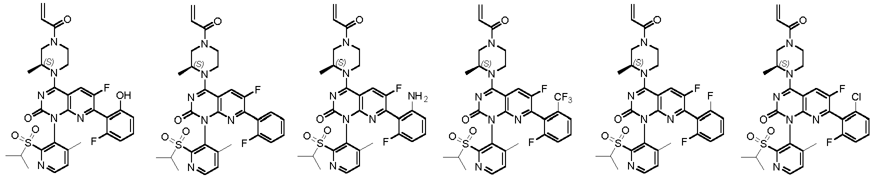

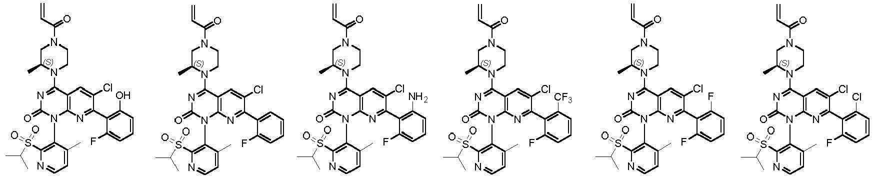

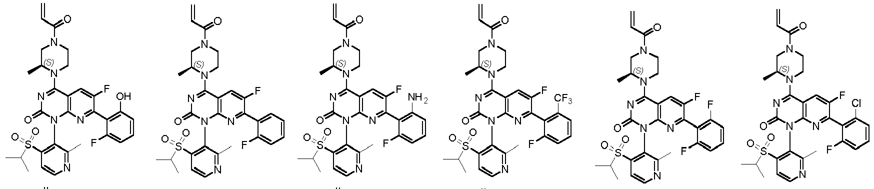

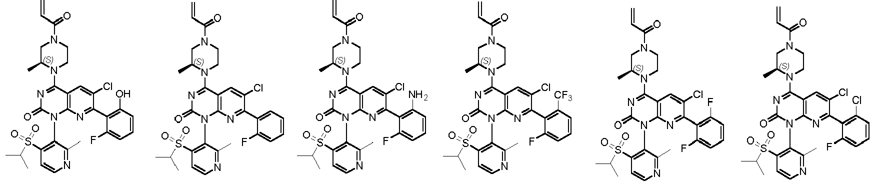

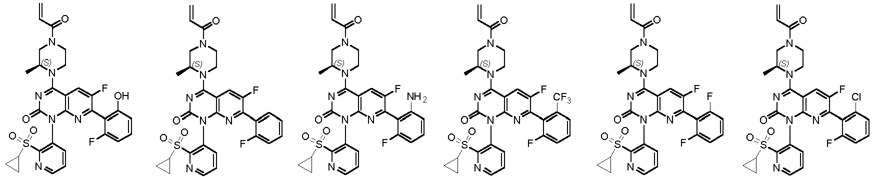

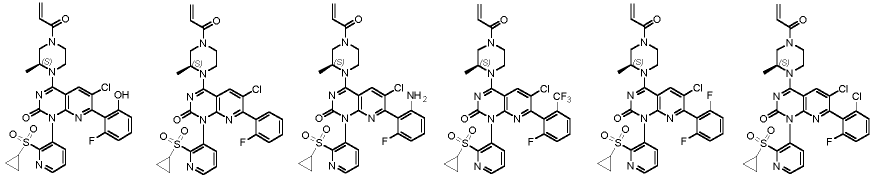

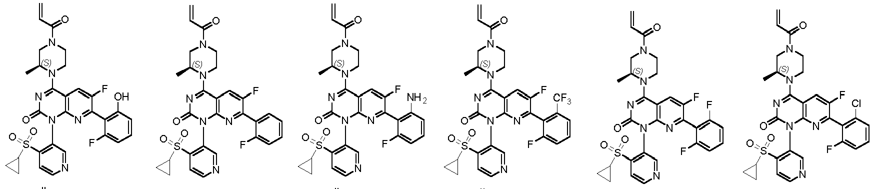

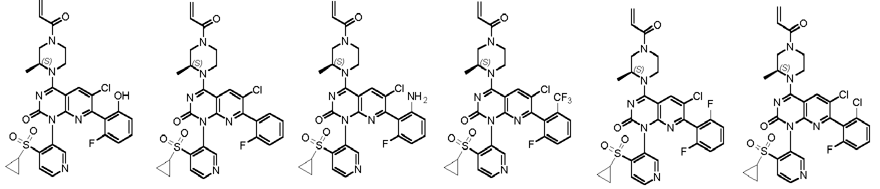

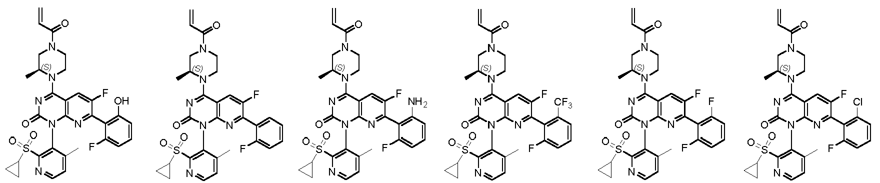

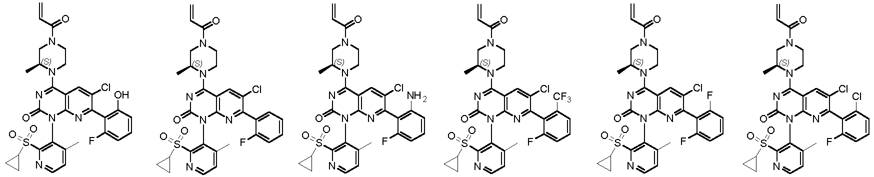

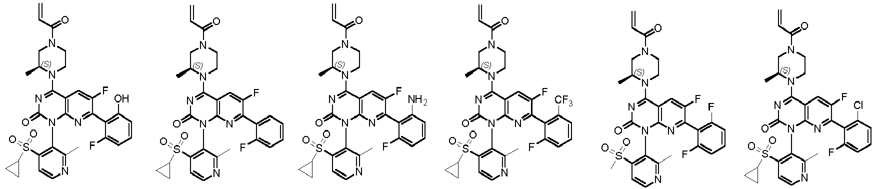

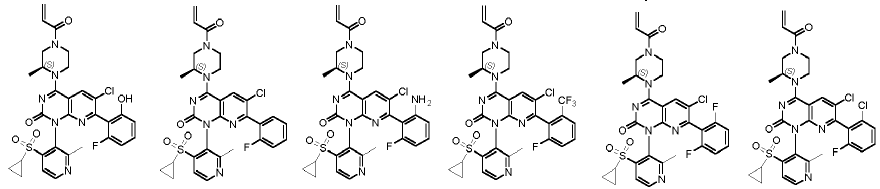

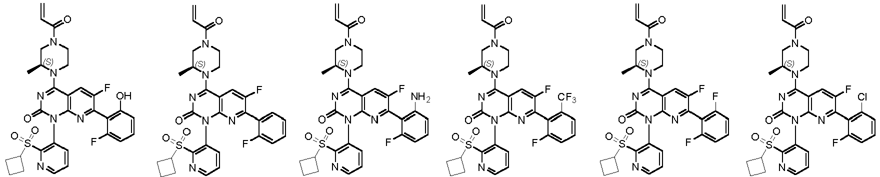

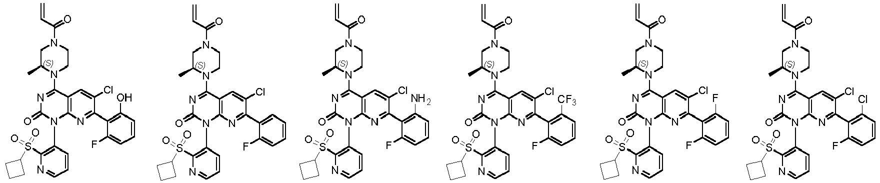

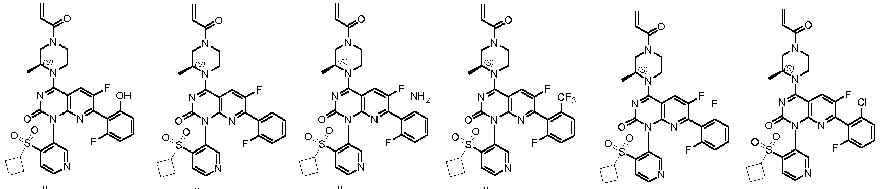

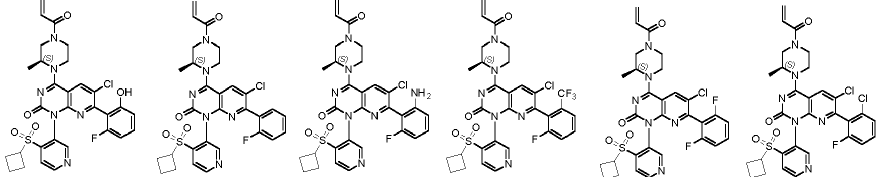

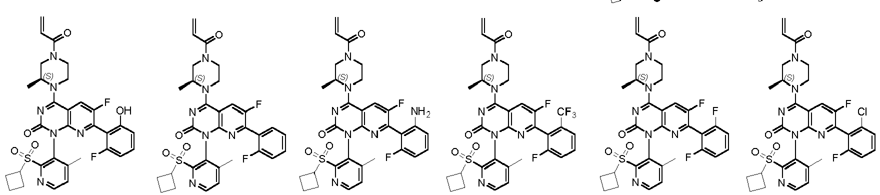

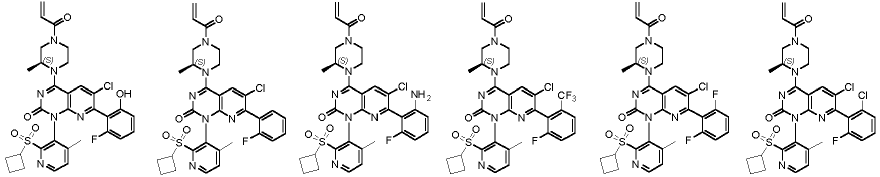

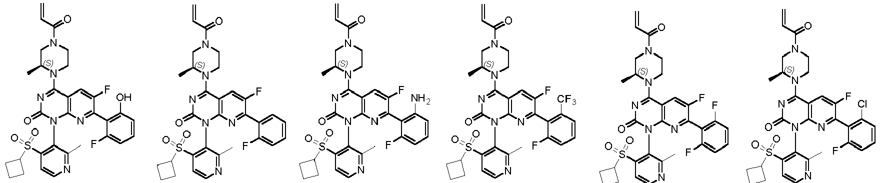

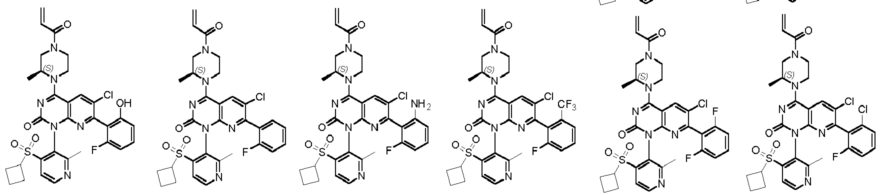

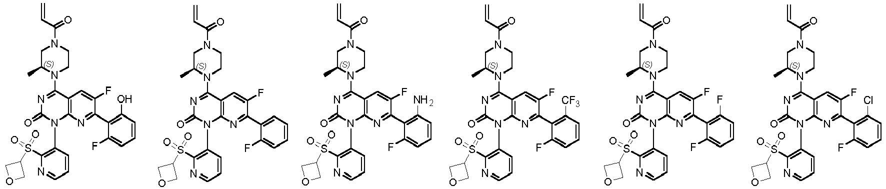

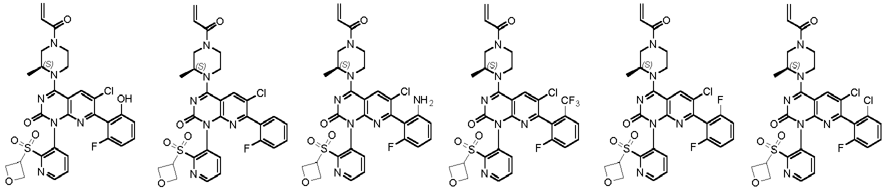

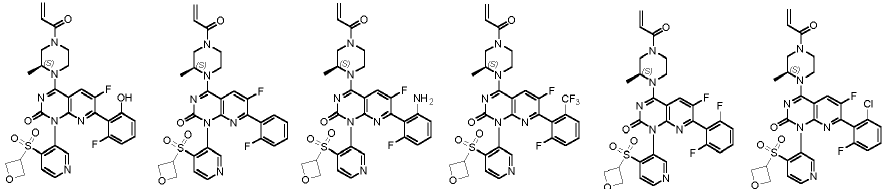

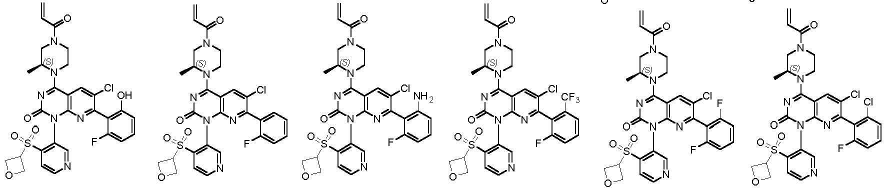

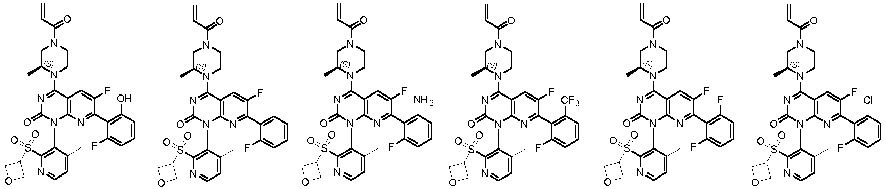

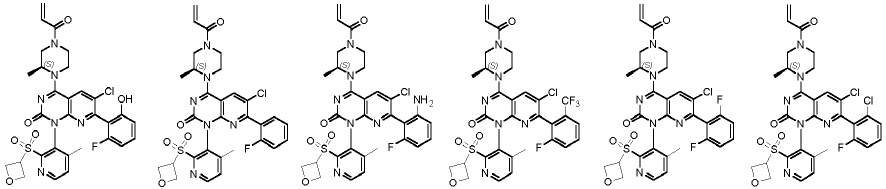

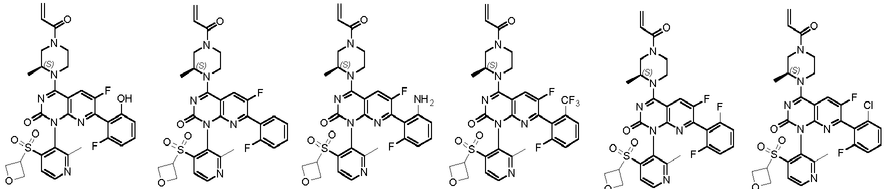

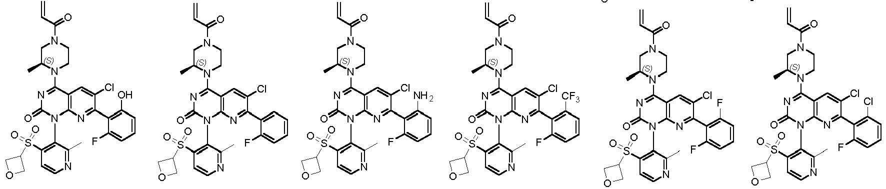

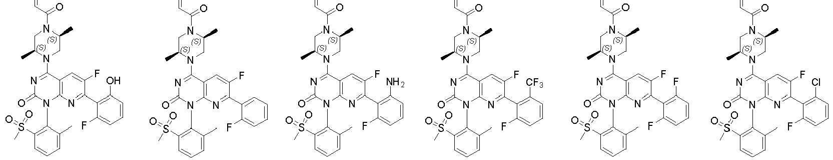

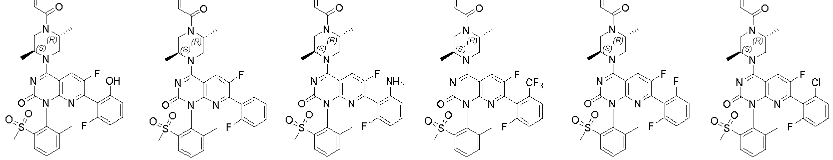

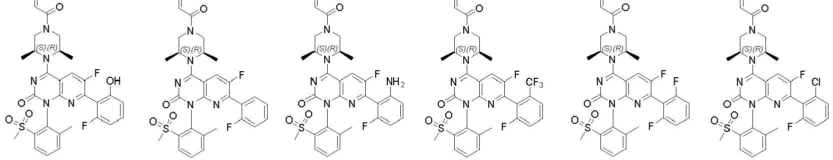

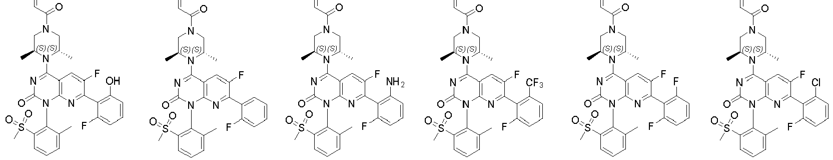

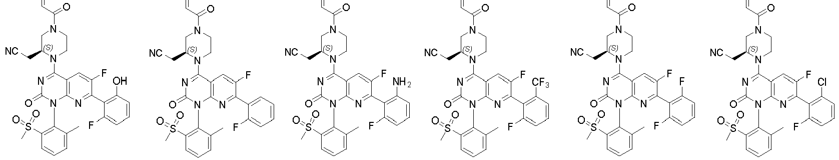

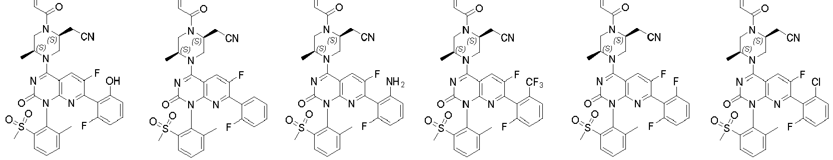

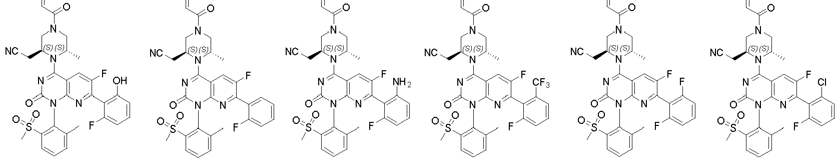

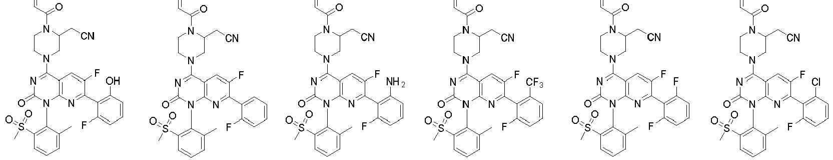

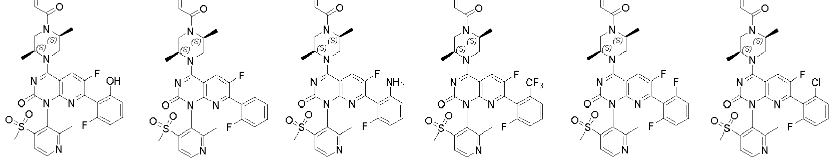

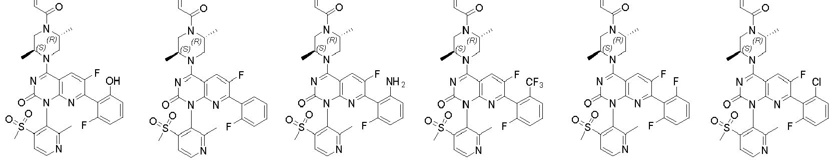

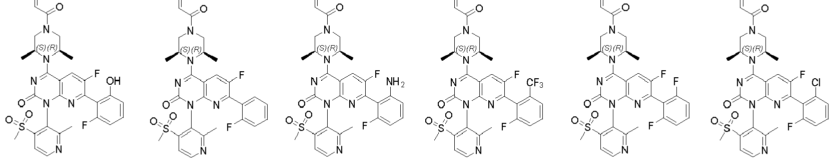

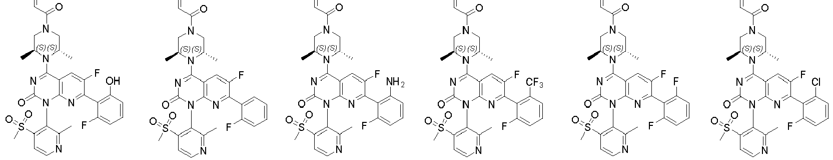

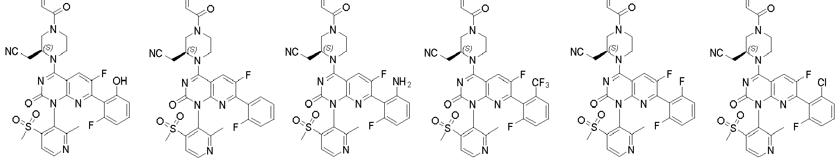

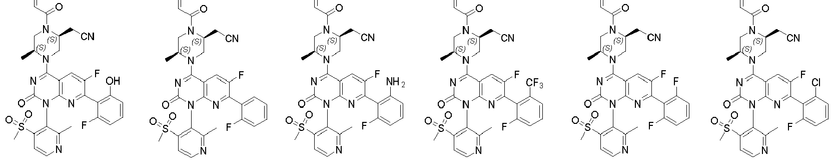

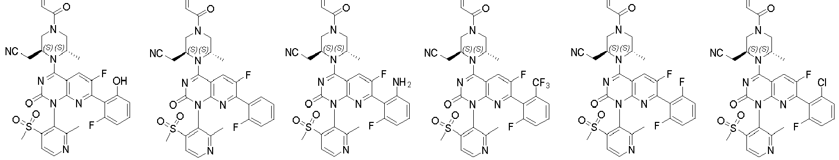

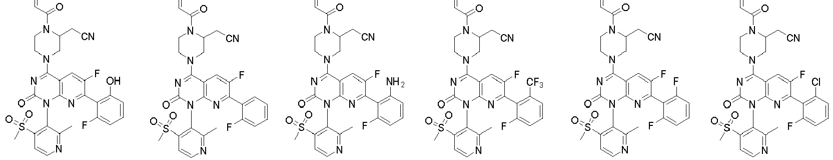

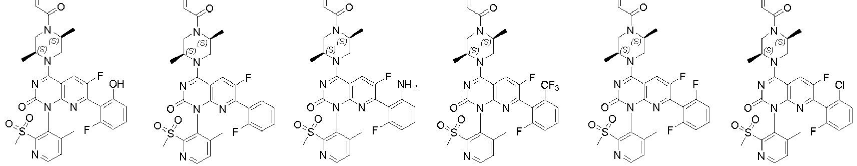

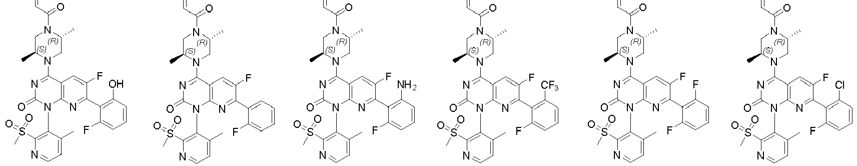

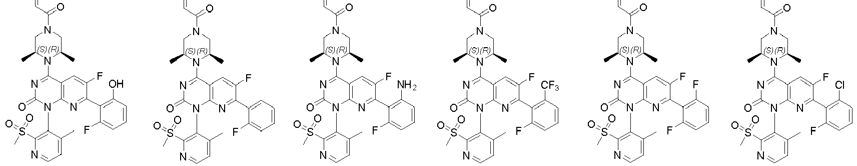

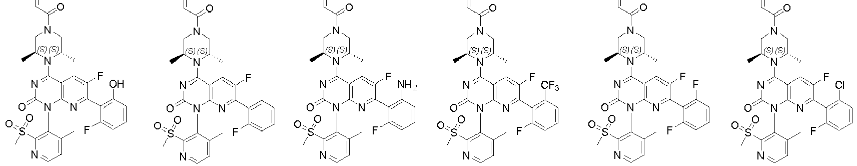

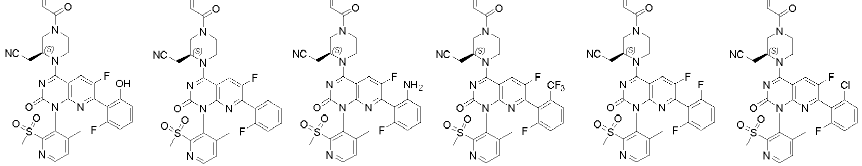

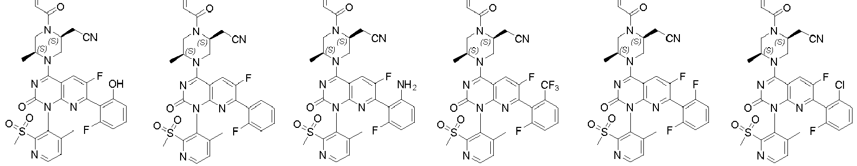

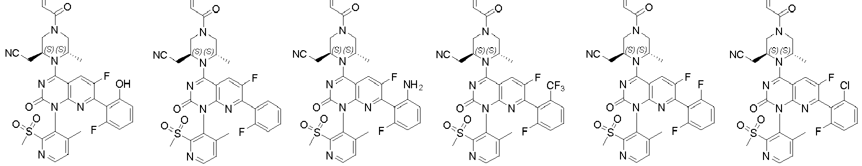

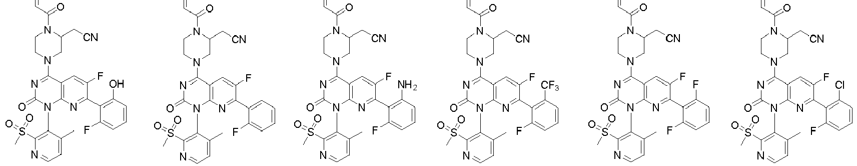

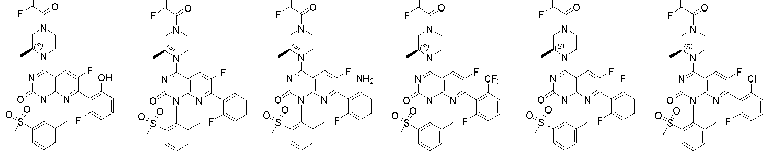

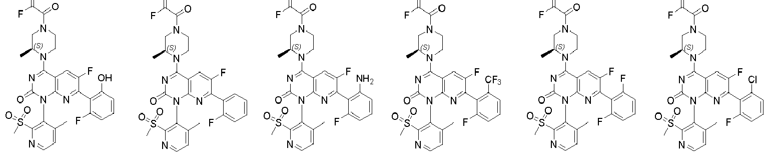

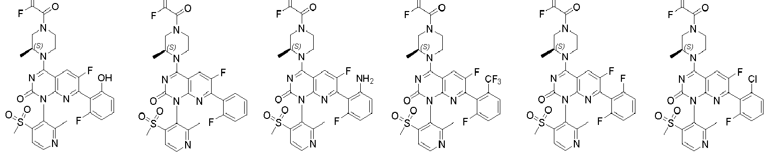

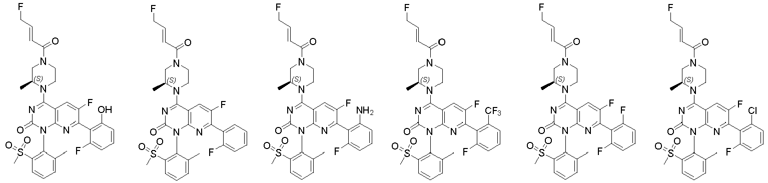

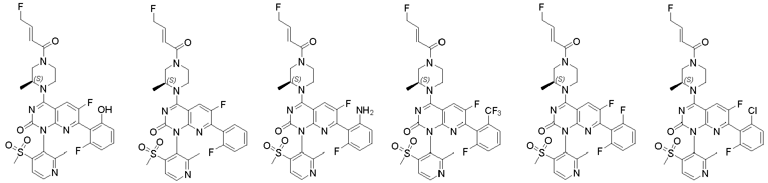

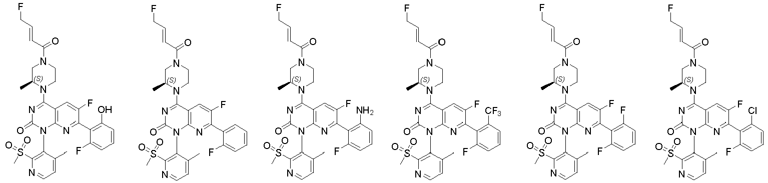

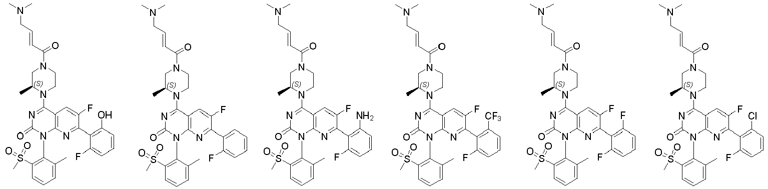

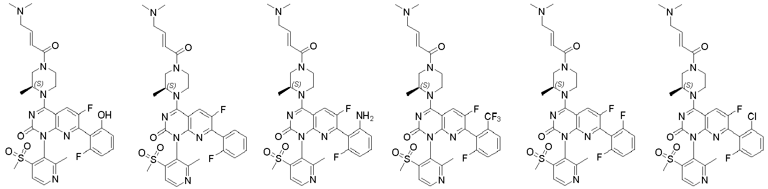

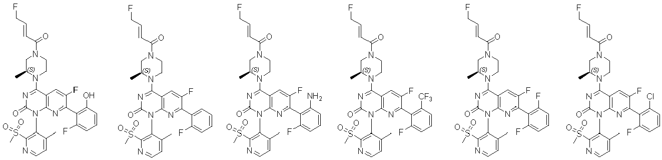

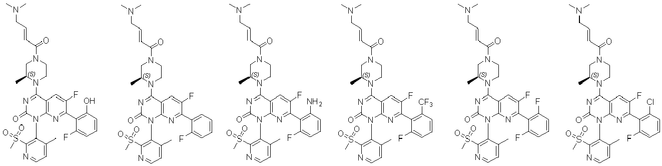

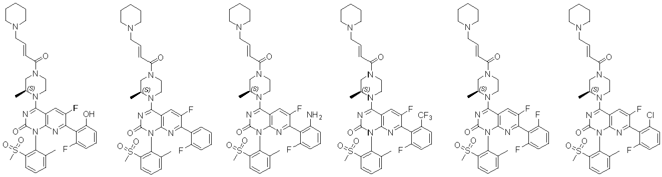

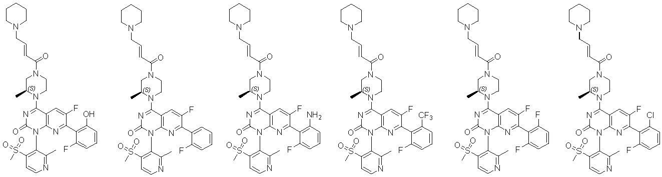

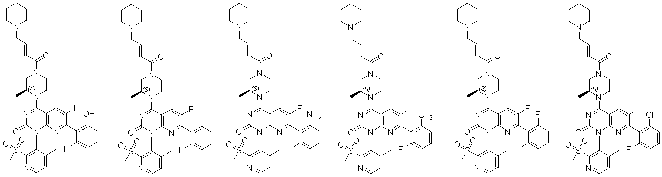

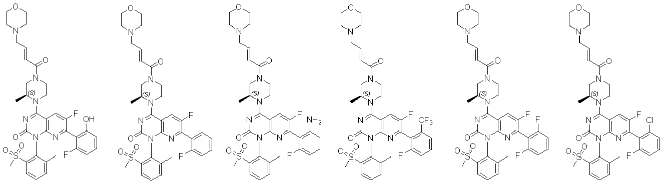

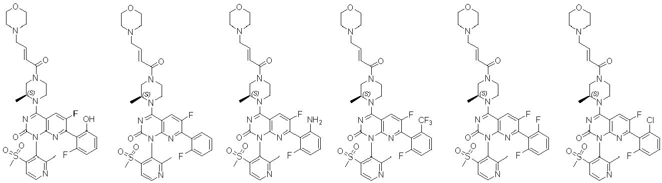

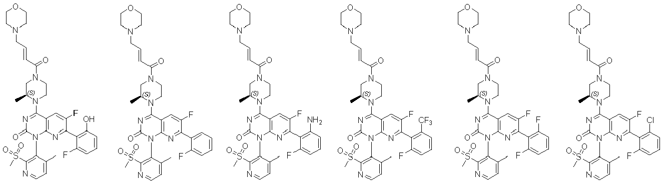

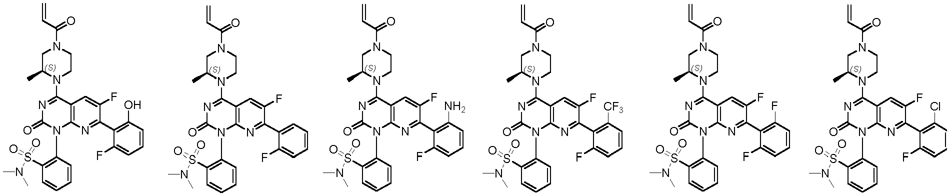

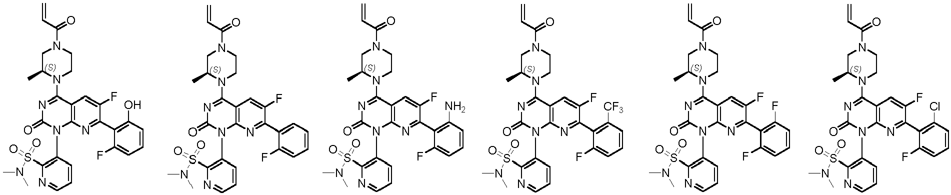

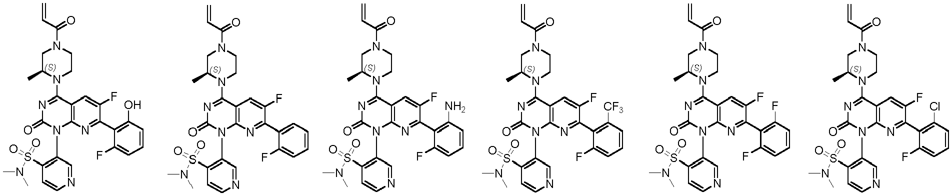

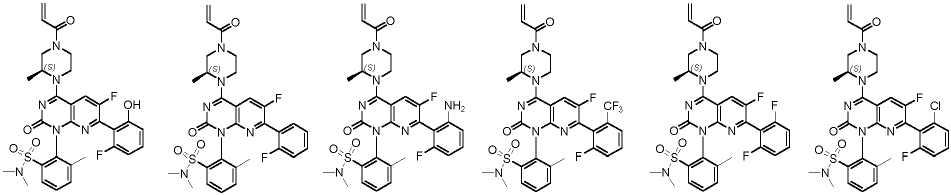

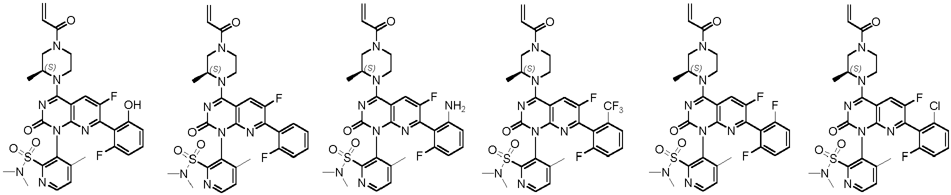

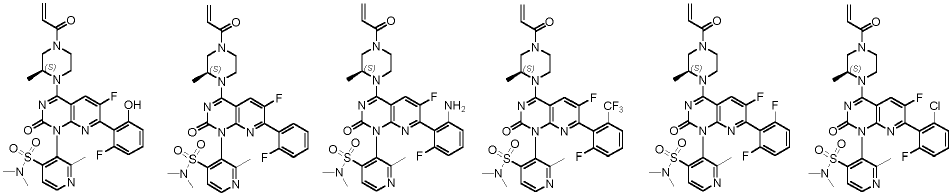

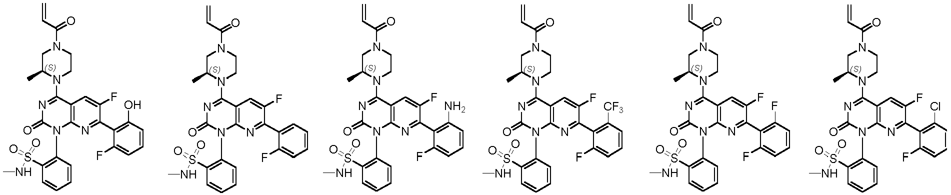

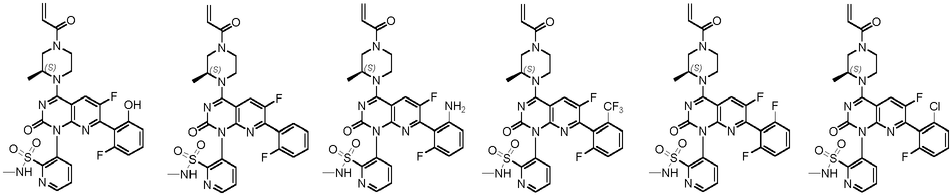

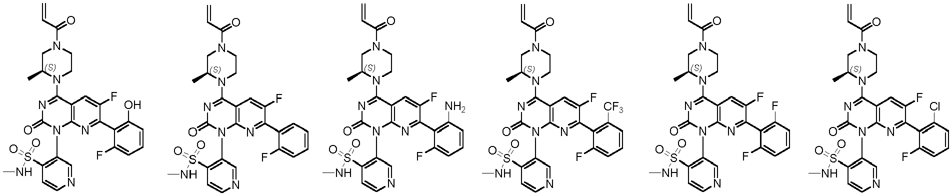

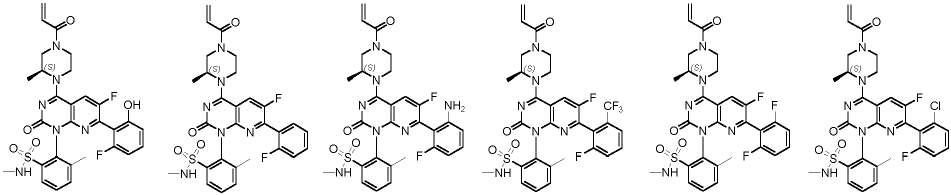

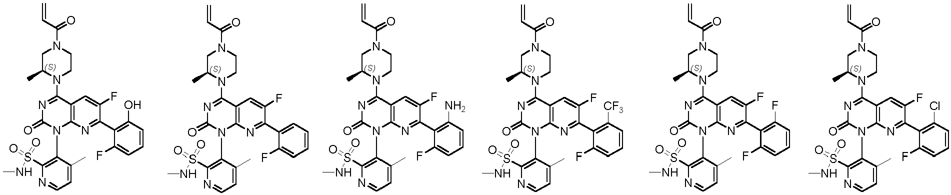

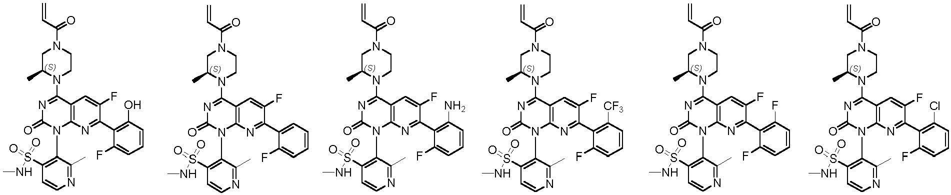

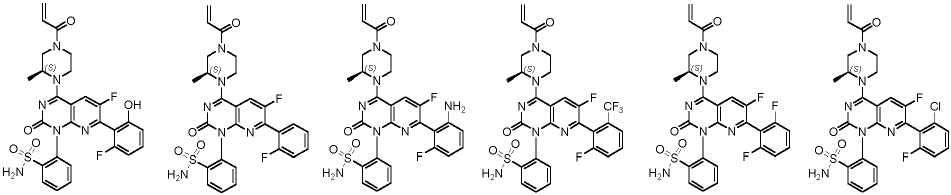

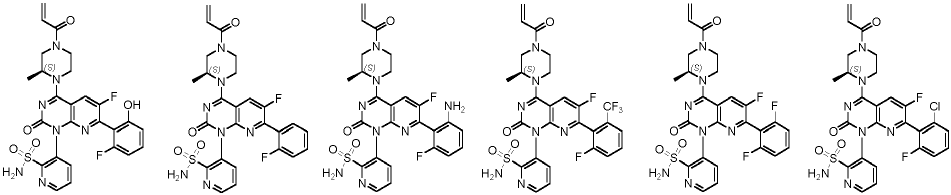

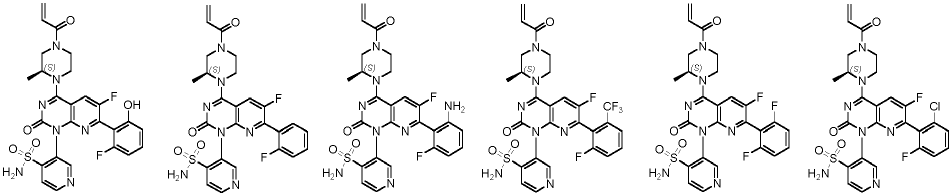

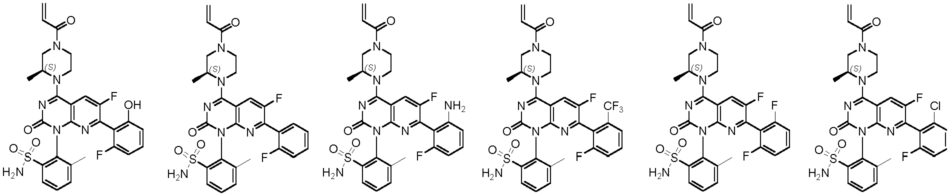

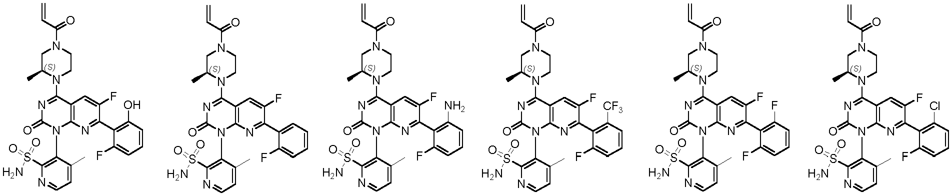

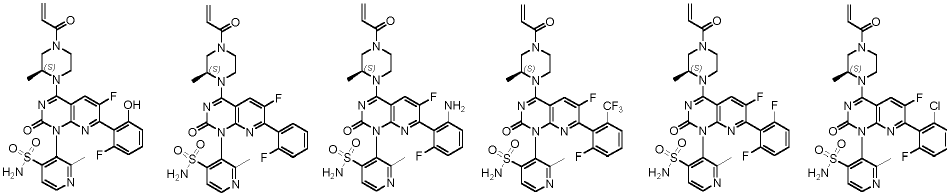

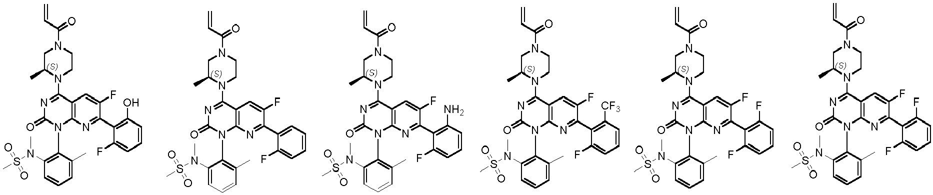

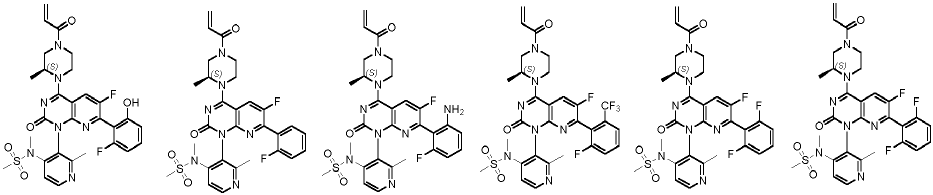

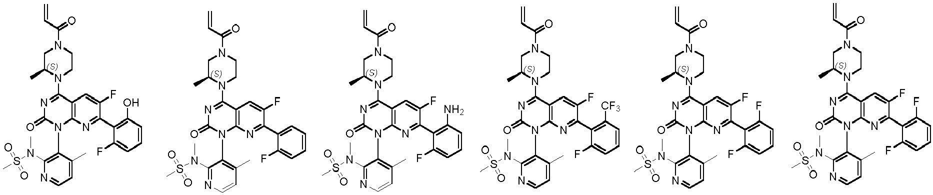

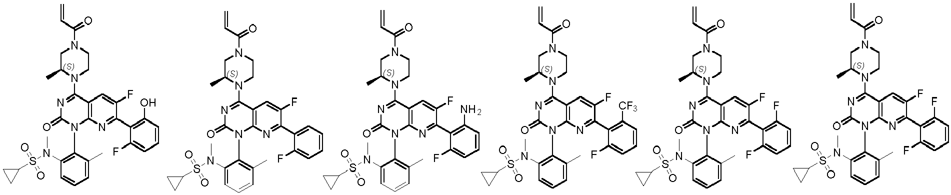

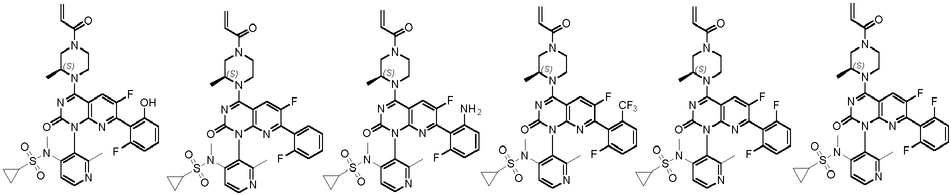

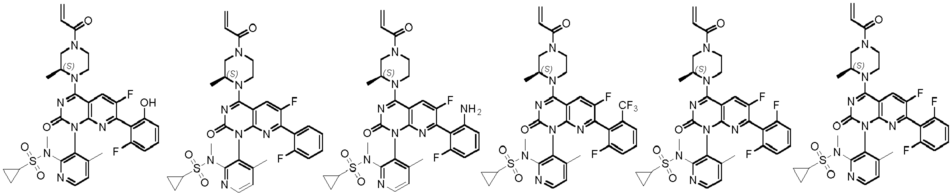

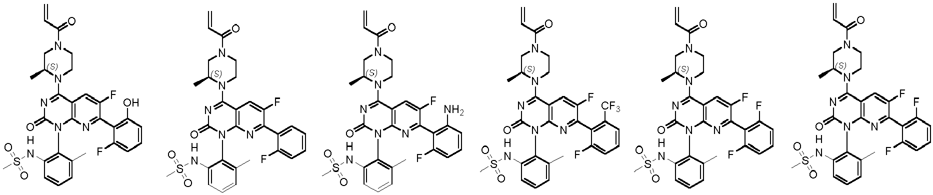

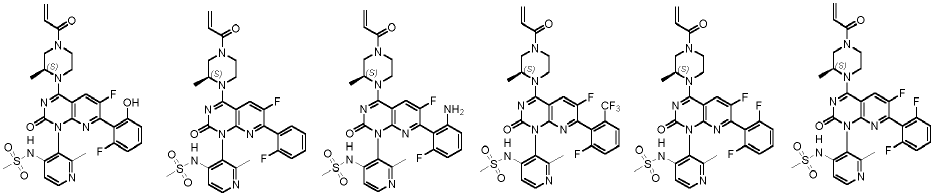

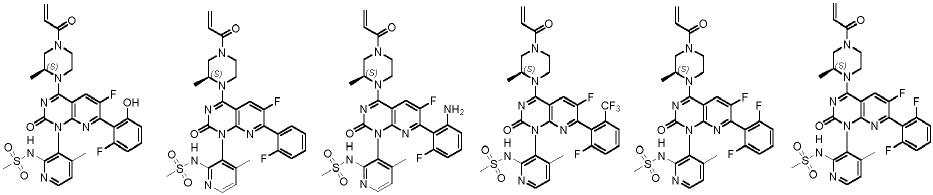

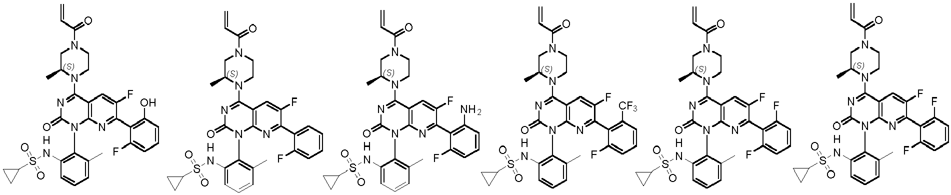

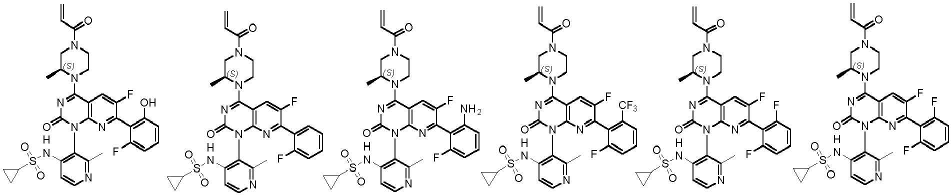

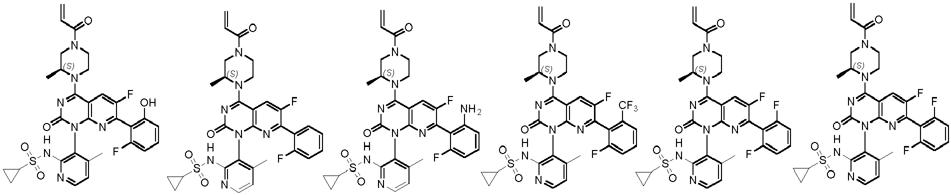

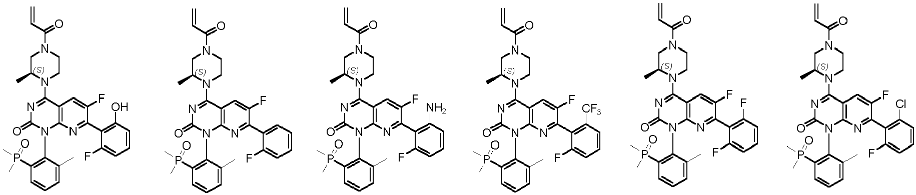

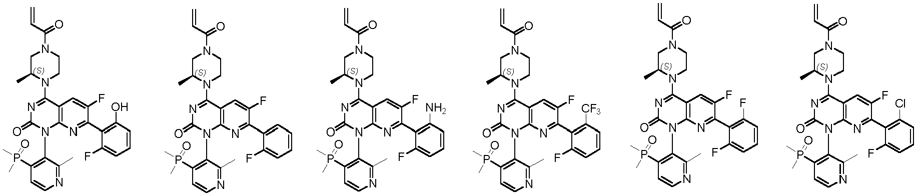

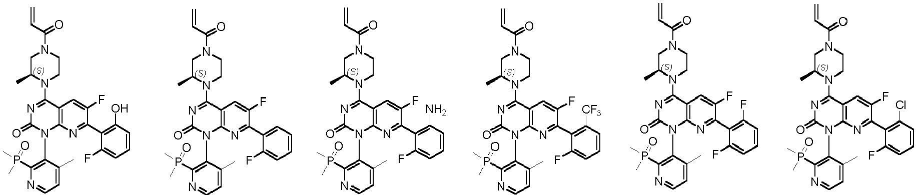

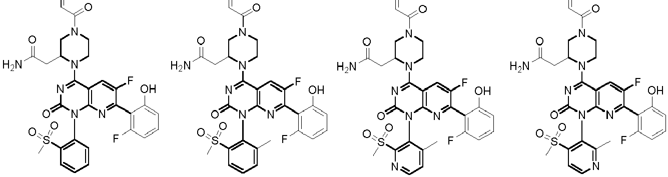

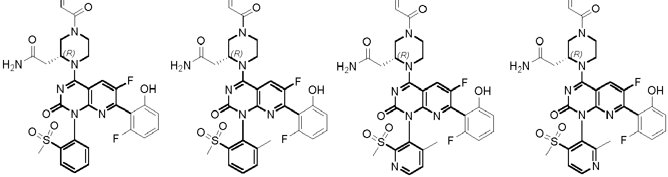

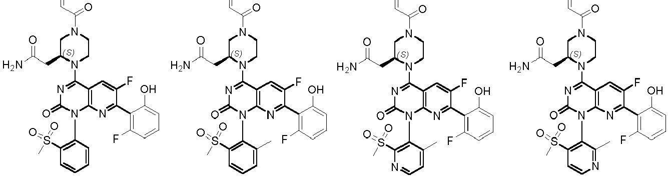

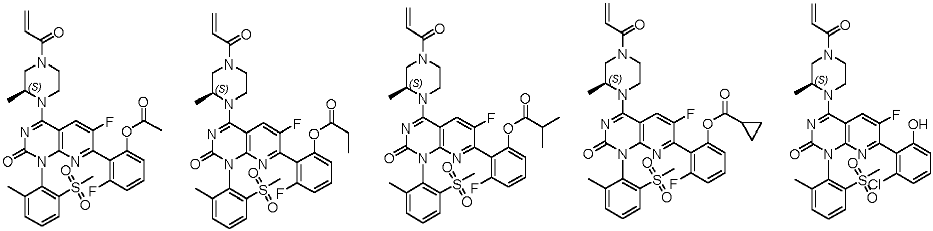

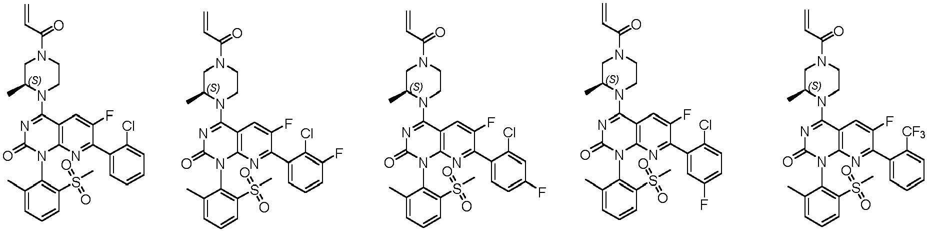

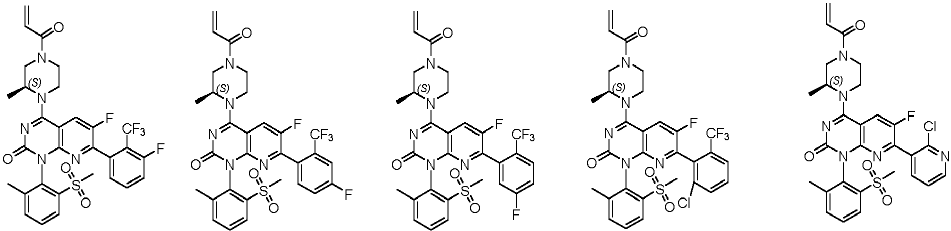

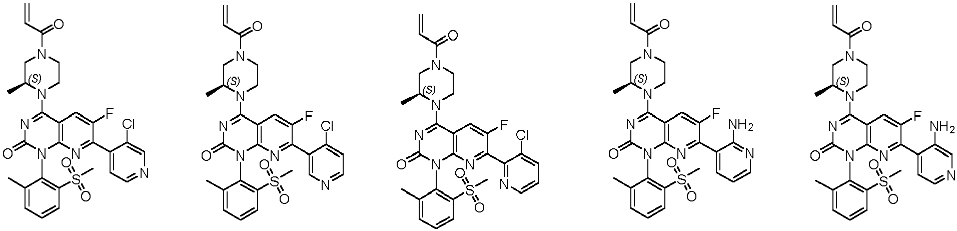

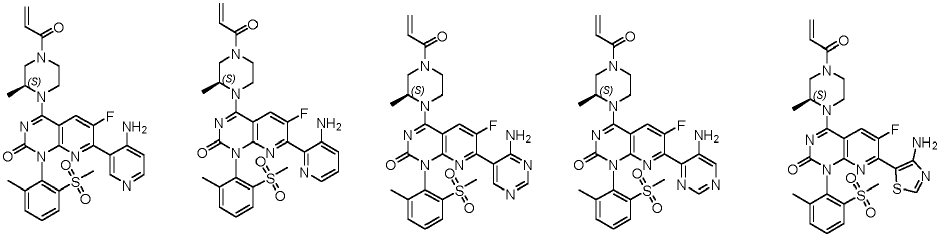

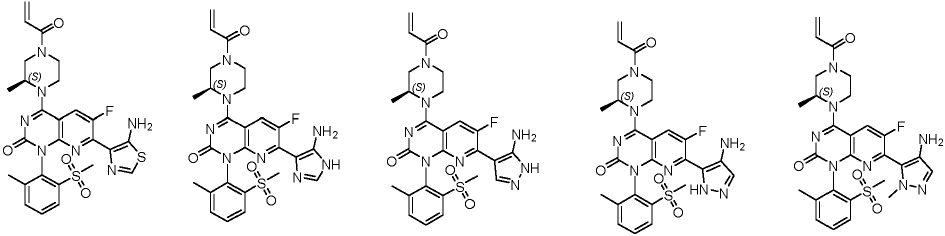

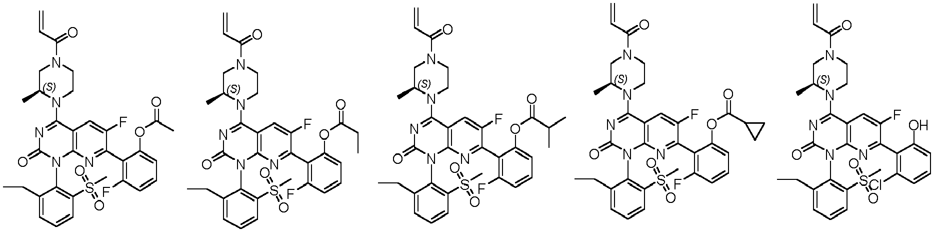

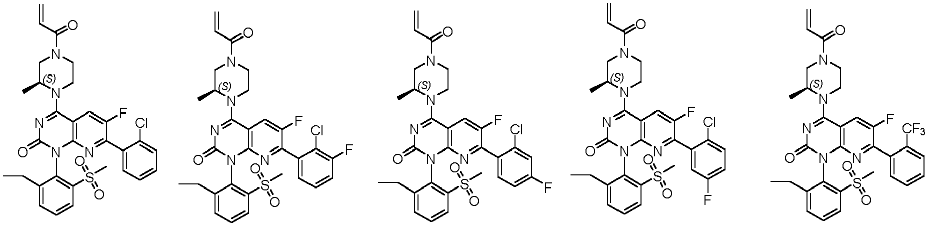

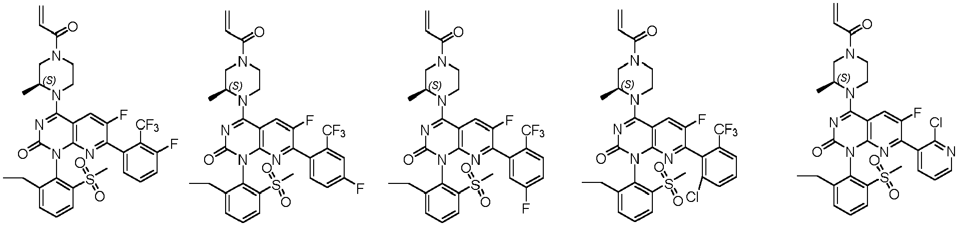

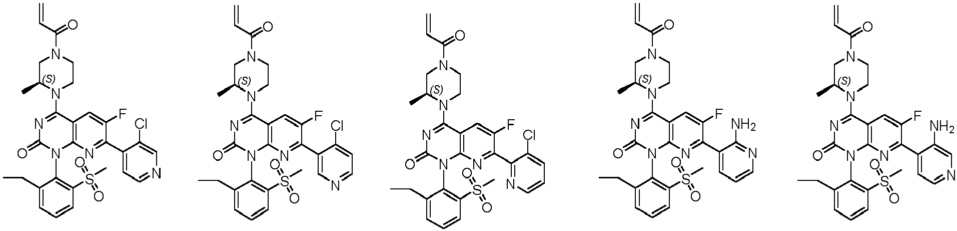

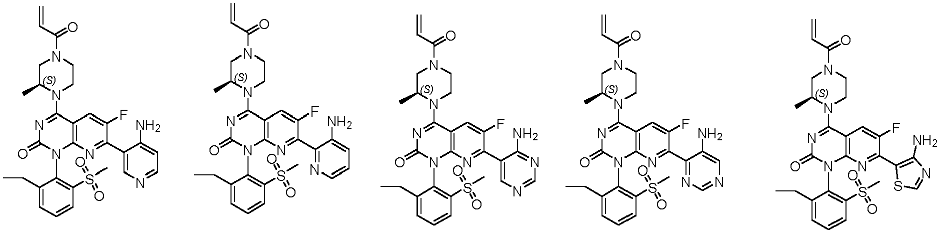

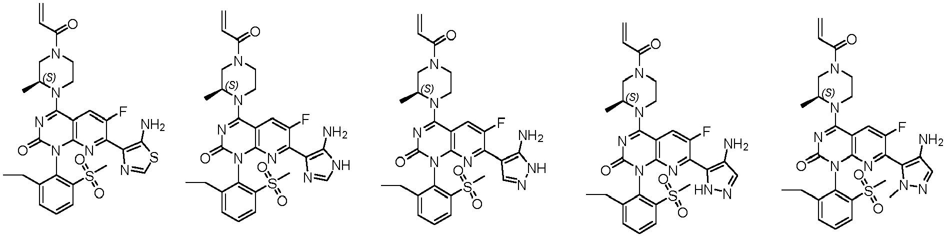

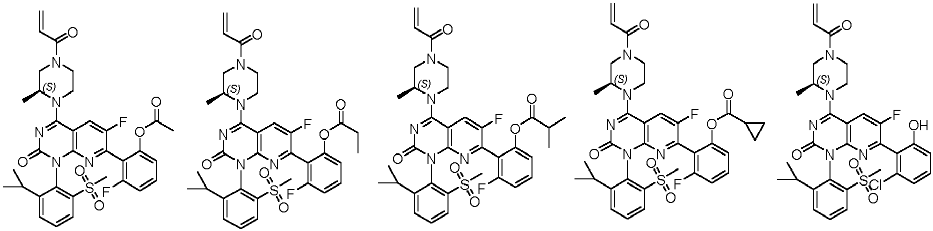

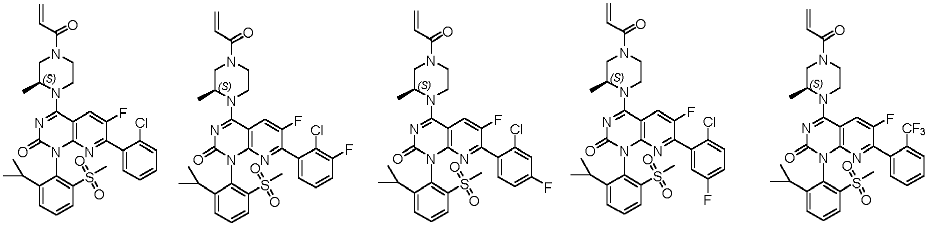

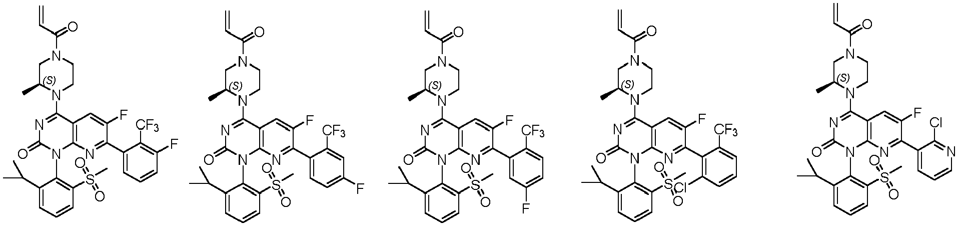

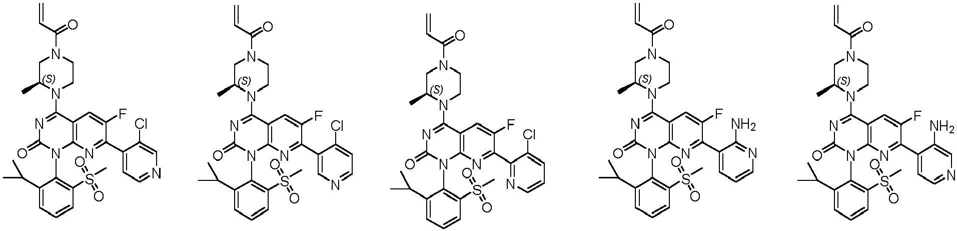

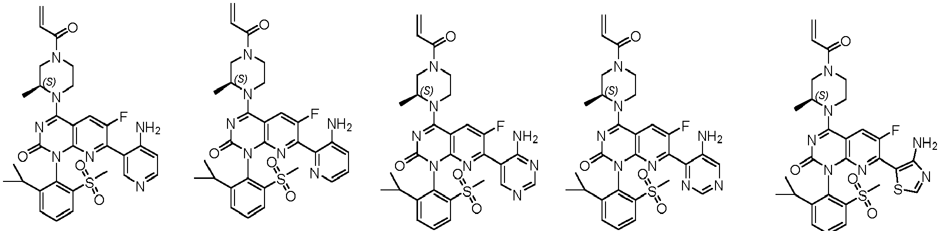

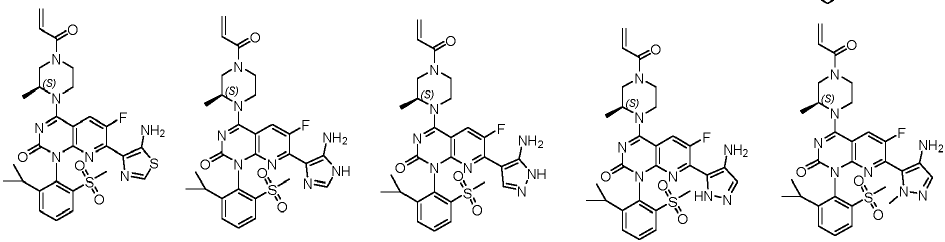

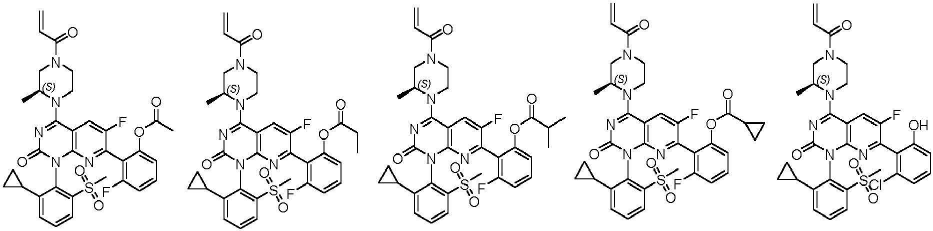

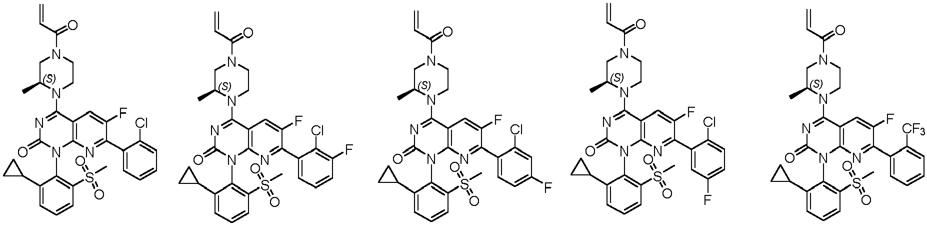

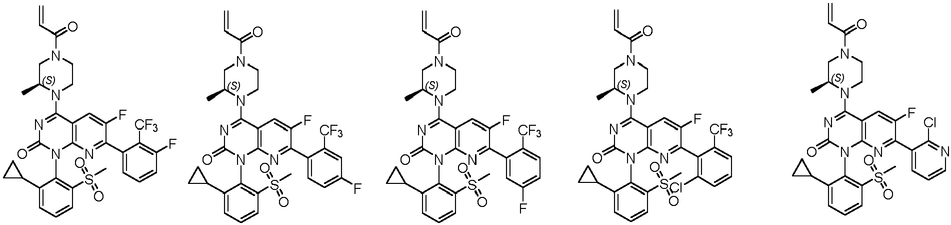

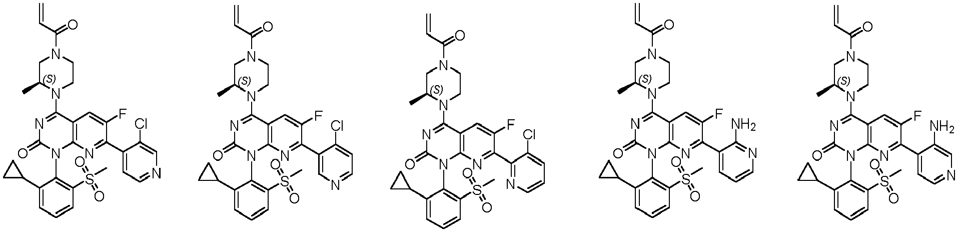

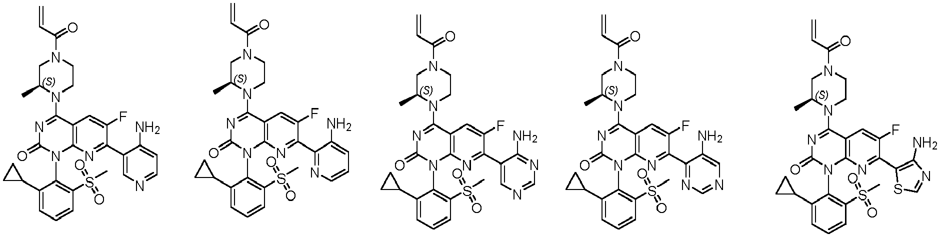

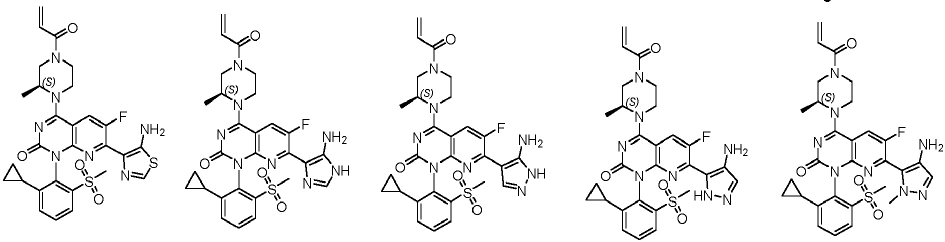

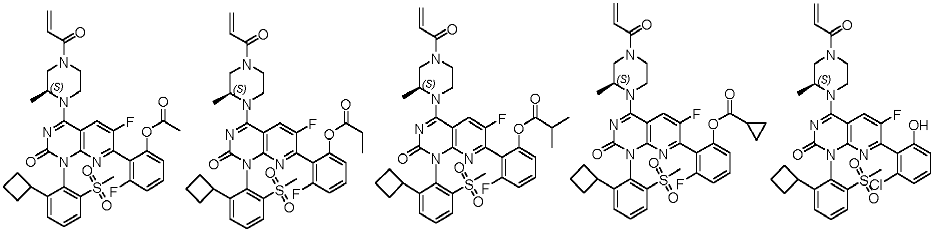

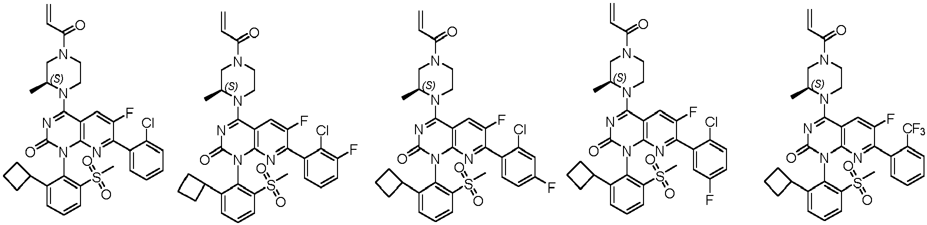

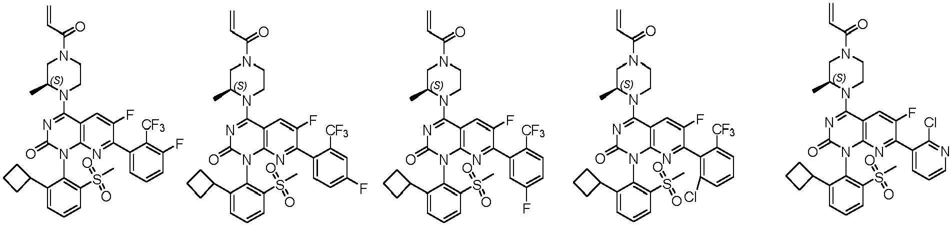

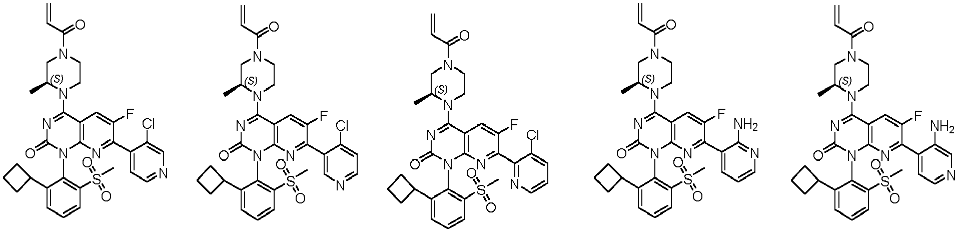

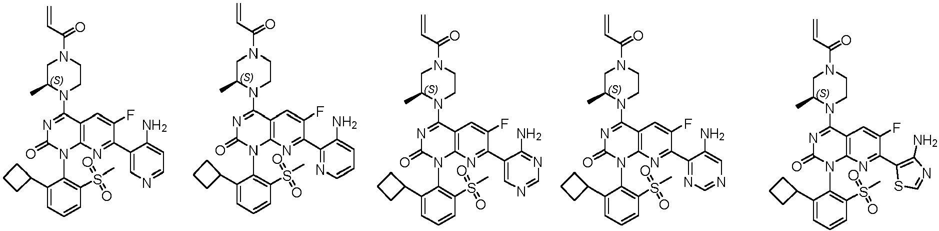

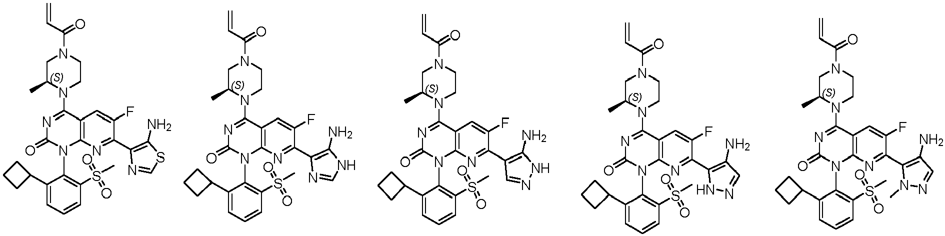

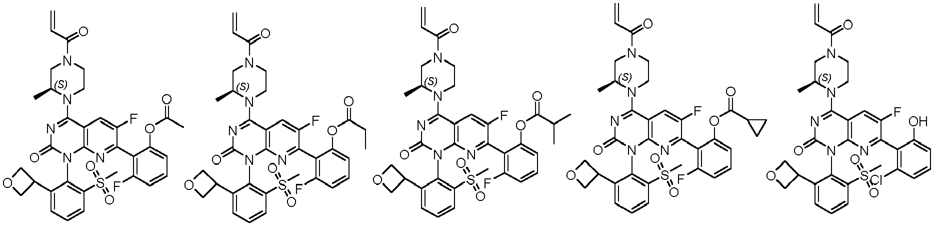

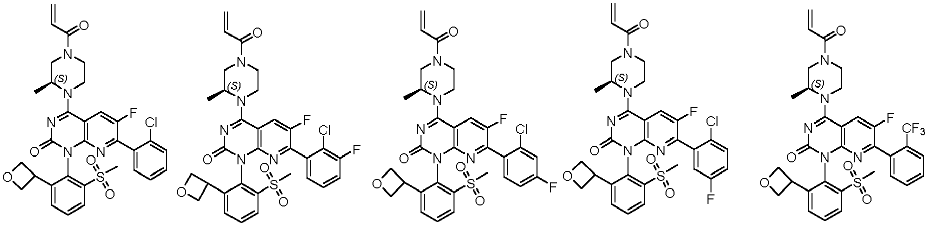

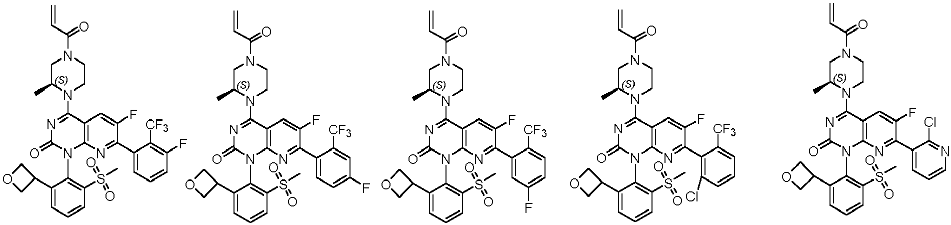

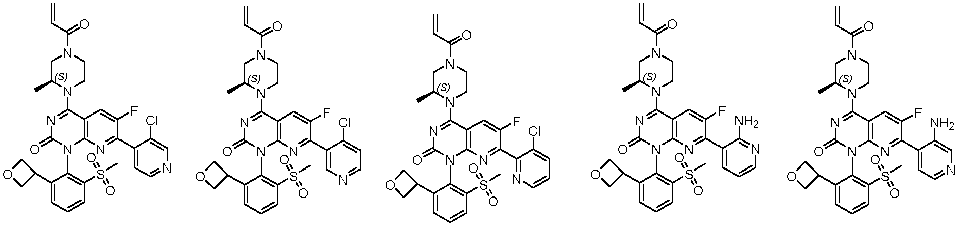

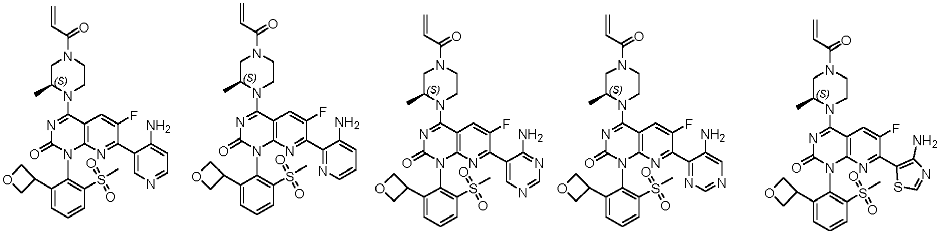

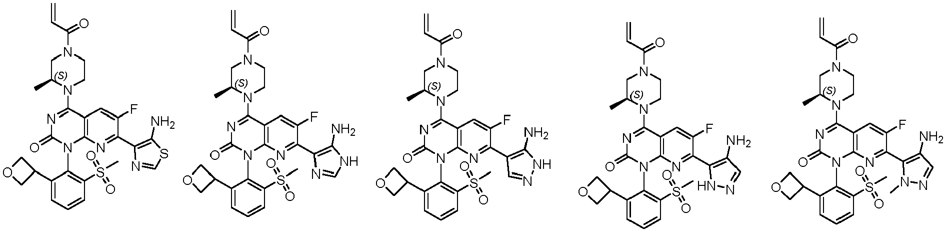

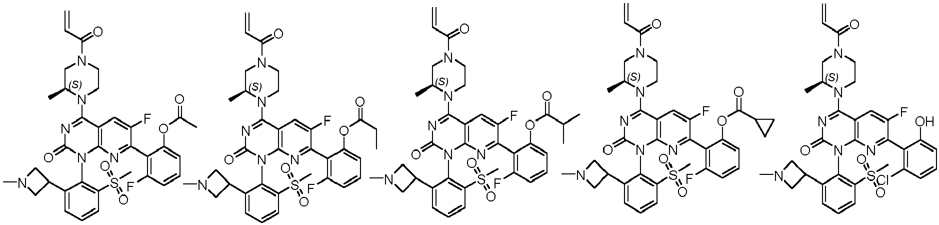

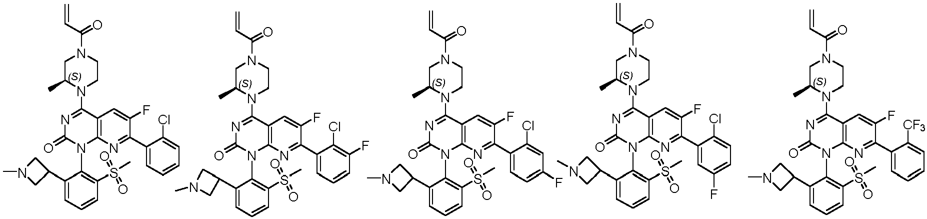

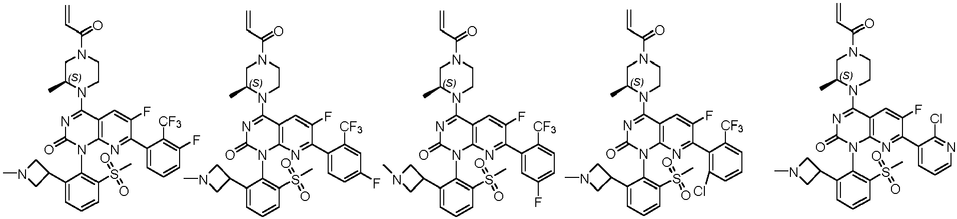

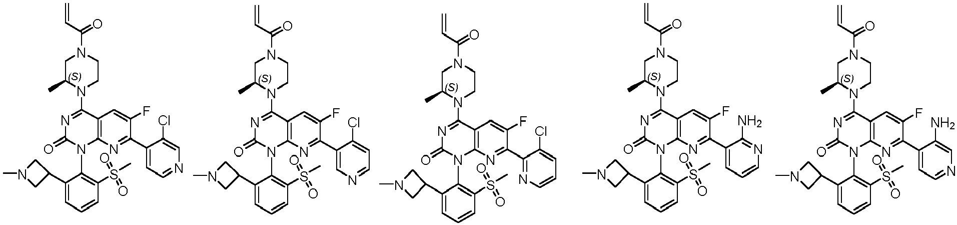

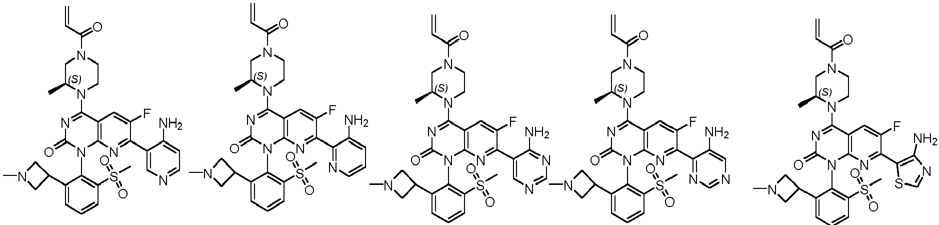

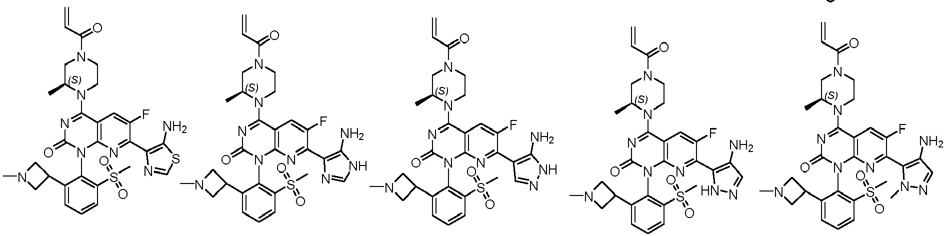

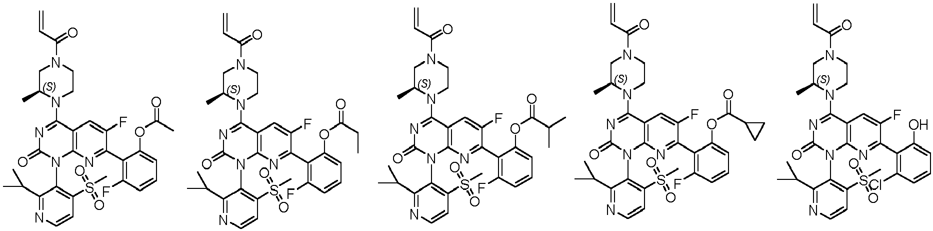

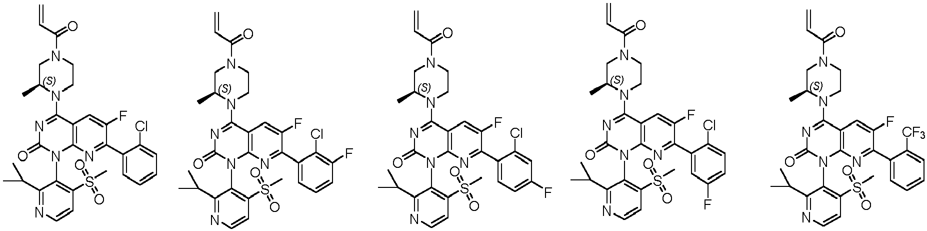

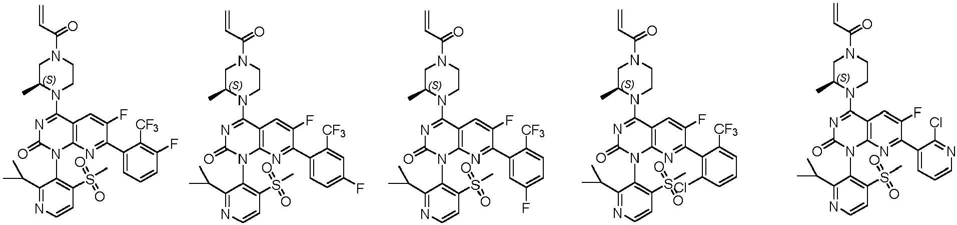

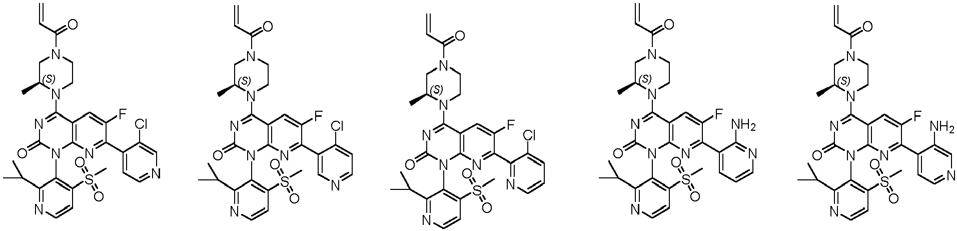

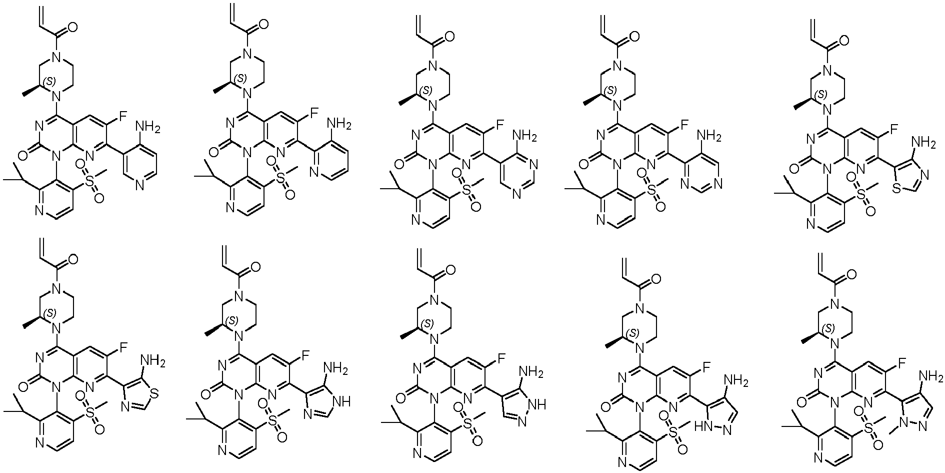

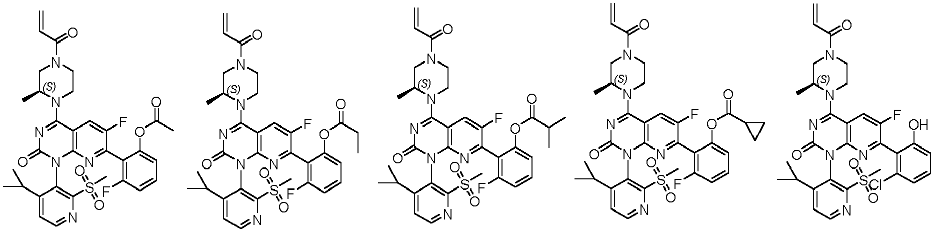

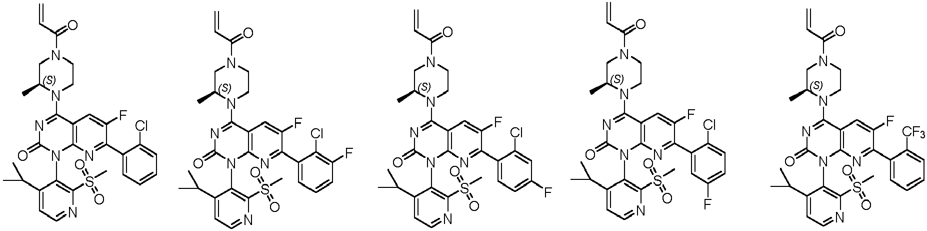

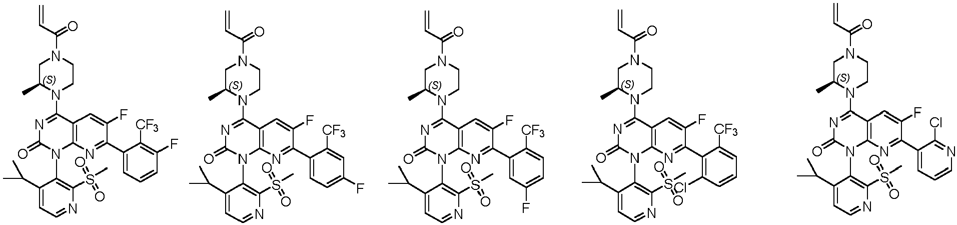

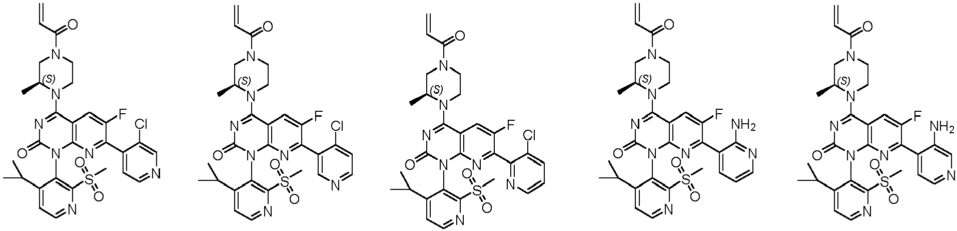

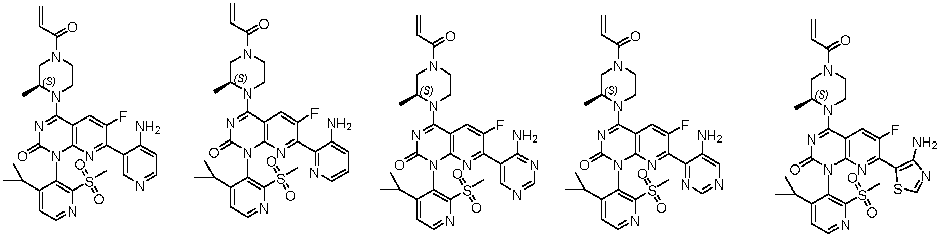

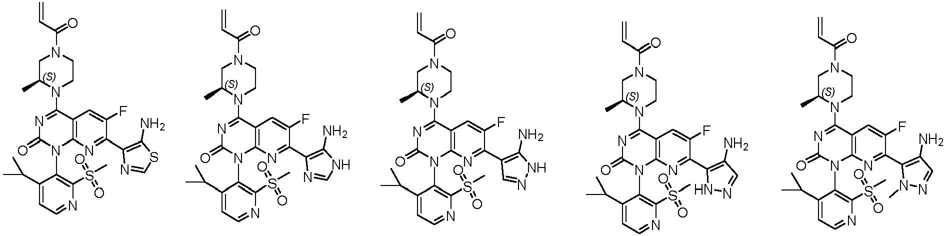

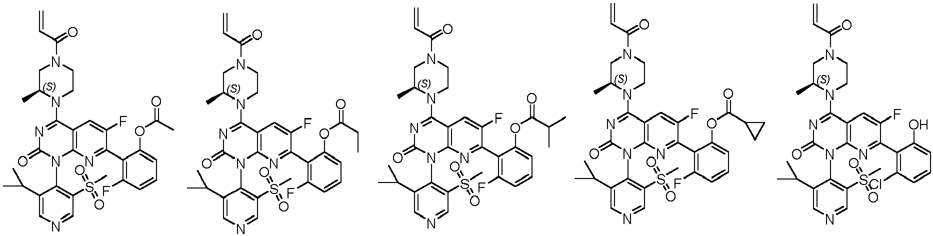

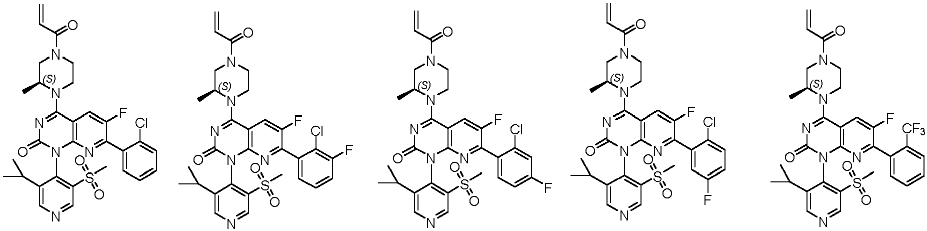

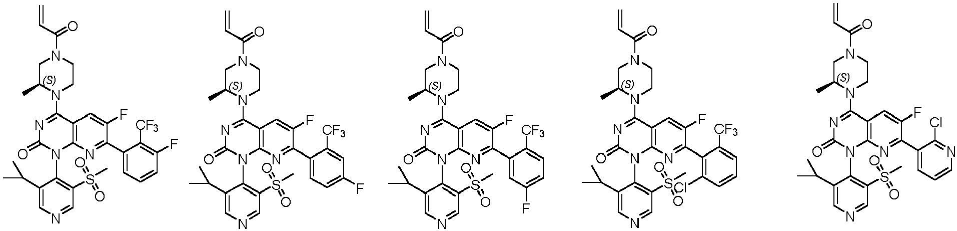

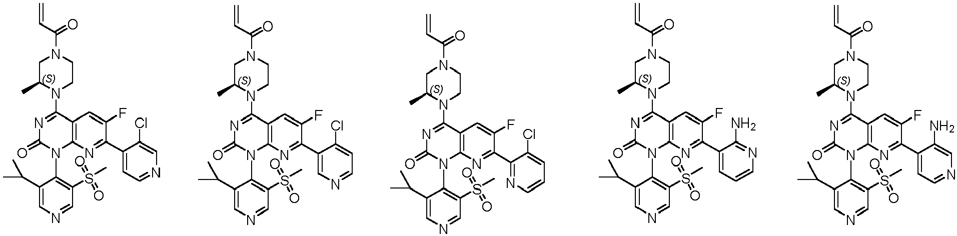

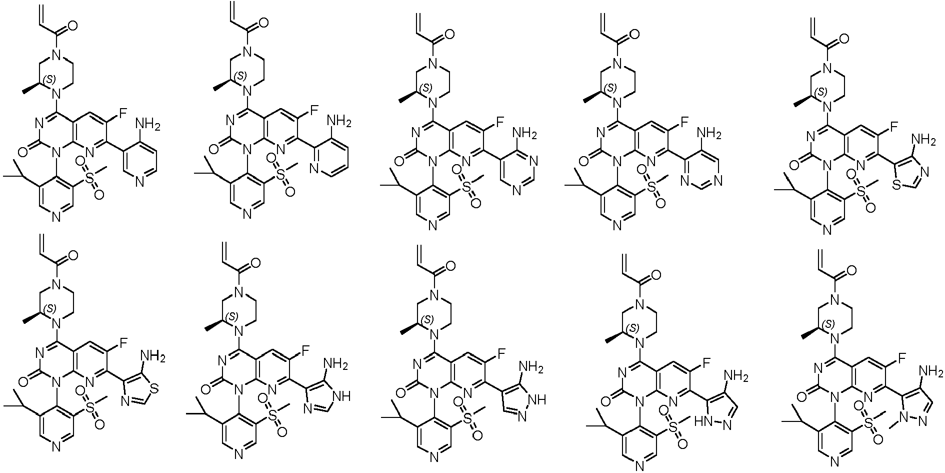

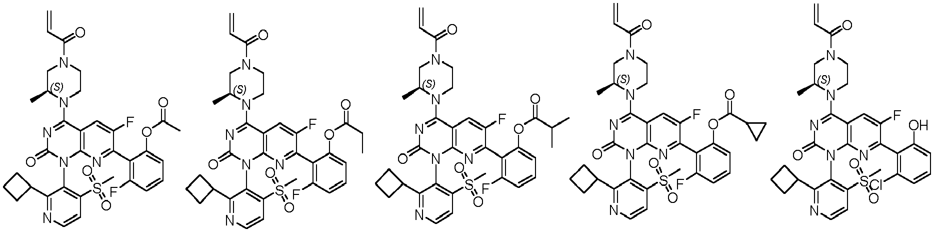

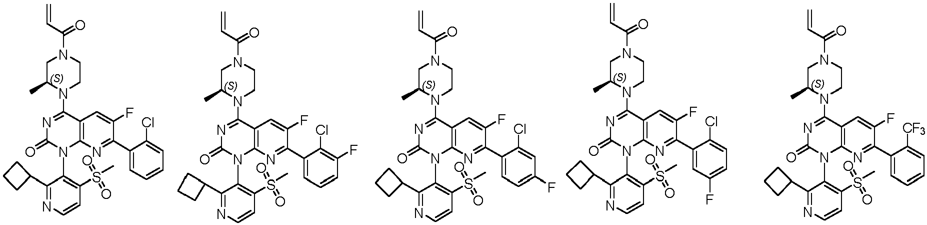

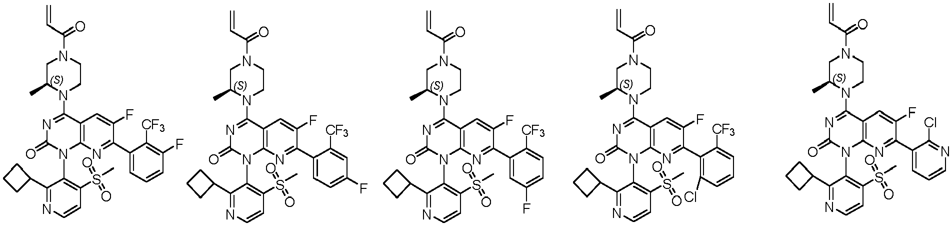

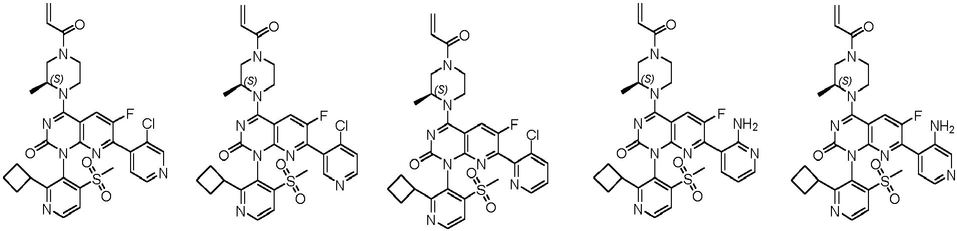

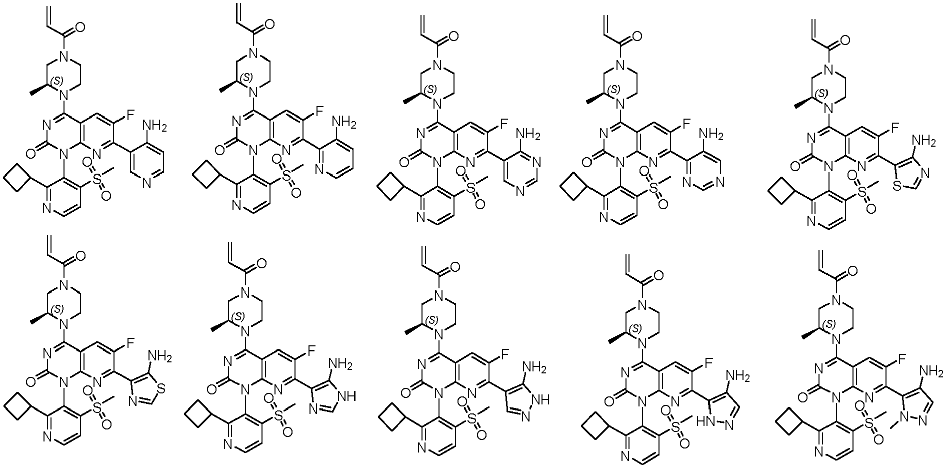

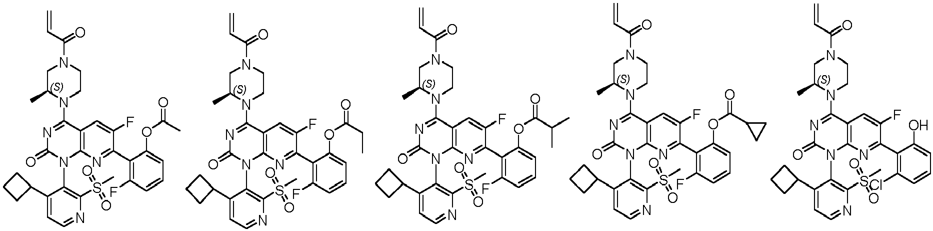

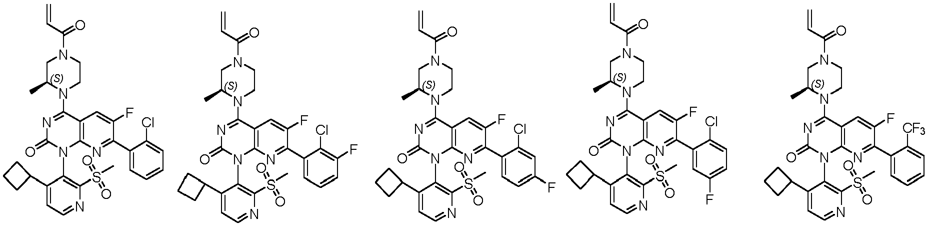

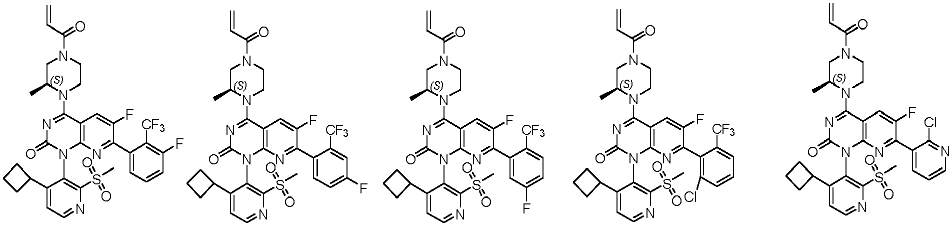

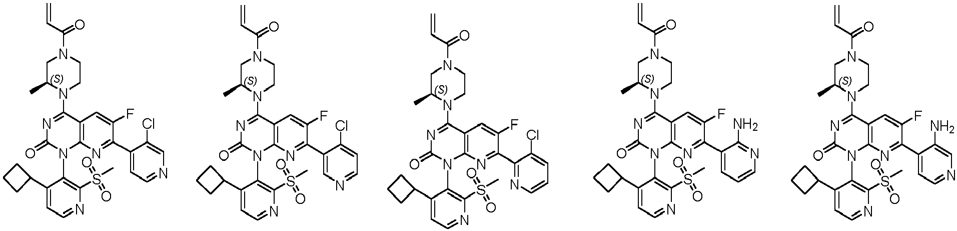

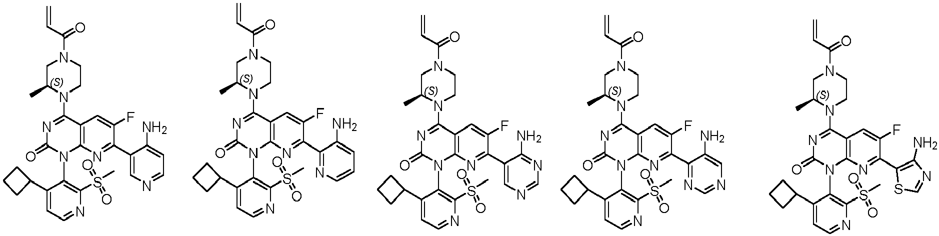

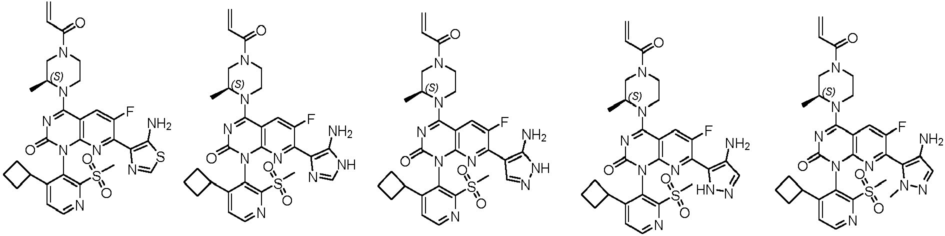

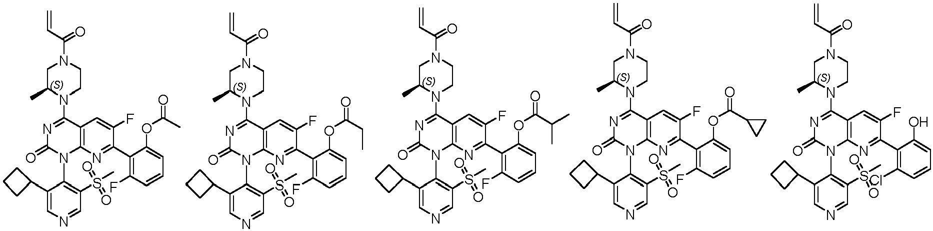

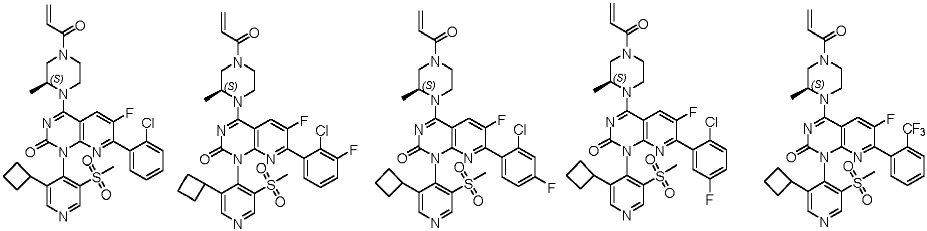

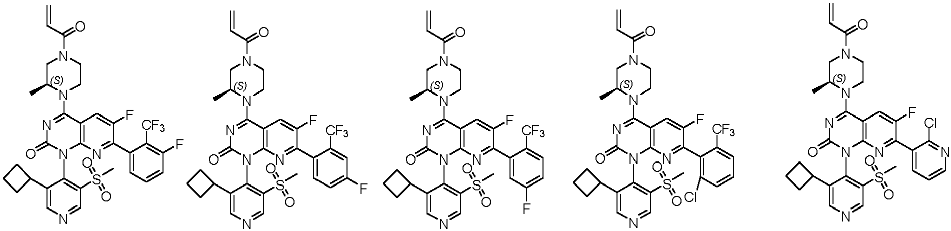

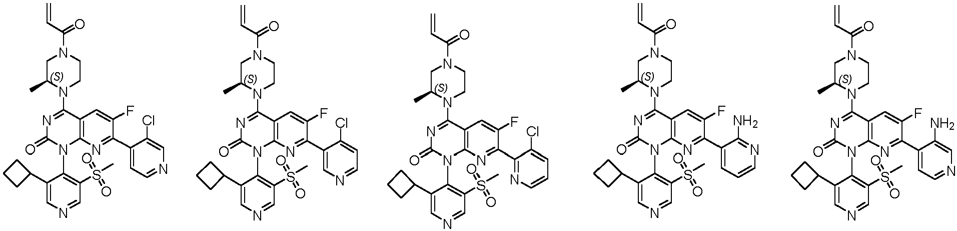

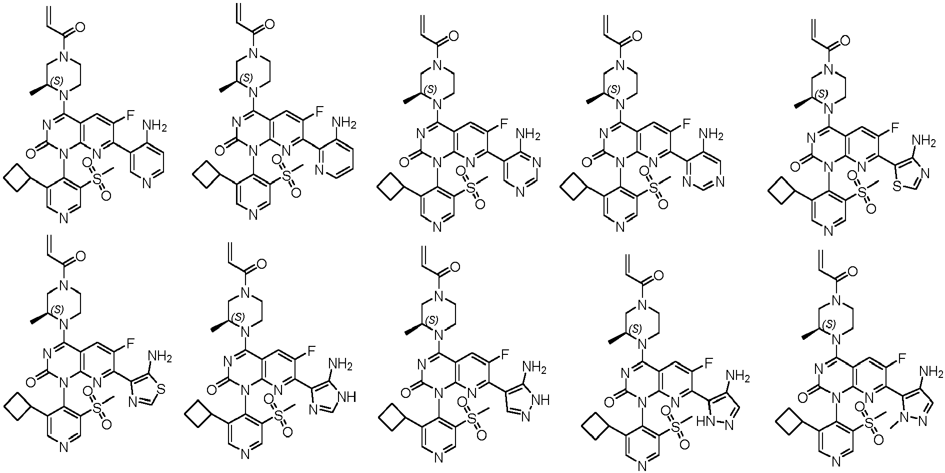

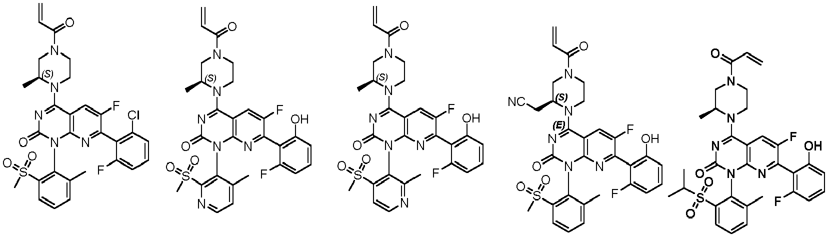

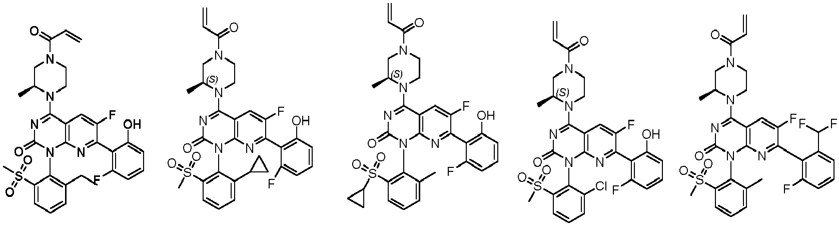

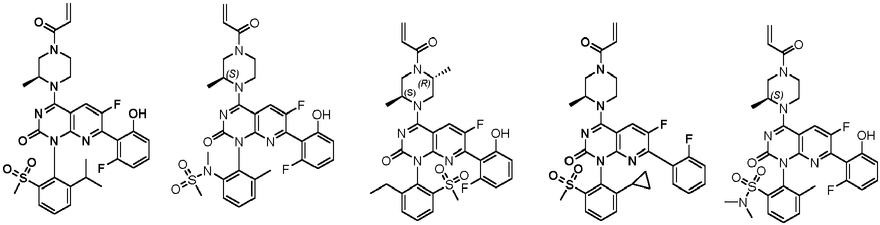

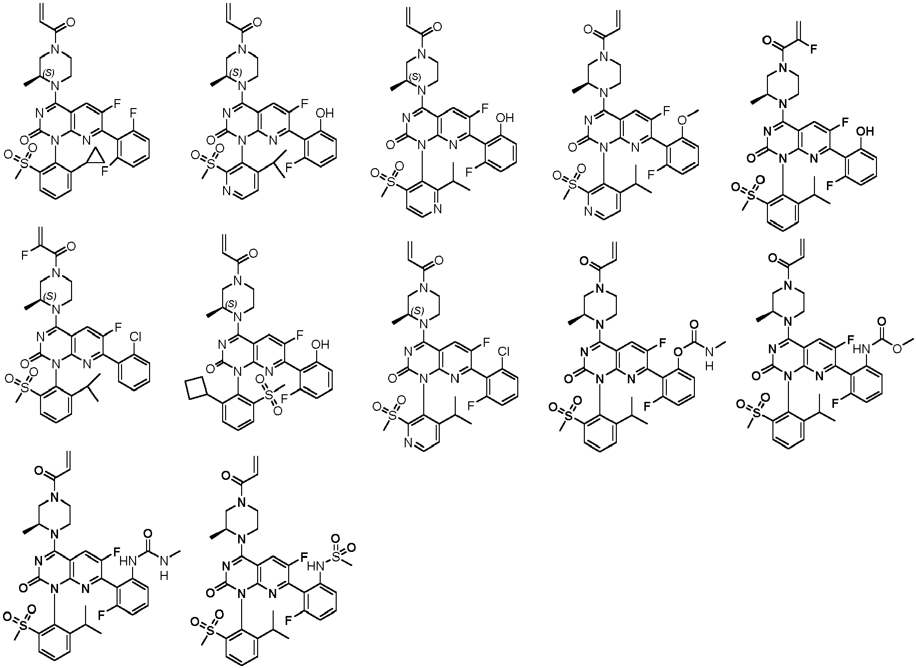

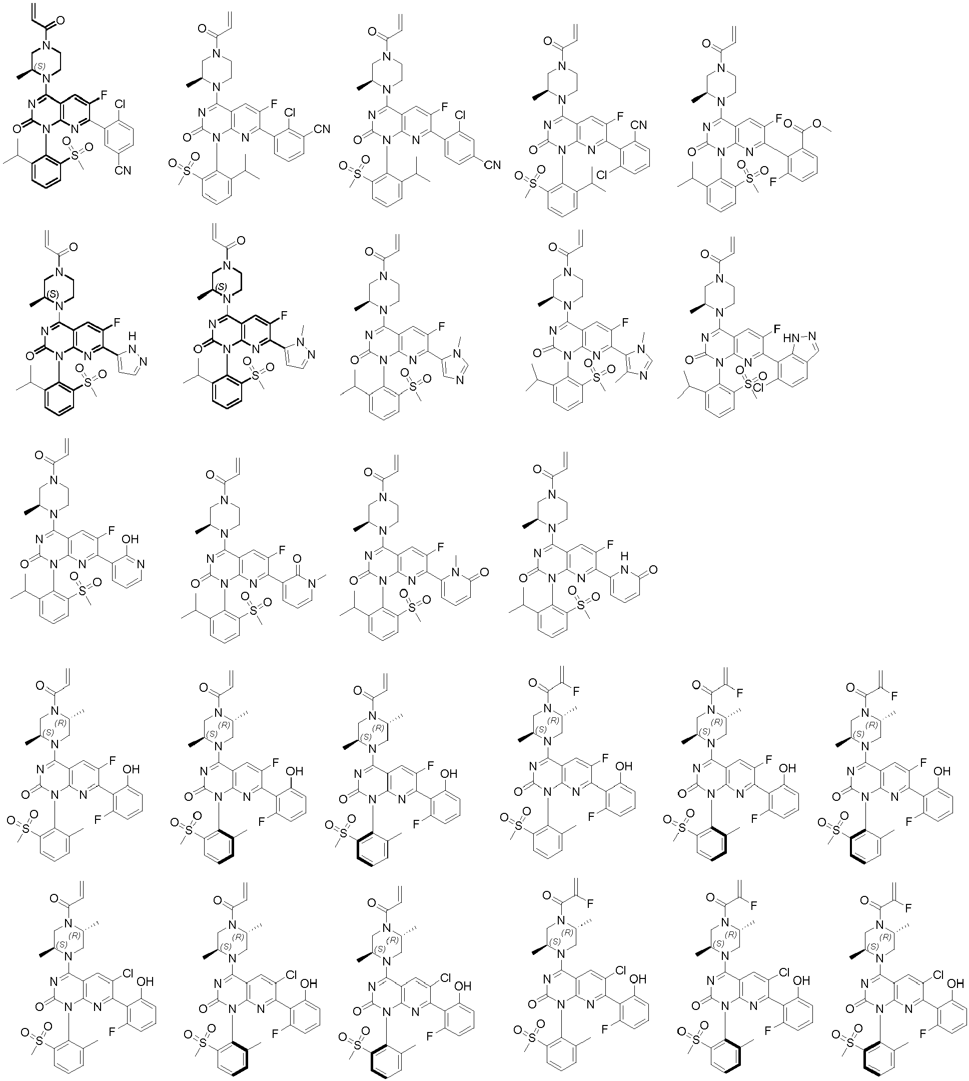

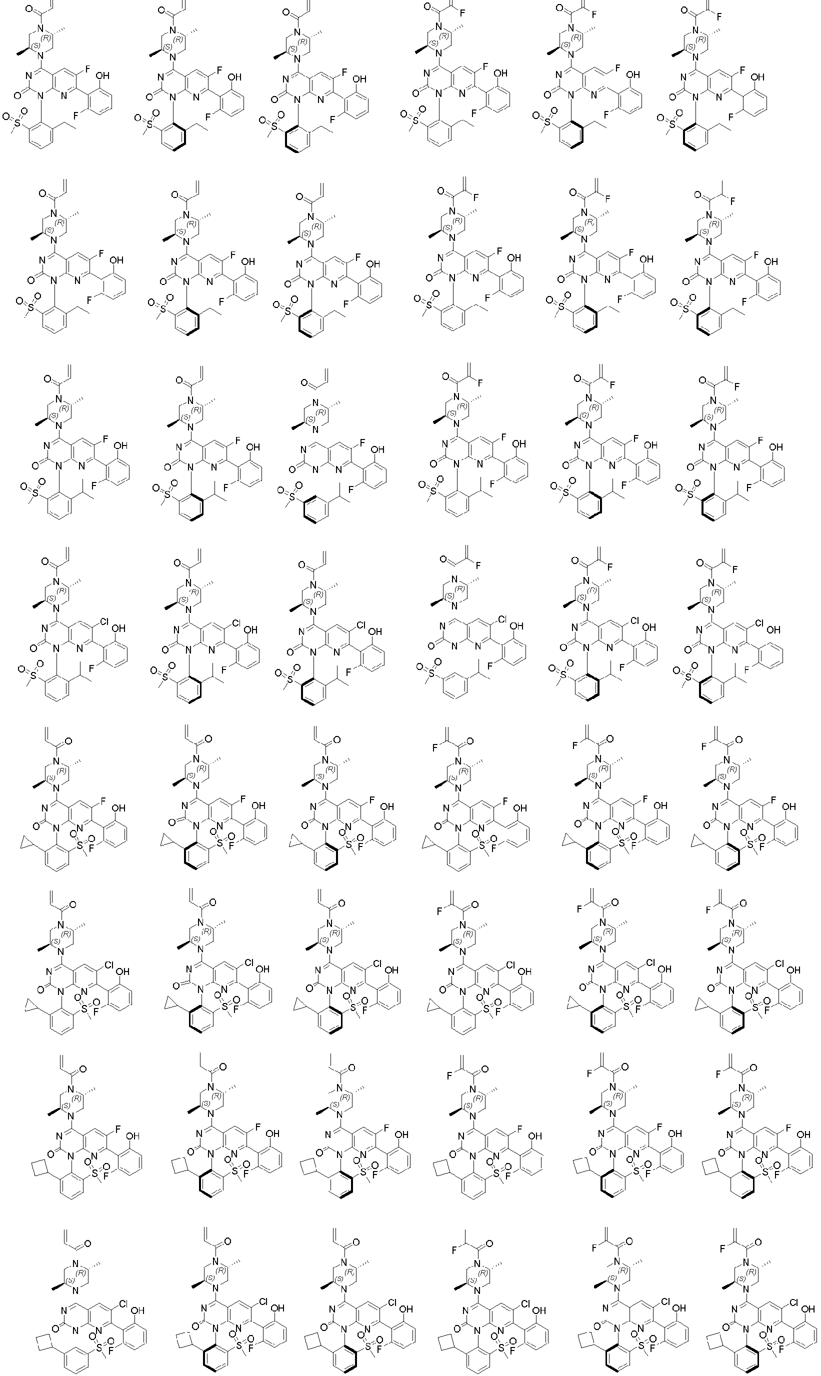

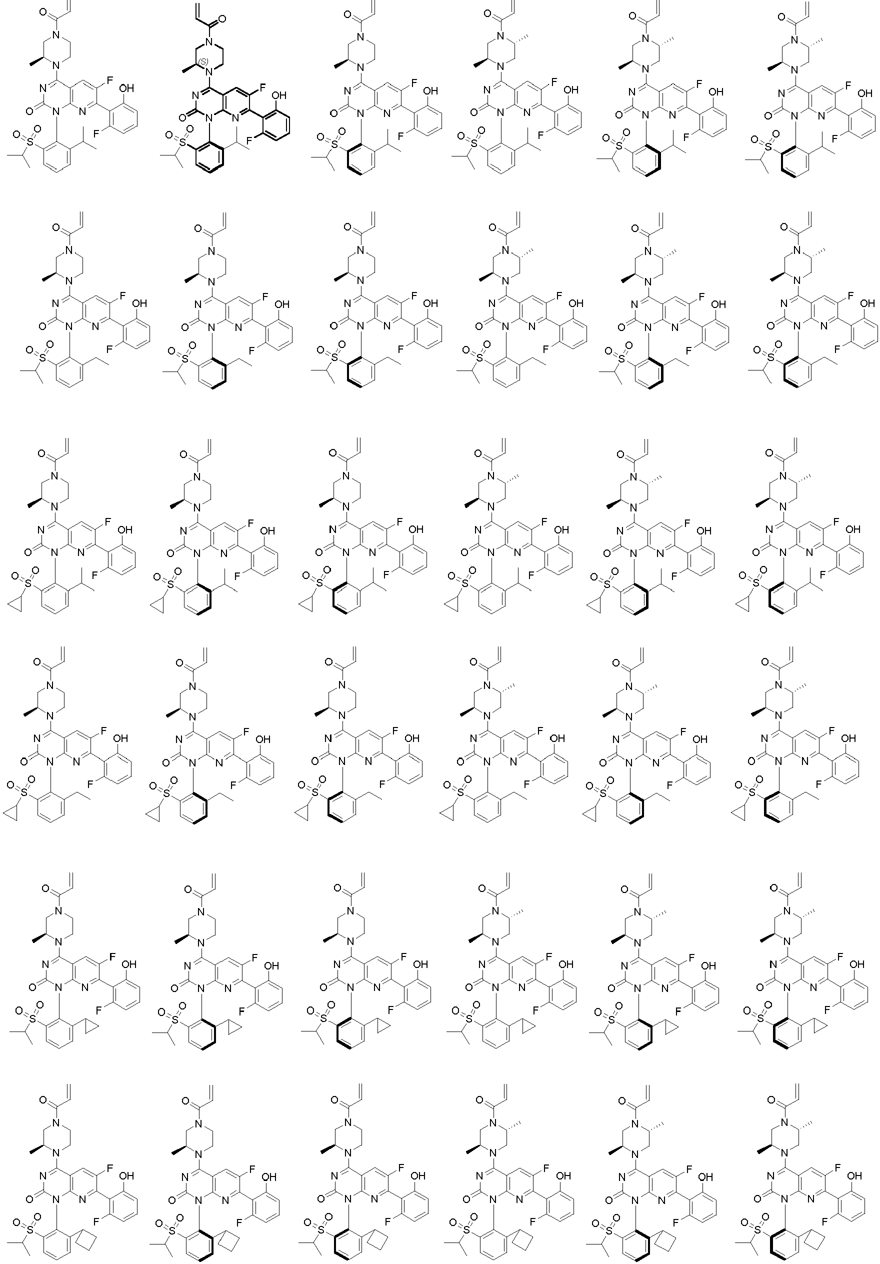

In another preferred embodiment, the compound of formula (I), the stereoisomer, tautomer, crystal form, pharmaceutically acceptable salt, hydrate, solvate or prodrug thereof does not comprise

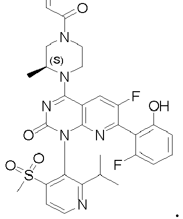

In another preferred embodiment, the compound of formula (I), or stereoisomer, tautomer, crystal form, pharmaceutically acceptable salt, hydrate, solvate or prodrug thereof is selected from the compounds shown in the examples.

In the second aspect of the present invention, it provides a method for preparing the compound of formula (I), or stereoisomer, tautomer, crystal form, pharmaceutically acceptable salt, hydrate, solvate or prodrug thereof, comprising steps: 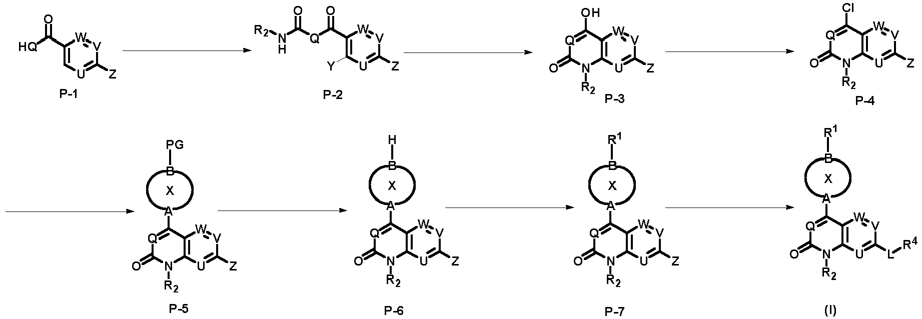
(i) in an inert solvent (such as tetrahydrofuran), the compound of formula P-1 first reacts with oxalyl chloride, and then reacts with the amino compound R₂-NH₂ to obtain the compound of formula P-2;
(ii) in an inert solvent (such as tetrahydrofuran), in the presence of a first base, the compound of formula P-2 undergoes a ring closing reaction to obtain the compound of formula P-3;
(iii) in an inert solvent (such as acetonitrile), in the presence of a second base, the compound of formula P-3 reacts with phosphorus oxychloride to obtain the compound of formula P-4;
(iv) in an inert solvent (such as acetonitrile), in the presence of a base (such as N,N-diisopropylethylamine), the compound of formula P-4 reacts with 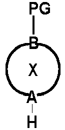 through coupling or substitution reaction to obtain the compound of formula P-5;
(v) in an inert solvent (such as dichloromethane), in the presence of an acid (such as trifluoroacetic acid), the compound of formula P-5 is deprotected to obtain the compound of formula P-6;
(vi) in an inert solvent (such as dichloromethane), in the presence of a base (such as N,N-diisopropylethylamine), the compound of formula P-6 reacts with R¹E through coupling, substitution or acylation reaction to obtain the compound of formula P-7;
(vii) in an inert solvent (such as dioxane/water), in the presence of a base (such as potassium acetate) and catalyst (such as [1,1 '-bis (diphenylphosphine) ferrocene] palladium dichloride), the compound of formula P-7 reacts with R₄-L-E₁ through coupling, substitution or acylation reaction to obtain the compound of formula (I);
wherein,
E is halogen, OH, OCOR¹, OCO(ⁱBu), etc;
E₁ is -BH₂, -B(OH)₂, 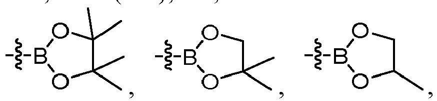 -Sn(Bu)₃, -ZnBr, etc.;
PG is an amino protection group, and the protection group is selected from the group consisting of Boc, Bn, Cbz and Fmoc;
Y and Z are leaving groups, and the leaving groups are selected from the group consisting of halogen and OTf;
the first base is selected from the group consisting of KHMDS, NaHMDS, LiHMDS, NaH, NaOMe, NaOEt, and ^{t}BuONa;
the second base is selected from the group consisting of TEA, DIPEA, DMAP and N,N-dimethylaniline;
R¹, R², R⁴, L, A, B, X, U, V, W, and Q are defined as in the first aspect.

In the third aspect of the invention, it provides a pharmaceutical composition comprising one or more compounds of the formula (I), the stereoisomer, tautomer, crystal form, pharmaceutically acceptable salt, hydrate, solvate or prodrug thereof of the first aspect; and pharmaceutically acceptable carriers.

In another preferred embodiment, the pharmaceutical composition further comprises a drug selected from the group consisting of: PD-1 inhibitor (e. g., nivolumab, pimumab, pidilizumab, cemiplimab, JS-001, SHR-120, BGB-A317, IBI-308, GLS-010, GB-226, STW204, HX008, HLX10, BAT1306, AK105, LZM 009 or the biological analogue thereof, etc.), PD-L1 inhibitor (e. g, dulvalumab, atezumab, avelumab, CS1001, KN035, HLX20, SHR-1316, BGB-A333, JS003, CS1003, KL -A167, F 520, GR1405, MSB2311 or the biological analogue thereof, etc.), CD20 antibody (e. g, rituximab, obinutuzumab, ofatumumab, veltuzumab, tositumomab, 131I-tositumomab, ibritumomab tiuxetan, 90Y-ibritumomab tiuxetan, 90In-ibritumomab tiuxetan, ibritumomab tiuxetan, etc.), CD47 antibody (e. g, Hu5F9-G4, CC-90002, TTI-621, TTI-622, OSE-172, SRF-231, ALX-148, NI-1701, SHR-1603, IBI188, IMM01), ALK inhibitor (e. g, Ceritinib, Alectinib, Brigatinib, Lorlatinib, Ocatinib), PI3K inhibitors (e. g, Idelalisib, Duvelisib, Dactolisib, Taselisib, Bimiralisib, Omipalisib, Buparlisib, etc.), BTK inhibitor (e. g, ibrutinib, Tirabrutinib, Acalabrutinib, Zanubrutinib, Vecabrutinib, etc.), EGFR inhibitor (e. g, Afatinib, Gefitinib, Erlotinib , Lapatinib, Dacomitinib, Icotinib, Canertinib, Sapitinib, Naquotinib, Pyrotinib, Rociletinib, Osimertinib, etc.), VEGFR inhibitor (e. g, Sorafenib, Pazopanib, Regorafenib, Sitravatinib, Ningetinib, Cabozantinib, Sunitinib, Donafenib, etc.), HDAC inhibitor (e. g, Givinostat, Tucidinostat, Vorinostat, Fimepinostat, Droxinostat, Entinostat, Dacinostat, Quisinostat, Tacedinaline, etc.), CDK inhibitor (e. g, Palbociclib, Ribociclib, Abemaciclib, Milciclib, Trilaciclib, Lerociclib, etc.), MEK inhibitor (e. g, Simetinib (AZD6244), Trametinib (GSK1120212), PD0325901, U0126, Pimasertib (AS-703026), PD184352 (CI-1040), etc.), mTOR inhibitor (e. g, Vistusertib, etc.), SHP2 inhibitor (e. g, RMC-4630, JAB-3068, TNO155, etc.), and a combination thereof.

In another preferred embodiment, it provides a method of preparing the pharmaceutical composition comprising the step of mixing pharmaceutically acceptable carriers with the compound of formula (I), or stereoisomer, tautomer, crystal form, pharmaceutically acceptable salt, hydrate, solvate or prodrug thereof of the first aspect, thereby forming the pharmaceutical composition.

In the fourth aspect of the invention, it provides a use of the compound of formula (I), or stereoisomer, tautomer, crystal form, pharmaceutically acceptable salt, hydrate, solvate or prodrug thereof of the first aspect, or the pharmaceutical composition of the third aspect for preparing a pharmaceutical composition for preventing and / or treating the disease related to the activity or expression of KRAS ^{G12C}.

In the fifth aspect of the invention, it provides a method for preventing and / or treating the disease related to the activity or expression of KRAS ^{G12C} comprising the step of administrating an effective amount of the compound of the formula (I), the stereoisomer, tautomer, crystal form, pharmaceutically acceptable salt, hydrate, solvate or prodrug thereof of the first aspect or the pharmaceutical composition of the third aspect to the subject in need thereof.

In another preferred embodiment, the disease is tumor or dysfunctional disease.

In another preferred embodiment, the disease is selected from the group consisting of lung cancer, breast cancer, prostate cancer, esophageal cancer, colorectal cancer, bone cancer, kidney cancer, gastric cancer, liver cancer, colon cancer, melanoma, lymphoma, leukemia, brain tumor, myeloma, soft tissue sarcoma, pancreatic cancer, skin cancer.

In the sixth aspect of the invention, it provides a non-diagnostic and non-therapeutic method for inhibiting KRAS^{G12C} comprising the step of administrating an effective amount of the compound of the formula (I), or stereoisomer, tautomer, crystal form, pharmaceutically acceptable salt, hydrate, solvate or prodrug thereof of the first aspect or the pharmaceutical composition of the third aspect to the subject in need thereof.

In the seventh aspect of the invention, it provides a method for inhibiting KRAS^{G12C} in vitro, comprising the steps of contacting the compound of formula (I), or stereoisomer, tautomer, crystal form, pharmaceutically acceptable salt, hydrate, solvate or prodrug thereof of the first aspect or the pharmaceutical composition of the third aspect with somatic cells.

In another preferred embodiment, the somatic cells are derived from a primate, such as a human.

It should be understood that in the present invention, any of the technical features specifically described above and below (such as in the Example) can be combined with each other, thereby constituting new or preferred technical solutions. Limited by space, it will not be repeated here.

### DETAILED DESCRIPTION OF THE INVENTION

After a long and intensive study, the inventor had accidentally prepared a new class of compounds with selective inhibition and/or better pharmacodynamic properties of KRAS^{G12C}. On this basis, the inventor completed the present invention.

### Terms

In the present invention, unless otherwise specified, the terms used have the general meanings known to those skilled in the art.

When a substituent is described by a conventional chemical formula written from left to right, the substituent also includes a chemically equivalent substituent obtained by writing a structural formula from right to left. For example, -CH₂O- is equivalent to -OCH₂-.

The term "alkyl" refers to a linear or branched chain alkane group, which may include any number of carbon atoms, wherein "C₁-C₁₈ alkyl" refers to alkyl containing 1, 2, 3, 4, 5, 6, 7, 8, 9, 10, 11, 12, 13, 14, 15, 16, 17 or 18 carbon atoms, preferably, for example, C₁-C₂, C₁-C₃, C₁-C₄, C₁-C₅, C₁-C₆, C₁-C₇, C₁-C₈, C₁-C₉, C₁-C₁₀, C₂-C₃, C₂-C₄, C₂-C₅, C₂-C₆, C₃-C₄, C₃-C₅, C₃-C₆, C₄-C₅, C₄-C₆ or C₅₋₆. Typical "alkyl" includes but is not limited to, methyl, ethyl, propyl, isopropyl, n-butyl, tert-butyl, isobutyl, 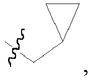 pentyl, isopentyl, heptyl, 4,4-dimethylpentyl, octyl, 2,2,4-trimethylpentyl, nonyl, decyl, undecyl , dodecyl, and the like. In the present invention, the alkyl further comprises substituted alkyl. "Substituted alkyl" refers to one or more positions in the alkyl are substituted, especially 1-4 substituents, which can be substituted at any position.

The term "cycloalkyl" refers to a completely saturated cyclic hydrocarbon group, wherein "C₃-C₂₀ cycloalkyl" refers to a completely saturated cyclic hydrocarbon group containing 3, 4, 5, 6, 7, 8, 9, 10, 11, 12, 13, 14, 15, 16, 17, 18, 19 or 20 carbon atoms, consisting of 1-4 rings, each containing 3-8 carbon atoms. Preferably C₃-C₄, C₃-C₅, C₃-C₆, C₃-C₇, C₃-C₈, C₃-C₉, C₃-C₁₀. "Substituted cycloalkyl" refers to one or more positions in the cycloalkyl are substituted, especially 1-4 substituents, which can be substituted at any position. In the present invention, "cycloalkyl" is intended to include "substituted cycloalkyl".

The term "heterocyclyl" refers to a completely saturated or partially unsaturated cyclic group, wherein, "3-20-membered heterocyclyl" refers to completely saturated or partially unsaturated cyclic group containing 3, 4, 5, 6, 7, 8, 9, 10, 11, 12, 13, 14, 15, 16, 17, 18, 19 or 20 ring atoms (including, but not limited to, such as 3-7 membered mono ring, 6-11 membered bicyclo, or 8-16 membered tricyclic system) in which at least one heteroatom exists in a ring containing at least one carbon atom. Each heterocyclyl containing heteroatom can have 1, 2, 3 or 4 heteroatoms, and these heteroatoms are selected from nitrogen, oxygen or sulfur, wherein the nitrogen or sulfur can be oxidized, and the nitrogen can also be quaternized. Heterocyclyl can be attached to the residue of any heteroatom or carbon atom of the ring or ring molecule. Typical monocyclic heterocyclyls include, but are not limited to azetidinyl, pyrrolidyl, oxetanyl, pyrazolinyl, imidazolinyl, imidazolidinyl, oxazolidinyl, isoxazolidinyl, thiazolidinyl, isothiazolidinyl, tetrahydrofuryl, piperidyl, piperazinyl, 2-oxoppiperazinyl, 2-oxopiperidyl, 2-oxopyrrolidyl, hexahydroacridheptyl, 4-piperidinone, tetrahydropyranyl, morpholinyl, thiomorpholinyl, thiomorpholinyl-sulfoxide, thiomorpholine-sulfonyl, 1,3-dioxoalkyl and tetrahydro-1,1-dioxothienyl, etc.. A polycyclic heterocyclyl includes spiro, fused, and bridged heterocyclyls. The spiro, fused, and bridged heterocyclyls involved are optionally connected with other groups by single bond, or are further fused with other cycloalkyl, heterocyclyl, aryl and heteroaryl by any two or more atoms of the ring; and the heterocyclyl can be substituted or unsubstituted.

The term "aryl" refers to an aromatic cyclic hydrocarbon group, wherein "C6-C14 aryl" refers to an aromatic cyclic hydrocarbon group containing 6, 7, 8, 9, 10, 11, 12, 13 or 14 ring carbon atoms having 1-5 rings, particularly monocyclic and bicyclic groups such as phenyl, biphenyl or naphthyl. Any aromatic ring having two or more aromatic rings (bicyclic, etc.) , the aromatic rings of aryl may be connected by single bond (such as biphenyl) or fused (such as naphthalene, anthracene, etc.). "Substituted aryl" refers to one or more positions in the aryl are substituted, especially 1-3 substituents, which can be substituted at any position.

The term "heteroaryl" refers to an aromatic cyclic hydrocarbon group containing 1 to 4 heteroatoms, wherein the heteroatoms are selected from oxygen, nitrogen, and sulfur. The term "5-14membered heteroaryl" refers to an aromatic cyclic hydrocarbon group containing 5, 6, 7, 8, 9, 10, 11, 12, 13, or 14 ring atoms, wherein the ring atom contains 1 to 4 heteroatoms selected from N, O, and S. The heteroaryl is preferably 5 to 10 membered, more preferably 5 or 6 membered, the heteroaryl includes, but is not limited to, pyrryl, pyrazolyl, imidazolyl, oxazolyl, isoxazolyl, thiazoly, thiadiazolyl, isothiazolyl, furanyl, pyridyl, pyrazinyl, pyrimidyl, pyridazinyl, triazinyl, triazolyl, and tetrazolyl, etc..

The term "alkoxy" refers to a linear or branched chain or cyclic alkoxy, wherein "C1-C18 alkoxy" refers to a linear or branched chain or cyclic alkoxy having 1 to 18 carbon atoms, comprising C1-C18 alkyl-O-, -C1-C6 alkyl-O-C1-C6 alkyl, preferably C1-C8 alkoxy, more preferably C1-C6 alkoxy, alkoxy includes, but is not limited to, methoxy, ethoxy, propoxy, isopropoxy, butoxy, and the like.

"Cycloalkenyl" refers to a cyclic hydrocarbon group having one or more double bonds, wherein "C₄-C₁₀ cycloalkenyl" refers to a cyclic hydrocarbon group having one or more double bonds containing 4, 5, 6, 7, 8, 9 or 10 carbon atoms, preferably C₄-C₆ cycloalkenyl, including but not limited to cyclobutenyl, cyclopentenyl, cyclopentadienyl, cyclohexenyl, cyclohexadienyl, etc.

The term "ester group" refers to a group with a structure of -COOR, wherein R represents hydrogen, alkyl or substituted alkyl, cycloalkyl or substituted cycloalkyl, cycloalkeny or substituted cycloalkenyl, aryl or substituted aryl, heteroaryl or substituted heteroaryl, heterocyclyl or substituted heterocyclyl. wherein, alkyl, cycloalkyl, cycloalkenyl, aryl, heteroaryl, heterocyclyl have the definitions described above.

The term "amino" refers to a group with a structure of -NRR', wherein R and R' can be independently hydrogen, alkyl or substituted alkyl, cycloalkyl or substituted cycloalkyl, cycloalkenyl or substituted cycloalkenyl, aryl or substituted aryl, heteroaryl or substituted heteroaryl, heterocyclyl or substituted heterocyclyl. In which, alkyl, cycloalkyl, cycloalkenyl, aryl, heteroaryl, heterocyclyl have the definitions described above. R and R' can be the same or different, when R and R' are H at the same time, the amino is -NH₂. Examples of amino includes, but are not limited to, methylamino, dimethylamino, ethylamino, diethylamino, propylamino, isopropylamino, butylamino, and the like.

The term "amido" refers to a group with a structure of -CONRR', wherein R and R' can be independently hydrogen, alkyl or substituted alkyl, cycloalkyl or substituted cycloalkyl, cycloalkenyl or substituted cycloalkenyl, aryl or substituted aryl, heteroaryl or substituted heteroaryl, heterocyclyl or substituted heterocyclyl. In which, alkyl, cycloalkyl, cycloalkenyl, aryl, heteroaryl, heterocyclyl have the definitions described above. R and R' can be the same or different.

The term "sulfonamido" refers to a group with a structure of -SO₂NRR', wherein R and R' can be independently hydrogen, alkyl or substituted alkyl, cycloalkyl or substituted cycloalkyl, cycloalkenyl or substituted cycloalkenyl, aryl or substituted aryl, heteroaryl or substituted heteroaryl, heterocyclyl or substituted heterocyclyl. In which, alkyl, cycloalkyl, cycloalkenyl, aryl, heteroaryl, heterocyclyl have the definitions described above. R and R' can be the same or different.

The term "aminosulfonyl" refers to a group with a structure of -NRSO₂R', wherein R and R' can be independently hydrogen, alkyl or substituted alkyl, cycloalkyl or substituted cycloalkyl, cycloalkenyl or substituted cycloalkenyl, aryl or substituted aryl, heteroaryl or substituted heteroaryl, heterocyclyl or substituted heterocyclyl. In which, alkyl, cycloalkyl, cycloalkenyl, aryl, heteroaryl, heterocyclyl have the definitions described above. R and R' can be the same or different.

The term "carbamido" refers to a group with a structure of -NRCONR'R", wherein R, R' and R" can be independently hydrogen, alkyl or substituted alkyl, cycloalkyl or substituted cycloalkyl, cycloalkenyl or substituted cycloalkenyl, aryl or substituted aryl, heteroaryl or substituted heteroaryl, heterocyclyl or substituted heterocyclyl. In which, alkyl, cycloalkyl, cycloalkenyl, aryl, heteroaryl, heterocyclyl have the definitions described above. R, R' and R" can be the same or different.

When the substituent is a non-terminal substituent or the related group removes an H atom, it is a subunit of the corresponding group, usually a divalent group. For example, after removing an H atom, the alkyl is alkylene (such as methylene, ethylidene, propylidene, isopropylidene (such as 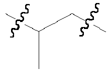 ), butylidene (such as 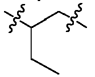 or 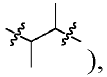 pentylidene (such as 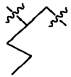 ), hexylidene (such as 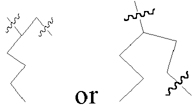 ), heptylidene (such as 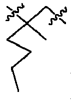 ), etc.), cycloalkyl corresponds to cycloalkylene (such as 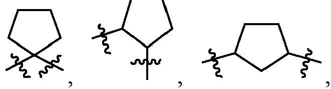 etc.), heterocyclyl corresponds to heterocyclylene (such as 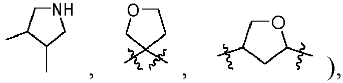 and alkoxy corresponds to alkyleneoxy (-CH₂O-, -CH₂CH₂-O-CH₂-, -CH₂OCH₂CH₂CH₂-), etc.

The term "halogen" or "halo" is chlorine, bromine, fluorine, and iodine.

The term "halogenated" means that H in a group is substituted by a halogen.

The term "deuterated" refers to that H in a group is substituted by a deuterium.

The term "hydroxy" refers to a group with a structure of OH.

The term "nitro" refers to a group with a structure of NO₂.

The term "cyano" refers to a group with a structure of CN.

The term "selected from the substituted or unsubstituted group consisting of" means that the H atom of the selected group is substituted or unsubstituted, while the selected group does not contain the H atom, it will not be substituted.

Unless otherwise stated, it is assumed that any heteroatom with a lower valence state has enough hydrogen atoms to replenish its valence state.

As described herein, the compound in the present invention may be substituted with any number of substituents or functional groups to extend its scope. In general, whether the term "substituted" appears before or after the term "optional", the general formula that includes substituents in the compound of the present invention means the substitution of a specified structural substituent for hydrogen radical. When multiple locations in a particular structure are substituted by multiple specific substituents, the substituents at each location can be the same or different. The term "substituted" as used herein includes all substitution that allows organic compounds to be substituted. Broadly speaking, the allowable substituents include non-cyclic, cyclic, branched, non-branched, carbocyclic and heterocyclic, aromatic ring and non-aromatic ring organic compounds. In the present invention, such as heteroatomic nitrogen, its valence state may be supplemented by a hydrogen substituent or by any permitted organic compound described above. Furthermore, the invention is unintentionally limited to the substituted organic compounds. The present invention considers that a combination of substituents and variable groups is good for the treatment of diseases (such as infectious or proliferative diseases) in the form of stable compounds. The term "stable" herein refers to a stable compound which is sufficient for maintaining the integrity of the compound structure within a sufficiently long time, preferably being effective in a sufficiently long time, which is hereby used for the above purposes.

In the present invention, the term "substituted" refers to one or more hydrogen atoms on a specific group being substituted by a specific substituent. The specific substituents are those described in the preceding paragraph or those present in each Example. Unless otherwise specified, a substituted group may have a substituent selected from a specific group at any substitutable position of the group, and the substituent may be the same or different in each position. In the present invention, unless otherwise stated, the group comprises a corresponding substituent group and a subunit, for example, alkyl comprises substituted alkyl, cycloalkyl comprises substituted cycloalkyl, aryl comprises substituted aryl, heteroaryl comprises substituted heteroaryl, and heterocyclyl comprises substituted heterocyclyl, etc.. Those skilled in the art should understand that the combinations of substituents contemplated by the present invention are those that are stable or chemically achievable. Typically substituents include, but are not limited to one or more of the following groups: such as hydrogen, deuterium, halogen (such as monohalogenated substituent or polyhalogenated substituents, and the latter such as trifluoromethyl or alkyl containing Cl₃), cyano, nitro, oxo (such as = O), trifluoromethyl, trifluoromethoxy, cycloalkyl, C2-C6 alkenyl, C4-C10 cycloalkenyl, C2-C6 alkynyl, heterocyclyl, aryl, heteroaryl, ORₐ, SRa, S(=O)Rₑ, S(=O)₂Rₑ, P(=O)₂Rₑ, S(=O)₂ORₑ, P(=O)₂ORₑ, NR_{b}Rₑ, NR_{b}S(=O)₂Rₑ, NR_{b}P(=O)₂Rₑ, S(=O)₂NR_{b}Rₑ, P(=O)₂NR_{b}Rₑ, C(=O)OR_{d}, C(=O)Rₐ, C(=O)NR_{b}R_{c}, OC(=O)Rₐ, OC(=O)NR_{b}Rₑ, NR_{b}C(=O)ORₑ, NR_{d}C(=O)NR_{b}Rₑ, NR_{d}S(=O)₂NR_{b}Rₑ, NR_{d}P(=O)₂NR_{b}Rₑ, NR_{b}C(=O)Rₐ or NR_{b}P(=O)₂Rₑ, wherein Rₐ appearing here can be independently hydrogen, deuterium, C1-C6 alkyl, C3-C8 cycloalkyl, C2-C6 alkenyl, C3-C10 cycloalkenyl, C2-C6 alkynyl, 4-8 membered heterocyclyl, 5-14 membered heteroaryl or C6-C14 aryl. R_{b}, Rₑ and R_{d} can be independently hydrogen, deuterium, C1-C6 alkyl, C3-C8 cycloalkyl, 4-8 membered heterocyclyl, 5-14 membered heteroaryl or C6-C14 aryl, or R_{b} and Rₑ together with the N atom can form a heterocycle. Rₑ can be independently hydrogen, C1-C6 alkyl, C3-C8 cycloalkyl, C2-C6 alkenyl, C3-C6 cycloalkenyl, C2-C6 alkynyl, 4-8 membered heterocyclyl, 5-14 membered heteroaryl or C6-C14 aryl. The above typical substituents, such as alkyl, cycloalkyl, alkenyl, cycloalkenyl, alkynyl, heterocyclyl, heteroaryl or aryl, and their corresponding substituent groups and subunits, may optionally be substituted, wherein the alkyl, cycloalkyl, cycloalkenyl, heterocyclyl, heteroaryl or aryl has the definitions described above.

### Active ingredient

As used herein, the terms "compounds of the invention" or "active ingredients of the invention" are used interchangeably and refer to compounds of formula (I), or pharmaceutically acceptable salt, hydrate, solvate, isotope compound (e.g. deuterated compound) or prodrug thereof. The term also includes racemate and optical isomer.

The compound of formula (I) has the following structure: 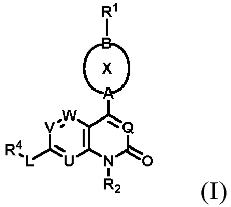 wherein, R¹, R², R⁴, L, U, V, W, Q, A, B and X are defined as above.

Preferably, the compound of formula (I) has a structure shown in formula (II-A) or (II-B): 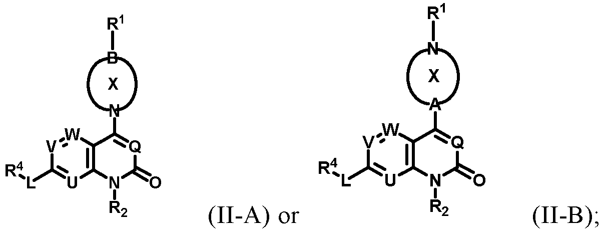 wherein:
R¹, R², R⁴, A, B, L, X, U, V, W and Q are defined as above.

Preferably, the compound of formula (I) has the structure shown in formula (III): 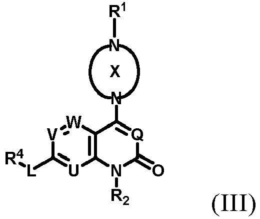 R¹, R², R⁴, X, L, U, V, W and Q are defined as above.

Preferably, the compound of formula (I) has a structure shown in formula (IV): 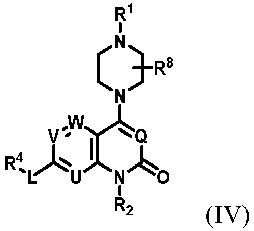 wherein:
R¹, R², R⁴, R⁸, L, U, V, W and Q are defined as above.

Preferably, the compound of formula (I) has a structure shown in formula (V): 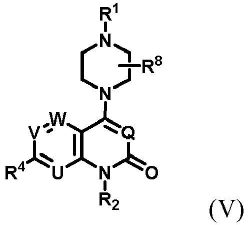 wherein:
R¹, R², R⁴, R⁸, U, V, W and Q are defined as above.

Preferably, the compound of formula (I) has the structure shown in formula (VI): 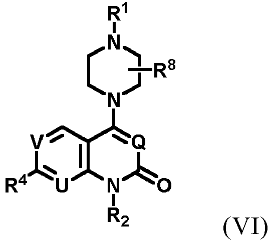 wherein:
R¹, R², R⁴, R⁸, U, V and Q are defined as above.

Preferably, the compound of formula (I) has a structure shown in formula (VII): 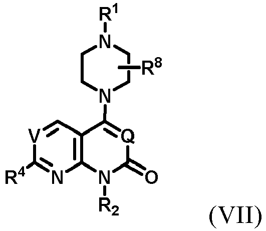 wherein:
R¹, R², R⁴, R⁸, V and Q are defined as above.

Preferably, in the above-mentioned formulas (i. e. I-VII), R¹ is selected from the group consisting of **-C(O)C(R*^{A}*)=C(R*^{B}*)*₂*, -S(O)₂C(R*^{A}*)=C(R*^{B}*)*₂***, **-NR⁶C(O)C(R*^{A}*)=C(R*^{B}*)*₂*** and **-NR⁶S(O)₂C(R*^{A}*)-C(R*^{B}*)*₂*;**
wherein, R*^{A}* is independently selected from the group consisting of hydrogen, deuterium, fluorine, cyano and C₁-C₃ alkyl; each R*^{B}* is the same or different, and is independently selected from the group consisting of hydrogen, deuterium, cyano and C₁-C₃ alkyl; wherein, the alkyl can be substituted by one or more (such as 2, 3, 4 or 5) substituents selected from the group consisting of deuterium, halogen, cyano, amino, C₃-C₇ cycloalkyl, 4-7-membered heterocyclyl, NHR⁹ and NR⁹R¹⁰; R⁹ and R¹⁰ are each independently C₁-C₃ alkyl; or R⁹ and R¹⁰ together with the N atom which they are attached to form a substituted or unsubstituted 4-8-membered heterocyclyl;

R⁶ is selected from the substituted or unsubstituted group consisting of hydrogen, deuterium, C₁-C₆ alkyl, deuterated C₁-C₆ alkyl, halogenated C₁-C₆ alkyl, C₃-C₈ cycloalkyl, C₁-C₆ alkoxy, deuterated C₁-C₆ alkoxy, halogenated C₁-C₆ alkoxy, 4-8 membered heterocyclyl, C₆-C₁₄ aryl and 5-14 membered heteroaryl;
wherein, unless otherwise stated, the "substituted" means being substituted by one or more substituents selected from the group consisting of hydrogen, deuterium, C₁-C₆ alkyl, deuterated C₁-C₆ alkyl, halogenated C₁-C₆ alkyl, C₃-C₈ cycloalkyl, C₁-C₆ alkoxy, deuterated C₁-C₆ alkoxy, halogenated C₁-C₆ alkoxy, C₆-C₁₀ aryl, 5-10 membered heteroaryl, 4-8 membered heterocyclyl, halogen, nitro, hydroxyl, cyano, ester group, amino, NR_{b}C(=O)ORₑ, OC(=O)NR_{b}R_{c}, amido, sulfonamido and carbamido; R_{b} and Rₑ can be independently hydrogen, deuterium, C1-C6 alkyl, C3-C8 cycloalkyl, 4-8 membered heterocyclyl, 5-14 membered heteroaryl or C6-C14 aryl, or R_{b} and Rₑ together with the N atom can form 4-8 membered heterocyclyl; Rₑ can be independently hydrogen, C1-C6 alkyl, C3-C8 cycloalkyl, C2-C6 alkenyl, C3-C6 cycloalkenyl, C2-C6 alkynyl, 4-8 membered heterocyclyl, 5-14 membered heteroaryl or C6-C14 aryl;
with the proviso that
when B is N, R¹ is selected from the group consisting of **-C(O)C(R*^{A}*)-C(R*^{B}*)*₂*** and **S(O)₂C(R*^{A}*)=C(R*^{B}*)₂;**
when B is CH or CR⁵, R¹ is selected from the group consisting of **-NR⁶C(O)C(R*^{A}*)=C(R*^{B}*)*₂*** and **-NR⁶S(O)₂C(R*^{A}*)-C(R*^{B}*)*₂*,** more preferably, R¹ is **-C(O)C(R*^{A}*)=C(R*^{B}*)*₂*,** wherein **R*^{A}*** is independently selected from the group consisting of hydrogen and fluorine; each **R*^{B}*** is the same or different, and is independently selected from the group consisting of hydrogen and C₁-C₃ alkyl, wherein the alkyl can be substituted by one or more substituents selected from the group consisting of deuterium, halogen, cyano, amino, C₃-C₇ cycloalkyl, 4-7 membered heterocyclyl, NHR⁹ and NR⁹R¹⁰; R⁹ and R¹⁰ are each independently C₁-C₃ alkyl; or R⁹ and R¹⁰ together with the N atom to which they are attached form a 4-8 membered heterocyclyl.

Preferably, in the above-mentioned formulas, R² is selected from the substituted group consisting of phenyl and 5-6-membered heteroaryl, wherein the substituted means being substituted by one or more (such as 2, 3, 4 or 5) substituents selected from the group consisting of R', -SO₂R',-SO₂NR'R", -NR'SO₂R" and -P(= O)R'R"; R' and R" are the same or different, and are each independently selected from the substituted or unsubstituted group consisting of hydrogen, deuterium, halogen, nitro, hydroxyl, cyano, ester group, amino, amido, C₁-C₆ alkyl, C₃-C₆ cycloalkyl, C₄-C₆ cycloalkenyl, 4-8 membered heterocyclyl, C₆-C₁₀ aryl and 5-10 membered heteroaryl; or when R' and R" are attached to the same N atom, R' and R" together with the N atom to which they are attached form a substituted or unsubstituted 4-6 membered heterocyclyl; the substituted means being substituted by one or more (such as 2, 3, 4 or 5) substituents selected from the group consisting of hydrogen, deuterium, C₁-C₆ alkyl, deuterated C₁-C₆ alkyl, halogenated C₁-C₆ alkyl, C₃-C₈ cycloalkyl, C₁-C₆ alkoxy, deuterated C₁-C₆ alkoxy, halogenated C₁-C₆ alkoxy, C₆-C₁₀ aryl, 5-10 membered heteroaryl, 4-8 membered heterocyclyl, halogen, nitro, hydroxyl, cyano, ester group, amino, amido, sulfonamido and carbamido.

Preferably, in the above-mentioned formulas, R⁴ is selected from the substituted or unsubstituted group consisting of phenyl and 5-6 membered heteroaryl, wherein the substituted means being substituted by one or more (such as 2, 3, 4 or 5) substituents selected from the group consisting of hydrogen, deuterium, halogen, ester group, cyano, NR_{b}C(=O)ORₑ, OC(= O)Rₑ, OC(= O)NR_{b}Rₑ, amino, C₁-C₁₈ alkyl (preferably C₁-C₆ alkyl, more preferably C₁-C₃ alkyl), halogenated C₁-C₁₈ alkyl (preferably halogenated C₁-C₆ alkyl, more preferably halogenated C₁-C₃ alkyl) and hydroxyl; R_{b} and Rₑ can be independently hydrogen, deuterium, C1-C6 alkyl, C3-C8 cycloalkyl, 4-8 membered heterocyclyl, 5-14 membered heteroaryl or C6-C14 aryl, or R_{b} and Rₑ together with the N atom can form 4-8 membered heterocyclyl; Rₑ can be independently hydrogen, C1-C6 alkyl, C3-C8 cycloalkyl, C2-C6 alkenyl, C3-C6 cycloalkenyl, C2-C6 alkynyl, 4-8 membered heterocyclyl, 5-14-membered heteroaryl or C6-C14 aryl.

Preferably, in the above-mentioned formulas, Q is N.

Preferably, in the above-mentioned formulas, V and W are each independently CR³, and R³ is H or halogen; preferably, R³ is halogen.

Preferably, in the formula (IV), (V), (VI), or (VII), R⁸ is independently selected from the substituted or unsubstituted group consisting of hydrogen, deuterium, C₁-C₆ alkyl, deuterated C₁-C₆ alkyl, halogenated C₁-C₆ alkyl, C₃-C₈ cycloalkyl, C₁-C₆ alkoxy, deuterated C₁-C₆ alkoxy, halogenated C₁-C₆ alkoxy, amino, hydroxyl and 4-8-membered heterocyclyl; the substituted means being substituted by one or more (such as 2, 3, 4, or 5) substituents selected from the group consisting of hydrogen, deuterium, halogen, nitro, hydroxyl, cyano, ester group, amino, amido, sulfonamido and carbamido; more preferably, R⁸ is independently selected from the substituted or unsubstituted group consisting of hydrogen, deuterium, C₁-C₆ alkyl, deuterated C₁-C₆ alkyl and halogenated C₁-C₆ alkyl; wherein the substituted means being substituted by cyano.

Preferably, R⁸ is methyl.

Preferably, the compound of formula (I), or stereoisomer, tautomer, crystal form, pharmaceutically acceptable salt, hydrate, solvate or prodrug thereof has the structure shown in formula (VIII): 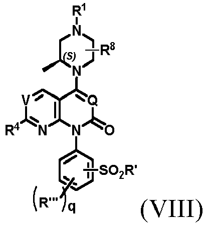 wherein,
R'" is selected from the substituted or unsubstituted group consisting of hydrogen, deuterium, halogen, nitro, hydroxyl, cyano, ester group, amino, amido, C₁-C₆ alkyl, C₃-C₈ cycloalkyl , C₄-C₁₀ cycloalkenyl, 4-8 membered heterocyclyl, C₆-C₁₄ aryl , 5-14 membered heteroaryl, wherein, the substituted means being substituted by one or more substituents selected from the group consisting of deuterium, halogen, nitro, hydroxyl, cyano, ester group, amino, amido, C₁-C₆ alkyl, C₃-C₈ cycloalkyl, C₄-C₁₀ cycloalkenyl, 4-8-membered heterocyclyl, C₆-C₁₄ aryl and 5-14 membered heteroaryl;
q is selected from 1, 2, 3, or 4;
R¹, R⁴, R⁸, R', V and Q are defined as above.

Preferably, the compound of formula (I), or stereoisomer, tautomer, crystal form, pharmaceutically acceptable salt, hydrate, solvate or prodrug thereof has the structure shown in formula (IX): 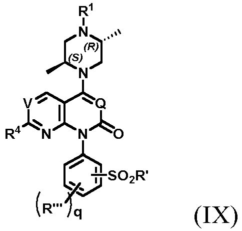 wherein,
R¹, R⁴, R', V, R‴, Q and q are defined as above.

Preferably, in the formulas II-VII, 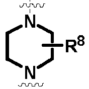 moiety is selected from 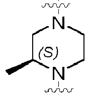 or

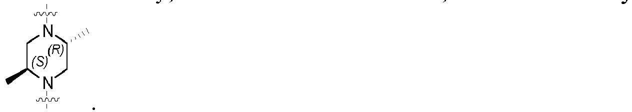

Preferably, in formula I, 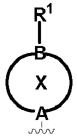 moiety is selected from

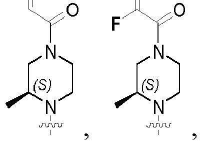

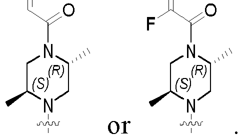

Preferably, in the formula I-II, 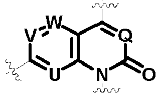 moiety is 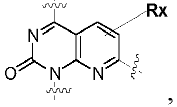 wherein, Rx is selected from hydrogen, deuterium, C₁-C₃ alkyl, deuterated C₁-C₃ alkyl, halogenated C₁-C₃ alkyl, C₃-C₆ cycloalkyl, C₁-C₃ alkoxy, halogen, nitro, hydroxyl, cyano, ester group, 4-6-membered heterocyclyl, preferably, 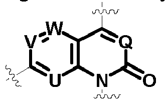 moiety is

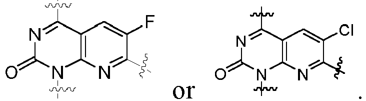

Preferably, in the formulas III-IX, 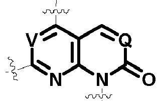 moiety is 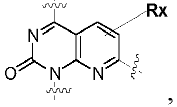 wherein, Rx is selected from hydrogen, deuterium, C₁-C₃ alkyl, deuterated C₁-C₃ alkyl, halogenated C₁-C₃ alkyl, C₃-C₆ cycloalkyl, C₁-C₃ alkoxy, halogen, nitro, hydroxyl, cyano, ester group, 4-6-membered heterocyclyl, preferably, 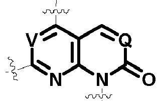 moiety is

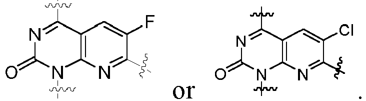

Preferably, in the formulas I-VII, R² is selected from the group consisting of 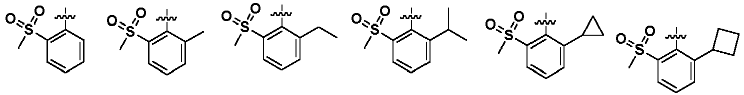 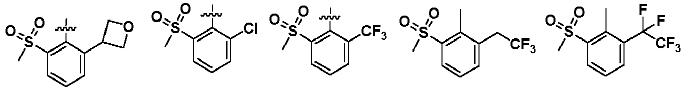 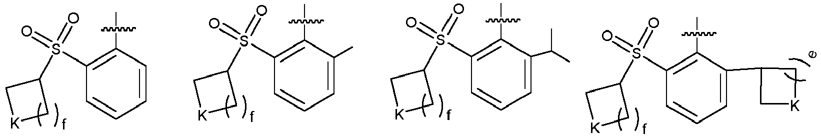 K is independently O, S, CH₂ or NH; e and f are each independently 0, 1 or 2, preferably H in the above-mentioned groups may be optionally substituted by deuterium, halogen, nitro, hydroxyl, cyano, ester group, amino, amido, or C₁-C₃ alkyl.

Preferably, the compound of formula (I), or stereoisomer, tautomer, crystal form, pharmaceutically acceptable salt, hydrate, solvate or prodrug thereof has the structure shown in formula (VIII): 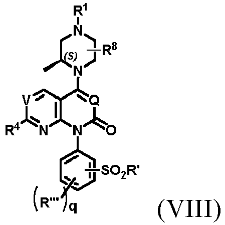 wherein,
R'" is selected from the substituted or unsubstituted group consisting of hydrogen, deuterium, halogen, nitro, hydroxyl, cyano, ester group, amino, amido, C₁-C₆ alkyl, C₃-C₈ cycloalkyl , C₄-C₁₀ cycloalkenyl, 4-8 membered heterocyclyl, C₆-C₁₄ aryl , 5-14 membered heteroaryl, wherein, the substituted means being substituted by one or more substituents selected from the group consisting of deuterium, halogen, nitro, hydroxyl, cyano, ester group, amino, amido, C₁-C₆ alkyl, C₃-C₈ cycloalkyl, C₄-C₁₀ cycloalkenyl, 4-8-membered heterocyclyl, C₆-C₁₄ aryl and 5-14 membered heteroaryl;
R¹, R⁴, R⁸, R', V, Q and q are defined as above.

Preferably, in formula VIII, R'" is selected from the substituted or unsubstituted group consisting of C₁-C₆ alkyl, C₃-C₈ cycloalkyl, C₄-C₁₀ cycloalkenyl, 4-8 membered heterocyclyl, C₆-C₁₀ aryl and 5-10 membered heteroaryl, wherein the substituted means being substituted by one or more substituents selected from the group consisting of deuterium, halogen, nitro, hydroxyl, cyano, ester group, amino, amido, C₁-C₆ alkyl, C₃-C₈ cycloalkyl and 4-8-membered heterocyclyl.

Preferably, in formula VIII, R'" is selected from the substituted or unsubstituted group consisting of C₁-C₆ alkyl, C₃-C₆ cycloalkyl, C₄-C₆ cycloalkenyl and 4-6 membered heterocyclyl, wherein the substituted means being substituted by one or more substituents selected from the group consisting of deuterium, halogen, nitro, hydroxyl, cyano, ester group, amino, amido and C₁-C₃ alkyl.

Preferably, in formulas VIII-IX, 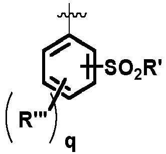 moiety is selected from the group consisting of 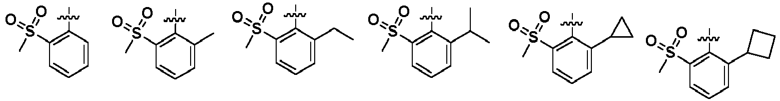 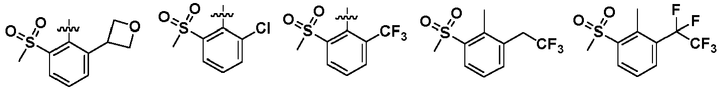 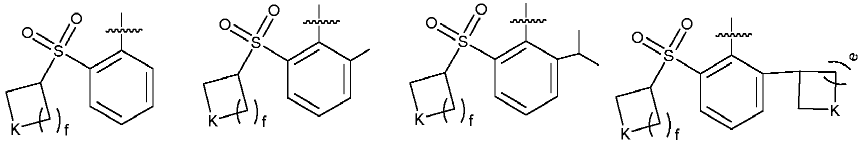 K is independently O, S, CH₂ or NH; e and f are each independently 0, 1 or 2 preferably H in the above-mentioned groups may be optionally substituted by deuterium, halogen, nitro, hydroxyl, cyano, ester group, amino, amido, or C₁-C₃ alkyl.

Preferably, the compound of formula (I), or stereoisomer, tautomer, crystal form, pharmaceutically acceptable salt, hydrate, solvate or prodrug thereof has the structure shown in formula (X) or (XI): 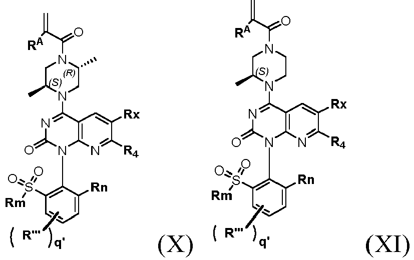 wherein,
R⁴ is selected from substituted or unsubstituted C6-C14 aryl or 5-10 membered heteroaryl, wherein the substituted means being substituted by one or more (such as 2, 3, 4 or 5) substituents selected from the group consisting of deuterium, halogen, ester group, cyano, NR_{b}C(=O)ORₑ, OC (= O)Rₑ, OC (= O)NR_{b}Rₑ, amino, C₁-C₆ alkyl, halogenated C₁-C₆ alkyl, and hydroxyl; R_{b} and Rₑ can be independently hydrogen, deuterium, C1-C6 alkyl, C3-C8 cycloalkyl, 4-8 membered heterocyclyl, 5-14 membered heteroaryl or C6-C14 aryl, or R_{b} and Rₑ together with the N atom can form 4-8 membered heterocyclyl; Rₑ can be independently hydrogen, C1-C6 alkyl, C3-C8 cycloalkyl, C2-C6 alkenyl, C3-C6 cycloalkenyl, C2-C6 alkynyl, 4-8 membered heterocyclyl, 5-14-membered heteroaryl or C6-C14 aryl;
Rm is selected from the substituted or unsubstituted group consisting of amino, C₁-C₆ alkyl, C₃-C₆ cycloalkyl and 4-6 membered heterocyclyl, wherein the substituted means being substituted by one or more substituents selected from the group consisting of deuterium, halogen, nitro, hydroxyl, cyano, ester group, amino, amido, C₁-C₃ alkyl, C3-C6 cycloalkyl and 4-6 membered heterocyclyl;
Rn is selected from the substituted or unsubstituted group consisting of amino, C₁-C₆ alkyl, C₁-C₆ alkoxy, C₃-C₆ cycloalkyl , -O-C₃-C₆ cycloalkyl, C₁-C₆ alkyl C₃-C₆ cycloalkyl, -O-C₁-C₆ alkyl C₃-C₆ cycloalkyl and 4-6 membered heterocyclyl, wherein the substituted means being substituted by one or more substituents selected from the group consisting of deuterium, halogen, nitro, hydroxyl, cyano, ester group, amino, amido, C₁-C₃ alkyl, halogenated C₁-C₃ alkyl, C3-C6 cycloalkyl and 4-6 membered heterocyclyl;
Rx is selected from F or Cl;
R^{A} is selected from H, D, halogen, or cyano, preferably R^{A} is selected from H or F. R‴ is defined as above;
q' is selected from 0, 1, 2, or 3.

Preferably, in the formulas X-XI, R⁴ is selected from the substituted or unsubstituted phenyl or 5-6-membered heteroaryl, wherein the substituted means being substituted by one or more (such as 2, 3, 4 or 5) substituents selected from the group consisting of deuterium, halogen, ester group, cyano, NR_{b}C(=O)ORₑ, OC (= O)Rₑ, OC (= O)NR_{b}Rₑ, amino, C₁-C₃ alkyl, halogenated C₁-C₃ alkyl, and hydroxyl; R_{b} and Rₑ can be independently hydrogen, deuterium, C₁-C₃ alkyl, C3-C6 cycloalkyl, 4-6 membered heterocyclyl, 5-6 membered heteroaryl or phenyl, or R_{b} and Rₑ together with the N atom can form 4-6 membered heterocyclyl; Rₑ can be independently hydrogen, C1-C3 alkyl or C3-C6 cycloalkyl.

Preferably, in the formulas X-XI, Rn is selected from the substituted or unsubstituted group consisting of ethyl, n-propyl, isopropyl, cyclopropyl, cyclobutyl, cyclopentyl, cyclohexyl, cyclopropylamino, azetidinyl, azacyclopentanyl, azacyclohexanyl, oxiranyl, oxetanyl, oxecyclopentanyl, oxacyclohexanyl, wherein the substituted means being substituted by one or more substituents selected from the group consisting of deuterium, halogen, nitro, hydroxyl, cyano, ester group, amino, amido, C₁-C₃ alkyl.

Preferably, in the formulas X-XI, Rm is selected from the substituted or unsubstituted group consisting of methyl, ethyl, n-propyl, isopropyl, cyclopropyl, cyclobutyl, cyclopentyl, cyclohexyl, cyclopropylamino, azetidinyl, azacyclopentanyl, azacyclohexanyl, oxiranyl, oxetanyl, oxecyclopentanyl, oxacyclohexanyl, wherein the substituted means being substituted by one or more substituents selected from the group consisting of deuterium, halogen, nitro, hydroxyl, cyano, ester group, amino, amido, C₁-C₃ alkyl.

The salt of the compound in the present invention may be formed which are also within the scope of the present invention. Unless otherwise stated, the compound in the present invention is understood to include its salt. The term "salt" as used herein refers to a salt formed in the form of acid or base from inorganic or organic acid and base. Further, when the compound in the present invention contains a base fragment which includes, but is not limited to pyridine or imidazole, when contains an acid segment which includes, but is not limited tocarboxylic acid. The zwitter-ion that may form " inner salt " is included within the range of the term "salt". Pharmaceutically acceptable (i.e., non-toxic, physiologically acceptable)salt is preferred, although other salts are also useful and may be used, for example, in the separation or purification steps of the preparation process. The compound of the present invention may form a salt, for example, compound I is reacted with a a certain amount (such as an equivalent amount) of an acid or base, and precipitated in a medium, or freeze-dried in aqueous solution.

The compounds in the present invention containing base fragment which includes but is not limited to amines or pyridine or imidazole rings, may form salt with organic or inorganic acid. Typical acids that form salts include acetate (such as acetate or trihalogenated acetic acid, such as trifluoroacetic acid), adipate, alginate, ascorbate, aspartate, benzoate, benzene sulfonate, disulfate, borate, butyrate, citrate, camphorate, camphor sulfonate, cyclopentane propionate, diethylene glycolate, lauryl sulfate, ethanesulphonate, fumarate, gluceptate, glycerophosphate, hemisulphate, enanthate, caproate, hydrochloride, hydrobromide, hydriodate, isethionate(e.g., 2-hydroxy-ethesulfonate), lactate, maleate, mesylate, naphthalenesulfonate (e.g., 2-naphthalenesulfonate), nicotinate, nitrate, oxalate, pectate, persulfate, phenylpropionate (e.g., 3-phenylpropionate), phosphate, picrate, pivalate, propionate, salicylate, succinate, sulfate (e.g., formed with sulfuric acid), sulfonate, tartrate, thiocyanate, toluenesulfonate(e.g., tosilate), dodecanoate, etc..

Some compounds of the invention may contain acidic fragments including, but not limited to carboxylic acid may form salts with various organic or inorganic bases. Salt formed by typical base includes ammonium salt, alkali metal salt (such as sodium, lithium and potassium salts), alkaline earth metal salt (such as calcium and magnesium salts), and salt formed by organic bases (such as organic amines), such as benzathine, dicyclohexylamine, hydrabamine (salt formed with N,N-bis(dehydroabietyl) ethylenediamine), *N*-methyl-D-glucanamine, N-methyl-D-glucoamide, tert-butyllamine, and the salt formed with amino acids such as arginine, lysine, etc.. Basic nitrogen-containing groups can form quaternary ammonium salts with halides, such as small molecular alkyl halides (such as chlorides, bromides and iodides of methyl, ethyl, propyl and butyl), dialkyl sulfate (such as dimethyl, diethyl, dibutyl, and dipentyl sulfates), long chain halides (such as asuch as chlorides, bromides and iodides of decyl, dodecyl, tetradecyl, and tetradecyl), aralkyl halides (such as bromides of benzyl and phenyl), etc..

The prodrug and solvate of the compound in the present invention are also included within the scope of the present invention. The term "prodrug" herein refers to a compound resulting from the chemical transformation of a metabolic or chemical process to produce a compound, salt, or solvate in the present invention for the treatment of an associated disease. The compounds of the invention include solvates such as hydrates.

Compound, salt or solvate in the present invention, may be present in tautomeric forms such as amide and imino ether. All of these tautomers are part of the present invention.

Stereisomers of all compounds (e.g., those asymmetric carbon atoms that may be present due to various substitutions), including their enantiomeric forms and non-enantiomed forms, all belong to the protection scope of the present invention. The independent stereoisomer in the present invention may not coexist with other isomers (e.g., as a pure or substantially pure optical isomer with special activity), or may be a mixture (e.g.,racemate), or a mixture formed with all other stereoisomers or a part thereof. The chiral center of the present invention has two configurations of S or R, which is defined by International Union of Pure and Applied Chemistry (IUPAC) founded in 1974. The racemization form can be solved by physical methods, such as fractional crystallization, or separation crystallization by derivation into diastereomers, or separation by chiral column chromatography. Individual optical isomer can be obtained from racemate by appropriate methods, including but not limited to conventional methods, such as recrystallization after salting with optically active acids.

Weight content of compound in the present invention obtained by preparation, separation and purification in turn is equal to or greater than 90%, such as equal to or greater than 95%, equal to or greater than 99% ("very pure"compound), and listed in the description of the text. In addition, the "very pure" compound of the present invention is also part of the present invention.

All configuration isomers of the compound of the present invention are within the scope, whether in mixture, pure or very pure form. The definition of the compound of the present invention comprises cis (Z) and trans (E) olefin isomers, and cis and trans isomers of carbocyclic and heterocyclic.

In the entire specification, the groups and substituents can be selected to provide stable fragments and compounds.

Specific functional groups and chemical term definitions are described in detail. For the purposes of the present invention, the chemical elements are consistent with Periodic Table of the Elements, CAS version, Handbook of Chemistry and Physics, 75th Ed.· The definition of a particular functional group is also described. In addition, the basic principles of Organic Chemistry as well as specific functional groups and reactivity described in "Organic Chemistry", Thomas Sorrell, University Science Books, Sausalito: 1999, the entire content of which is incorporated herein by reference.

Some compounds of the present invention may exist in specific geometric or stereoisomer forms. The present invention covers all compounds, including their cis and trans isomers, R and S enantiomers, diastereomers, (D) type isomers, (L) type isomers, racemic mixtures and other mixtures. In addition, asymmetric carbon atom can represent substituent, such as alkyl. All isomers and mixtures thereof are included in the present invention.

According to the invention, mixtures of isomers may contain a variety ratios of isomers. For example, mixtures with only two isomers may hanve the following combinations: 50:50, 60:40, 70:30, 80:20, 90:10, 95:5, 96:4, 97:3, 98:2, 99:1, or 100:0, all ratios of the isomers are within the scope of the present invention. Similar ratio and the ratio of mixtures of more complex isomers, which are readily understood by general skill of the art are also within the scope of the invention.

The present invention also includes the isotope labeled compound, which is equivalent to the original compound herein. However, in fact, the substitution of one or more atoms by an atom with a different atomic weight or mass number usually occurs. Examples of compound isotopes that may be listed in the present invention include hydrogen, carbon, nitrogen, oxygen, phosphorus, sulfur, fluorine, and chlorine isotopes such as ²H, ³H, ¹³C, ¹¹C, ¹⁴C, ¹⁵N, ¹⁸O, ¹⁷O, ³¹P, ³²P, ³⁵S, ¹⁸F and ³⁶Cl, respectively. Compound, or enantiomer, diastereomer, isomer, or pharmaceutically acceptable salt or solvate, the above compound containing isotopes or other isotope atoms are all within the scope of the invention. Some isotope-labeled compounds in the present invention, such as the radioactive isotopes of ³H and ¹⁴C, are also included and are useful in experiments on the tissue distribution of drugs and substrates. Tritium (³H) and Carbon-14 (¹⁴C), which are relatively easy to prepare and detect. Which are the preferred choice. In addition, heavier isotope substitutions such as deuterium, i.e. ²H, have advantages in certain therapies due to their good metabolic stability, such as increased half-life or reduced dosage in vivo, and thus may be preferred in certain situations. Isotope-labeled compounds can be prepared by conventional methods through replacing readily available isotope-labeled reagents with non-isotopic reagents that can be prepared using the disclosed scheme shown in the Example.

If the synthesis of the compound of the invention is to be designed, it can be prepared by asymmetric synthesis, or derivatized with chiral auxiliary reagent, separating the resulting diastereomeric mixture and removing the chiral adjunct to obtain a pure enantiomer. In addition, if a molecule contains a basic functional group, such as an amino acid, or an acidic functional group, such as a carboxyl group, a diastereomer can be formed with a salt of suitable optically active acids or bases, which can be separated by conventional means, such as crystallization or chromatography, to obtain a pure enantiomer.

The metabolite of the compounds of the present application and their pharmaceutically acceptable salts, and prodrugs that can be converted into the compounds of the present application and their pharmaceutically acceptable salts in vivo, also included in the claims.

### Preparation method

The preparation method of the compound of the formula (I) of the present invention is more specifically described below, but these specific methods do not constitute any limitation of the invention. The compound of the invention may also optionally be conveniently prepared by combining the various synthetic methods described in this specification or known in the art, such a combination may be easily performed by a skilled person in the art to which the invention belongs.

Typically, the preparation process for the compounds of the present invention is as follows, in which the raw materials and reagents used may be commercially purchased or synthesized according to the reported literature unless otherwise specified. 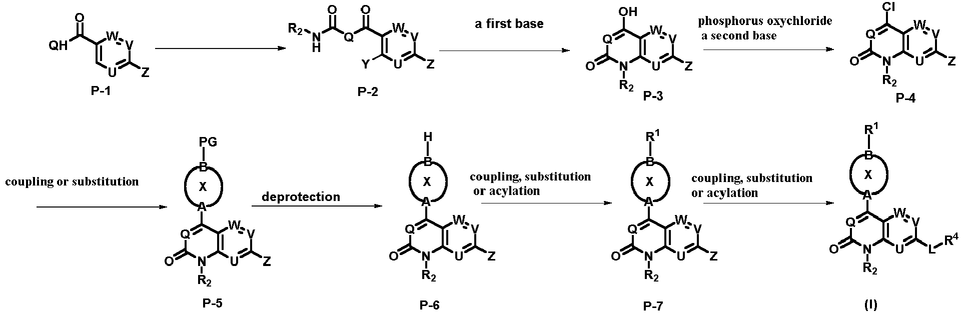
(i) in an inert solvent (such as tetrahydrofuran), the compound of formula P-1 first reacts with oxalyl chloride, and then reacts with the amino compound R₂-NH₂ to obtain the compound of formula P-2;
(ii) in an inert solvent (such as tetrahydrofuran), under the action of a first base, the compound of formula P-2 undergoes a ring closing reaction to obtain the compound of formula P-3;
(iii) in an inert solvent (such as acetonitrile), under the action of a second base, the compound of formula P-3 reacts with phosphorus oxychloride to obtain the compound of formula P-4;
(iv) in an inert solvent (such as acetonitrile), in the presence of a base (such as N,N-diisopropylethylamine), the compound of formula P-4 reacts with 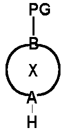 through coupling or substitution reaction to obtain the compound of formula P-5;
(v) in an inert solvent (such as dichloromethane), in the presence of an acid (such as trifluoroacetic acid), the compound of formula P-5 is deprotected to obtain the compound of formula P-6;
(vi) in an inert solvent (such as dichloromethane), in the presence of a base (such as N,N-diisopropylethylamine), the compound of formula P-6 reacts with R₁E through coupling, substitution or acylation reaction to obtain the compound of formula P-7;
(vii) in an inert solvent (such as dioxane/water), in the presence of base (such as potassium acetate) and catalyst (such as [1,1 '-bis (diphenylphosphine) ferrocene] palladium dichloride), the compound of formula P-7 reacts with R₄-L-E₁ through coupling, substitution or acylation reaction to obtain the compound of formula (I);
wherein,
E is halogen, OH, OCOR¹, OCO(ⁱBu), etc;
E₁ is -BH₂, -B(OH)₂, 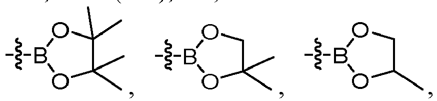 -Sn(Bu)₃, -ZnBr, etc.;
PG is an amino protection group, and the protection group is selected from the group consisting of Boc, Bn, Cbz and Fmoc;
Y and Z are leaving groups, and the leaving groups are selected from the group consisting of halogen and OTf;
the first base is selected from the group consisting of KHMDS, NaHMDS, LiHMDS, NaH, NaOMe, NaOEt, and ^{t}BuONa;
the second base is selected from the group consisting of TEA, DIPEA, DMAP and N,N-dimethylaniline;
R¹, R², R⁴, L, A, B, X, U, V, W, and Q are defined as above.

In the above reaction steps, the reaction solvent, the reaction catalyst, the base used in the reaction, the reaction temperature, the reaction time, etc., can be selected by those skilled in the art according to the specific reactants.

### Pharmaceutical composition and method of administration

The pharmaceutical compositions of the present invention are used to prevent and / or treat the following diseases: inflammation, cancer, cardiovascular disease, infection, immunological disease, metabolic disease.

The compounds of the formula (I) may be used in combination with other drugs known to treat or improve similar conditions. When administered in combination, the original administration for the drug can remain unchanged, while compound of formula (I) may be administered simultaneously or subsequently. Pharmaceutical composition containing one or more known drugs and the compound of formula (I) may be preferred when administered in combination with one or more other drugs. The drug combination also includes administering the compound of formula (I) and other one or more known drugs at overlapping time. When the compound of formula (I) is combined with other one or more drugs, the dose of the compound or known drug may be lower than that of their individual use.

The drug or active ingredients that can be used in pharmaceutical use with the compounds of the formula (I) include but are not limited to PD-1 inhibitor (e. g., nivolumab, pimumab, pidilizumab, cemiplimab, JS-001, SHR-120, BGB-A317, IBI-308, GLS-010, GB-226, STW204, HX008, HLX10, BAT1306, AK105, LZM 009 or the biological analogue thereof, etc.), PD-L1 inhibitor (e. g, dulvalumab, atezumab, avelumab, CS1001, KN035, HLX20, SHR-1316, BGB-A333, JS003, CS1003, KL -A167, F 520, GR1405, MSB2311 or the biological analogue thereof, etc.), CD20 antibody (e. g, rituximab, obinutuzumab, ofatumumab, veltuzumab, tositumomab, 131I-tositumomab, ibritumomab tiuxetan, 90Y-ibritumomab tiuxetan, 90In-ibritumomab tiuxetan, ibritumomab tiuxetan, etc.), CD47 antibody (e. g, Hu5F9-G4, CC-90002, TTI-621, TTI-622, OSE-172, SRF-231, ALX-148, NI-1701, SHR-1603, IBI188, IMM01), ALK inhibitor (e. g, Ceritinib, Alectinib, Brigatinib, Lorlatinib, Ocatinib), PI3K inhibitors (e. g, Idelalisib, Duvelisib, Dactolisib, Taselisib, Bimiralisib, Omipalisib, Buparlisib, etc.), BTK inhibitor (e. g, ibrutinib, Tirabrutinib, Acalabrutinib, Zanubrutinib, Vecabrutinib, etc.), EGFR inhibitor (e. g, Afatinib, Gefitinib, Erlotinib , Lapatinib, Dacomitinib, Icotinib, Canertinib, Sapitinib, Naquotinib, Pyrotinib, Rociletinib, Osimertinib, etc.), VEGFR inhibitor (e. g, Sorafenib, Pazopanib, Regorafenib, Sitravatinib, Ningetinib, Cabozantinib, Sunitinib, Donafenib, etc.), HDAC inhibitor (e. g, Givinostat, Tucidinostat, Vorinostat, Fimepinostat, Droxinostat, Entinostat, Dacinostat, Quisinostat, Tacedinaline, etc.), CDK inhibitor (e. g, Palbociclib, Ribociclib, Abemaciclib, Milciclib, Trilaciclib, Lerociclib, etc.), MEK inhibitor (e. g, Simetinib (AZD6244), Trametinib (GSK1120212), PD0325901, U0126, Pimasertib (AS-703026), PD184352 (CI-1040), etc.), mTOR inhibitor (e. g, Vistusertib, etc.), SHP2 inhibitor (e. g, RMC-4630, JAB-3068, TNO155, etc.), and a combination thereof.

The dosage forms of the pharmaceutical composition of the prensent invention include (but are not limited to) : injection, tablet, capsule, aerosol, suppository, pellicle, pill, liniment for external use, controlled release or sustained-release or nano formulation.

The pharmaceutical composition of the present invention comprises a compound of the present invention or a pharmaceutically acceptable salt and a pharmaceutically acceptable excipient or carrier with safe and effective amount. wherein "safe and effective amount" refers to the amount of compound is sufficient to significantly improve the condition, not to produce severe side effects. Typically, the pharmaceutical composition contains 1-2000 mg of the compound / dosage of the present invention, and preferrably contains 10-1000 mg of the compound / dosage of the present invention. Preferably, "one dosage" is a capsule or a pill.

"Pharmaceutically acceptable carrier" refers to one or more compatible solid or liquid filler or gel substances, which are suitable for human use, and must be sufficiently pure and of sufficiently low toxicity. "Compatible" herein refers to ability of each component of a composition can be mixed with the compound of the present invention and can be mixed with each other without appreciably reducing the efficacy of the compound. Examples of pharmaceutically acceptable carrier include cellulose and derivatives thereof (such as sodium carboxymethylcellulose, sodium ethylcellulose, cellulose acetate, etc.), gelatin, talc, solid lubricant (such as stearic acid, magnesium stearate), calcium sulfate, vegetable oil (such as soybean oil, sesame oil, peanut oil, olive oil, etc.), polyol (such as propylene glycol, glycerol, mannitol, sorbitol, etc.), emulsifier (such as Tween^{®}), wetting agent (such as lauryl sodium sulfate), colorant, flavoring, stabilizer, antioxidant, preservative, pyrogen-free water, etc..

There is no special limitation of administration mode for the compound or pharmaceutical compositions of the present invention, and the representative administration mode includes (but is not limited to): oral, intratumorally, rectal, parenteral (intravenous, intramuscular or subcutaneous), and topical administration.

Solid dosage forms for oral administration include capsules, tablets, pills, powders and granules. In these solid dosage forms, the active compounds are mixed with at least one conventional inert excipient (or carrier), such as sodium citrate or dicalcium phosphate, or mixed with any of the following components: (a) fillers or compatibilizer,such as starch, lactose, sucrose, glucose, mannitol and silicic acid; (b) binders, such as hydroxymethyl cellulose, alginate, gelatin, polyvinylpyrrolidone, sucrose and arabic gum; (c) humectant, such as, glycerol; (d) disintegrating agent, such as agar, calcium carbonate, potato starch or tapioca starch, alginic acid, certain composite silicates, and sodium carbonate; (e) dissolution-retarding agents, such as paraffin; (f) absorption accelerators, such as quaternary ammonium compounds; (g) wetting agents, such as cetyl alcohol and glyceryl monostearate; (h) adsorbents, for example, kaolin; and (i) lubricants such as talc, stearin calcium, magnesium stearate, solid polyethylene glycol, lauryl sodium sulfate, or the mixtures thereof. In capsules, tablets and pills, the dosage forms may also contain buffering agents.

The solid dosage forms such as tablets, sugar pills, capsules, pills and granules can be prepared by using coating and shell materials, such as enteric coatings and any other materials known in the art. They can contain an opaque agent. The release of the active compounds or compounds in the compositions can be released in a delayed mode in a given portion of the digestive tract. Examples of the embedding components include polymers and waxes. If necessary, the active compounds and one or more above excipients can form microcapsules.

Liquid dosage forms for oral administration include pharmaceutically acceptable emulsions, solutions, suspensions, syrups or tinctures. In addition to the active compounds, the liquid dosage forms may contain any conventional inert diluents known in the art such as water or other solvents, solubilizers and emulsifiers, such as ethanol, isopropanol, ethyl carbonate, ethyl acetate, propylene glycol, 1,3-butanediol, dimethyl formamide, as well as oil, in particular, cottonseed oil, peanut oil, corn germ oil, olive oil, castor oil and sesame oil, or the combination thereof.

Besides these inert diluents, the composition may also contain additives such as wetting agents, emulsifiers, and suspending agent, sweetener, flavoring agents and perfume.

In addition to the active compounds, the suspension may contain suspending agent, for example, ethoxylated isooctadecanol, polyoxyethylene sorbitol and sorbitan esters, microcrystalline cellulose, methanol aluminum and agar, or the combination thereof.

The compositions for parenteral injection may comprise physiologically acceptable sterile aqueous or anhydrous solutions, dispersions, suspensions or emulsions, and sterile powders which can be re-dissolved into sterile injectable solutions or dispersions. Suitable aqueous and non-aqueous carriers, diluents, solvents or excipients include water, ethanol, polyols and any suitable mixtures thereof.

The dosage forms for topical administration of compounds of the invention include ointments, powders, patches, aerosol, and inhalants. The active ingredients are mixed with physiologically acceptable carriers and any preservatives, buffers, or propellant if necessary, under sterile conditions.

Compounds of the present invention can be administrated alone, or in combination with any other pharmaceutically acceptable compounds.

When the pharmaceutical compositions are used, a safe and effective amount of compound of the present invention is administrated to a mammal (such as human) in need thereof, wherein the dose of administration is a pharmaceutically effective dose. For a person weighed 60 kg, the daily dose is usually 1-2000 mg, preferably 50-1000mg. Of course, the particular dose should also depend on various factors, such as the route of administration, patient healthy status, which are well within the skills of an experienced physician..

The present invention also provides a preparation method of pharmaceutical composition comprising the step of mixing a pharmaceutically acceptable carrier with the compound of formula (I) or crystal form, pharmacically acceptable salt, hydrate or solvate thereof of the present invention.

The invention also provides a treatment method comprising the step of administering the compound of formula (I), or its crystalline form, pharmaceutically acceptable salt, hydrate or solvate thereof, or the pharmaceutical composition of the invention to a subject in need thereof to selectively inhibit KRAS^{G12C}.

### Compared with the prior art, the present invention has the following main advantages:

(1) The compound has a good selective inhibition on KRAS^{G12C};
(2) The compound has better pharmacodynamics, pharmacokinetic properties and lower toxic and side effects.

The present invention will be further illustrated below with reference to the specific examples. It should be understood that these examples are only to illustrate the invention but not to limit the scope of the invention. The experimental methods without specific conditions in the following examples usually follow conventional conditions, or according to the conditions recommended by the manufacturer. Unless otherwise stated, percentage and parts are calculated by weight.

All major and scientific terms used herein are the same as those familiar to those skilled in the art, unless otherwise defined. In addition, any method or material which is similar or equal to the recorded content may be applied to the method of the present invention. The preferred methods and materials described herein are described only for demonstration purposes.

The compound structure of the present invention was determined by nuclear magnetic resonance (NMR) and Liquid-mass chromatography (LC-MS).

NMR was detected by the Bruker AVANCE-400 NMR instrument, and the solvent included DMSO-d₆, CD₃COCD₃, CDCl₃ and CD₃OD, etc. The internal standard was tetramethylsilane (TMS), and the chemical shift was measured in percent per million (ppm).

LC-MS was detected by using Waters SQD2 mass spectrometry. HPLC was detected by using Agilent 1100 high voltage chromatograph (Microsorb 5 micron C18 100 × 3.0mm column).

Qingdao GF254 silica gel plate was used for thin layer chromatography, 0.15-0.20mm was used for TLC, and 0.4mm-0.5mm was used for preparative thin layer chromatography. Generally, Qingdao silica gel 200-300 mesh silica gel was used as carrier in column chromatography.

The starting materials in the Example of the present invention are known and commercially available, or may be synthesized by or in accordance with the literature reported in the art.

Unless otherwise specified, all reactions in the present invention are carried out by continuous magnetic stirring under the protection of dry inert gas (such as nitrogen or argon), and the reaction temperature is Celsius.

### EXAMPLE

### Example 1 Preparation of 4-((S)-4-acroloyl-2-methylpiperazin-1-yl) -6-fluoro-7-(2-fluoro-6-hydroxyphenyl)-1-(4-methyl-2-(methylsulfonyl) pyridin-3-yl) pyridino [2,3-d] pyrimidin-2 (1H)-one

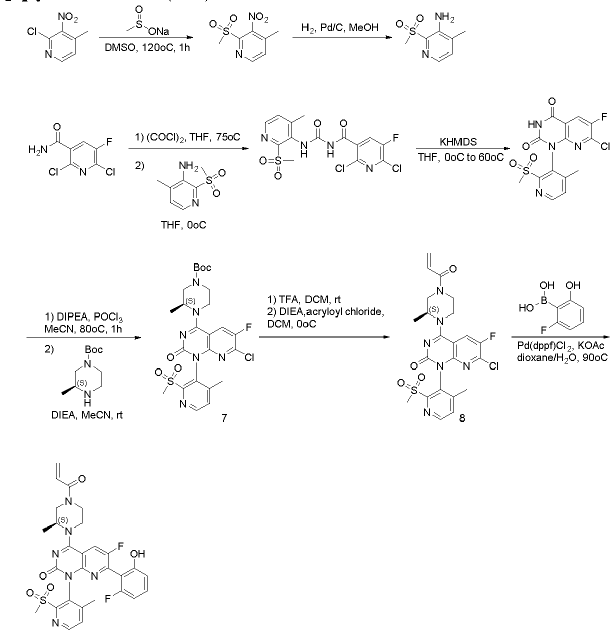

### Step 1. Preparation of 4-methyl -2-(methylsulfonyl)-3-nitropyridine

2-Chloro-4-methyl-3-nitropyridine (3g, 17.4 mmol) was dissolved in 25 mL of dimethyl sulfoxide, and sodium methylsulfinate (2.7g, 26.2 mmol) was added to the solution. The mixture was stirred at 120 °C for 1 hour, quenched with 100 mL of water, and then extracted with 100 mL of ethyl acetate three times. The combined organic phase was washed 3 times with 50 mL brine, then the organic phase was dried and concentrated. The residual solid was slurried with ethanol/methanol/ethyl acetate (40 mlL/5 mL/5 mL), filtered and dried to obtain the target compound (2.3g, 61%).

### Step 2. Preparation of 4-methyl-2-(methylsulfonyl) pyridin-3-amine

4-Methyl -2-(methylsulfonyl)-3-nitropyridine (2.2g, 10.2 mmol) was dissolved in 110 mL of methanol, and 10% palladium carbon (50% w/w, 660 mg) was added to the solution. The mixture was stirred at room temperature under a hydrogen atmosphere for 5 hours. The catalyst was removed by suction filtration, the filtrate was concentrated, and separated by silica gel column chromatography (petroleum ether: ethyl acetate = 5:1 to 2:1) to obtain the target compound (820 mg, yield: 41%).

LC-MS:m/z 187 (M + H)⁺.

### Step 3. Preparation of 2, 6-dichloro-5-fluoro-N-(((4-methyl-2-(methylsulfonyl) pyridin-3-yl) carbamoyl) nicotinamide

2, 6-Dichloro-5-fluoronicotinamide (873 mg, 4.2 mmol) was dissolved in 15 mL of anhydrous tetrahydrofuran, and a solution of oxalyl chloride (3.6 mL, 42.0 mmol) in dichloromethane (4.5 mL) was slowly added to the solution. After the addition was completed, the mixture was refluxed and stirred at 75 °C for 2 hours, and then concentrated to dryness under reduced pressure. The residue was diluted with 15 mL of anhydrous tetrahydrofuran and cooled to 0 °C. 4-Methyl-2-(methylsulfonyl) pyridin-3-amine (820 mg, 4.4 mmol) was dissolved in 6 mL of anhydrous tetrahydrofuran, and then added dropwise into the above solution. The reaction solution was stirred at 0 °C for 2 hours, quenched with saturated ammonium chloride/saturated salt water (V:V = 1:1,30 mL), and then extracted with dichloromethane/methanol (V:V = 10:1,30 mL) for 3 times. The combined organic phase was dried and concentrated. The residual solid was slurried with petroleum ether/ethyl acetate (2:1, 25 mL), filtered and dried to obtain the target compound (1.36g, yield: 77%).

LC-MS:m/z 421(M + H)⁺.

### Step 4. Preparation of 7-chloro-6-fluoro-1-(4-methyl-2-(methylsulfonyl) pyridin-3-yl) pyridino[2,3-d] pyrimidin -2,4(1H,3H)-dione

2, 6-dichloro-5-fluoro-N-(((4-methyl -2-(methylsulfonyl) pyridin-3-yl) carbamoyl) nicotinamide (1.13g, 2.7 mmol) was suspended in 34 mL of tetrahydrofuran, and potassium bis(trimethylsilyl)amino (1 M tetrahydrofuran solution, 6.2 mL, 6.2 mmol) was added dropwise under an ice bath. After dropping, the reaction liquid became clear. The reaction solution was stirred at 60 °C for 2 hours, quenched with 40 mL of saturated ammonium chloride, and then extracted with 40 mL of ethyl acetate for 3 times. The combined ethyl acetate layer was dried and concentrated. The residual solid was slurried with petroleum ether/ethyl acetate (V:V=1:1, 25 mL), filtered and dried to obtain the target compound (870 mg, yield: 84%).

LC-MS:m/z 385(M + H)⁺.

### Step 5. Preparation of (S)-tert-butyl 4-(7-chloro-6-fluoro-1-(4-methyl-2-(methylsulfonyl)pyridin-3-yl)-2-oxo-1, 2-dihydropyridino [2,3-d] pyrimidin-4-yl)-3-methylpiperazin-1-carboxylate

7-Chloro-6-fluoro-1-(4-methyl-2-(methylsulfonyl) pyridin-3-yl) pyridino[2,3-d]pyrimidin-2,4(1H,3H)-dione (300 mg, 0.8 mmol) was suspended in 9 mL acetonitrile, and N,N-diisopropylethylamine (0.77 mL, 4.7 mmol) and phosphorus oxychloride (0.36 mL,3.9 mmol) were added. The reaction solution became clear. The reaction solution was stirred at 80 °C for 1 hour and concentrated to dryness under reduced pressure. The residue was dissolved in 12 mL of acetonitrile, cooled to 0°C, N,N-diisopropylethylamine (0.4 mL, 2.3 mmol) and tert-butyl (S)-3-methylpiperazin-1-carboxylate(188 mg,0.9 mmol) were added. The reaction solution was stirred at room temperature for 1 hour, quenched with saturated sodium bicarbonate solution (20 mL), and then extracted with 20 mL ethyl acetate for 3 times. The combined ethyl acetate layer was dried, concentrated and separated by silica gel column chromatography (petroleum ether: ethyl acetate = 3:1 to pure ethyl acetate) to obtain the target compound (328 mg, yield: 74%).

LC-MS:m/z 567 (M + H)⁺.

### Step 6. Preparation of (S)-4-(4-acroloyl-2-methylpiperazin -1-yl)-7-chloro-6-fluoro-1-(4-methyl-2-(methylsulfonyl) pyridin-3-yl) pyridino[2,3-d]pyrimidin-2(1H)-one

(S)-tert-butyl 4-(7-chloro-6-fluoro-1-(4-methyl-2-(methylsulfonyl) pyridin-3-yl) -2-oxo-1, 2-dihydropyridino [2,3-d]pyrimidin-4-yl)-3-methylpiperazin-1-carboxylate (328 mg, 0.6 mmol) was dissolved in 4 mL of dichloromethane, 1 mL of trifluoroacetic acid was added. The reaction solution was stirred at room temperature for 2 hours, and concentrated to dryness. The residue was steamed with 15 mL of dichloromethane for 3 times to obtain the crude product. The crude product was dissolved in 6 mL of dichloromethane, cooled to 0°C, and a solution of N,N-diisopropylethylamine (0.38 mL,2.3 mmol) and acryloyl chloride (63 mg, 0.7 mmol) in dichloromethane (1 mL) was added dropwise. The reaction solution was stirred at 0°C for 30 minutes, quenched with 20 mL of saturated sodium bicarbonate, and extracted with 20 mL of dichloromethane for 3 times. The dichloromethane layers were combined, dried, concentrated, and the residue was separated by silica gel column chromatography (dichloromethane: methanol = 60:1) to obtain the target compound (200 mg, yield: 67%).

LC-MS:m/z 521 (M + H)⁺.

### Step 7. Preparation of 4-((S)-4-acroloyl-2-methylpiperazin-1-yl) -6-fluoro-7-(2-fluoro-6-hydroxyphenyl)-1-(4-methyl-2-(methylsulfonyl)pyridin-3-yl)p yridino [2,3-d] pyrimidin-2 (1H)-one

### (S)-4-(4-acroloyl-2-methylpiperazin-1-yl)

-7-chloro-6-fluoro-1-(4-methyl-2-(methylsulfonyl) pyridin-3-yl) pyridino [2,3-d] pyrimidin-2(1H)-one (126 mg, 0.24 mmol), (2-fluoro-6-hydroxyphenyl) boric acid (49 mg,0.31 mmol), [1,1 '-bis (diphenylphosphine) ferrocene] palladium dichloride dichloromethane complex (20 mg, 0.024 mmol) and potassium acetate (95 mg, 0.97 mmol) were suspended in a mixed solvent of dioxane/water (7 mL/0.7 mL), and replaced with nitrogen for three times, heated and stirred at 90 °C for 2.5 hours. After the reaction solution was cooled to room temperature, 20 mL of semi-saturated sodium bicarbonate solution was added, and extracted with 20 mL of ethyl acetate for 3 times. The ethyl acetate layers were combined, dried, concentrated, and the residue was separated by silica gel column chromatography (dichloromethane: methanol = 100:1 to 60:1) to obtain the target compound (45 mg, yield: 31%).

LC-MS:m/z 597 (M + H)⁺. ¹H NMR (400 MHz, CDCl₃) δ 8.65-8.63 (m, 1H), 8.57 (brs, 1H), 7.89 (t, *J* = 8.8 Hz, 1H), 7.60 (d, *J* = 4.4 Hz, 1H), 7.26 (m, 1H), 6.71-6.56 (m, 3H), 6.40 (d, *J=* 16.4 Hz, 1H), 5.81 (d, *J=* 10.4 Hz, 1H), 5.10-4.50 (m, 3H), 4.10-3.50 (m, 3H), 3.27 (d, *J=* 2.4 Hz, 3H), 3.26-3.00 (m, 1H), 2.28 (d, *J=* 6.8 Hz, 3H), 1.53 (m, 3H).

### The following compounds were synthesized from different starting materials

### according to the method of Example 1:

### Example 2 4-((S)-4-acroloyl-2-methylpiperazin-1-yl) -6-fluoro-7-(2-fluoro-6-hydroxyphenyl)-1-(2-methyl-4-(methylsulfonyl) pyridin-3-yl) pyridino [2,3-d] pyrimidin-2 (1H)-one

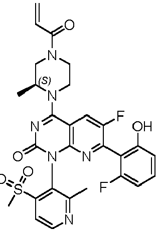

LC-MS:m/z 597 (M + H)⁺. ¹H NMR (400 MHz, CDCl₃) δ 8.93 (d, *J* = 5.2 Hz, 1H), 8.65 (brs, 1H), 7.95-7.89 (m, 2H), 7.26 (m, 1H), 6.72-6.75 (m, 3H), 6.45-6.39 (m, 1H), 5.85-5.81 (m, 1H), 5.25-4.30 (m, 3H), 4.20-3.50 (m, 3H), 3.30-3.00 (m, 4H), 2.46 (d, *J* = 10.0 Hz, 3H), 1.51-1.48 (m, 3H).

### Example 3 2-((2S)-4-acryloyl -1-(6-fluoro-7-(2-fluoro-6-hydroxyphenyl)-1-(2-methyl-6-(methylsulfonyl)phenyl) -2-oxo-1,2-dihydropyridino[2,3-d] pyrimidin-4-yl) piperazin-2-yl) acetonitrile

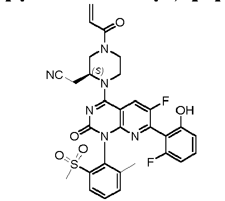

LC-MS:m/z 621 (M + H)⁺. ¹H NMR (400 MHz, CDCl₃) 8 9.01-8.91 (m, 1H), 8.11 (t, *J=* 7.2 Hz, 1H), 7.95 (brs, 1H), 7.75 (t, *J=* 8.4 Hz, 1H), 7.70-7.64 (m, 1H), 7.26 (m, 1H), 6.71-6.59 (m, 3H), 6.46 (d, *J=* 16.4 Hz, 1H), 5.88 (d, *J=* 10.4 Hz, 1H), 5.50-5.30 (m, 1H), 4.75-3.40 (m, 6H), 3.10 (d, *J=* 4.8 Hz, 3H), 3.05-2.80 (m, 2H), 2.18 (d, *J=* 10.8 Hz, 3H).

### Example 4 4-((S)-4-acryloyl-2-methylpiperazin-1-yl) -6-fluoro-7-(2-fluoro-6-hydroxyphenyl)-1-(2-(isopropylsulfonyl)-6-methylphenyl) pyridino[2,3-d] pyrimidin -2(1H)-one

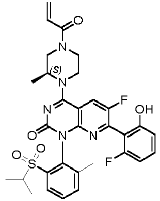

LC-MS:m/z 624 (M + H)⁺. ¹H NMR (400 MHz, CDCl₃) δ 8.98-8.94 (m, 1H), 8.04 (d, *J* = 8.0 Hz, 1H), 7.87 (t, *J* = 8.0 Hz, 1H), 7.72 (d, *J* = 7.6 Hz, 1H), 7.62 (t, *J* = 8.0 Hz, 1H), 7.26 (m, 1H), 6.71-6.54 (m, 3H), 6.44-6.39 (m, 1H), 5.83-5.80 (m, 1H), 5.10-4.30 (m, 3H), 4.10-3.00 (m, 5H), 2.16 (d, *J=* 8.4 Hz, 3H), 1.52 (brs, 3H), 1.31-1.28 (m, 3H), 1.16-1.14 (m, 3H).

### Example 5 4-((S)-4-acrylo-2-methylpiperazin-1-yl)-1-(2-ethyl-6-(methylsulfonyl) phenyl) -6-fluoro-7-(2-fluoro-6-hydroxyphenyl) pyridino[2,3-d] pyrimidin -2(1H)-one

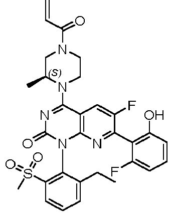

LC-MS:m/z 610 (M + H)⁺. ¹H NMR (400 MHz, CDCl₃) δ 8.99 (s, 1H), 8.12 (d, *J* = 7.6 Hz, 1H), 7.87 (dd, *J* = 9.2 Hz, 3.2 Hz, 1H), 7.78 (d, *J* = 7.6 Hz, 1H), 7.71 (t, *J* = 7.6 Hz, 1H), 7.26 (m, 1H), 6.69-6.54 (m, 3H), 6.44-6.38 (m, 1H), 5.83-5.80 (m, 1H), 5.10-4.38 (m, 3H), 4.10-3.50 (m, 3H), 3.30-3.00 (m, 4H), 2.59-2.41 (m, 2H), 1.58-1.49 (m, 3H), 1.21-1.16 (m, 3H).

### Example 5-1 two isomers of Example 5A and Example 5B were obtained by chiral separation:

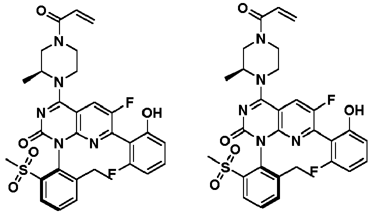

**Example 5A**:LC-MS:m/z 610(M+H)⁺. ¹H NMR (400 MHz, CDCl₃) δ 9.02 (s, 1H), 8.14 (d, *J* = 7.3 Hz, 1H), 7.90 (d, *J* = 8.5 Hz, 1H), 7.81 (d, *J* = 7.1 Hz, 1H), 7.73 (t, *J* = 7.8 Hz, 1H), 7.33 - 7.23 (m, 1H), 6.76 - 6.53 (m, 3H), 6.44 (d, *J* = 16.3 Hz, 1H), 5.84 (d, *J* = 10.0 Hz, 1H), 5.03 (m, 1H), 4.57 (m, 2H), 4.22 - 3.41 (m, 3H), 3.35 - 3.00 (m, 4H), 2.48 (m, 2H), 1.51 (s, 3H), 1.21 (t, *J* = 6.7 Hz, 3H).

**Example 5B:**LC-MS:m/z 610(M+H)⁺. ¹H NMR (400 MHz, CDCl₃) δ 9.01 (s, 1H), 8.21 - 8.07 (m, 1H), 7.90 (d, *J* = 9.3 Hz, 1H), 7.81 (d, *J* = 7.0 Hz, 1H), 7.73 (t, *J* = 7.8 Hz, 1H), 7.35 - 7.23 (m, 1H), 6.68 (m, 3H), 6.44 (d, *J* = 16.5 Hz, 1H), 5.84 (d, *J* = 10.2 Hz, 1H), 5.12 - 4.29 (m, 3H), 3.99 (m, 1H), 3.69 (m, 2H), 3.22 (m, 4H), 2.69 - 2.37 (m, 2H), 1.66 - 1.43 (m, 3H), 1.25 - 1.12 (m, 3H).

### Example 6 4-((S)-4-acryloyl-2-methylpiperazin-1-yl)-1-(2-cyclopropyl-6-(methylsulfonyl)phenyl) -6-fluoro-7-(2-fluoro-6-hydroxyphenyl) pyridino [2,3-d] pyrimidin-2(1H)-one

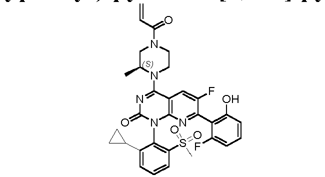

LC-MS:m/z 622 (M+H)⁺. ¹H NMR (400 MHz, CDCl₃) δ 9.00 (s, 1H), 8.08 (d, *J* = 7.6 Hz, 1H), 7.88 (d, *J* = 9.2 Hz, 1H), 7.67-7.63 (m, 1H), 7.46 (d, *J* = 7.6 Hz, 1H), 7.29-7.23 (m, 1H), 6.75-6.50 (m, 3H), 6.44-6.39 (m, 1H), 5.83-5.80 (m, 1H), 5.04-4.38 (m, 3H), 4.06-3.55 (m, 3H), 3.24-3.02 (m, 4H), 1.55-1.49 (m, 4H), 0.85-0.56 (m, 4H).

### Example 6-1 two isomers of Example 6A and Example 6B were obtained by chiral searation:

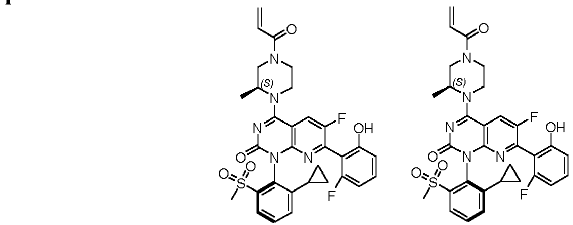

**Example 6A:**LC-MS:m/z 622(M+H)⁺. ¹H NMR (400 MHz, CDCl₃) δ 9.03 (s, 1H), 8.10 (d, *J=* 7.2 Hz, 1H), 7.90 (d, *J=* 8.9 Hz, 1H), 7.67 (t, *J=* 7.9 Hz, 1H), 7.48 (dd, *J=* 7.9, 1.0 Hz, 1H), 7.40 - 7.17 (m, 1H), 6.81 - 6.54 (m, 3H), 6.43 (dd, *J=* 16.7, 1.3 Hz, 1H), 5.84 (d, *J=* 10.3 Hz, 1H), 5.08 (m, 1H), 4.59 (m, 2H), 4.25 - 3.44 (m, 3H), 3.31 - 2.99 (m, 4H), 1.70 (m, 1H), 1.51 (s, 3H), 0.96 - 0.78 (m, 2H), 0.65 (m, 2H).

**Example 6B:**LC-MS:m/z 622(M+H)⁺. ¹H NMR (400 MHz, CDCl₃) δ 9.02 (s, 1H), 8.10 (d, *J=* 7.5 Hz, 1H), 7.91 (d, *J=* 9.2 Hz, 1H), 7.67 (t, *J=* 7.9 Hz, 1H), 7.47 (d, *J=* 7.7 Hz, 1H), 7.36 - 7.19 (m, 1H), 6.68 (m, 3H), 6.43 (d, *J=* 16.6 Hz, 1H), 5.84 (d, *J=* 10.3 Hz, 1H), 4.69 (m, 3H), 3.98 (m, 1H), 3.70 (m, 2H), 3.35 - 3.02 (m, 4H), 1.63 (m, 4H), 0.87 (m, 2H), 0.67 (m, 2H).

### Example 7 4-((S)-4-acryloyl-2-methylpiperazin-1-yl)-1-(2-(cyclopropylsulfonyl)-6-methylphenyl) -6-fluoro-7-(2-fluoro-6-hydroxyphenyl) pyridino [2,3-d] pyrimidin-2(1H)-one

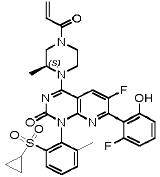

LC-MS:m/z 622 (M+H)⁺. ¹H NMR (400 MHz, CDCl₃) δ 9.00 (s, 1H), 7.97-7.85 (m, 2H), 7.71 (d, *J* = 7.6 Hz, 1H), 7.61 (t, *J* = 8.0 Hz, 1H), 7.29-7.23 (m, 1H), 6.69-6.63 (m, 3H), 6.44-6.39 (m, 1H), 5.83-5.80 (m, 1H), 5.05-4.37 (m, 3H), 4.02-3.63 (m, 3H), 3.23-2.78 (m, 2H), 2.19 (d, *J=* 10.8 Hz, 3H), 1.41-1.37 (m, 4H). 1.03-0.96 (m, 3H).

### Example 8 4-((S)-4-acryloyl-2-methylpiperazin-1-yl)-1-(2-chloro-6-(methylsulfonyl)phenyl) -6-fluoro-7-(2-fluoro-6-hydroxyphenyl) pyridino [2,3-d] pyrimidin-2 (1H)-one

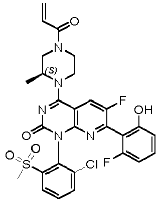

LC-MS:m/z 616 (M+H)⁺. ¹H NMR (400 MHz, CDCl₃) δ 8.81(s, 1H), 8.19 (d, *J* = 8.0 Hz, 1H), 7.95-7.85 (m, 2H), 7.70 (t, *J* = 8.4 Hz, 1H), 7.33-7.23 (m, 1H), 6.74-6.50 (m, 3H), 6.10 (d, *J* = 16.8 Hz, 1H), 6.10 (dd, *J* = 10.4 Hz, 8 Hz; 1H), 5.15-4.30 (m, 3H), 4.15-3.39 (m, 3H), 3.31-2.95 (m, 4H), 1.51 (s, 3H).

### Example 9 4-((S)-4-acryloyl-2-methylpiperazin-1-yl)-7-(2-(difluoromethyl)-6-fluorophenyl) -6-fluoro-1-(2-methyl-6-(methylsulfonyl)phenyl) pyridino [2,3-d] pyrimidin-2 (1H)-one

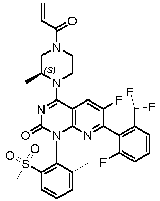

LC-MS:m/z 630 (M+H)⁺. ¹H NMR (400 MHz, CDCl₃) δ 8.01 (d, *J* = 7.6 Hz, 1H), 7.87-7.83 (m, 1H), 7.64-7.62 (m, 1H), 7.56-7.46 (m, 3H), 7.26-7.22 (m, 1H), 6.69-6.30 (m, 3H), 5.82 (dd, *J* = 10.4 Hz, 1.6 Hz, 1H), 5.04-4.36 (m, 3H), 4.05-3.62 (m, 3H), 3.27-3.08 (m, 4H), 2.16-2.13 (m, 3H), 1.60-1.49 (m, 3H).

### Example 10 4-((S)-4-acryloyl-2-methylpiperazin-1-yl) -6-fluoro-7-(2-fluoro-6-hydroxyphenyl)-1-(2-isopropyl-6-(methylsulfonyl) phenyl) pyridino[2,3-d] pyrimidin-2(1H)-one

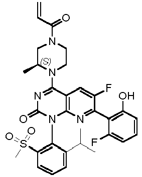

LC-MS:m/z 624 (M+H)⁺. ¹H NMR (400 MHz, CDCl₃) δ 8.98 (s, 1H), 8.12 (dd, *J* = 8.0 Hz, 1.6 Hz, 1H), 7.89-7.82 (m, 2H), 7.74 (t, *J* = 8.0 Hz, 1H), 7.26 (m, 1H), 6.69-6.62 (m, 3H), 6.42 (dd, *J* = 16.8 Hz, 1.6 Hz, 1H), 5.82 (dd, *J* = 10.4 Hz, 1.6 Hz, 1H), 5.15-4.65 (m, 3H), 4.15-3.50 (m, 3H), 3.30-3.00 (m, 4H), 2.85-2.70 (m, 1H), 1.56-1.48 (m, 3H), 1.25 (dd, *J* = 6.8 Hz, 2.4 Hz, 3H), 1.05 (t, *J* = 6.4 Hz, 3H).

### Example 10-1 two isomers of Example 10A and Example 10B were obtained by chiral separation:

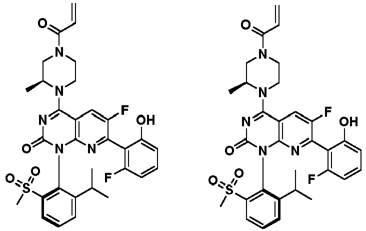

**Example 10A:**LC-MS:m/z 624 (M+H)⁺. ¹H NMR (400 MHz, DMSO) δ 10.23 (s, 1H), 8.31 (t, *J* = 10.5 Hz, 1H), 7.95 - 7.75 (m, 2H), 7.66 (t, *J* = 7.9 Hz, 1H), 7.26 (m, 1H), 6.98 - 6.78 (m, 1H), 6.77 - 6.59 (m, 2H), 6.22 (d, *J* = 16.6 Hz, 1H), 5.78 (dd, *J* = 10.5, 2.1 Hz, 1H), 4.92 (s, 1H), 4.37 (m, 2H), 4.10 (m, 1H), 3.88 - 3.46 (m, 2H), 3.18 (m, 1H), 2.65 (d, *J* = 6.0 Hz, 1H), 1.34 (d, *J* = 6.6 Hz, 3H), 1.10 (d, *J* = 6.8 Hz, 3H), 1.00 (d, *J* = 6.8 Hz, 3H).

**Example 10B:**LC-MS:m/z 624 (M+H)⁺. ¹H NMR (400 MHz, DMSO) δ 10.28 (s, 1H), 8.33 (t, *J* = 9.2 Hz, 1H), 7.98 - 7.77 (m, 2H), 7.66 (t, *J* = 7.8 Hz, 1H), 7.26 m, 1H), 6.88 (m, 1H), 6.79 - 6.52 (m, 2H), 6.22 (dd, *J* = 16.6, 6.0 Hz, 1H), 5.78 (dd, *J* = 10.4, 2.2 Hz, 1H), 4.89 (s, 1H), 4.55 - 3.91 (m, 3H), 3.65 (m, 1H), 3.47 (m, 1H), 3.08 (m, 1H), 2.67 (m, 1H), 1.30 (s, 3H), 1.11 (d, *J* = 6.8 Hz, 3H), 1.02 (m, 3H).

### Example 11 N-(2-(4-((S))-4-acryloyl-2-methylpiperazin-1-yl) -6-fluoro-7-(2-fluoro-6-hydroxyphenyl)-2-oxopyrano [2, 3-d] pyrimidin-1(2H)-yl)-3-methylphenyl)-N-methylsulfonamide

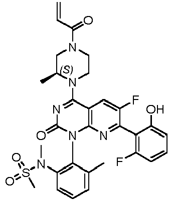

LC-MS:m/z 625 (M+H)⁺. ¹H NMR (400 MHz, CDCl₃) δ 8.64 (brs, 1H), 7.86-7.78 (m, 1H), 7.47 (td, *J* = 7.6 Hz, 1.6 Hz; 1H), 7.40 (d, *J* = 7.2 Hz, 1H), 7.32-7.21 (m, 2H), 6.70-6.50 (m, 3H), 6.40 (dd, *J* = 16.8 Hz, 1.6 Hz; 1H), 6.10 (dd, *J* = 10.4 Hz, 1.6 Hz; 1H), 5.05-4.25 (m, 3H), 4.10-3.46 (m, 3H), 3.31-2.99 (m, 4H), 2.85 (d, *J* = 2.0 Hz, 3H), 2.17 (s, 3H), 1.54-1.42 (m, 3H).

### Example 12 4-((2S,5R)-4-acryloyl-2, 5-dimethylpiperazin-1-yl)-1-(2-ethyl-6-(methylsulfonyl)phenyl)-6-fluoro-7-(2-fluoro-6 -hydroxyphenyl) pyridino [2,3-d] pyrimidin-2 (1H)-one

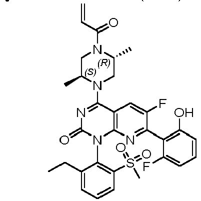

LC-MS:m/z 624 (M+H)⁺. ¹H NMR (400 MHz, CDCl₃) δ 9.03 (t, *J* = 18.4 Hz, 1H), 8.14-8.10 (m, 1H), 7.89-7.68 (m, 3H), 7.28-7.23 (m, 1H), 6.71-6.36 (m, 4H),, 5.83-5.79 (m, 1H), 5.12-3.47 (m, 6H), 3.14-3.10 (m, 3H), 2.55-2.37 (m, 2H), 1.51-1.16(m, 9H).

### Example 13 (S)-4-(4-acryloyl-2-methylpiperazin-1-yl)-1-(2-cyclopropyl-6-(methylsulfonyl) phenyl) -6-fluoro-7-(2-fluorophenyl) pyridino [2,3-d] pyrimidin-2(1H)-one

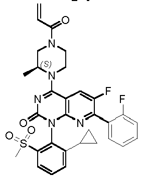

LC-MS:m/z 606 (M+H)⁺. ¹H NMR (400 MHz, CDCl₃) δ 7.99 (d, *J* = 7.6 Hz, 1H), 7.80-7.77 (m, 1H), 7.55 (t, *J* = 8.0 Hz, 1H), 7.42-7.38 (m, 2H), 7.31-7.26 (m, 1H), 7.16-7.08 (m, 2H), 6.70-6.52 (m, 1H), 6.41 (dd, *J* = 16.8 Hz, 1.6 Hz, 1H), 5.81 (dd, *J* = 10.4 Hz, 1.6 Hz, 1H), 5.04-4.36 (m, 3H), 4.05-3.58 (m, 3H), 3.24-3.04 (m, 4H), 1.67-1.60 (m, 1H), 1.54-1.47 (m, 3H), 0.82-0.76 (m, 2H), 0.66-0.53 (m, 2H).

### Example 14 2-(4-((S))-4-acryloyl-2-methylpiperazin-1-yl) -6-fluoro-7-(2-fluoro-6-hydroxyphenyl)-2-oxopyrano [2,3-d] pyrimidin-1 (2H)-yl)-N,N, 3-trimethylbenzenesulfonamide

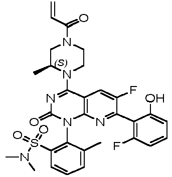

LC-MS:m/z 625 (M+H)⁺. ¹H NMR (400 MHz, CDCl₃) δ 9.04 (brs, 1H), 7.96 (d, *J* = 8.0 Hz, 1H), 7.89-7.81 (m, 1H), 7.80 (d, *J* = 7.6 Hz, 1H), 7.57 (t, *J* = 7.6 Hz, 1H), 7.30-7.22 (m, 1H), 6.71-6.51 (m, 3H), 6.41 (dd, *J* = 16.4 Hz, 1.6 Hz; 1H), 5.82 (dd, *J* = 10.4 Hz, 1.6 Hz; 1H), 5.16-2.92 (m, 7H), 2.73 (s, 6H), 2.50 (d, *J=* 7.2 Hz, 3H), 1.50-1.41 (m, 3H).

### Example 15 (S)-4-(4-acryloyl-2-methylpiperazin-1-yl)-1-(2-cyclopropyl-6-(methylsulfonyl) phenyl)-7-(2,6-difluorohenl-6-fluororidino[2,3-d] pyrimidin-2(1H)-one

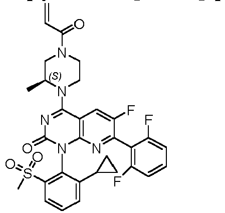

LC-MS:m/z 624 (M+H)⁺. ¹H NMR (400 MHz, CDCl₃) δ 7.95 (d, *J* = 8.0 Hz, 1H), 7.83-7.78 (m, 1H), 7.50 (t, *J* = 8.0 Hz, 1H), 7.42-7.33 (m, 2H), 6.92 (t, *J* = 8.4 Hz, 2H), 6.70-6.53 (m, 1H), 6.41 (dd, *J=* 16.4 Hz, 1.6 Hz, 1H), 5.81 (dd, *J=* 10.4 Hz, 2.0 Hz, 1H), 5.08-4.28 (m, 3H), 4.09-3.58 (m, 3H), 3.31-3.07 (m, 4H), 1.65-1.57 (m, 1H), 1.57-1.45 (m, 3H), 0.78-0.74 (m, 2H), 0.66-0.55 (m, 2H).

### Example 16 4-((S)-4-acryloyl-2-methylpiperazin-1-yl) -6-fluoro-7-(2-fluoro-6-hydroxyphenyl)-1-(4-isopropyl-2-(methylsulfonyl) pyridin-3-yl) pyridino [2,3-d] pyrimidin-2 (1H)-one

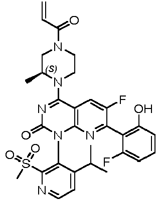

LC-MS:m/z 625 (M+H)⁺. ¹H NMR (400 MHz, CDCl₃) δ 8.73-8.70 (m, 1H), 8.50-8.41 (m, 1H), 7.89 (dd, *J* = 9.2 Hz, 2.4 Hz, 1H), 7.67 (d, *J* = 5.2 Hz, 1H), 7.26 (m, 1H), 6.70-6.50 (m, 3H), 6.40 (d, *J=* 16.8 Hz, 1H), 5.81 (d, *J=* 10.4 Hz, 1H), 5.10-4.34 (m, 3H), 4.04-3.60 (m, 3H), 3.28-3.09 (m, 4H), 3.00-2.80 (m, 1H), 1.57-1.49 (m, 3H), 1.27 (dd, *J* = 6.8 Hz, 1.6 Hz, 3H), 1.07 (t, *J* = 6.4 Hz, 3H).

### Example 16-1 two isomers of Example 16A and Example 16B were obtained by chiral separation:

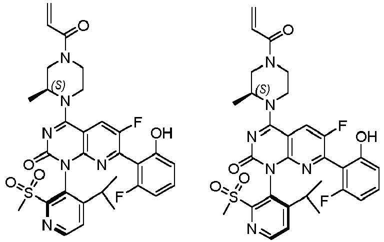

**Example 16A:**LC-MS:m/z 625 (M+H)⁺. ¹H NMR (400 MHz, DMSO) δ 10.22 (s, 1H), 8.70 (d, *J* = 4.7 Hz, 1H), 8.34 (d, *J* = 9.3 Hz, 1H), 7.87 (d, *J* = 4.8 Hz, 1H), 7.26 (dd, *J* = 15.2, 7.8 Hz, 1H), 6.85 (dd, *J* = 15.8, 10.0 Hz, 1H), 6.79 - 6.56 (m, 2H), 6.21 (d, *J* = 16.2 Hz, 1H), 5.77 (d, *J* = 10.5 Hz, 1H), 4.97 (s, 1H), 4.22 (m, 3H), 3.61 (m, 2H), 3.30 - 2.90 (m, 4H), 2.81 (m, 1H), 1.31 (d, *J* = 6.3 Hz, 3H), 1.12 (d, *J* = 6.6 Hz, 3H), 1.03 (d, J= 6.5 Hz, 3H).

**Example 16B:**LC-MS:m/z 625 (M+H)⁺. ¹H NMR (400 MHz, DMSO) δ 10.22 (s, 1H), 8.70 (d, *J* = 5.0 Hz, 1H), 8.31 (t, *J* = 10.7 Hz, 1H), 7.87 (d, *J* = 5.0 Hz, 1H), 7.26 (dd, *J* = 15.4, 8.1 Hz, 1H), 7.01 - 6.77 (m, 1H), 6.76 - 6.57 (m, 2H), 6.29 - 6.11 (m, 1H), 5.77 (dd, *J* = 10.5, 2.0 Hz, 1H), 4.86 (s, 1H), 4.50 - 3.93 (m, 3H), 3.78 - 3.40 (m, 2H), 3.30 - 2.94 (m, 4H), 2.80 (m, 1H), 1.31 (d, *J* = 6.5 Hz, 3H), 1.13 (d, *J* = 6.8 Hz, 3H), 1.03 (d, J= 6.7 Hz, 3H).

### Example 17 (S)-4-(4-acryloyl-2-methylpiperazin-1-yl)-7-(2-chlorophenyl)-1-(2-cyclopropyl-6-(met hylsulfonyl)phenyl)-6-fluoropyridino[2, 3-d] pyrimidin-2(1H)-one

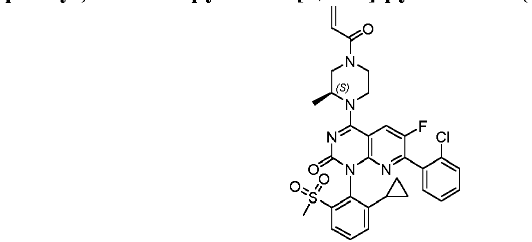

LC-MS:m/z 622 (M+H)⁺. ¹H NMR (400 MHz, CDCl₃) δ 7.96 (d, *J* = 7.6 Hz, 1H), 7.80-7.77 (m, 1H), 7.51 (t, *J* = 8.0 Hz, 1H), 7.41-7.28 (m, 3H), 7.26 (m, 2H), 6.68-6.54 (m, 1H), 6.42 (dd, *J* = 16.8 Hz, 2.0 Hz, 1H), 5.81 (dd, *J* = 10.4 Hz, 1.6 Hz, 1H), 5.08-4.33 (m, 3H), 4.07-3.59 (m, 3H), 3.28-3.09 (m, 4H), 1.69-1.65 (m, 1H), 1.57-1.47 (m, 3H), 0.82-0.76 (m, 2H), 0.67-0.51 (m, 2H).

### Example 18 4-((S)-4-acryloyl-2-methylpiperazin-1-yl) -6-fluoro-7-(2-fluoro-6-hydroxyphenyl)-1-(2-isopropyl-4-(methylsulfonyl) pyridin-3-yl)pyridino[2,3-d]pyrimidin-2 (1H)-one

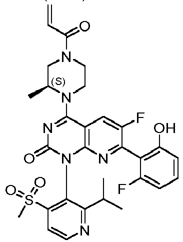

LC-MS:m/z 625 (M+H)⁺. ¹H NMR (400 MHz, CDCl₃) δ 9.02 (t, *J* = 8.8 Hz, 1H), 8.67 (t, *J* = 4.8 Hz, 1H), 7.93-7.89 (m, 2H), 7.28-7.26 (m, 1H), 6.71-6.40 (m, 4H), 5.84-5.81 (m, 1H), 4.48-3.68 (m, 7H), 3.15 (s, 3H), 2.97-2.93 (m, 1H), 1.59-1.07(m, 9H).

### Example 18-1 two isomers of Example 18A and Example 18B were obtained by chiral separation:

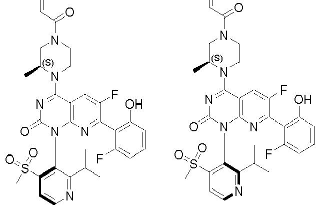

**Example 18A:**LC-MS:m/z 625 (M+H)⁺. ¹H NMR (400 MHz, DMSO) δ 10.31 (s, 1H), 8.96 (d, *J=* 5.0 Hz, 1H), 8.40 (t, *J=* 10.2 Hz, 1H), 7.89 (d, *J=* 5.0 Hz, 1H), 7.32 (dd, *J=* 15.3, 8.3 Hz, 1H), 7.02 - 6.84 (m, 1H), 6.83 - 6.64 (m, 2H), 6.26 (dd, *J=* 16.6, 5.1 Hz, 1H), 5.82 (dd, *J* = 10.4, 2.3 Hz, 1H), 4.96 (s, 1H), 4.29 (m, 3H), 3.58 (m, 2H), 3.36 - 2.87 (m, 5H), 1.43 - 1.33 (m, 3H), 1.17 (d, *J* = 6.7 Hz, 3H), 1.10 - 1.00 (m, 3H).

**Example 18B:**LC-MS:m/z 625 (M+H)⁺. ¹H NMR (400 MHz, DMSO) δ 10.24 (s, 1H), 8.90 (d, *J=* 5.0 Hz, 1H), 8.35 (t, *J=* 10.4 Hz, 1H), 7.83 (d, *J=* 5.0 Hz, 1H), 7.26 (dd, *J=* 15.3, 8.2 Hz, 1H), 6.95 - 6.79 (m, 1H), 6.78 - 6.57 (m, 2H), 6.21 (d, *J=* 16.6 Hz, 1H), 5.77 (dd, *J* = 10.5, 2.1 Hz, 1H), 4.97 (s, 1H), 4.22 (m, 3H), 3.86 - 3.43 (m, 2H), 3.29 - 2.81 (m, 5H), 1.32 (t, *J* = 7.0 Hz, 3H), 1.12 (t, *J* = 8.2 Hz, 3H), 1.02 (d, *J* = 6.6 Hz, 3H).

### Example 19 4-((S)-4-acryloyl-2-methylpiperazin-1-yl) -6-fluoro-7-(2-fluoro-6-methoxyphenyl)-1-(4-isopropyl-2-(methylsulfonyl) pyridin-3-yl) pyridino[2,3-d]pyrimidin -2(1H)-one

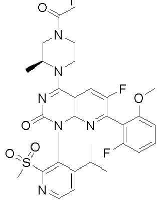

LC-MS:m/z 639 (M+H)⁺. ¹H NMR (400 MHz, CDCl₃) δ 8.66 (d, *J* = 5.2 Hz, 1H), 7.79-7.75 (m, 1H), 7.58 (d, *J=* 4.4 Hz, 1H), 7.33-7.29 (m, 1H), 6.71-6.60 (m, 3H), 6.41-6.38 (m, 1H), 5.80 (d, *J=* 10.4 Hz, 1H), 5.04-4.35 (m, 3H), 4.00-3.60 (m, 6H), 3.18 (m, 4H), 2.92 (m, 1H), 1.48 (m, 3H), 1.23 (d, *J=* 6.8 Hz, 3H), 1.09 (m, 3H) .

### Example 20 (S)-4-(4-acryloyl-2-methylpiperazin-1-yl)-7-(2-chlorophenyl) -6-fluoro-1-(4-isopropyl-2-(methylsulfonyl) pyridin-3-yl) pyridino[2,3-d] pyrimidin-2(1H)-one

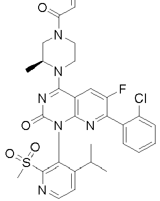

LC-MS:m/z 625 (M+H)⁺. δ 8.67-8.65 (m, 1H), 7.81-7.77 (m, 1H), 7.57 (d, *J=* 4.8 Hz, 1H), 7.40-7.27 (m, 4H), 6.61 (m, 1H), 6.42-6.38 (m, 1H), 5.80 (d, *J* = 10.4 Hz, 1H), 5.16-4.36 (m, 3H), 4.02-3.59 (m, 3H), 3.17 (m, 4H), 2.95 (m, 1H), 1.49 (m, 3H), 1.25 (d, *J* = 6.8 Hz, 3H), 1.06 (d, *J=* 6.8 Hz, 3H) .

### Example 20-1 two isomers of Example 20A and Example 20B were obtained by chiral separation:

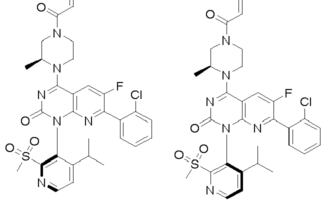

**Example 20A:**LC-MS:m/z 625 (M+H)⁺. ¹H NMR (400 MHz, DMSO) δ 8.71 (d, *J* = 5.0 Hz, 1H), 8.41 (t, *J* = 9.3 Hz, 1H), 7.88 (d, *J* = 5.0 Hz, 1H), 7.56 (d, *J* = 7.1 Hz, 1H), 7.50 (dd, *J* = 7.4, 1.6 Hz, 1H), 7.42 (t, *J=* 7.5 Hz, 1H), 7.38 - 7.25 (m, 1H), 6.86 (dd, *J* = 16.6, 9.3 Hz, 1H), 6.22 (d, *J* = 16.4 Hz, 1H), 5.89 - 5.69 (m, 1H), 5.02 (s, 1H), 4.23 (m, 3H), 3.93 - 3.57 (m, 2H), 3.30 - 3.01 (m, 4H), 2.88 (m, 1H), 1.31 (d, *J* = 6.5 Hz, 3H), 1.15 (d, *J=* 6.8 Hz, 3H), 1.02 (d, *J=* 6.8 Hz, 3H).

**Example 20B:**LC-MS:m/z 625 (M+H)⁺. ¹H NMR (400 MHz, DMSO) δ 8.71 (d, *J* = 5.0 Hz, 1H), 8.37 (t, *J=* 10.5 Hz, 1H), 7.88 (d, *J=* 5.0 Hz, 1H), 7.48 (m, 3H), 7.30 (d, *J* = 7.3 Hz, 1H), 6.87 (d, *J=* 10.4 Hz, 1H), 6.22 (d, *J=* 16.4 Hz, 1H), 5.78 (d, *J=* 12.5 Hz, 1H), 4.86 (s, 1H), 4.62 - 3.99 (m, 3H), 3.58 (m, 2H), 3.11 (m, 4H), 2.94 - 2.81 (m, 1H), 1.33 (d, *J=* 6.6 Hz, 3H), 1.15 (d, *J=* 6.8 Hz, 3H), 1.02 (d, *J=* 6.8 Hz, 3H).

### Example 21 6-fluoro-7-(2-fluoro-6-hydroxyphenyl)-4-((S)-4-(2-fluoroacryloyl)-2-methylpiperazin-1-yl)-1-(2-isopropyl-6-(methylsulfoxyl)phenyl)pyridino[2,3-d]pyrimidin-2(1H)-one

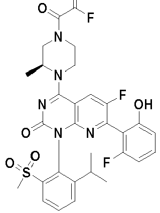

LC-MS:m/z 642 (M+H)⁺. ¹H NMR (400 MHz, CDCl₃) δ 8.97 (s, 1H), 8.13-8.11 (m, 1H), 7.88-7.82 (m, 2H), 7.76-7.72 (m, 1H), 7.28-7.22 (m, 1H), 6.66-6.62 (m, 2H), 5.48-5.35 (m, 1H), 5.26-5.22 (m, 1H), 5.02-4.90 (m, 1H), 4.53-3.65 (m, 6H), 3.11 (s, 3H), 2.82-2.73 (m, 1H), 1.60-1.03 (m, 9H).

### Example 22 (S)-4-(4-acryloyl-2-methylpiperazin-1-yl)-7 -(2-chloro-4-fluorophenyl)-6-fluoro-1-(4-is opropyl-2-(methylsulfonyl) pyridin-3-yl) pyridino[2,3-d] pyrimidin-2(1H)-one

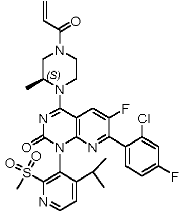

LC-MS:m/z 643 (M+H)⁺. ¹H NMR (400 MHz, CDCl₃) δ 8.68-8.67 (m, 1H), 7.81-7.78 (m, 1H), 7.59 (d, *J* = 5.2 Hz, 1H), 7.34 (m, 1H), 7.14 (dd, *J* = 4.4, 2.4Hz, 1H), 7.02-6.99 (m, 1H), 6.61-6.58 (m, 1H), 6.4-6.38 (m, 1H), 5.82-5.80 (m, 1H), 5.01-3.62 (m, 6H), 3.18 (m, 4H), 2.96 (m, 1H), 1.48 (m, 3H), 1.26 (d, *J=* 6.8 Hz, 3H), 1.07-1.05 (m, 3H).

### Example 23 (S)-7-(2-chlorophenyl)-6-fluoro-4-(4-(2-fluoroacryloyl)-2-methylpiperazin-1-yl)-1-(2-i sopropyl-6-(methylsulfonyl)phenyl)pyridino[2,3-d]pyrimidin-2(1H)-one

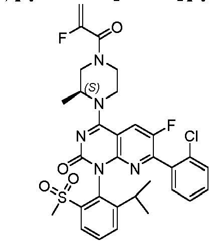

LC-MS:m/z 642 (M+H)⁺. ¹H NMR (400 MHz, CDCl₃) δ 7.98 (d, *J* = 6.8 Hz, 1H), 7.79-7.75 (m, 1H), 7.70-7.69 (m, 1H), 7.60-7.56 (m, 1H), 7.39-7.19 (m, 4H), 5.46-5.33 (m, 1H), 5.25-5.20 (m, 1H), 4.93 (m, 1H), 4.65-3.20 (m, 6H), 3.07 (s, 3H), 2.81-2.75 (m, 1H), 1.52 (d, *J=* 6.8 Hz, 3H),1.23 (d, *J=* 6.8 Hz, 3H), 1.02-1.00 (m, 3H).

### Example 24 2-(4-((S))-4-acryloyl-2-methylpiperazin-1-yl) -6-fluoro-1-(4-isopropyl-2-(methylsulfonyl)pyridin-3-yl)-2-oxo-1, 2-dihydropyridino[2,3-d]pyrimidin-7-yl)-3-fluorophenylacetate

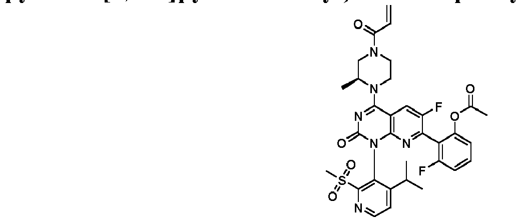

LC-MS:m/z 667 (M+H)⁺. ¹H NMR (400 MHz, CDCl₃) δ 8.68-8.67 (m, 1H), 7.85-7.79 (m, 1H), 7.56 (d, *J* = 4.8 Hz, 1H), 7.43-7.37 (m, 1H), 7.02-6.95 (m, 2H), 6.70-6.53 (m, 1H), 6.42-6.38 (m, 1H), 5.81-5.79 (m, 1H), 5.08-3.63 (m, 6H), 3.17 (m, 4H), 2.76 (m, 1H), 2.02 (s, 3H), 1.46 (m, 3H), 1.20 (d, *J=* 6.8 Hz, 3H), 0.99 (m, 3H).

### Example 25 4-((S)-4-acryloyl-2-methylpiperazin-1-yl)-1-(2-cyclobutyl-6-(methylsulfonyl)phenyl)-6 -fluoro-7-(2-fluoro-6-hydroxyphenyl) pyridino[2,3-d]pyrimidin-2(1H)-one

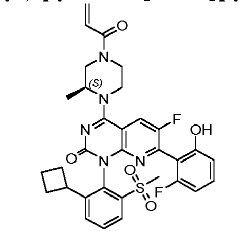

LC-MS:m/z 636 (M+H)⁺. ¹H NMR (400 MHz, CDCl₃) δ 8.97 (s, 1H), 8.11 (d, *J* = 6.8 Hz, 1H), 8.10-7.70 (m, 3H), 7.28-7.22 (m, 1H), 6.69-6.60 (m, 3H), 6.42-6.39 (m, 1H), 5.83-5.80 (m, 1H), 5.05-3.64 (m, 6H), 3.12 (m, 5H), 2.30-2.25 (m, 1H), 2.11-2.08 (m, 2H), 1.85-1.71 (m, 3H), 1.47 (d, *J=* 6.4 Hz, 3H).

### Example 25-1 two isomers of Example 25A and Example 25B were obtained by chiral separation:

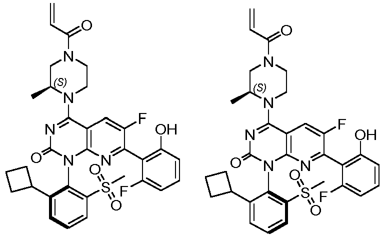

**Example 25A:**LC-MS:m/z 636 (M+H)⁺. ¹H NMR (400 MHz, DMSO) δ 10.24 (s, 1H), 8.32 (t, *J=* 9.3 Hz, 1H), 7.89 (dd, *J=* 7.8, 1.1 Hz, 1H), 7.76 (d, *J=* 7.3 Hz, 1H), 7.65 (m, 1H), 7.27 (m, 1H), 6.87 (m, 1H), 6.79 - 6.58 (m, 2H), 6.22 (m, 1H), 5.78 (m, 1H), 4.87 (m, 1H), 4.21 (m, 3H), 3.78 - 3.42 (m, 2H), 3.31 - 2.91 (m, 5H), 2.19 - 1.87 (m, 3H), 1.87 - 1.57 (m, 3H), 1.33 (m, 3H).

**Example 25B:LC-MS:m/z** 636 (M+H)⁺. ¹H NMR (400 MHz, DMSO) δ 10.24 (s, 1H), 8.33 (t, *J* = 10.1 Hz, 1H), 7.89 (dd, *J* = 7.8, 1.1 Hz, 1H), 7.76 (d, *J* = 7.3 Hz, 1H), 7.66 (m, 1H), 7.27 (m, 1H), 6.99 - 6.79 (m, 1H), 6.79 - 6.58 (m, 2H), 6.21 (m, 1H), 5.77 (m, 1H), 4.88 (s, 1H), 4.55 - 3.97 (m, 3H), 3.77 - 3.42 (m, 2H), 3.30 - 2.89 (m, 5H), 2.13 - 1.88 (m, 3H), 1.89 - 1.57 (m, 3H), 1.29 (m, 3H).

### Example 26 (S)-4-(4-acryloyl-2-methylpiperazin-1-yl)-7-(2-chloro-3-fluorophenyl)-6-fluoro-1-(4-is opropyl-2-(methylsulfonyl) pyridin-3-yl) pyridino[2,3-d] pyrimidin-2(1H)-one

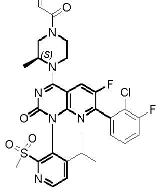

LC-MS:m/z 643 (M+H)⁺. ¹H NMR (400 MHz, CDCl₃) δ 8.67-8.66 (m, 1H), 7.83-7.80 (m, 1H), 7.58 (d, *J=* 4.2 Hz, 1H), 7.28-7.13 (m, 3H), 6.61-6.58(m, 1H), 6.42-6.39 (m, 1H), 5.83-5.80 (m, 1H), 5.34-3.62 (m, 6H), 3.18 (m, 4H), 2.97 (m, 1H), 1.49 (m, 3H), 1.27-1.25 (m, 3H), 1.07-1.06 (m, 3H).

### Example 27 4-((S)-4-acryloyl-2-methylpiperazin-1-yl)-7-(2-chloro-6-hydroxyphenyl)-6-fluoro-1-(4 -isopropyl-2-(methylsulfonyl) pyridin-3-yl) pyridino[2,3-d] pyrimidin-2(1H)-one

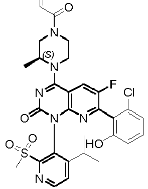

LC-MS:m/z 641 (M+H)⁺. ¹H NMR (400 MHz, CDCl₃) δ 8.61 (m, 1H), 7.90-7.86 (m, 1H), 7.61-7.60 (m, 1H), 7.26-7.18 (m, 1H), 6.96 (d, *J=* 8.0 Hz, 1H), 6.84 (d, *J=* 8.4 Hz, 1H), 6.60-6.57 (m, 1H), 6.42-6.38 (m, 1H), 5.82-5.79 (m, 1H), 5.06-3.61 (m, 6H), 3.30 (s, 3H), 2.95-2.88 (m, 2H), 1.54-1.50 (m, 3H), 1.28-1.26 (m, 3H), 1.02 (m, 3H).

### Example 28 4-((S)-4-acryloyl-2-methylpiperazin-1-yl)-7-(2-chloro-6-fluorophenyl)-6-fluoro-1-(4-is opropyl-2-(methylsulfonyl)pyridin-3-yl) pyridino[2,3-d] pyrimidin-2(1H)-one

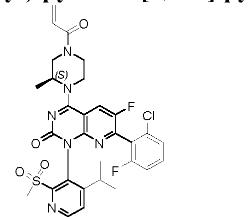

LC-MS:m/z 643 (M+H)⁺. ¹H NMR (400 MHz, CDCl₃) δ 8.66 (m, 1H), 7.87-7.83 (m, 1H), 7.58 (d, *J=* 4.8 Hz, 1H), 7.36-7.31 (m, 1H), 7.23-7.21 (m, 1H), 7.03-7.02 (m, 1H), 6.61-6.58 (m, 1H), 6.42-6.38 (m, 1H), 5.82-5.79 (m, 1H), 5.06-3.61 (m, 6H), 3.27-3.12 (m, 4H), 2.92 (m, 1H), 1.53-1.50 (m, 3H), 1.25-1.23 (m, 3H), 1.08 (m, 3H).

### Example 29 4-((S)-4-acryloyl-2-methylpiperazin-1-yl) -6-fluoro-7-(2-fluoro-6-hydroxyphenyl)-1-(3-isopropyl-5-(methylsulfonyl)pyrimidin-4 -yl) pyridino[2,3-d]pyrimidin -2(1H)-one

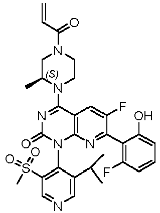

LC-MS:m/z 625 (M+H)⁺. ¹H NMR (400 MHz, CDCl₃) δ 9.25 (s, 1H), 9.06 (s, 1H), 8.58 (s, 1H), 7.90 (d, *J=* 9.3 Hz, 1H), 7.32 - 7.23 (m, 1H), 6.74 - 6.51 (m, 3H), 6.42 (dd, *J* = 16.7, 1.6 Hz, 1H), 5.89 - 5.77 (m, 1H), 5.27 - 3.45 (m, 6H), 3.17 (s, 4H), 2.83 (m, 1H), 1.56 - 1.41 (m, 3H), 1.30 (m, 3H), 1.14 (t, *J* = 6.5 Hz, 3H).

### Example 29-1 two isomers of Example 29A and Example 29B were obtained by chiral separation:

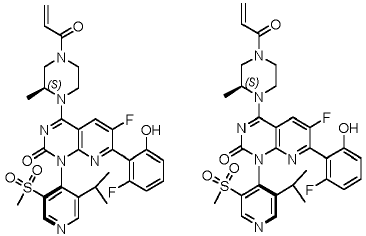

**Example 29A:**LC-MS:m/z 625 (M+H)⁺.

**Example 29B:**LC-MS:m/z 625 (M+H)⁺.

### Example 30 (S)-4-(4-acryloyl-2-methylpiperazin-1-yl)-7 -(2-chlorophenyl)-6-fluoro-1-(2-isopropyl-6-(methylsulfonyl) phenyl) pyridino[2,3-d]pyrimidin-2(1H)-one

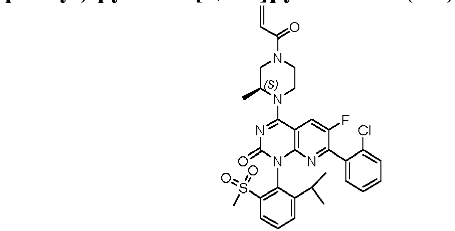

LC-MS:m/z 624 (M+H)⁺. ¹H NMR (400 MHz, CDCl₃) δ 7.98 (dd, *J* = 7.8, 1.3 Hz, 1H), 7.84 - 7.73 (m, 1H), 7.70 (d, *J* = 6.8 Hz, 1H), 7.58 (t, *J* = 7.9 Hz, 1H), 7.44 - 7.30 (m, 2H), 7.28 - 7.15 (m, 2H), 6.61 (m, 1H), 6.41 (dd, *J* = 16.8, 1.7 Hz, 1H), 5.81 (d, *J* = 10.5 Hz, 1H), 5.13 - 3.47 (m, 6H), 3.08 (m, 4H), 2.80 (m, 1H), 1.50 (m, 3H), 1.24 (t, *J* = 6.4 Hz, 3H), 1.01 (d, *J=* 6.8 Hz, 3H).

### Example 30-1 two isomers of Example 30A and Example 30B were obtained by chiral separation:

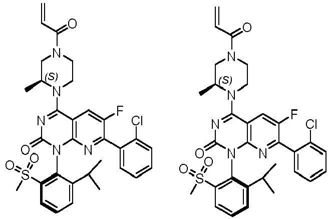

**Example 30A:**LC-MS:m/z 624 (M+H)⁺. ¹H NMR (400 MHz, DMSO) δ 8.38 (t, *J* = 9.3 Hz, 1H), 7.86 (m, 2H), 7.66 (t, *J=* 7.8 Hz, 1H), 7.47 (m, 3H), 7.22 (d, *J=* 7.2 Hz, 1H), 6.92 - 6.74 (m, 1H), 6.22 *(d, J* = 16.6 Hz, 1H), 5.77 *(d, J* = 10.7 Hz, 1H), 4.96 (brs, 1H), 4.23 (m, 3H), 3.87 - 3.44 (m, 2H), 3.28 - 3.07 (m, 1H), 3.01 (s, 3H), 2.71 (d, *J* = 6.1 Hz, 1H), 1.30 (t, *J=* 14.4 Hz, 3H), 1.11 (d, *J* = 6.7 Hz, 3H), 0.94 (t, *J* = 28.8 Hz, 3H).

**Example 30B:**LC-MS**:** m/z 624 (M+H)⁺. ¹H NMR (400 MHz, DMSO) δ 8.37 (m, 1H), 7.86 (m, 2H), 7.66 (t, *J=* 7.5 Hz, 1H), 7.47 (m, 3H), 7.22 (d, *J=* 7.0 Hz, 1H), 6.96 - 6.68 (m, 1H), 6.22 (d, *J=* 15.4 Hz, 1H), 5.77 (d, *J=* 10.3 Hz, 1H), 4.88 (brs, 1H), 4.53 - 3.94 (m, 3H), 3.76 - 3.45 (m, 2H), 3.33 - 2.60 (m, 5H), 1.30 *(t, J* = 15.2 Hz, 3H), 1.09 (t, *J* = 15.7 Hz, 3H), 0.95 (t, *J* = 27.8 Hz, 3H).

### Example 31 2-(4-((S))-4-acryloyl-2-methylpiperazin-1-yl) -6-fluoro-1-(2-isopropyl-6-(methylsulfonyl)phenyl)-2-oxo-1, 2-dihydropyridino[2,3-d]pyrimidin-7-yl)-3-fluorophenyl acetate

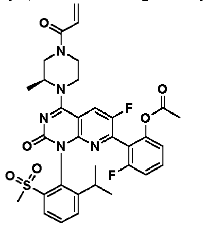

LC-MS: m/z 666(M+H)⁺. ¹H NMR (400 MHz, CDCl₃) δ 7.91 (d, *J* = 7.3 Hz, 1H), 7.76 (s, 1H), 7.60 (d, *J=* 7.4 Hz, 1H), 7.52 (t, *J=* 7.6 Hz, 1H), 7.32 (m, 1H), 6.99 - 6.76 (m, 2H), 6.55 (m, 1H), 6.34 m, 1H), 5.75 (m, 1H), 5.23 - 3.42 (m, 6H), 3.15 (m, 4H), 2.54 (s, 1H), 1.94 (s, 3H), 1.57 - 1.30 (m, 3H), 1.07 (m, 3H), 0.85 (m, 3H).

### Example 31-1 two isomers of Example 31A and Example 31B were obtained by chiral separation:

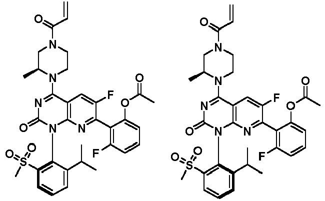

**Example 31A:**LC-MS:m/z 666(M+H)⁺. ¹H NMR (400 MHz, DMSO) δ 8.38 (t, *J* = 9.3 Hz, 1H), 7.89 (dd, *J=* 7.8, 1.3 Hz, 1H), 7.80 (dt, *J=* 14.3, 7.2 Hz, 1H), 7.66 (t, *J=* 7.8 Hz, 1H), 7.56 (td, *J* = 8.4, 6.6 Hz, 1H), 7.25 (t, *J* = 8.7 Hz, 1H), 7.12 (d, *J* = 7.8 Hz, 1H), 6.87 (m, 1H), 6.22 (d, *J=* 16.7 Hz, 1H), 5.78 (d, *J=* 10.6 Hz, 1H), 4.93 (brs, 1H), 4.35 (m, 2H), 4.10 (m, 1H), 3.86 - 3.46 (m, 2H), 3.24 (m, 1H), 2.99 (m, 3H), 2.60 (m, 1H), 2.13 - 1.86 (m, 3H), 1.35 (d, *J=* 6.5 Hz, 3H), 1.09 (d, *J=* 6.6 Hz, 3H), 0.90 (t, *J=* 12.5 Hz, 3H).

**Example 31B:**LC-MS:m/z 666(M+H)⁺. ¹H NMR (400 MHz, DMSO) δ 8.41 (t, *J* = 8.8 Hz, 1H), 7.89 (dd, *J* = 7.8, 1.4 Hz, 1H), 7.82 (dt, *J* = 7.9, 3.9 Hz, 1H), 7.66 (t, *J* = 7.8 Hz, 1H), 7.56 (td, *J* = 8.4, 6.6 Hz, 1H), 7.25 (t, *J* = 8.8 Hz, 1H), 7.12 (d, *J* = 8.0 Hz, 1H), 7.00 - 6.78 (m, 1H), 6.34 - 6.09 (m, 1H), 5.78 (dd, *J* = 10.4, 2.3 Hz, 1H), 4.90 (brs, 1H), 4.56 - 3.94 (m, 3H), 3.63 (m, 2H), 3.31 - 2.88 (m, 4H), 2.66 (m, 1H), 2.09 - 1.87 (m, 3H), 1.31 (m, 3H), 1.09 (d, *J* = 6.6 Hz, 3H), 0.93 (m, 3H).

### Example 32 (S)-4-(4-acryloyl-2-methylpiperazin-1-yl)-7 -(2-chloro-3-fluorophenyl)-6-fluoro-1-(2-is opropyl-6-(methylsulfonyl) phenyl) pyridino[2,3-d]pyrimidin-2(1H)-one

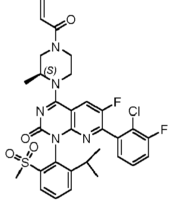

LC-MS:m/z 642 (M+H)⁺. ¹H NMR (400 MHz, CDCl₃) δ 8.03 - 7.91 (m, 1H), 7.80 (dd, *J* = 8.6, 5.5 Hz, 1H), 7.70 (d, *J* = 7.0 Hz, 1H), 7.59 (t, *J* = 7.8 Hz, 1H), 7.21 (m, 2H), 7.02 (d, *J* = 7.4 Hz, 1H), 6.61 (s, 1H), 6.41 (dd, *J* = 16.8, 1.6 Hz, 1H), 5.81 (d, *J* = 10.5 Hz, 1H), 5.24 - 3.42 (m, 6H), 3.08 (s, 4H), 2.80 (m, 1H), 1.47 (m, 3H), 1.25 (m, 3H), 1.06 - 0.93 (m, 3H).

### Example 32-1 two isomers of Example 32A and Example 32B were obtained by chiral separation:

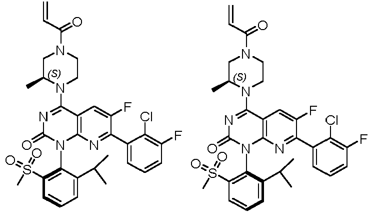

**Example 32A**:LC-MS:m/z 642 (M+H)⁺. ¹H NMR (400 MHz, DMSO) δ 8.42 (t, *J* = 9.4 Hz, 1H), 7.86 (m, 2H), 7.67 (t, *J* = 7.8 Hz, 1H), 7.58 - 7.23 (m, 2H), 7.12 (m, 1H), 6.91 (m, 1H), 6.22 (d, *J* = 16.9 Hz, 1H), 5.78 (d, *J* = 11.3 Hz, 1H), 4.97 (brs, 1H), 4.23 (m, 3H), 3.90 - 3.56 (m, 2H), 3.20 (m, 1H), 3.01 (s, 3H), 2.73 (m, 1H), 1.32 (d, *J* = 6.4 Hz, 3H), 1.11 (d, *J* = 6.7 Hz, 3H), 0.98 (d, *J* = 6.7 Hz, 3H).

**Example 32B:** LC-MS:m/z 642 (M+H)⁺. ¹H NMR (400 MHz, DMSO) δ 8.40 (t, *J* = 9.9 Hz, 1H), 7.87 (m, 2H), 7.67 (t, *J* = 7.8 Hz, 1H), 7.59 - 7.38 (m, 2H), 7.10 (d, *J* = 7.5 Hz, 1H), 6.87 (dd, *J* = 26.4, 15.8 Hz, 1H), 6.22 (d, *J* = 16.5 Hz, 1H), 5.90 - 5.66 (m, 1H), 4.87 (brs, 1H), 4.24 (m, 3H), 3.82 - 3.45 (m, 2H), 3.18 (m, 1H), 3.01 (s, 3H), 2.74 (m, 1H), 1.31 *(t, J* = 6.4 Hz, 3H), 1.11 (d, *J* = 6.8 Hz, 3H), 0.98 (d, *J* = 6.8 Hz, 3H).

### Example 33 (S)-4-(4-acryloyl-2-methylpiperazin-1-yl)-7 -(2-chlorophenyl)-6-fluoro-1-(2-isopropyl-4-(methylsulfonyl) pyridin-3-yl) pyridino[2,3-d] pyrimidin-2(1H)-one

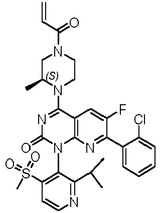

LC-MS:m/z 625 (M+H)⁺.

### Example 33-1 two isomers of Example 33A and Example 33B were obtained by chiral separation:

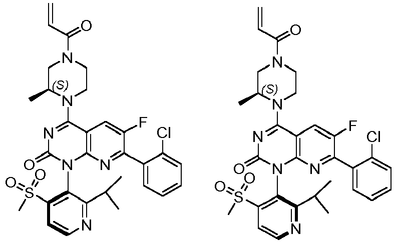

**Example 33A**:LC-MS:m/z 625 (M+H)⁺.

**Example 33B:**LC-MS:m/z 625 (M+H)⁺.

### Example 34 (S)-4-(4-acryloyl-2-methylpiperazin-1-yl)-7 -(2-chloro-3-fluorophenyl)-6-fluoro-1-(2-is opropyl-4-(methylsulfonyl) pyridin-3-yl) pyridino[2,3-d] pyrimidin-2(1H)-one

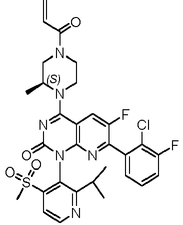

LC-MS:m/z 643 (M+H)⁺.

### Example 34-1 two isomers of Example 34A and Example 34B were obtained by chiral separation:

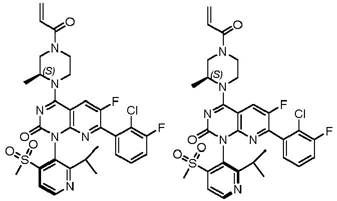

**Example 34**A:LC-MS:m/z 643 (M+H)⁺.

**Example 34B**:LC-MS:m/z 643 (M+H)⁺.

### Example 35 (S)-4-(4-acryloyl-2-methylpiperazin-1-yl)-7 -(2-chlorophenyl)-1-(2-cyclo butyl-6-(meth ylsulfonyl) phenyl)-6-fluoropyridino[2,3-d]pyrimidin-2(1H)-one

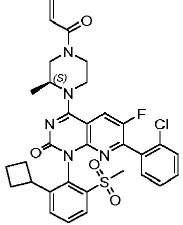

LC-MS:m/z 636 (M+H)⁺.

### Example 35-1 two isomers of Example 35A and Example 35B were obtained by chiral separation:

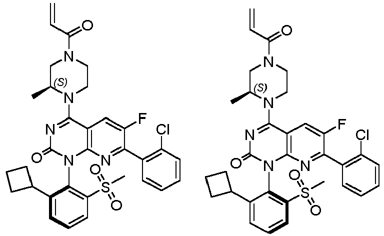

**Example 35A:**LC-MS:m/z 636 (M+H)⁺.

**Example 35B:**LC-MS:m/z 636 (M+H)⁺.

### Example 36 (S)-4-(4-acryloyl-2-methylpiperazin-1-yl)-7 -(2-chloro-3-fluorophenyl)-1-(2-cyclobutyl -6-(methylsulfonyl) phenyl)-6-fluoropyridino[2,3-d]pyrimidin-2(1H)-one

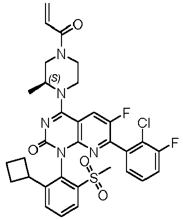

LC-MS:m/z 654 (M+H)⁺.

### Example 36-1 two isomers of Example 36A and Example 36B were obtained by chiral separation:

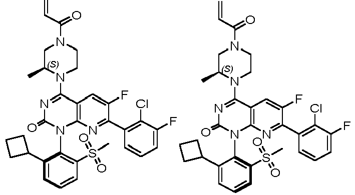

**Example 36A:**LC-MS:m/z 654 (M+H)⁺.

**Example 36B:**LC-MS:m/z 654 (M+H)⁺.

### Example 37 (S)-4-(4-acryloyl-2-methylpiperazin-1-yl)-7 -(2-chloropyridin-3-yl)-6-fluoro-1-(2-isopr opyl-6-(methylsulfonyl)phenyl) pyridino[2,3-d]pyrimidin-2(1H)-one

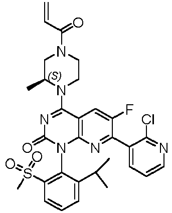

LC-MS:m/z 625 (M+H)⁺.

### Example 37-1 two isomers of Example 37A and Example 37B were obtained by chiral separation:

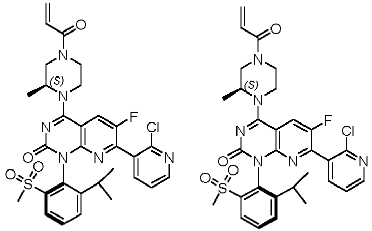

**Example 37A:**LC-MS:m/z 625 (M+H)⁺.

**Example 37B:**LC-MS:m/z 625 (M+H)⁺.

### Example 38 (S)-4-(4-acryloyl-2-methylpiperazin-1-yl)-7 -(3-chloropyridin-4-yl)-6-fluoro-1-(2-isopr opyt-6-(methytsutfonyt)phenyl) pyridinor2,3-dlpyrimidin-2(1H)-one

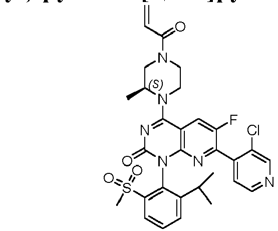

LC-MS:m/z 625 (M+H)⁺.

### Example 38-1 two isomers of Example 38A and Example 38B were obtained by chiral separation:

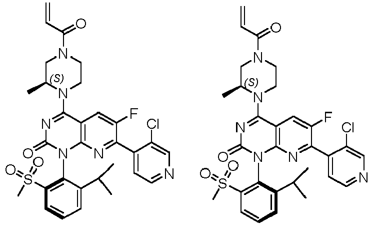

**Example 38A:**LC-MS:m/z 625 (M+H)⁺.

**Example 38B:**LC-MS:m/z 625 (M+H)⁺.

### Example 39 (S)-4-(4-acryloyl-2-methylpiperazin-1-yl)-7-(4-chloropyridin-3-yl)-6-fluoro-1-(2-isopr opyl-6-(methylsulfonyl)phenyl) pyridinor2,3-dlpyrimidin-2(1H)-one

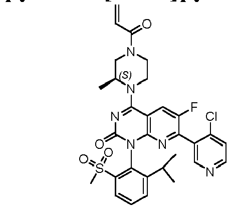

LC-MS:m/z 625 (M+H)⁺.

### Example 39-1 two isomers of Example 39A and Example 39B were obtained by chiral separation:

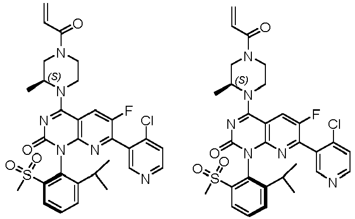

**Example 39A:**LC-MS:m/z 625 (M+H)⁺.

**Example 39B:**LC-MS:m/z 625 (M+H)⁺.

### Example 40 (S)-4-(4-acryloyl-2-methylpiperazin-1-yl)-7 -(3-chloropyridin-2-yl)-6-fluoro-1-(2-isopr opyl-6-(methylsulfonyl)phenyl) pyridino[2,3-d]pyrimidin-2(1H)-one

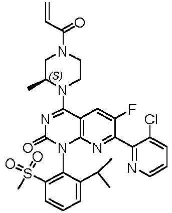

LC-MS:m/z 625 (M+H)⁺.

### Example 40-1 two isomers of Example 40A and Example 40B were obtained by chiral separation:

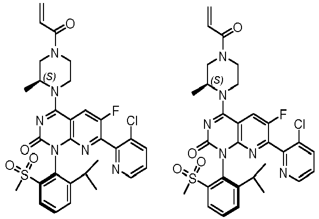

**Example 40A:**LC-MS:m/z 625 (M+H)⁺.

**Example 40B:**LC-MS:m/z 625 (M+H)⁺.

### Example 41 (S)-4-(4-acryloyl-2-methylpiperazin-1-yl)-7 -(2-aminopyridin-3-yl)-6-fluoro-1-(2-isopr opyt-6-(methytsutfonyt)phenyl) pyridino[2,3-d]pyrimidin-2(1H)-one

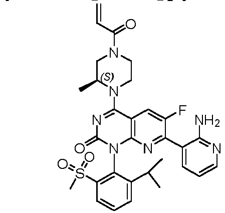

LC-MS:m/z 606 (M+H)⁺.

### Example 41-1 two isomers of Example 41A and Example 41B were obtained by chiral separation:

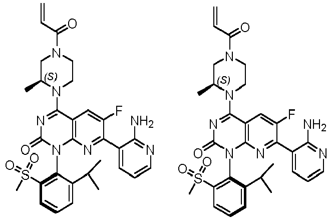

**Example 41A:**LC-MS:m/z 606 (M+H)⁺.

**Example 41B:**LC-MS:m/z 606 (M+H)⁺.

### Example 42 (S)-4-(4-acryloyl-2-methylpiperazin-1-yl)-7-(3-aminopyridin-4-yl)-6-fluoro-1-(2-isopr opyl-6-(methylsulfonyl)phenyl) pyridino[2,3-d]pyrimidin-2(1H)-one

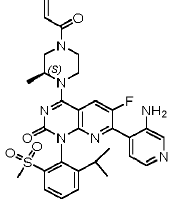

LC-MS:m/z 606 (M+H)⁺.

### Example 42-1 two isomers of Example 42A and Example 42B were obtained by chiral separation:

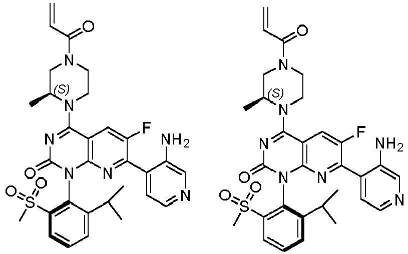

**Example 42A:**LC-MS:m/z 606 (M+H)⁺.

**Example 42B:**LC-MS:m/z 606 (M+H)⁺.

### Example 43 (S)-4-(4-acryloyl-2-methylpiperazin-1-yl)-7-(4-aminopyridin-3-yl)-6-fluoro-1-(2-isopr opyl-6-(methylsulfonyl)phenyl) pyridino[2,3-d]pyrimidin-2(1H)-one

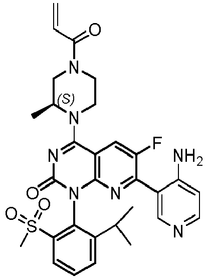

LC-MS:m/z 606 (M+H)⁺.

### Example 43-1 two isomers of Example 43A and Example 43B were obtained by chiral separation:

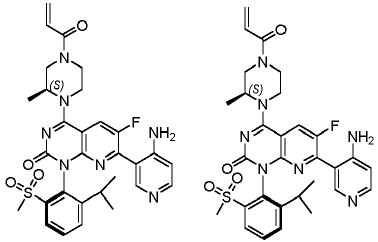

**Example 43A:**LC-MS:m/z 606 (M+H)⁺.

**Example 43B:**LC-MS:m/z 606 (M+H)⁺.

### Example 44 (S)-4-(4-acryloyl-2-methylpiperazin-1-yl)-7-(3-aminopyridin-2-yl)-6-fluoro-1-(2-isopr opyl-6-(methylsulfonyl)phenyl) pyridino[2,3-d]pyrimidin-2(1H)-one

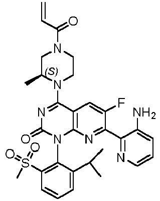

LC-MS:m/z 606 (M+H)⁺.

### Example 44-1 two isomers of Example 44A and Example 44B were obtained by chiral separation:

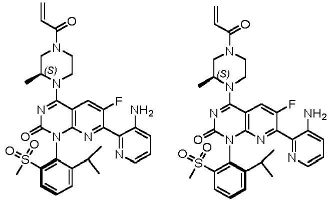

**Example 44A:**LC-MS:m/z 606 (M+H)⁺.

**Example 44B:**LC-MS:m/z 606 (M+H)⁺.

### Example 45 2-(4-((S))-4-acryloyl-2-methylpiperazin-1-yl) -6-fluoro-1-(2-isopropyl-6-(methylsulfonyl)phenyl)-2-oxo-1, 2-dihydropyridino[2,3-d]pyrimidin-7-yl)-3-fluorophenyl propionate

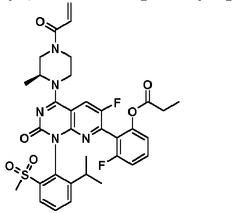

LC-MS:m/z 680 (M+H)⁺.

### Example 45-1 two isomers of Example 45A and Example 45B were obtained by chiral separation:

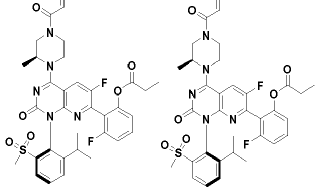

**Example 45A:**LC-MS:m/z 680 (M+H)⁺.

**Example 45B:**LC-MS:m/z 680 (M+H)⁺.

### Example 46 2-(4-((S))-4-acryloyl-2-methylpiperazin-1-yl) -6-fluoro-1-(2-isopropyl-6-(methylsulfonyl) phenyl)-2-oxo-1, 2-dihydropyridino[2,3-d] pyrimidin-7-yl)-3-fluorophenyl isobutyrate

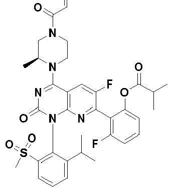

LC-MS:m/z 694 (M+H)⁺.

### Example 46-1 two isomers of Example 46A and Example 46B were obtained by chiral separation:

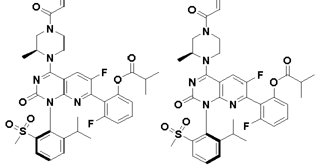

**Example 46A:**LC-MS:m/z 694 (M + H)⁺.

**Example 46B:**LC-MS:m/z 694 (M + H)⁺.

### Example 47 2-(4-((S))-4-acryloyl-2-methylpiperazin-1-yl) -6-fluoro-1-(2-isopropyl-6-(methylsulfonyl)phenyl)-2-oxo-1, 2-dihydropyridino[2,3-d] pyrimidin-7-yl)-3-fluorophenylmethylcarbamate

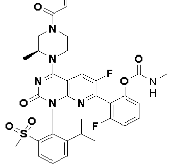

LC-MS:m/z 681 (M+H)⁺.

### Example 47-1 two isomers of Example 47A and Example 47B were obtained by chiral separation:

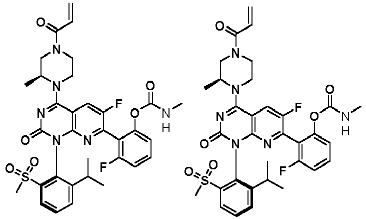

**Example 47A:**LC-MS:m/z 681 (M+H)⁺.

**Example 47B:**LC-MS:m/z 681 (M+H)⁺.

### Example 48 (2-(4-((S))-4-acryloyl-2-methylpiperazin-1-yl) -6-fluoro-1-(2-isopropyl-6-(methylsulfonyl)phenyl)-2-oxo-1, 2-dihydropyridino[2,3-d]pyrimidin-7-yl)-3-fluorophenyl) methyl carbamate

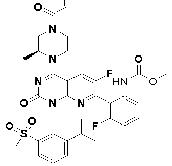

LC-MS:m/z 681 (M+H)⁺.

### Example 48-1 two isomers of Example 48A and Example 48B were obtained by chiral separation:

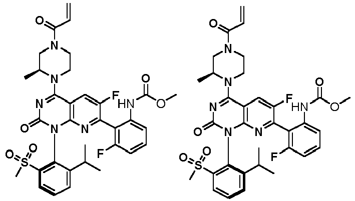

**Example 48A:**LC-MS:m/z 681 (M+H)⁺.

**Example 48B:**LC-MS:m/z 681 (M+H)⁺.

### Example 49 1-(2-(4-((S))-4-acryloyl-2-methylpiperazin-1-yl) -6-fluoro-1-(2-isopropyl-6-(methylsulfonyl)phenyl)-2-oxo-1, 2-dihydropyridino[2,3-d]pyrimidin-7-yl)-3-fluorophenyl)-3-methylurea

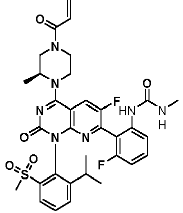

LC-MS:m/z 680 (M+H)⁺.

### Example 49-1 two isomers of Example 49A and Example 49B were obtained by chiral separation:

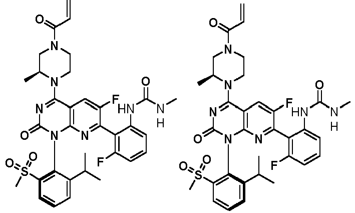

**Example 49A:**LC-MS:m/z 680 (M+H)⁺.

**Example 49B:**LC-MS:m/z 680 (M+H)⁺.

### Example 50 N-(2-(4-((S))-4-acryloyl-2-methylpiperazin-1-yl) -6-fluoro-1-(2-isopropyl-6-(methylsulfonyl)phenyl)-2-oxo-1,2-dihydropyridino[2,3-d] pyrimidin-7-yl)-3-fluorophenyl)-methylsulfonamide

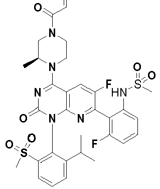

LC-MS:m/z 701 (M+H)⁺.

### Example 50-1 two isomers of Example 50A and Example 50B were obtained by chiral separation:

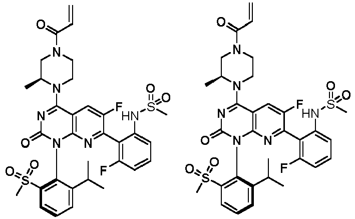

**Example 50A:**LC-MS:m/z 701 (M + H)⁺.

**Example 50B:**LC-MS:m/z 701 (M + H)⁺.

### Example 51 Preparation of 4-((S)-4-acryloyl-2-methylpiperazin-1-yl) -6-fluoro-7-(2-fluoro-6-hydroxyphenyl)-1-(2-(methylsulfonyl)phenyl) pyridino[2,3-d]pyrimidin-2(1H)-one

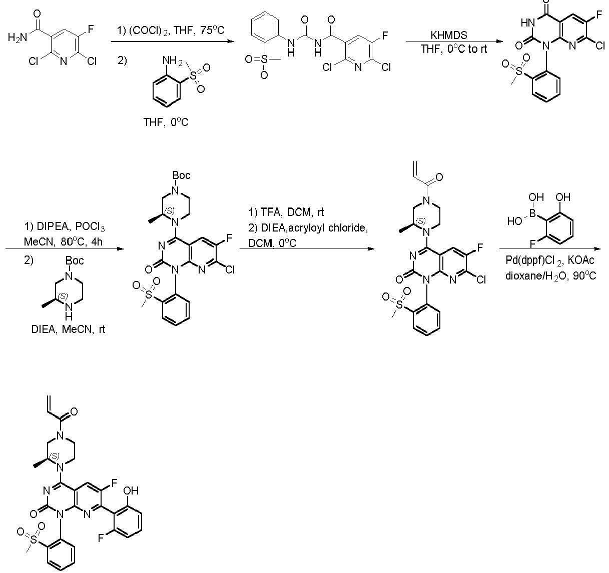

### Step 1. Preparation of 2, 6-dichloro-5-fluoro-N-(((2-(methylsulfonyl) phenyl) carbamoyl) nicotinamide

2, 6-Dichloro-5-fluoronicotinamide (420 mg, 2.0 mmol) was dissolved in anhydrous tetrahydrofuran (7 mL), and a solution of oxalyl chloride (1.7 mL, 20.0 mmol) in dichloromethane (2 mL) was slowly added to the solution. After the addition was completed, the mixture was refluxed and stirred at 75°C for 2h, and then concentrated to dryness under reduced pressure. The residue was diluted with anhydrous tetrahydrofuran (7 mL) and cooled to 0°C. 2-(Methylsulfonyl) aniline (360 mg, 2.1 mmol) was dissolved in anhydrous tetrahydrofuran (3 mL) and then was added dropwise to the above solution. The reaction solution was stirred at 0 °C for 2h, quenched with saturated ammonium chloride/saturated saline (V/V = 1/1,20 mL), and then extracted with dichloromethane/methanol (V/V = 10/1,20 mL) for 3 times. The combined organic phase was dried, concentrated, the residual solid was slurried with petroleum ether/ethyl acetate (V/V = 3/1,15 mL), filtered and dried to obtain the target product (645 mg, yield: 79%).

LC-MS:m/z 406 (M+H)⁺.

### Step 2. Preparation of 7-chloro-6-fluoro-1-(2-(methylsulfonyl) phenyl) pyridino [2,3-d] pyrimidin-2,4(1H,3H)-dione

2, 6-Dichloro-5-fluoro-N-((2-(methylsulfonyl) phenyl) carbamoyl) nicotinamide (645 mg,1.6 mmol) was suspended in tetrahydrofuran (15 mL) and potassium bis (trimethylsilyl) amide (1 M tetrahydrofuran solution, 3.6 mL, 3.6 mmol) was added dropwise under an ice bath. After dropping, the reaction liquid became clear. The reaction solution was stirred at room temperature for 16h, quenched with saturated ammonium chloride (20 mL), and then extracted with ethyl acetate (20 mL) for 3 times. The combined ethyl acetate layer was dried and concentrated. The residual solid was slurried with petroleum ether/ethyl acetate (V/V = 3/1,10 mL), pumped and filtered, and dried to obtain the target product (500 mg, yield: 85%).

LC-MS:m/z 370 (M+H)⁺. ¹H NMR (400 MHz, DMSO-*d*₆) *δ* 12.32 (s, 1H), 8.50 (d, *J* = 7.2 Hz, 1H), 8.15 (dd, *J* = 8.0 Hz, 1.2 Hz, 1H), 7.97-7.92 (m, 1H), 7.86-7.81 (m, 1H), 7.66 (dd, *J=* 8.0 Hz, 1.2 Hz, 1H), 3.09 (s, 3H).

### Step 3. Preparation of (S)-tert-butyl 4-(7-chloro-6-fluoro-1-(2-(methylsulfonyl) phenyl)-2-oxo-1, 2-dihydropyridino [2,3-d] pyrimidin-4-yl)-3-methylpiperazin-1-carboxylic acid

7-Chloro -6-fluoro -1-(2-(methylsulfonyl) phenyl) pyridino[2,3-d]pyrimidin-2,4(1H,3H)-dione (450 mg,1.2 mmol) was suspended in acetonitrile (10mL), and N,N-diisopropylethylamine (1.2 mL,7.3 mmol) and phosphorus oxychloride (0.6 mL) were added dropwise, 6.1 mmol), and the reaction liquid became clear. The reaction solution was stirred at 80 °C for 4h and concentrated to dryness under reduced pressure. The residue was dissolved in acetonitrile (10mL), cooled to 0 °C, N,N-diisopropylethylamine (0.6 mL,3.7 mmol) and tert-butyl(S)-3-methylpiperazin-1-carboxylate (290 mg,1.5 mmol) were added, the reaction solution was stirred at room temperature for 1h, and quenched with semi-saturated sodium bicarbonate solution (40 mL), extracted with ethyl acetate (30 mL) for 3 times. The combined ethyl acetate layer was dried and concentrated, and purified by silica gel column chromatography(petroleum ether: ethyl acetate = 3/1 to 1/2.5) to obtain the target product (460 mg, yield: 68%).

LC-MS:m/z 552 (M+H)⁺.

### Step 4. Preparation of (S)-4-(4-acryloyl -2-methylpiperazin -1-yl) -7-chloro-6-fluoro-1-(2-(methylsulfonyl) phenyl) pyridino [2,3-d] pyrimidin-2(1H)-one

(S)-tert-butyl 4-(7-chloro-6-fluoro-1-(2-(methylsulfonyl) phenyl) -2-oxo-1, 2-dihydropyridino [2,3-d] pyrimidin-4-yl)-3-methylpiperazin-1-carboxylic acid (500 mg,0.9 mmol) was dissolved in dichloromethane (10 mL), and trifluoroacetic acid (2 mL) was added. The reaction solution was stirred at room temperature for 2 hours, concentrated to dryness, and the residue was steamed with dichloromethane (15mL) for 3 times to obtain crude product. The crude product was dissolved in dichloromethane (8 mL), cooled to 0°C, and a solution of N,N-diisopropylethylamine (0.6 mL,3.6 mmol) and acryloyl chloride (110 mg,1.2 mmol) in dichloromethane (1 mL) was added dropwise. The reaction solution was stirred at 0 °C for 30min, quenched with saturated sodium bicarbonate (30 mL), extracted with dichloromethane (20 mL) for 3 times. The dichloromethane layers were combined, dried and concentrated, and the residue was purified by silica gel column chromatography(dichloromethane/methanol = 60/1) to obtain the target product (380 mg, yield: 83%).

LC-MS:m/z 506 (M+H)⁺.

### Step 5. Preparation of 4-((S)-4-acryloyl-2-methylpiperazin-1-yl) -6-fluoro-7-(2-fluoro-6-hydroxyphenyl)-1-(2-(methylsulfonyl) phenyl) pyridino [2,3-d] pyrimidin-2(1H)-one

(S)-4-(4-acroloyl-2-methylpiperazin-1-yl)-7-chloro-6-fluoro-1-(2-(methylsulfonyl) phenyl) pyridino [2,3-d] pyrimidin-2(1H)-one (150 mg, 0.3 mmol), (2-fluoro-6-hydroxyphenyl) boric acid (60 mg,0.4 mmol), [1,1 '-bis (diphenylphosphine) ferrocene] palladium dichloride dichloromethane complex (24 mg, 0.03 mmol) and potassium acetate (120 mg, 1.2 mmol) were suspended in a mixed solvent of dioxane/water (7.5 mL/0.75 mL), and replaced with nitrogen for three times, heated and stirred at 90 °C for 2 hours. After the reaction solution was cooled to room temperature, a semi-saturated sodium bicarbonate solution (20 mL) was added and extracted with ethyl acetate (20 mL) for 3 times. The ethyl acetate layers were combined, dried, concentrated, and the residue was purified by silica gel column chromatography (dichloromethane: methanol = 100:1 to 60:1) to obtain the target product (90 mg, yield: 52%).

LC-MS:m/z 582 (M+H)⁺. ¹H NMR (400 MHz, CDCl₃) δ 8.97-8.94 (m, 1H), 8.27 (d, *J* = 8.0 Hz, 1H), 7.90-7.84 (m, 2H), 7.79-7.74 (m, 1H), 7.46-7.42 (m, 1H), 7.29-7.23 (m, 1H), 6.71-6.57 (m, 3H), 6.44-6.38 (m, 1H), 5.82 (d, *J=* 11.2 Hz, 1H), 5.12-4.32 (m, 3H), 4.07-3.63 (m, 3H), 3.24-3.01 (m, 4H), 1.50 (s, 3H).

### The following compounds were synthesized from different starting materials according to the method of Example 51:

### Example 52 4-((S)-4-acryloyl-2-methylpiperazin-1-yl) -6-fluoro-7-(2-fluoro-6-hydroxyphenyl)-1-(2-(isopropylsulfonyl) phenyl) pyridino[2,3-d] pyrimidin -2(1H)-one

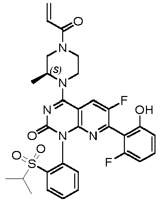

LC-MS:m/z 610 (M+H)⁺. ¹H NMR (400 MHz, CDCl₃) δ 8.91-8.86 (m, 1H), 8.22-8.19 (m, 1H), 7.88-7.82 (m, 2H), 7.75-7.72 (m, 1H), 7.45-7.42 (m, 1H), 7.28-7.23 (m, 1H), 6.71-6.63 (m, 3H), 6.44-6.39 (m, 1H), 5.83-5.80 (m, 1H), 5.04-4.79 (m, 3H), 4.56-3.90 (m, 4H), 3.71-3.48 (m, 1H), 1.50 (s, 3H), 1.33-1.30 (m, 3H), 1.18-1.16 (m, 3H).

### Example 53 4-((S)-4-acryloyl-2-methylpiperazin-1-yl) -6-fluoro-7-(2-fluoro-6-hydroxyphenyl)-1-(2-methyl-6-(methylsulfonyl) phenyl) pyridino[2,3-d] pyrimidin-2(1H)-one

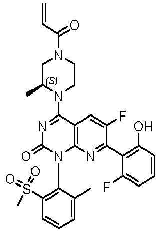

LC-MS:m/z 596 (M+H)⁺. ¹H NMR (400 MHz, CDCl₃) δ 8.12-8.11 (m, 1H), 7.89-7.87 (m, 1H), 7.75-7.63 (m, 2H), 7.26 (m, 1H), 6.69-6.64 (m 3H), 6.44-6.40 (m, 1H), 5.84-5.81 (m, 1H), 5.00-4.40 (m, 3H), 4.07-3.65 (m, 3H), 3.25-3.16 (m, 4H), 2.20-2.18 (m, 3H), 1.58-1.50 (m, 3H).

### Example 53-1 two isomers of Example 53A and Example 53B were obtained by chiral separation:

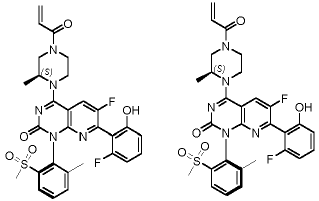

**Example 53A:**LC-MS:m/z 596 (M+H)⁺.

**Example 53B:**LC-MS:m/z 596 (M+H)⁺.

### Example 54 4-((S)-4-acryloyl-2-methylpiperazin-1-yl) -6-fluoro-7-(2-fluoro-6-hydroxyphenyl)-1-( 2-(methylsulfonyl) pyridin-3-yl) pyridino [2,3-d] pyrimidin -2(1H)-one

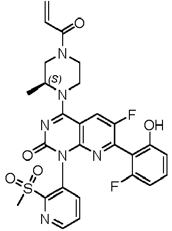

LC-MS: m/z 583 (M+H)⁺. ¹H NMR (400 MHz, CDCl₃) δ 8.80 (dd, *J* = 4.8, 1.2 Hz, 1H), 8.37-8.25 (m, 1H), 8.05 (dd, *J* = 8.0, 1.2 Hz, 1H), 7.90-7.87 (m, 1H), 7.26 (dd, *J* = 14.6, 7.6 Hz, 1H), 6.92 (m, 1H), 6.82-6.65 (m, 2H), 6.23-6.18 (m, 1H), 5.76 (dd, *J* = 6.4, 2.4 Hz, 1H), 4.98-4.82 (m, 1H), 4.44-4.00 (m, 3H), 3.81-3.62 (m, 2H), 3.24-3.00 (m, 4H), 1.34-1.29 (m, 3H).

### Example 55 4-((S)-4-acryloyl-2-methylpiperazin-1-yl) -6-fluoro-7-(2-fluoro-6-hydroxyphenyl)-1-(4-methylsulfonyl) pyridin-3-yl) pyridino [2,3-d] pyrimidin -2(1H)-one

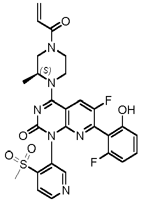

LC-MS:m/z 583 (M+H)⁺. ¹H NMR (400 MHz, CDCl₃) δ 9.06 (d, *J* = 4.2 Hz), 8.75-8.74 (m, 1H), 8.59-8.57 (m, 1H), 8.10 (d, *J* = 4.2 Hz), 7.87 (d, *J* = 11.2 Hz, 1H), 7.30-7.25 (m , 1H), 6.72-6.57 (m, 3H), 6.64-6.58 (m, 1H), 5.84-5.82 (m, 1H), 5.29-4.28 (m, 3H), 4.10-3.61 (m, 3H), 3.22-2.92 (m, 4H0, 1.59-1.48 (m, 3H).

### Example 56 2-(1-acryloyl -4-(6-fluoro-7-(2-fluoro-6-hydroxyphenyl)-1-(2-(methylsulfonyl) phenyl) -2-oxo-1, 2-dihydropyridino [2,3-d] pyrimidin-4-yl) piperazin-2-yl) acetonitrile

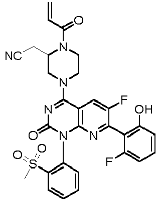

LC-MS: m/z 607 (M+H)⁺. ¹H NMR (400 MHz, CDCl₃) δ 8.29-8.24 (m, 1H), 7.96-7.76 (m, 3H), 7.47-7.43 (m, 1H), 7.29 (m, 1H), 6.70-6.56 (m, 3H), 6.45-6.41 (m, 1H), 5.00 (brs, 1H), 4.56-3.70 (m, 6H), 3.16 (m, 3H), 3.16-3.10 (m,1H), 2.98-2.79 (m, 1H).

### Example 57 2-(1-acryloyl -4-(6-fluoro-7-(2-fluoro-6-hydroxyphenyl)-1-(2-methyl-6-(methylsulfonyl) phenyl) -2-oxo-1, 2-dihydropyridino [2,3-d] pyrimidin-4-yl) piperazin-2-yl) acetonitrile

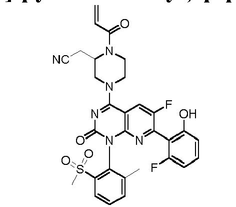

LC-MS: m/z 621 (M+H)⁺. ¹H NMR (400 MHz, CDCl₃) δ 8.12-7.97 (m, 2H), 7.76-7.72 (m, 1H), 7.67-7.64 (m, 1H), 7.29 (m, 1H), 6.69-6.46 (m, 3H), 6.42-6.41 (m, 1H), 5.88-5.86 (m, 1H), 5.00 (brs, 1H), 4.54-3.77 (m, 6H), 3.12 (m, 4H), 2.81-2.77 (m, 1H), 2.20-2.17 (m, 3H).

### Example 58 2-(1-acryloyl -4-(6-fluoro-7-(2-fluoro-6-hydroxyphenyl)-1-(2-(methylsulfonyl) pyridin-3-yl) -2-oxo-1, 2-dihydropyridino [2,3-d] pyrimidin-4-yl) piperazin-2-yl) acetonitrile

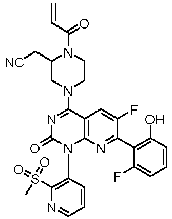

LC-MS: m/z 608 (M+H)⁺. ¹H NMR (400 MHz, CDCl₃) δ 8.82-8.81 (m, 1H), 7.99-7.76 (m, 3H), 7.30 (m, 1H), 6.70-6.56 (m, 3H), 6.44-6.40 (m, 1H), 5.87-5.84 (m, 1H), 5.01 (brs, 1H), 4.61-3.60 (m, 6H), 3.29 (m, 3H), 2.96-2.76 (m, 2H).

### Example 59 4-((S)-4-acryloyl -2-methylpiperazin-1-yl)-7-(2-chloro-6-fluorophenyl) -6-fluoro-1-(2-methyl-6-(methylsulfonyl) phenyl) pyridino [2,3-d] pyrimidin-2(1H)-one

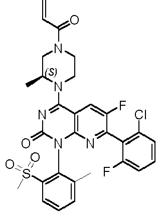

LC-MS: m/z 614 (M+H)⁺. ¹H NMR (400 MHz, CDCl₃) δ 7.95 (d, *J =* 3.6 Hz, 1H), 7.82 (t, *J* = 8.4 Hz, 1H), 7.60 (d, *J* = 3.2 Hz, 1H), 7.50 (t, *J* = 8.4 Hz, 1H), 7.34-7.29 (m, 1H), 7.20 (d, *J* = 8 Hz, 1H), 7.02 (m, 1H), 6.60 (m, 1H), 6.39 (dd, *J* = 1.6 Hz, 17.2 Hz, 1H), 5.80 (dd, *J* = 1.2 Hz, 10.4 Hz, 1H), 5.20-4.20 (m, 3H), 4.10-3.55 (m, 3H), 3.40-3.05 (m, 4H), 2.17-2.15 (m, 3H), 1,.51 (m, 3H).

### Example 60 2-(4-acryloyl-1-(6-fluoro-7-(2-fluoro-6-hydroxyphenyl)-1-(2-(methylsulfonyl) phenyl) -2-oxo-1, 2-dihydropyridino [2,3-d] pyrimidin-4-yl) piperazin-2-yl) acetonitrile

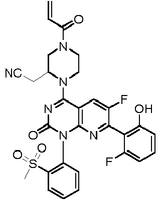

LC-MS: m/z 607 (M+H)⁺. ¹H NMR (400 MHz, CDCl₃) δ 8.87-8.77 (m, 1H), 8.22-8.19 (m, 1H), 7.86-7.80 (m, 3H), 7.69 (m, 1H), 7.37-7.36 (m, 1H), 7.25 (m, 1H), 6.66-6.53 (m, 3H), 6.39 (d, *J=* 17.2 Hz, 1H), 5.81 (d, *J=* 10 Hz, 1H), 5.30-5.20 (m, 1H), 4.80-3.95 (m, 3H), 3.90-3.30 (m, 3H), 3.09-2.88 (m, 5H).

### Example 61 2-(4-acryloyl-1-(6-fluoro-7-(2-fluoro-6-hydroxyphenyl)-1-(2-(methylsulfonyl) phenyl) -2-oxo-1, 2-dihydropyridino [2,3-d] pyrimidin-4-yl) piperazin-2-yl) acetamide

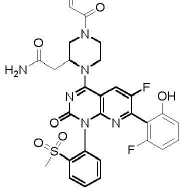

LC-MS: m/z 625 (M+H)⁺. ¹H NMR (400 MHz, CDCl₃) δ 8.20-7.75 (m, 2H), 7.60-7.40 (m, 2H), 7.11-7.01 (m, 2H), 6.84-7.53 (m, 5H), 6.36-6.29 (m, 1H), 5.79 (m, 1H), 5.44-5.23 (m, 1H), 5.50-3.98 (m, 4H), 3.81-3.070 (m, 6H), 2.85-2.63 (m, 2H).

### Example 62 2-(4-((S))-4-acryloyl-2-methylpiperazin-1-yl) -6-fluoro-1-(2-isopropyl-6-(methylsulfonyl) phenyl)-2-oxo-1, 2-dihydropyridino [2,3-d] pyrimidin-7-yl)-3-fluorophenyl cyclopropionate

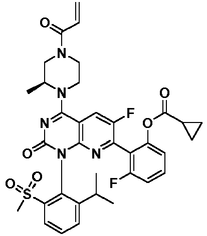

LC-MS : m/z 692 (M+H)⁺.

### Example 62-1 two isomers of Example 62A and Example 62B were obtained by chiral separation:

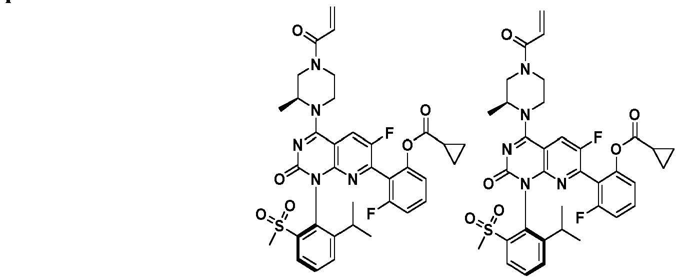

**Example 62A:**LC-MS:m/z 692 (M + H)⁺.

**Example 62B:**LC-MS:m/z 692 (M + H)⁺.

### Example 63 Preparation of 4-((2S,5R)-4-acryloyl -2, 5-dimethylpiperazin-1-yl)-1-(2-cyclobutyl-6-(methylsulfonyl) phenyl)-6-fluoro-7-(2-fluoro-6-hydroxyphenyl) pyridino [2,3-d] pyrimidin-2 (1H)-one

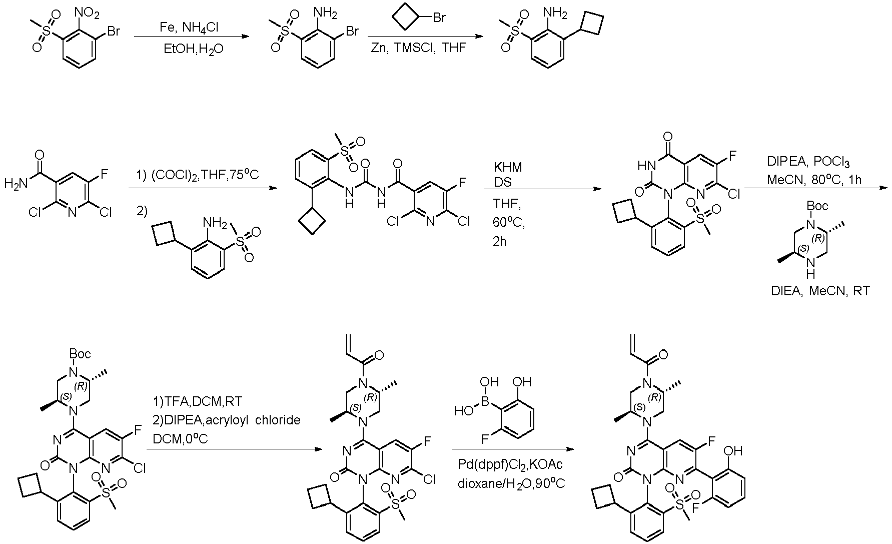

### Step 1. Preparation of 2-bromo-6-(methylsulfonyl) aniline

1-bromo-3-(methylsulfonyl)-2-nitrobenzene (44g,158 mmol), iron powder (44g, 788 mmol), ammonium chloride (83g, 1.6 mol) were suspended in a mixed solvent of ethanol/water (600 mL/120 mL), heated and stirred at 75 °C for 2 hours. After the reaction solution was cooled to room temperature, the solid was removed by suction filtration. 500 mL of water was added to the filtrate, and extracted twice with 500 mL of ethyl acetate. The ethyl acetate layers were combined, washed with 500 mL brine, dried and concentrated, and the residue was separated by silica gel column chromatography(petroleum ether: ethyl acetate = 10:1 to 2:1) to obtain the target product (30g, yield: 77%).

### Step 2. Preparation of 2-cyclobutyl -6-(methylsulfonyl) aniline

Zinc powder (52g, 800 mmol, Acros) was suspended in tetrahydrofuran (100 mL), then trimethylchlorosilane (8.7g, 80 mmol) was added, and heated and stirred at 75 °C for half an hour. Then bromocyclobutane (54g, 400 mmol) was added. The reaction solution was cooled to room temperature, 2-bromo-6-(methylsulfonyl) aniline (10g, 40 mmol) and [1,1 '-bis (diphenylphosphine) ferrocene] palladium dichloride dichloromethane complex (3.3g, 4 mmol) were added, and heated and stirred at 75 °C for three hours. The reaction solution was quenched with 30 mL of 1M phosphoric acid and extracted twice with ethyl acetate. The ethyl acetate layers were combined, dried and concentrated, and the residue was separated by silica gel column chromatography(petroleum ether: ethyl acetate = 10:1 to 2:1) to obtain the target product (8.1g, yield: 90%).

LC-MS: m/z 226 (M+H)⁺.

### Step 3. Preparation of 2, 6-dichloro-N-(((2-cyclobutyl-6-(methylsulfonyl) phenyl) carbamoyl)-5-fluoronicotinamide

2, 6-Dichloro-5-fluoronicotinamide (7.7 g, 37 mmol) was dissolved in 100 mL of anhydrous tetrahydrofuran, and a solution of oxalyl chloride (47g, 370 mmol) was slowly added to the solution. After the addition was completed, the mixture was refluxed and stirred at 75 °C for 2 hours, and then concentrated to dryness under reduced pressure. The residue was diluted with 100 mL of anhydrous tetrahydrofuran and cooled to 0 °C. 2-Cyclobutyl-6-(methylsulfonyl) aniline (8.8g, 39 mmol) was dissolved in 50 mL of anhydrous tetrahydrofuran, and then was added dropwise into the above solution. The reaction solution was stirred at 0 °C for 2 hours, quenched with saturated ammonium chloride/saturated salt water (1:1, 100 mL), and then extracted with ethyl acetate (50 mL) for 2 times. The combined organic phase was dried, concentrated, the residual solid was slurried with petroleum ether/ethyl acetate (5:1, 200 mL), filtered and dried to obtain the target product (12.7 g, yield: 75%).

LC-MS: m/z 460 (M+H)⁺.

### Step 4. Preparation of 7-chloro-1-(2-cyclobutyl-6-(methylsulfonyl) phenyl)-6-fluoropyridino [2,3-d] pyrimidin -2,4(1H,3H)-dione

2, 6-dichloro-N-(((2-cyclobutyl-6-(methylsulfonyl) phenyl) carbamoyl)-5-fluoronicotinamide 5(37.3g, 81.1 mmol) was dissolved in 550 mL of tetrahydrofuran, and potassium bis (trimethylsilyl) amino (1mol/L, 186.5 mL,186.5 mmol) was added dropwise under an ice bath. After dropping, the reaction solution was stirred at 25 °C for 16 hours, quenched with 200 mL of saturated ammonium chloride, and then extracted twice with 1000 mL of ethyl acetate. The combined ethyl acetate layer was dried and concentrated. The residual solid was slurried with petroleum ether/ethyl acetate (3:1, 300 mL), filtered, and dried to obtain the target product (23.2 g, yield: 68%).

LC-MS:m/z 424(M + H)⁺.

### Step 5. Preparation of (2R,5S)-tert-butyl 4-(7-chloro-1-(2-cyclobutyl -6-(methylsulfonyl) phenyl) -6-fluoro-2-oxo-1, 2-dihydropyridino[2,3-d] pyrimidin-4-yl)-2, 5-dimethylpiperazin-1-formate

7-Chloro-1-(2-cyclobutyl-6-(methylsulfonyl) phenyl)-6-fluoropyridino[2,3-d]pyrimidin-2,4(1H,3H)-dione (23.2g, 54.7 mmol) was suspended in 350 mL of acetonitrile, and N,N-diisopropylethylamine (42.3g, 328 mmol) and phosphorus oxychloride (33.5g,219 mmol) were added dropwise. The reaction solution became clear. The reaction solution was stirred at 80 °C for 1 hour and concentrated to dryness under reduced pressure. The residue was dissolved in 350 mL of acetonitrile, cooled to 0 °C, and N,N-diisopropylethylamine (42.3g, 328 mmol) and (2R,5S)-tert-butyl 2, 5-dimethylpiperazin-1-formate (14g, 65.6 mmol) were added. The reaction solution was stirred at room temperature for 1 hour, quenched with semi-saturated sodium bicarbonate solution (400 mL), and then extracted twice with 1000 mL ethyl acetate. The ethyl acetate layers were combined, dried and concentrated, and separated by silica gel column chromatography(petroleum ether: ethyl acetate = 5:1 to 1:1) to obtain the target product (21.2 g, yield: 63 %).

LC-MS: m/z 620 (M+H)⁺.

### Step 6. Preparation of 4-((2S,5R)-4-acryloyl -2, 5-dimethylpiperazin-1-yl)-7-chloro-1-(2-cyclobutyl-6-(methylsulfonyl) phenyl)-6-fluoropyridino[2,3-d] pyrimidin-2(1H)-one

(2R,5S)-tert-butyl 4-(7-chloro-1-(2-cyclobutyl-6-(methylsulfonyl) phenyl)-6-fluoro-2-oxo-1, 2-dihydropyridino [2,3-d]pyrimidin-4-yl)-2, 5-dimethylpiperazin-1-formate (21.2g,34.2 mmol) was dissolved in 200 mL of dichloromethane, and 65 mL of trifluoroacetic acid was added. The reaction solution was stirred at room temperature for 2 hours, and concentrated to dryness. The residue was steamed with 100 mL of dichloromethane for 2 times to obtain the crude product. The crude product was dissolved in 200 mL of dichloromethane, cooled to 0°C, and a solution of triethylamine (17.3g, 171 mmol) and acryloyl chloride (4g, 44.5 mmol) in dichloromethane (20 mL) was added dropwise. The reaction solution was stirred at 0°C for 30 minutes and at room temperature for 30 minutes. The reaction solution was quenched with 200 mL of saturated sodium bicarbonate and extracted twice with 800 mL of dichloromethane. The organic phase was dried and concentrated, and the residue was separated by silica gel column chromatography(dichloromethane: methanol = 60:1) to obtain the target product (13.6g, yield: 69%).

LC-MS: m/z 574 (M+H)⁺.

### Step 7. Preparation of 4-((2S,5R)-4-acryloyl-2, 5-dimethylpiperazin-1-yl)-1-(2-cyclobutyl-6-(methylsulfonyl) phenyl)-6-fluoro-7-(2-fluoro-6-hydroxyphenyl) pyridino [2,3-d] pyrimidin-2 (1H)-one

4-((2S,5R)-4-acryloyl-2, 5-dimethylpiperazin-1-yl)-7-chloro-1-(2-cyclobutyl-6-(methylsulfonyl) phenyl)-6-fluoropyridino [2, 3-d] pyrimidin-2(1H)-one (13.6g, 23.7 mmol), (2-fluoro-6-hydroxyphenyl) boric acid (5.2 g, 33.1 mmol), [1,1 '-bis (diphenylphosphine) ferrocene] palladium dichloride dichloromethane complex (1.94 g, 2.4 mmol) and potassium acetate (9.3 g, 95 mmol) were suspended in a mixed solvent of dioxane/water (200 mL/20 mL), and replaced with nitrogen for three times, heated and stirred at 90 °C for 4 hours. After the reaction solution was cooled to room temperature, 200 mL of semi-saturated sodium bicarbonate solution was added, and extracted with 500 mL of ethyl acetate for 2 times. The ethyl acetate layers were combined, dried, concentrated, and the residue was separated by silica gel column chromatography (dichloromethane: methanol = 100:1 to 50:1) to obtain the target product (8.6 g, yield: 56%).

LC-MS:m/z 650(M + H)⁺. ¹H NMR (400 MHz, CDCl₃) *δ* 9.07-8.93 (m, 1H) ,8.14-8.09 (m, 1H), 7.90-7.67 (m, 3H), 7.29-7.23 (m, 1H), 6.71-6.52 (m, 3H), 6.40 (m, 1H), 5.80 (m, 1H), 5.23-3.30 (m, 6H), 3.15-3.08 (m, 3H), 2.33-2.20 (m, 1H), 2.17-2.05 (m, 1H), 1.96-1.65(m, 3H), 1.60-1.32 (m, 8H).

### Example 63-1 two isomers of Example 63A and 63B were obtained by chiral separation:

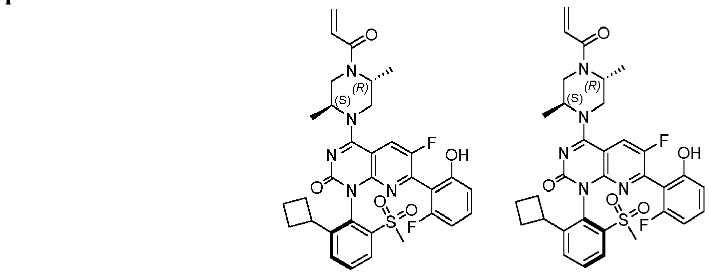

### Compound 63A

LC-MS: m/z 650 (M+H)⁺. ¹H NMR (400 MHz, DMSO) δ 10.21 (s, 1H), 8.32 (d, *J* = 9.1 Hz, 1H), 7.88 (d, *J* = 7.7 Hz, 1H), 7.75 - 7.59 (m, 2H), 7.27 (m, 1H), 6.93 - 6.60 (m, 3H), 6.19 (m, 1H), 5.74 (m, 1H), 4.93 - 4.38 (m, 2H), 4.23 - 3.40 (m, 3H), 3.30 - 3.23 (m, 1H), 2.99 (s, 3H), 2.18 - 1.87 (m, 3H), 1.85 - 1.52 (m, 3H), 1.38 - 1.10 (m, 7H).

### Compound 63B

LC-MS: m/z 650 (M+H)⁺. ¹H NMR (400 MHz, DMSO) δ 10.23 (s, 1H), 8.34 (m, 1H), 7.89 (d, *J=* 7.7 Hz, 1H), 7.77 (m, 1H), 7.66 (t, *J=* 7.8 Hz, 1H), 7.27 (m, 1H), 6.95 - 6.60 (m, 3H), 6.19 (m, 1H), 5.75 (m, 1H), 4.93 - 4.38 (m, 2H), 4.18 - 3.45 (m, 3H), 3.28 (m, 1H), 2.96 (s, 3H), 2.15 - 1.86 (m, 3H), 1.86 - 1.56 (m, 3H), 1.35 - 0.98 (m, 7H).

### The following compounds were synthesized from different starting materials according to the method of Example 63:

### Example 64 (S)-3-(4-(4-acryloyl-2-methylpiperazin-1-yl) -6-fluoro-1-(2-isopropyl-6-(methylsulfonyl) phenyl)-2-oxo-1, 2-dihydropyridino[2,3-d] pyrimidin-7-yl)-4-chlorobenzonitrile

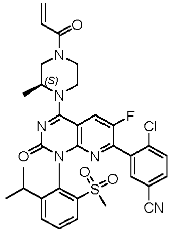

LC-MS: m/z 649 (M+H)⁺. ¹H NMR (400 MHz, CDCl₃) δ 7.99 (d, *J=* 7.6 Hz, 1H), 7.84 (dd, *J* = 8.8 Hz, 6.4 Hz, 1H), 7.72 (d, *J=* 7.6 Hz, 1H), 7.64-7.60 (m, 2H), 7.53-7.48 (m, 2H), 6.61 (s, 1H), 6.40 (d, *J=* 16.8 Hz, 1H), 5.80 (d, *J=* 12.0 Hz, 1H), 5.06-3.62 (m, 6H), 3.33-3.01 (m, 4H), 2.80 (s, 1H), 1.51 (s, 3H), 1.24 (d, *J=* 6.8 Hz, 3H), 1.03 (d, *J=* 6.8 Hz, 1H).

### Example 65 (S)-3-(4-(4-acryloyl-2-methylpiperazin-1-yl) -6-fluoro-1-(2-isopropyl-6-(methylsulfonyl) phenyl)-2-oxo-1, 2-dihydropyridino[2,3-d] pyrimidin-7-yl)-2-chlorobenzonitrile

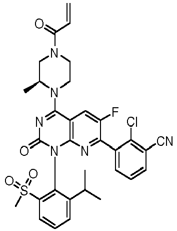

LC-MS: m/z 649 (M+H)⁺. ¹H NMR (400 MHz, CDCl₃) δ 8.34 (d, *J=* 2.4 Hz, 1H), 8.07-7.98 (m, 2H), 7.91-7.63 (m, 5H), 7.43 (t, *J* = 7.6 Hz, 1H), 7.03-6.81 (m, 1H), 5.10-3.51 (m, 6H), 3.44-3.14 (m, 1H), 3.06 (s, 3H), 2.78-2.58 (m, 1H), 1.56-1.45 (m, 3H), 1.26-1.18 (m, 3H), 1.13-1.05 (m, 3H).

### Example 66 (S)-4-(4-(4-acryloyl-2-methylpiperazin-1-yl) -6-fluoro-1-(2-isopropyl-6-(methylsulfonyl) phenyl)-2-oxo-1, 2-dihydropyridino [2,3-d] pyrimidin-7-yl)-3-chlorobenzonitrile

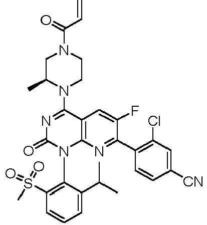

LC-MS: m/z 649 (M+H)⁺. ¹H NMR (400 MHz, CDCl₃) δ 8.34 (d, *J* = 2.0 Hz, 1H), 8.07-7.99 (m, 2H), 7.82-7.77 (m, 1H), 7.76-7.64 (m, 4H), 7.59 (d, *J* = 8.4 Hz, 1H), 7.05-6.84 (m, 1H), 5.10-3.12 (m, 7H), 3.06 (s, 3H), 2.79-2.58 (m, 1H), 1.56-1.41 (m, 3H), 1.26-1.18 (m, 3H), 1.13-1.05 (m, 3H).

### Example 67 2-(4-((S))-4-acryloyl-2-methylpiperazin-1-yl) -6-fluoro-1-(2-isopropyl-6-(methylsulfonyl) phenyl)-2-oxo-1, 2-dihydropyridino [2,3-d] pyrimidin-7-yl)-3-chlorobenzonitrile

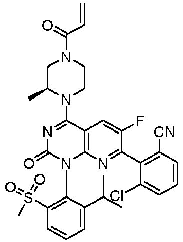

LC-MS: m/z 649 (M+H)⁺. ¹H NMR (400 MHz, CDCl₃) δ 8.34 (d, *J* = 2.4 Hz, 1H), 8.03 (d, *J* = 8.0 Hz, 1H), 7.94 (d, *J=* 15.6 Hz, 1H), 7.79 (d, *J* = 7.2 Hz, 1H), 7.76-7.63 (m, 4H), 7.41 (t, *J* = 8.0 Hz, 1H), 7.25-7.16 (m, 1H), 5.01-3.60 (m, 6H), 3.38-3.10 (m, 1H), 3.07 (s, 3H), 2.78-2.62 (m, 1H), 1.56-1.46 (m, 3H), 1.26-1.18 (m, 3H), 1.13-1.05 (m, 3H).

### Example 68 (S)-4-(4-acryloyl-2-methylpiperazin-1-yl) -6-fluoro-1-(2-isopropyl-6-(methylsulfonyl) phenyl)-7-(1-methyl-1H-pyrazol-5-yl) pyridino [2,3-d] pyrimidin-2(1H)-one

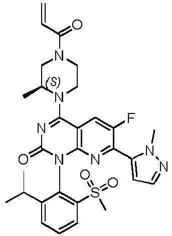

LC-MS: m/z 594 (M+H)⁺. ¹H NMR (400 MHz, CDCl₃) δ 8.05 (d, *J=* 7.8 Hz, 1H), 7.82-7.78 (m, 2H), 7.73 (t, *J=* 7.8 Hz, 1H), 7.47 (d, *J* = 1.8 Hz, 1H), 6.94 (s, 1H), 6.59 (d, *J* = 17.9 Hz, 1H), 6.42 (dd, *J=* 16.7, 1.6 Hz, 1H), 5.81 (d, *J=* 10.4 Hz, 1H), 5.01-3.67 (m, 7H), 3.61 (s, 3H), 3.19 (s, 3H), 2.75 (s, 1H), 1.52 (s, 3H), 1.28-1.23 (m, 3H), 1.04 (d, *J* = 5.8 Hz, 3H).

### Example 69 (S)-4-(4-acryloyl-2-methylpiperazin-1-yl) -6-fluoro-1-(2-isopropyl-6-(methylsulfonyl) phenyl)-7-(1H-pyrazol-5-yl) pyridino[2,3-d] pyrimidin-2(1H)-one

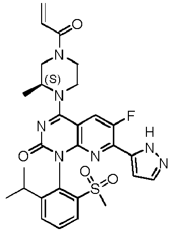

LC-MS:m/z 580(M + H)⁺. H NMR (400 MHz, CDCl₃) δ 8.07 (d, *J* = 7.8 Hz, 1H), 7.86-7.77 (m, 2H), 7.73 (t, *J* = 7.8 Hz, 1H), 7.61 (d, *J* = 1.8 Hz, 1H), 6.83 (s, 1H), 6.58 (d, *J* = 17.9 Hz, 1H), 6.40 (dd, *J* = 16.7 Hz, 1.6 Hz, 1H), 5.81 (d, *J* = 10.4 Hz, 1H), 5.01-3.67 (m, 6H), 3.09 (s, 4H), 2.75 (s, 1H), 1.52 (s, 3H), 1.28-1.23 (m, 3H), 1.04 (d, *J* = 5.8 Hz, 3H).

### Example 70 (S)-4-(4-acryloyl-2-methylpiperazin-1-yl) -6-fluoro-1-(2-isopropyl-6-(methylsulfonyl) phenyl)-7-(1-methyl-1H-imidazol-5-yl) pyridino [2,3-d] pyrimidin-2 (1H)-one

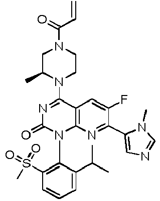

LC-MS: m/z 594 (M+H)⁺. ¹H NMR (400 MHz, CDCl₃) δ 8.34 (d, *J* = 2.4 Hz, 1H), 8.03 (d, *J* = 8.0 Hz, 1H), 7.79 (d, *J* = 7.6 Hz, 1H), 7.76-7.60 (m, 3H), 7.57-7.45 (m, 2H), 6.86-6.63 (m, 1H), 5.06-3.12 (m, 10H), 3.07 (s, 3H), 2.78-2.59 (m, 1H), 1.56-1.41 (m, 3H), 1.26-1.18 (m, 3H), 1.13-1.05 (m, 3H).

### Example 71 (S)-4-(4-acryloyl-2-methylpiperazin-1-yl) -6-fluoro-1-(2-isopropyl-6-(methylsulfonyl) phenyl)-7-(2-oxo-1, 2-dihydropyridin-3-yl) pyridino[2,3-d] pyrimidin-2 (1H)-one

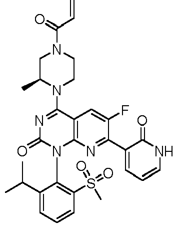

LC-MS: m/z 607 (M+H)⁺. ¹H NMR (400 MHz, CDCl₃) δ 11.34 (s, 1H), 8.33 (d, *J* = 2.8 Hz, 1H), 8.06-7.88 (m, 2H), 7.79 (d, *J* = 7.6 Hz, 1H), 7.76-7.60 (m, 3H), 7.56 (d, *J* = 15.2 Hz, 1H), 7.37 (d, *J* = 6.0 Hz, 1H), 6.39 (t, *J* = 6.4 Hz, 1H), 5.01-3.52 (m, 7H), 3.07 (s, 3H), 2.78-2.62 (m, 1H), 1.56-1.46 (m, 3H), 1.26-1.18 (m, 3H), 1.13-1.05 (m, 3H).

### Example 72 (S)-4-(4-acryloyl-2-methylpiperazin-1-yl) -6-fluoro-1-(2-isopropyl-6-(methylsulfonyl) phenyl)-7-(6-oxo-1, 6-dihydropyridin-2-yl) pyridino[2,3-d] pyrimidin-2 (1H)-one

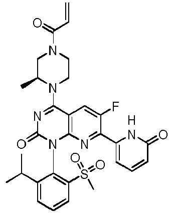

LC-MS: m/z 607 (M+H)⁺. ¹H NMR (400 MHz, CDCl₃) δ 13.26 (brs, 1H), 8.35 (d, *J* = 2.4 Hz, 1H), 8.04 (d, *J* = 8.0 Hz, 1H), 7.87-7.66 (m, 4H), 7.56-7.52 (m, 1H), 7.42-7.37 (m, 1H), 6.52 (d, *J* = 9.2 Hz, 1H), 6.46 (d, *J* = 6.8 Hz, 1H), 5.14-4.24 (m, 4H), 3.80-3.66 (m, 2H), 3.33-3.08 (m, 4H), 2.73-2.68 (m, 1H), 1.56-1.50 (m, 3H), 1.26-1.18 (m, 3H), 1.13-1.05 (m, 3H).

### Example 73 (S)-4-(4-acryloyl-2-methylpiperazin-1-yl) -6-fluoro-1-(2-isopropyl-6-(methylsulfonyl) phenyl)-7-(1-methyl-2-oxo-1,2-dihydropyridin-3-yl) pyridino[2,3-d] pyrimidin-2 (1H)-one

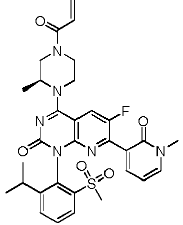

LC-MS: m/z 621(M+H)⁺. ¹H NMR (400 MHz, CDCl₃) δ 8.33 (d, *J* = 2.4 Hz, 1H), 8.11-7.94 (m, 2H), 7.90 (d, *J* = 8.0 Hz, 1H), 7.75-7.63 (m, 2H), 7.59-7.51 (m, 2H), 7.38 (d, *J* = 6.4 Hz, 1H), 6.30 (t, *J* = 6.8 Hz, 1H), 4.99-4.02 (m, 4H), 3.77-3.48 (m, 5H), 3.31-3.10 (m, 1H), 3.07 (s, 3H), 2.78-2.62 (m, 1H), 1.55-1.43 (m, 3H), 1.26-1.18 (m, 3H), 1.13-1.05 (m, 3H).

### Example 74 (S)-4-(4-acryloyl-2-methylpiperazin-1-yl) -6-fluoro-1-(2-isopropyl-6-(methylsulfonyl) phenyl)-7-(1-methyl-6-oxo-1, 6-dihydropyridin-2-yl) pyridino[2,3-d] pyrimidin-2 (1H)-one

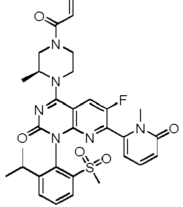

LC-MS: m/z 621(M+H)⁺.¹H NMR (400 MHz, CDCl₃) δ 8.35 (d, *J* = 2.4 Hz, 1H), 8.03 (d, *J* = 7.6 Hz, 1H), 7.80 (d, *J* = 7.2 Hz, 1H), 7.76-7.65 (m, 3H), 7.31 (t, *J* = 8.0 Hz, 1H), 6.94-6.74 (m, 1H), 6.63 (d, *J* = 9.2 Hz, 1H), 6.46-6.35 (m, 1H), 5.10-3.64 (m, 6H), 3.60 (s, 3H), 3.45-3.09 (m, 1H), 3.06 (s, 3H), 2.78-2.62 (m, 1H), 1.59-1.43 (m, 3H), 1.23 (d, *J* = 6.8 Hz, 3H), 1.10 (d, *J* = 5.6 Hz, 3H).

### Example 75 4-((S))-4-acryloyl-methylpiperazin-1-yl)-7-(6-chloro-1H-indazol-7-yl) -6-fluoro-1-(2-isopropyl-6-(methylsulfonyl) phenyl) pyridino [2,3-d] pyrimidin -2(1H)-one

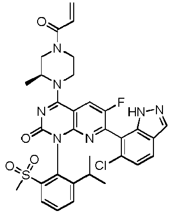

LC-MS: m/z 664 (M+H)⁺. ¹H NMR (400 MHz, CDCl₃) δ 12.99 (brs, 1H), 8.57-8.20 (m, 3H), 8.02 (d, *J* = 7.2 Hz, 1H), 7.86-7.72 (m, 2H), 7.67 (t, *J* = 8.0 Hz, 1H), 7.59 (d, *J* = 7.6 Hz, 1H), 7.23-7.03 (m, 2H), 5.19-3.24 (m, 7H), 3.06 (s, 3H), 2.78-2.58 (m, 1H), 1.60-1.42 (m, 3H), 1.30-1.15 (m, 3H), 1.14-0.99 (m, 3H).

### Example 76 4-((S)-4-acryloyl-methylpiperazin-1-yl)-7-(1, 4-dimethyl-1H-imidazol-5-yl)-6-fluoro-1-(2-isopropyl-6-(methylsulfonyl) phenyl) pyridino [2,3-d] pyrimidin-2 (1H)-one

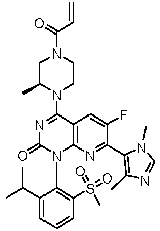

LC-MS: m/z 608 (M+H)⁺. ¹H NMR (400 MHz, CDCl₃) δ 8.34 (d, *J* = 2.4 Hz, 1H), 8.03 (d, *J* = 8.0 Hz, 1H), 7.79 (d, *J* = 6.8 Hz, 1H), 7.76-7.64 (m, 3H), 7.43 (s, 1H), 6.61-6.44 (m, 1H), 5.10-3.37 (m, 10H), 3.07 (s, 3H), 2.78-2.59 (m, 1H), 2.42 (s, 3H), 1.56-1.41 (m, 3H), 1.28-1.18 (m, 3H), 1.16-1.02 (m, 3H).

### Example 77 methyl 2-(4-((S))-4-acryloyl-2-methylpiperazin-1-yl) -6-fluoro-1-(2-isopropyl-6-(methylsulfonyl) phenyl)-2-oxo-1, 2-dihydropyridino[2,3-d] pyrimidin-7-yl)-fluorobenzoate

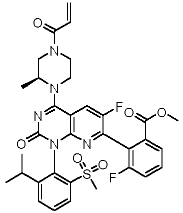

LC-MS: m/z 666 (M+H)⁺. ¹H NMR (400 MHz, CDCl₃) δ 8.34 (d, *J* = 2.4 Hz, 1H), 8.04-7.96 (m, 2H), 7.80-7.65 (m, 4H), 7.41-7.36 (m, 1H), 7.33-7.26 (m, 1H), 7.00-6.87 (m, 1H), 4.97-4.14 (m, 4H), 3.94 (s, 3H), 3.76-3.65 (m, 2H), 3.30-3.07 (m, 4H), 2.71 (s, 1H), 1.55-1.49 (m, 3H), 1.29-1.23 (m, 3H), 1.11-1.08 (m, 3H).

### Example 78 Preparation of 4-((2S,5R)-4-acryloyl -2, 5-dimethylpiperazin-1-yl)-1-(2-isopropyl-6-(methylsulfonyl) phenyl)-6-fluoro-7-(2-fluoro-6-hydroxyphenyl) pyridino [2,3-d] pyrimidin-2(1H)-one

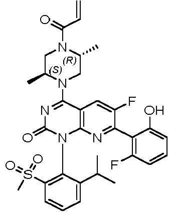

LC-MS:m/z 638 (M+H)⁺.

### Example 78-1 two isomers of Example 78A and 78B were obtained by chiral separation:

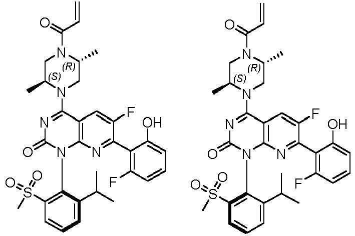

### Example 78A

LC-MS: m/z 638 (M+H)⁺. ¹H NMR (400 MHz, DMSO) δ 10.21 (brs, 1H), 8.28 (m, 1H), 7.85 (m, 2H), 7.65 (t, *J* = 7.8 Hz, 1H), 7.26 (m, 1H), 6.95 - 6.58 (m, 3H), 6.19 (m, 1H), 5.82 - 5.69 (m, 1H), 4.96 - 4.41 (m, 2H), 4.30 - 3.38 (m, 4H), 2.95 (s, 3H), 2.72 - 2.55 (m, 1H), 1.40 - 0.93 (m, 12H).

### Example 78B

LC-MS: m/z 638 (M+H)⁺. ¹H NMR (400 MHz, DMSO) δ 10.28 (brs, 1H), 8.51 - 8.33 (m, 1H), 8.00 - 7.81 (m, 2H), 7.70 (t, *J=* 7.8 Hz, 1H), 7.31 (m, 1H), 7.00 - 6.65 (m, 3H), 6.24 (m, 1H), 5.80 (m, 1H), 5.02 - 4.46 (m, 2H), 4.27 - 3.57 (m, 4H), 2.97 (s, 3H), 2.70 - 2.58 (m, 1H), 1.43 - 0.97 (m, 12H).

### Example 79 4-((2S,5R)-4-acryloyl -2, 5-dimethylpiperazin-1-yl)-6-chloro-7-(2-fluoro-6-hydroxyphenyl)-1-(2-isopropyl-6-(m ethylsulfonyl) phenyl) pyridino[2,3-d] pyrimidin-2 (1H)-one

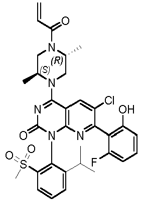

LC-MS:m/z 654 (M+H)⁺.

### Example 79-1 two isomers of Example 79A and 79B were obtained by chiral separation:

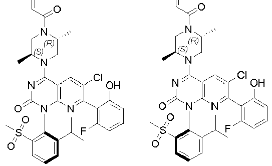

### Example 79A

LC-MS: m/z 638 (M+H)⁺. ¹H NMR (400 MHz, CDCl₃) δ 8.11 (t, *J* = 7.0 Hz, 2H), 7.78 (d, *J=* 6.8 Hz, 1H), 7.68 (t, *J=* 7.8 Hz, 1H), 7.26 - 7.20 (m, 1H), 6.78 - 6.49 (m, 3H), 6.40 (m, 1H), 5.81 (m, 1H), 5.11 (m, 2H), 4.53 - 4.19 (m, 1H), 4.17 - 3.80 (m, 2H), 3.61 (m, 1H), 3.13 (d, *J* = 5.6 Hz, 3H), 2.81 - 2.61 (m, 1H), 1.52 - 0.94 (m, 12H).

### Example 79B

LC-MS: m/z 638 (M+H)⁺. ¹H NMR (400 MHz, DMSO) δ 10.12 (brs, 1H), 8.48 (s, 1H), 7.92 - 7.75 (m, 2H), 7.65 (t, *J=* 7.8 Hz, 1H), 7.22 (m, 1H), 6.92 - 6.53 (m, 3H), 6.19 (m, 1H), 5.84 - 5.69 (m, 1H), 4.70 (m, 2H), 4.19 - 3.47 (m, 4H), 2.90 (s, 3H), 2.57 (m, 1H), 1.37 - 0.90 (m, 12H).

### Example 80 4-((S)-4-acryloyl-2-methylpiperazin-1-yl) -6-fluoro-7-(2-fluoro-6-hydroxyphenyl)-1-(2-isopropyl-6-(isopropylsulfonyl) phenyl) pyridino [2,3-d] pyrimidin-2 (1H)-one

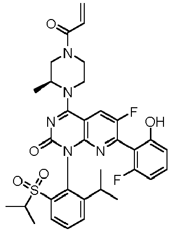

LC-MS: m/z 652 (M+H)⁺. ¹H NMR (400 MHz, CDCl₃) δ 8.91 (brs, 1H), 8.04 (d, *J* = 7.2 Hz, 1H), 7.87 (dd, *J* = 9.2 Hz, 3.2 Hz, 1H), 7.81 (dd, *J* = 8.0 Hz, 1.2 Hz, 1H), 7.72-7.67 (m, 1H), 7.26 (overlap, 1H), 6.69-6.54 (m, 3H), 6.42 (dd, *J* = 16.8 Hz, 1.6 Hz, 1H), 5.81 (dd, *J* = 10.8 Hz, 1.6 Hz, 1H), 5.10-3.40 (m, 7H), 3.30-3.00 (m, 1H), 2.75-2.60 (m, 1H), 1.50-1.48 (m, 3H), 1.31-1.24 (m, 6H), 1.17-1.15 (m, 3H), 1.02-0.99 (m, 3H).

### Example 80-1 two isomers of Example 80A and 80B were obtained by chiral separation:

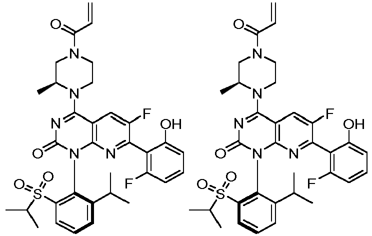

### Example 80A

LC-MS: m/z 652 (M+H)⁺. ¹H NMR (400 MHz, DMSO) δ 10.19 (brs, 1H), 8.28 (t, *J* = 9.7 Hz, 1H), 7.90 - 7.76 (m, 2H), 7.64 (t, *J=* 7.8 Hz, 1H), 7.26 (m, 1H), 6.97 - 6.58 (m, 3H), 6.21 (m, 1H), 5.77 (m, 1H), 4.86 (m, 1H), 4.49 - 3.96 (m, 3H), 3.83 - 3.48 (m, 2H), 3.28 - 3.02 (m, 2H), 2.59 (m, 1H), 1.34 (m, 3H), 1.18 - 0.89 (m, 12H).

### Example 80B

LC-MS: m/z 652 (M+H)⁺. ¹H NMR (400 MHz, DMSO) δ 10.21 (brs, 1H), 8.38 (s, 1H), 7.82 (m, 2H), 7.74 - 7.58 (m, 1H), 7.26 (m, 1H), 7.05 - 6.58 (m, 3H), 6.22 (m, 1H), 5.88 - 5.67 (m, 1H), 4.92 (m, 1H), 4.63 - 3.95 (m, 3H), 3.74 (m, 2H), 3.29 (m, 1H), 3.05 (m, 1H), 2.62 (m, 1H), 1.35 - 1.19 (m, 3H), 1.18 - 0.82 (m, 12H).

### Example 81 4-((2S,5R)-4-acryloyl-2, 5-dimethylpiperazin-1-yl)-1-(2-cyclopropyl-6-(methylsulfonyl) phenyl)-6-fluoro-7-(2-fluoro-6-hydroxyphenyl) pyridino [2,3-d] pyrimidin-2(1H)-one

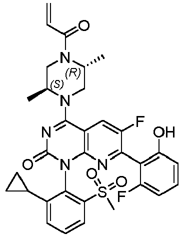

LC-MS: m/z 636(M+H)⁺. ¹H NMR (400 MHz, CDCl₃) δ 9.16-8.90 (m, 1H), 8.14-8.02 (m, 1H), 7.92-7.82 (m, 1H), 7.65 (t, *J* = 8.0 Hz, 1H), 7.54-7.41 (m, 1H), 7.30-7.21 (m, 1H), 6.73-6.50 (m, 3H), 6.40 (t, *J* = 15.2 Hz, 1H), 5.81 (t, *J* = 8.8 Hz, 1H), 5.18-3.67 (m, 6H), 3.18-3.07 (m, 3H), 1.53-1.19 (m, 7H), 0.93-0.53 (m, 4H).

### Example 81-1 two isomers of Example 81A and 81B were obtained by chiral separation:

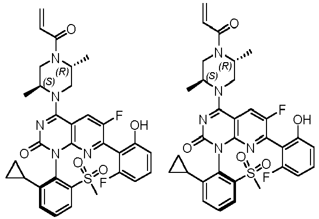

### Example 81A

LC-MS: m/z 636 (M+H)⁺. ¹H NMR (400 MHz, DMSO) δ 10.22 (brs, 1H), 8.31 (d, *J* = 90 Hz, 1H), 7.86 (d, *J* = 7.6 Hz, 1H), 7.58 (t, *J* = 7.8 Hz, 1H), 7.54 - 7.44 (m, 1H), 7.26 (dd, *J=* 15.3, 8.0 Hz, 1H), 6.96 - 6.52 (m, 3H), 6.19 (m, 1H), 5.79 - 5.65 (m, 1H), 4.97 - 4.36 (m, 2H), 4.16 (m, 1H), 3.95 - 3.41 (m, 3H), 3.00 (s, 3H), 1.67 - 1.46 (m, 1H), 1.24 (m, 6H), 0.75 - 0.43 (m, 4H).

### Example 81B

LC-MS: m/z 636 (M+H)⁺. ¹H NMR (400 MHz, DMSO) δ 10.29 (brs, 1H), 8.34 (dd, *J* = 8.9, 4.0 Hz, 1H), 7.85 (d, *J* = 7.5 Hz, 1H), 7.58 (t, *J* = 7.8 Hz, 1H), 7.43 (d, *J* = 7.7 Hz, 1H), 7.26 (dd, *J* = 15.4, 8.0 Hz, 1H), 6.95 - 6.55 (m, 3H), 6.19 (m, 1H), 5.75 (m, 1H), 4.74 (m, 2H), 4.22 - 3.51 (m, 4H), 2.96 (s, 3H), 1.64 - 1.48 (m, 1H), 1.25 (m, 6H), 0.63 (m, 4H).

### Example 82 4-((2S,5R)-4-acryloyl-2, 5-dimethylpiperazin-1-yl)-1-(2-ethyl-6-(isopropylsulfonyl) phenyl)-6-fluoro-7-(2-fluoro-6-hydroxyphenyl) pyridino[2,3-d] pyrimidin-2(1H)-one

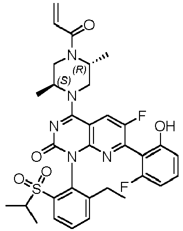

LC-MS: m/z 652 (M+H)⁺. ¹H NMR (400 MHz, CDCl₃) δ 9.10-8.09 (m, 1H), 8.06-8.03 (m, 1H), 7.88-7.85 (m, 1H), 7.80-7.76 (m, 1H), 7.70-7.65 (m, 1H), 7.28-7.22 (m, 1H), 6.70-6.55 (m, 3H), 6.39 (t, *J=* 13.6 Hz, 1H), 5.80-5.78 (m, 1H), 5.07-3.40 (m, 7H), 2.52-2.49 (m, 1H), 2.36-2.33 (m, 1H), 1.50-1.41 (m, 3H), 1.40-1.29 (m, 6H), 1.27-1.17 (m, 6H).

### Example 82-1 two isomers of Example 82A and 82B were obtained by chiral separation:

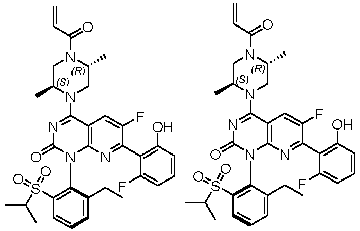

### Example 82A

LC-MS: m/z 652 (M+H)⁺. ¹H NMR (400 MHz, DMSO) δ 10.16 (brs, 1H), 8.29 - 8.11 (m, 1H), 7.78 (t, *J* = 7.0 Hz, 2H), 7.63 (t, *J* = 7.8 Hz, 1H), 7.27 (m, 1H), 6.96 - 6.58 (m, 3H), 6.19 (m, 1H), 5.75 (m, 1H), 4.66 (m, 2H), 4.46 - 4.13 (m, 2H), 3.94 - 3.57 (m, 2H), 3.23 (m, 1H), 2.31 (dd, *J=* 14.7, 7.2 Hz, 2H), 1.38 (d, *J=* 6.5 Hz, 3H), 1.22 (m, 3H), 1.10 - 0.86 (m, 9H).

### Example 82B

LC-MS: m/z 652 (M+H)⁺. ¹H NMR (400 MHz, DMSO) δ 10.13 (brs, 1H), 8.37 (t, *J* = 8.2 Hz, 1H), 7.79 (t, *J=* 6.4 Hz, 2H), 7.64 (t, *J=* 7.7 Hz, 1H), 7.26 (m, 1H), 6.93 - 6.57 (m, 3H), 6.19 (m, 1H), 5.75 (m, 1H), 5.05 - 4.31 (m, 2H), 4.21 - 3.52 (m, 4H), 3.17 - 2.99 (m, 1H), 2.30 (d, *J=* 6.9 Hz, 2H), 1.40 - 0.84 (m, 15H).

### Example 83 4-((S)-4-acryloyl-2-methylpiperazin-1-yl)-1-(2-ethyl-6-(isopropylsulfonyl) phenyl)-6-fluoro-7-(2-fluoro-6-hydroxyphenyl) pyridino [2,3-d]pyrimidin -2(1H)-one

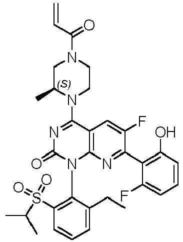

LC-MS: m/z 638 (M+H)⁺. ¹H NMR (400 MHz, CDCl₃) δ 8.96 (brs, 1H), 8.05 (d, *J=* 7.6 Hz, 1H), 7.89-7.85 (m, 1H), 7.78 (d, *J* = 8 Hz, 1H), 7.70-7.68 (m, 1H), 7.28-7.22 (m, 1H), 6.70-6.61 (m, 3H), 6.43-6.39 (m, 1H), 5.83-5.80 (m, 1H), 5.00-3.06 (m, 8H), 2.58-2.50 (m, 1H), 2.39-2.35 (m, 1H), 1.33-1.28 (m, 6H), 1.26-1.15 (m, 6H).

### Example 83-1 two isomers of Example 83A and 83B were obtained by chiral separation:

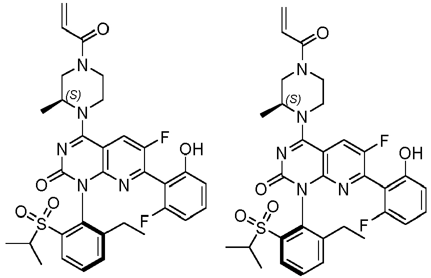

### Example 83A

LC-MS: m/z 638 (M+H)⁺. ¹H NMR (400 MHz, DMSO) δ 10.19 (brs, 1H), 8.28 (d, *J* = 8.8 Hz, 1H), 7.78 (t, *J* = 6.9 Hz, 2H), 7.63 (t, *J* = 7.7 Hz, 1H), 7.26 (m, 1H), 6.75 (m, 3H), 6.21 (m, 1H), 5.77 (m, 1H), 4.84 (s, 1H), 4.57 - 3.99 (m, 3H), 3.78 - 3.49 (m, 2H), 3.19 (m, 2H), 2.41 - 2.18 (m, 2H), 1.36 (d, *J* = 6.4 Hz, 3H), 1.12 - 0.81 (m, 9H).

### Example 83B

LC-MS: m/z 638 (M+H)⁺. ¹H NMR (400 MHz, DMSO) δ 9.31 (br, 1H), 8.35 (s, 1H), 7.84 - 7.70 (m, 2H), 7.63 (t, *J* = 7.8 Hz, 1H), 7.25 (m, 1H), 6.96 - 6.55 (m, 3H), 6.21 (m, 1H), 5.77 (m, 1H), 4.90 (m, 1H), 4.59 - 3.94 (m, 3H), 3.66 (m, 2H), 3.07 (m, 2H), 2.39 - 2.23 (m, 2H), 1.31 - 1.15 (m, 3H), 1.15 - 0.89 (m, 9H).

### Example 84 4-((2S,5R)-4-acryloyl -2, 5-dimethylpiperazin-1-yl) -6-chloro-1-(2-ethyl-6-(methylsulfonyl) phenyl)-7-(2-fluoro-6-hydroxyphenyl) pyridino [2,3-d] pyrimidin-2(1H)-one

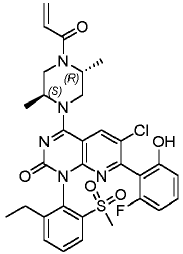

LC-MS: m/z 640 (M+H)⁺. ¹H NMR (400 MHz, CDCl₃) δ 8.19-8.06 (m, 2H), 8.06-7.83 (m, 1H), 7.75 (t, *J* = 8.0 Hz, 1H), 7.66 (dt, *J* = 8.0 Hz, 2.0 Hz, 1H), 7.26-7.20 (m, 1H), 6.77-6.50 (m, 3H), 6.40 (t, *J* = 15.2 Hz, 1H), 5.81 (t, *J* = 8.8 Hz, 1H), 5.21-3.64 (m, 6H), 3.17-3.05 (m, 3H), 2.60-2.31 (m, 2H), 1.55-1.34(m, 6H), 1.22-1.11(m, 3H).

### Example 84-1 two isomers of Example 84A and 84B were obtained by chiral separation:

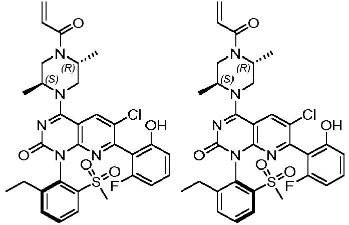

### Example 84A

LC-MS: m/z 640 (M+H)⁺. ¹H NMR (400 MHz, DMSO) δ 10.20 (brs, 1H), 8.42 (d, *J* = 10.9 Hz, 1H), 7.88 (d, *J* = 7.7 Hz, 1H), 7.73 (s, 1H), 7.60 m, 1H), 7.22 (m, 1H), 6.94 - 6.52 (m, 3H), 6.19 (m, 1H), 5.85 - 5.69 (m, 1H), 4.65 (m, 2H), 4.37 - 4.05 (m, 2H), 3.74 (m, 2H), 2.97 (s, 3H), 2.33 (m, 2H), 1.45 - 0.86 (m, 9H).

### Example 84B

LC-MS: m/z 640 (M+H)⁺. ¹H NMR (400 MHz, DMSO) δ 10.21 (brs, 1H), 8.48 (m, 1H), 7.88 (d, *J* = 7.7 Hz, 1H), 7.74 (s, 1H), 7.63 (t, *J* = 7.8 Hz, 1H), 7.23 (dd, *J* = 15.4, 8.0 Hz, 1H), 6.82 (dt, *J* = 16.8, 10.0 Hz, 1H), 6.74 - 6.54 (m, 2H), 6.19 (m, 1H), 5.85 - 5.64 (m, 1H), 4.71 (m, 2H), 3.95 (m, 4H), 2.95 (s, 3H), 2.31 (m, 2H), 1.39 - 0.97 (m, 9H).

### Example 85 Preparation of 4-((S)-4-acryloyl-2-methylpiperazin-1-yl) -6-fluoro-7-(2-fluoro-6-hydroxyphenyl)-1-(2-(methylsulfonyl) phenyl) pyridino[2,3-d] pyrimidin-2(1H)-one

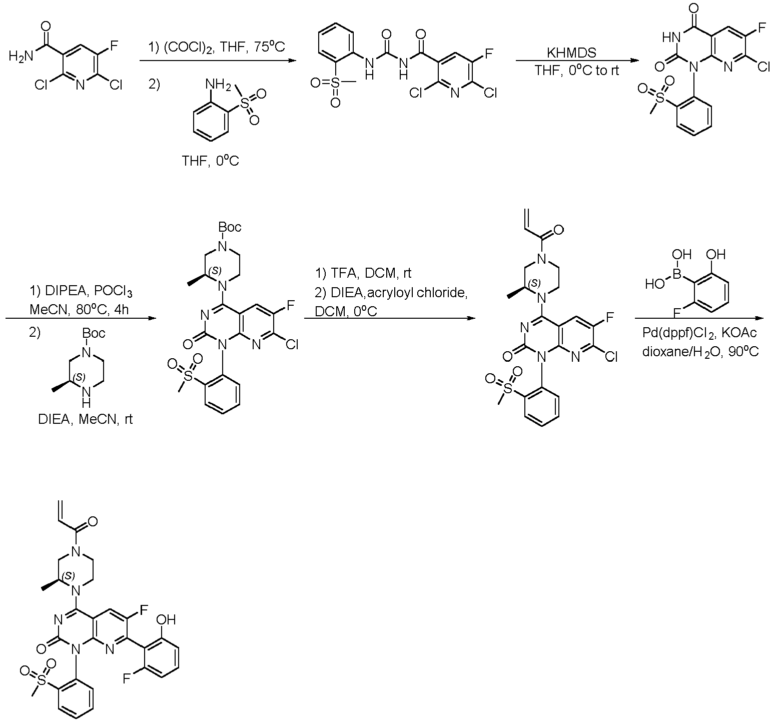

### Step 1. Preparation of 2, 6-dichloro-5-fluoro-N-(((2-(methylsulfonyl) phenyl) carbamoyl) nicotinamide

2, 6-Dichloro-5-fluoronicotinamide (420 mg, 2.0 mmol) was dissolved in anhydrous tetrahydrofuran (7 mL), and a solution of oxalyl chloride (1.7 mL, 20.0 mmol) in dichloromethane (2 mL) was slowly added to the solution. After the addition was completed, the mixture was refluxed and stirred at 75°C for 2h, and then concentrated to dryness under reduced pressure. The residue was diluted with anhydrous tetrahydrofuran (7 mL) and cooled to 0°C. 2-(Methylsulfonyl) aniline (360 mg, 2.1 mmol) was dissolved in anhydrous tetrahydrofuran (3 mL) and then was added dropwise to the above solution. The reaction solution was stirred at 0 °C for 2h, quenched with saturated ammonium chloride/saturated saline (V/V = 1/1,20 mL), and then extracted with dichloromethane/methanol (V/V = 10/1,20 mL) for 3 times. The combined organic phase was dried, concentrated, the residual solid was slurried with petroleum ether/ethyl acetate (V/V = 3/1,15 mL), filtered and dried to obtain the target product (645 mg, yield: 79%).

LC-MS: m/z 406 (M+H)⁺.

### Step 2. Preparation of 7-chloro-6-fluoro-1-(2-(methylsulfonyl) phenyl) pyridino [2,3-d] pyrimidin-2,4(1H,3H)-dione

2, 6-Dichloro-5-fluoro-N-((2-(methylsulfonyl) phenyl) carbamoyl) nicotinamide (645 mg, 1.6 mmol) was suspended in tetrahydrofuran (15 mL) and potassium bis (trimethylsilyl) amide (1 M tetrahydrofuran solution, 3.6 mL, 3.6 mmol) was added dropwise under an ice bath. After dropping, the reaction solution became clear. The reaction solution was stirred at room temperature for 16h, quenched with saturated ammonium chloride (20 mL), and then extracted with ethyl acetate (20 mL) for 3 times. The combined ethyl acetate layer was dried and concentrated. The residual solid was slurried with petroleum ether/ethyl acetate (V/V = 3/1,10 mL), filtered, and dried to obtain the target product (500 mg, yield: 85%).

LC-MS: m/z 370 (M+H)⁺. ¹H NMR (400 MHz, DMSO-*d*₆) *δ* 12.32 (s, 1H), 8.50 (d, *J* = 7.2 Hz, 1H), 8.15 (dd, *J* = 8.0 Hz, 1.2 Hz, 1H), 7.97-7.92 (m, 1H), 7.86-7.81 (m, 1H), 7.66 (dd, *J* = 8.0 Hz, 1.2 Hz, 1H), 3.09 (s, 3H).

### Step 3. Preparation of (S)-tert-butyl 4-(7-chloro-6-fluoro-1-(2-(methylsulfonyl) phenyl)-2-oxo-1, 2-dihydropyridino [2,3-d] pyrimidin-4-yl)-3-methylpiperazin-1-carboxylic acid

7-Chloro -6-fluoro -1-(2-(methylsulfonyl) phenyl) pyridino[2,3-d]pyrimidin-2,4(1H,3H)-dione (450 mg,1.2 mmol) was suspended in acetonitrile (10mL), and N,N-diisopropylethylamine (1.2 mL,7.3 mmol) and phosphorus oxychloride (0.6 mL, 6.1 mmol) were added dropwise, and the reaction solution became clear. The reaction solution was stirred at 80 °C for 4h and concentrated to dryness under reduced pressure. The residue was dissolved in acetonitrile (10mL), cooled to 0 °C, N,N-diisopropylethylamine (0.6 mL,3.7 mmol) and tert-butyl (S)-3-methylpiperazin-1-carboxylate (290 mg,1.5 mmol) were added, the reaction solution was stirred at room temperature for 1h, and quenched with semi-saturated sodium bicarbonate solution (40 mL), extracted with ethyl acetate (30 mL) for 3 times. The ethyl acetate layers were combined, dried and concentrated, and purified by silica gel column chromatography(petroleum ether: ethyl acetate = 3/1 to 1/2.5) to obtain the target product (460 mg, yield: 68%).

LC-MS: m/z 552 (M+H)⁺.

### Step 4. Preparation of (S)-4-(4-acryloyl -2-methylpiperazin -1-yl) -7-chloro-6-fluoro-1-(2-(methylsulfonyl) phenyl) pyridino [2,3-d] pyrimidin-2(1H)-one

(S)-tert-butyl 4-(7-chloro-6-fluoro-1-(2-(methylsulfonyl) phenyl) -2-oxo-1, 2-dihydropyridino [2,3-d] pyrimidin-4-yl)-3-methylpiperazin-1-carboxylic acid (500 mg,0.9 mmol) was dissolved in dichloromethane (10 mL), and trifluoroacetic acid (2 mL) was added. The reaction solution was stirred at room temperature for 2 hours, concentrated and dried, and the residue was steamed with dichloromethane (15mL) for 3 times to obtain crude product. The crude product was dissolved in dichloromethane (8 mL), cooled to 0°C, and a solution of N,N-diisopropylethylamine (0.6 mL, 3.6 mmol) and acryloyl chloride (110 mg,1.2 mmol) in dichloromethane (1 mL) was added dropwise. The reaction solution was stirred at 0 °C for 30min, quenched with saturated sodium bicarbonate (30 mL), extracted with dichloromethane (20 mL) for 3 times. The combined dichloromethane layer was dried and concentrated, and the residue was purified by silica gel column chromatography(dichloromethane/methanol = 60/1) to obtain the target product (380 mg, yield: 83%).

LC-MS: m/z 506 (M+H)⁺.

### Step 5. Preparation of 4-((S)-4-acryloyl-2-methylpiperazin-1-yl) -6-fluoro-7-(2-fluoro-6-hydroxyphenyl)-1-(2-(methylsulfonyl) phenyl) pyridino [2,3-d] pyrimidin-2(1H)-one

(S)-4-(4-acroloyl-2-methylpiperazin-1-yl) -7-chloro-6-fluoro-1-(2-(methylsulfonyl) phenyl) pyridino [2,3-d] pyrimidin-2(1H)-one (150 mg, 0.3 mmol), (2-fluoro-6-hydroxyphenyl) boric acid (60 mg,0.4 mmol), [1,1 '-bis (diphenylphosphine) ferrocene] palladium dichloride dichloromethane complex (24 mg, 0.03 mmol) and potassium acetate (120 mg, 1.2 mmol) were suspended in a mixed solvent of dioxane/water (7.5 mL/0.75 mL), and replaced with nitrogen for three times, heated and stirred at 90 °C for 2 hours. After the reaction solution was cooled to room temperature, a semi-saturated sodium bicarbonate solution (20 mL) was added and extracted with ethyl acetate (20 mL) for 3 times. The ethyl acetate layers were combined, dried, concentrated, and the residue was purified by silica gel column chromatography (dichloromethane: methanol = 100:1 to 60:1) to obtain the target product (90 mg, yield: 52%).

LC-MS: m/z 582 (M+H)⁺. ¹H NMR (400 MHz, CDCl₃) δ 8.97-8.94 (m, 1H), 8.27 (d, *J* = 8.0 Hz, 1H), 7.90-7.84 (m, 2H), 7.79-7.74 (m, 1H), 7.46-7.42 (m, 1H), 7.29-7.23 (m, 1H), 6.71-6.57 (m, 3H), 6.44-6.38 (m, 1H), 5.82 (d, *J* = 11.2 Hz, 1H), 5.12-4.32 (m, 3H), 4.07-3.63 (m, 3H), 3.24-3.01 (m, 4H), 1.50 (s, 3H).

### The following compounds were synthesized from different starting materials according to the method of Example 85:

### Example 86 4-((S)-4-acryloyl-2-methylpiperazin-1-yl) -6-fluoro-7-(2-fluoro-6-hydroxyphenyl)-1-(2-(isopropylsulfonyl) phenyl) pyridino[2,3-d] pyrimidin -2(1H)-one

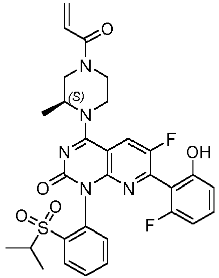

LC-MS: m/z 610 (M+H)⁺. ¹H NMR (400 MHz, CDCl₃) δ 8.91-8.86 (m, 1H), 8.22-8.19 (m, 1H), 7.88-7.82 (m, 2H), 7.75-7.72 (m, 1H), 7.45-7.42 (m, 1H), 7.28-7.23 (m, 1H), 6.71-6.63 (m, 3H), 6.44-6.39 (m, 1H), 5.83-5.80 (m, 1H), 5.04-4.79 (m, 3H), 4.56-3.90 (m, 4H), 3.71-3.48 (m, 1H), 1.50 (s, 3H), 1.33-1.30 (m, 3H), 1.18-1.16 (m, 3H).

### Example 87 4-((S)-4-acryloyl-2-methylpiperazin-1-yl) -6-fluoro-7-(2-fluoro-6-hydroxyphenyl)-1-(2-methyl-6-(methylsulfonyl) phenyl) pyridino[2,3-d] pyrimidin-2(1H)-one

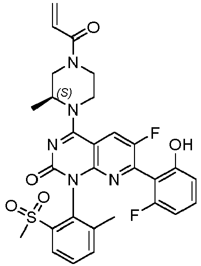

LC-MS: m/z 596 (M+H)⁺. ¹H NMR (400 MHz, CDCl₃) δ 8.12-8.11 (m, 1H), 7.89-7.87 (m, 1H), 7.75-7.63 (m, 2H), 7.26 (m, 1H), 6.69-6.64 (m 3H), 6.44-6.40 (m, 1H), 5.84-5.81 (m, 1H), 5.00-4.40 (m, 3H), 4.07-3.65 (m, 3H), 3.25-3.16 (m, 4H), 2.20-2.18 (m, 3H), 1.58-1.50 (m, 3H).

### Example 87-1 two isomers of Example 87A and Example 87B were obtained by chiral separation:

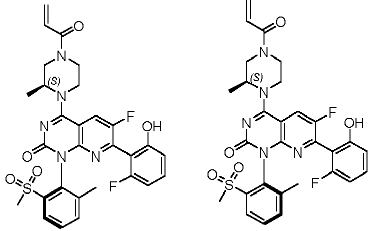

**Example 87A:**LC-MS:m/z 596 (M+H)⁺.

**Example 87B:**LC-MS:m/z 596 (M+H)⁺.

### Example 88 4-((S)-4-acryloyl-2-methylpiperazin-1-yl) -6-fluoro-7-(2-fluoro-6-hydroxyphenyl)-1-( 2-(methylsulfonyl) pyridin-3-yl) pyridino [2,3-d] pyrimidin -2(1H)-one

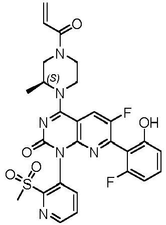

LC-MS: m/z 583 (M+H)⁺. ¹H NMR (400 MHz, CDCl₃) δ 8.80 (dd, *J* = 4.8, 1.2 Hz, 1H), 8.37-8.25 (m, 1H), 8.05 (dd, *J* = 8.0, 1.2 Hz, 1H), 7.90-7.87 (m, 1H), 7.26 (dd, *J* = 14.6, 7.6 Hz, 1H), 6.92 (m, 1H), 6.82-6.65 (m, 2H), 6.23-6.18 (m, 1H), 5.76 (dd, *J* = 6.4, 2.4 Hz, 1H), 4.98-4.82 (m, 1H), 4.44-4.00 (m, 3H), 3.81-3.62 (m, 2H), 3.24-3.00 (m, 4H), 1.34-1.29 (m, 3H).

### Example 89 4-((S)-4-acryloyl-2-methylpiperazin-1-yl) -6-fluoro-7-(2-fluoro-6-hydroxyphenyl)-1-(4-methylsulfonyl) pyridin-3-yl) pyridino [2,3-d] pyrimidin -2(1H)-one

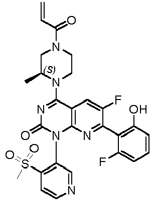

LC-MS: m/z 583 (M+H)⁺. ¹H NMR (400 MHz, CDCl₃) δ 9.06 (d, *J* = 4.2 Hz), 8.75-8.74 (m, 1H), 8.59-8.57 (m, 1H), 8.10 (d, *J* = 4.2 Hz), 7.87 (d, *J* = 11.2 Hz, 1H), 7.30-7.25 (m , 1H), 6.72-6.57 (m, 3H), 6.64-6.58 (m, 1H), 5.84-5.82 (m, 1H), 5.29-4.28 (m, 3H), 4.10-3.61 (m, 3H), 3.22-2.92 (m, 4H0, 1.59-1.48 (m, 3H).

### Example 90 2-(1-acryloyl -4-(6-fluoro-7-(2-fluoro-6-hydroxyphenyl)-1-(2-(methylsulfonyl) phenyl) -2-oxo-1, 2-dihydropyridino [2,3-d] pyrimidin-4-yl) piperazin-2-yl) acetonitrile

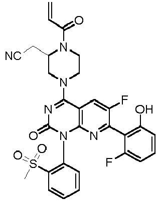

LC-MS: m/z 607 (M+H)⁺. ¹H NMR (400 MHz, CDCl₃) δ 8.29-8.24 (m, 1H), 7.96-7.76 (m, 3H), 7.47-7.43 (m, 1H), 7.29 (m, 1H), 6.70-6.56 (m, 3H), 6.45-6.41 (m, 1H), 5.00 (brs, 1H), 4.56-3.70 (m, 6H), 3.16 (m, 3H), 3.16-3.10 (m,1H), 2.98-2.79 (m, 1H).

### Example 91 2-(1-acryloyl -4-(6-fluoro-7-(2-fluoro-6-hydroxyphenyl)-1-(2-methyl-6-(methylsulfonyl) phenyl) -2-oxo-1, 2-dihydropyridino [2,3-d] pyrimidin-4-yl) piperazin-2-yl) acetonitrile

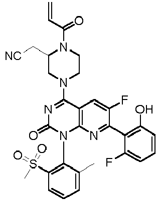

LC-MS: m/z 621 (M+H)⁺. ¹H NMR (400 MHz, CDCl₃) δ 8.12-7.97 (m, 2H), 7.76-7.72 (m, 1H), 7.67-7.64 (m, 1H), 7.29 (m, 1H), 6.69-6.46 (m, 3H), 6.42-6.41 (m, 1H), 5.88-5.86 (m, 1H), 5.00 (brs, 1H), 4.54-3.77 (m, 6H), 3.12 (m, 4H), 2.81-2.77 (m, 1H), 2.20-2.17 (m, 3H).

### Example 92 2-(1-acryloyl -4-(6-fluoro-7-(2-fluoro-6-hydroxyphenyl)-1-(2-(methylsulfonyl) pyridin-3-yl) -2-oxo-1, 2-dihydropyridino [2,3-d] pyrimidin-4-yl) piperazin-2-yl) acetonitrile

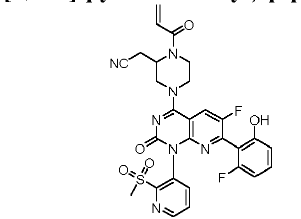

LC-MS: m/z 608 (M+H)⁺. ¹H NMR (400 MHz, CDCl₃) δ 8.82-8.81 (m, 1H), 7.99-7.76 (m, 3H), 7.30 (m, 1H), 6.70-6.56 (m, 3H), 6.44-6.40 (m, 1H), 5.87-5.84 (m, 1H), 5.01 (brs, 1H), 4.61-3.60 (m, 6H), 3.29 (m, 3H), 2.96-2.76 (m, 2H).

### Example 93 4-((S)-4-acryloyl-2-methylpiperazin-1-yl)-7-(2-chloro-6-fluorophenyl)-6-fluoro-1-(2-methyl-6-(methylsulfonyl) phenyl) pyridino [2,3-d] pyrimidin-2(1H)-one

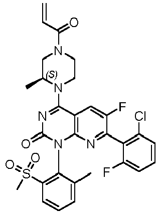

LC-MS: m/z 614 (M+H)⁺. ¹H NMR (400 MHz, CDCl₃) δ 7.95 (d, *J* = 3.6 Hz, 1H), 7.82 (t, *J* = 8.4 Hz, 1H), 7.60 (d, *J* = 3.2 Hz, 1H), 7.50 (t, *J* = 8.4 Hz, 1H), 7.34-7.29 (m, 1H), 7.20 (d, *J* = 8 Hz, 1H), 7.02 (m, 1H), 6.60 (m, 1H), 6.39 (dd, *J* = 1.6 Hz, 17.2 Hz, 1H), 5.80 (dd, *J* = 1.2 Hz, 10.4 Hz, 1H), 5.20-4.20 (m, 3H), 4.10-3.55 (m, 3H), 3.40-3.05 (m, 4H), 2.17-2.15 (m, 3H), 1,.51 (m, 3H).

### Example 95 2-(4-acryloyl-1-(6-fluoro-7-(2-fluoro-6-hydroxyphenyl)-1-(2-(methylsulfonyl) phenyl) -2-oxo-1, 2-dihydropyridino [2,3-d] pyrimidin-4-yl) piperazin-2-yl) acetonitrile

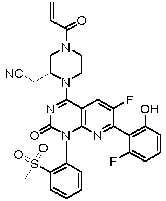

LC-MS: m/z 607 (M+H)⁺. ¹H NMR (400 MHz, CDCl₃) δ 8.87-8.77 (m, 1H), 8.22-8.19 (m, 1H), 7.86-7.80 (m, 3H), 7.69 (m, 1H), 7.37-7.36 (m, 1H), 7.25 (m, 1H), 6.66-6.53 (m, 3H), 6.39 (d, *J* = 17.2 Hz, 1H), 5.81 (d, *J* = 10 Hz, 1H), 5.30-5.20 (m, 1H), 4.80-3.95 (m, 3H), 3.90-3.30 (m, 3H), 3.09-2.88 (m, 5H).

### Example 96 2-(4-acryloyl-1-(6-fluoro-7-(2-fluoro-6-hydroxyphenyl)-1-(2-(methylsulfonyl) phenyl) -2-oxo-1, 2-dihydropyridino [2,3-d] pyrimidin-4-yl) piperazin-2-yl) acetamide

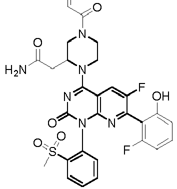

LC-MS: m/z 625 (M+H)⁺. ¹H NMR (400 MHz, CDCl₃) δ 8.20-7.75 (m, 2H), 7.60-7.40 (m, 2H), 7.11-7.01 (m, 2H), 6.84-7.53 (m, 5H), 6.36-6.29 (m, 1H), 5.79 (m, 1H), 5.44-5.23 (m, 1H), 5.50-3.98 (m, 4H), 3.81-3.070 (m, 6H), 2.85-2.63 (m, 2H).

### Biological test evaluation

The following biological test examples further describe the present invention, but these examples are not intended to limit the scope of the invention.

**Cell assay of antiproliferative activity of compounds against NCI-H358(KRAS ^{G12C} mutation) cell.**

### Experimental steps

40µL phosphate buffer was added to the outer wells of the 384 microplate, and 40µL cell suspension to be tested was added to the other wells. Then the microplate was incubated in a carbon dioxide incubator overnight.

The compounds to be tested were gradiently diluted, each compound was performed 10 concentration gradients (diluted from 50 µM to 0.003 µM) and added 100nL to the corresponding wells in the microplate. After addition, 40 µl of phosphate buffer was added to each well in rows A and P and columns 1 and 24, and then the microplate was incubated in a carbon dioxide incubator for 5 days.

20µL of Promega Celltiter-Glo reagent was added to each well of the microplate, and then the luminescence signal was stabilized by oscillating at room temperature for 10min. Then PekinElmer Envision multi-label analyzer was used to read.

Finally, GraphPad Prism software was used to calculate the IC₅₀ value of the compound and the fitting curve was drawn.

Anti-proliferative activity of the compounds of the prensent invention against NCI-H358(KRAS ^{G12C} mutation) cell was shown in Table 1.

**Table 1 Anti-proliferative activity of the compound of the present invention**

| **IC₅₀** | **NCI-H358(**µM**)** |
|---|---|
| **Example 1** | 0.09 |
| **Example 2** | 0.54 |
| **Example 3** | 0.87 |
| **Example 4** | 0.42 |
| **Example 5** | 0.028 |
| **Example 5A** | 0.23 |
| **Example 5B** | 0.011 |
| **Example 6** | 0.028 |
| **Example 6A** | 0.2 |
| **Example 6B** | 0.015 |
| **Example 7** | 0.24 |
| **Example 8** | 0.17 |
| **Example 9** | 0.23 |
| **Example 10** | 0.010 |
| **Example 10A** | 0.28 |
| **Example 10B** | 0.005 |
| **Example 11** | 0.18 |
| **Example 12** | 0.041 |
| **Example 13** | 0.015 |
| **Example 14** | 0.36 |
| **Example 15** | 0.058 |
| **Example 16** | 0.012 |
| **Example 16A** | 0.72 |
| **Example 16B** | 0.007 |
| **Example 17** | 0.031 |
| **Example 18** | 0.030 |
| **Example 18A** | 0.013 |
| **Example 18B** | 0.46 |
| **Example 19** | 0.048 |
| **Example 20** | 0.023 |
| **Example 20A** | 1.5 |
| **Example 20B** | 0.013 |
| **Example 21** | 1.2 |
| **Example 22** | 0.039 |
| **Example 23** | 1.1 |
| **Example 24** | 0.028 |
| **Example 25** | 0.009 |
| **Example 25A** | 0.35 |
| **Example 25B** | 0.006 |
| **Example 26** | 0.018 |
| **Example 27** | 0.03 |
| **Example 28** | 0.026 |
| **Example 29** | 0.053 |
| **Example 30** | 0.018 |
| **Example 30A** | 0.74 |
| **Example 30B** | 0.011 |
| **Example 31** | 0.008 |
| **Example 31A** | 0.30 |
| **Example 31B** | 0.006 |
| **Example 53B** | 0.066 |
| **Example 54** | 2.5 |
| **Example 55** | 5.3 |
| **Example 56** | 8.0 |
| **Example 57** | 10 |
| **Example 58** | 10 |
| **Example 59** | 0.20 |
| **Example 60** | > 10 |
| **Example 61** | > 10 |
| **Example 63A** | 0.37 |
| **Example 63 B** | 0.008 |
| **Example 64** | 0.12 |
| **Example 65** | > 10 |
| **Example 66** | > 10 |
| **Example 67** | > 10 |
| **Example 68** | 2.2 |
| **Example 69** | > 10 |
| **Example 70** | > 10 |
| **Example 71** | > 10 |
| **Example 72** | > 10 |
| **Example 73** | > 10 |
| **Example 74** | > 10 |
| **Example 75** | > 10 |
| **Example 76** | > 10 |
| **Example 77** | > 10 |
| **Example 78A** | 0.17 |
| **Example 78B** | 0.007 |
| **Example 79A** | 0.048 |
| **Example 79B** | 0.002 |
| **Example 80B** | 0.007 |
| **Example 81B** | 0.009 |
| **Example 82B** | 0.050 |
| **Example 83B** | 0.026 |
| **Example 84B** | 0.006 |
| **Example 85** | < 1 |
| **Example 86** | < 1 |
| **Example 87** | < 0.1 |
| **Example 87A** | < 0.03 |
| **Example 87B** | < 0.1 |
| **Example 88** | >1-<10 uM |
| **Example 89** | >1-<10 uM |
| **Example 90** | >1-<10 uM |
| **Example 91** | >1-<10 uM |
| **Example 92** | >10 uM |

It can be seen from Table 1:
the compounds of the present invention showed good cell anti-proliferative activity for KRAS ^{G12C} mutant NCI-H358 cell, especially the compounds containing the structure of phenylsulfone structure.

### Pharmacokinetics test evaluation

### Evaluation of pharmacokinetic tests in mice

Male ICR mice with a body weight of about 20-30g, were given 30 mg/kg of solutions of compounds of Examples 10B, 16B, 18A, 63B and 78B of the present invention [CMC/TW80 as carrier] by gavage after fasting overnight. Blood samples were collected at 0.5, 1.0, 2.0, 4.0, 6.0, 8.0, 12 and 24 hours after the compound was administrated, and the concentrations of the compound in plasma were determined by LC/MS/MS.

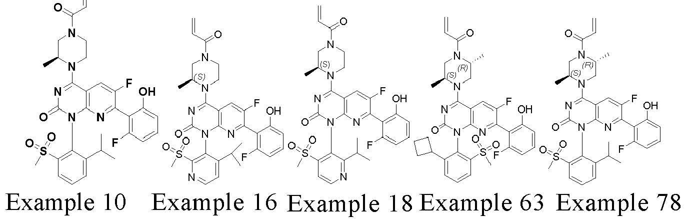

The results are shown in Table 2.

**Table 2 Summary of pharmacokinetics parameters: (n = 4, mean)**

| **parameter** | **unit** | **Example 10B** | **Example 16B** | **Example 18A** | **Example 63B** | **Example 78B** |
|---|---|---|---|---|---|---|
| Dose | | 30mg/kg | 30mg/kg | 30mg/kg | 30mg/kg | 30mg/kg |
| Cmax | ng/mL | 1570 | 26.1 | 336 | 796 | 722 |
| AUC₀₋ₜ | ng.h/mL | 1548 | 46.1 | 219 | 1540 | 688 |

It can be seen from the test results that compounds with similar structures, among which, compounds containing phenylsulfone have better pharmacokinetic properties than those containing pyridine sulfone.

### Evaluation of pharmacokinetic tests in rat

Male SD rats with a body weight of about 220g, were given 15 mg/kg of the solution of the compound of the present invention or the control compound AMG510 [DMSO/PEG400 as the carrier] by gavage after fasting overnight. Blood samples were collected at 0.5, 1.0, 2.0, 4.0, 6.0, 8.0, 12 and 24 after administration of the compound of the present invention, respectively, or at 0.5, 1.0, 2.0, 4.0, 6.0, 8.0, 12 and 24 after 7 days of continuous administration, and the concentration of the compound of the invention or control compound AMG 510 in plasma was determined by LC/MS/MS.

The test results show that the compound of the present invention has good pharmacokinetic characteristics compared to AMG 510.

### Pharmacodynamic testing evaluation of anti-tumor activity

### 1. H358 CDX Tumor Model

100 uL suspension containing 5×10⁶ NCI-H358 tumor cells was subcutaneously injected into the right posterior back of nude mice. The health of the mice was monitored daily. Measurements were started when the tumor grew palpable. The tumor volume calculation formula was adopted: 0.5xLxW2, where L and W represented tumor length and width, respectively. The tumor grew to about 200 mm³, the mice were randomly divided into groups. Mice were intragastrically administrated the corresponding dose (20mg/Kg) of the compound's solution every day, and their general status was monitored at the same time. The tumor was measured 2 times a week, and the weight was measured twice a week. The results are shown in Table 3.

**Table 3 Pharmacodynamic testing evaluation of anti-tumor activity**

| **Group** | **TV (mm³) Day** 0 | **TV (mm³) Day 20** | **% Tumor Growth** | **%TGI** |
|---|---|---|---|---|
| Blank control group | 196 | 1920 | 886 | / |
| Example 63B | 196 | 263 | 19 | 97.8% |
| Example 78B | 196 | 231 | 13 | 98.5% |
| AMG510 | 196 | 437 | 117 | 86.8% |

The results show that the compounds containing the structure of phenylsulfone in the present invention (such as Examples 63B and 78B) have better efficacy than the control compound AMG 510.

### 2. MIA PaCa-2 CDX Tumor Model

100 uL suspension containing 5×10⁶ MIA PaCa-2 tumor cells was subcutaneously injected into the right posterior abdomen of nude mice. The health of the mice was monitored daily. Measurements were started when the tumor grew palpable. The tumor volume calculation formula was adopted: 0.5×L×W², where L and W represented tumor length and width, respectively. The tumor grew to about 150 mm³, the mice were randomly divided into groups. Mice were intragastrically administrated the corresponding dose (3, 10mg/Kg) of the compound's CMC-Na suspension every day, and their general status was monitored at the same time. The tumor was measured 3 times a week, and the weight was measured twice a week.

It can be seen from the test results that the compound of the present invention has a good anti-tumor effect.

All literatures mentioned in the present application are incorporated by reference herein, as though individually incorporated by reference. Additionally, it should be understood that after reading the above teaching, many variations and modifications may be made by the skilled in the art, and these equivalents also fall within the scope as defined by the appended claims.

## Claims

1. A compound of formula (I), or stereoisomer, tautomer, crystal form, pharmaceutically acceptable salt, hydrate, solvate or prodrug thereof: 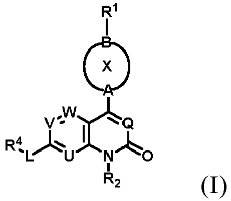 wherein:
A and B are the same or different, and are each independently selected from the group consisting of CH, CR⁵ and N;
X is selected from the group consisting of 4-14 membered saturated or unsaturated heterocyclyl, C₄-C₁₄ cycloalkyl, C₆-C₁₄ aryl and 5-14 membered heteroaryl, wherein the heterocyclyl, cycloalkyl, aryl or heteroaryl can optionally be substituted by one or more R⁸;
U, V, W and Q are the same or different, and each independently selected from the group consisting of CH, CR³ and N;
R¹ is selected from the group consisting of **-C(O)C(R*^{A}*)** 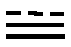 **C(R*^{B}*)*ₚ*, -S(O)₂C(R*^{A}*)** 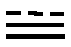 **C(R*^{B}*)*ₚ***, **-NR⁶C(O)C(R*^{A}*)** 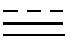 **C(R*^{B}*)*****ₚ*** and **-NR⁶S(O)₂C(R*^{A}*)** 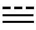 **C(R*^{B}*)*ₚ***; wherein " 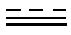 " represents double bond " 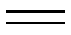 " or triple bond " 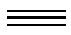 ";
R*^{A}* is absent, or is independently selected from the group consisting of hydrogen, deuterium, fluorine, cyano and C₁-C₃ alkyl; R*^{B}* is each independently selected from the group consisting of hydrogen, deuterium, cyano and C₁-C₃ alkyl; wherein, the alkyl can be substituted by one or more substituents selected from the group consisting of deuterium, halogen, cyano, amino, C₃-C₇ cycloalkyl, 4-7-membered heterocyclyl, NHR⁹ and NR⁹R¹⁰; R⁹ and R¹⁰ are each independently C₁-C₃ alkyl; or R⁹ and R¹⁰ together with the N atom to which they are attached form a substituted or unsubstituted 4-8-membered heterocyclyl;
p is an integer of 1 or 2;
R² is selected from the substituted group consisting of C₆-C₁₄ aryl and 5-14-membered heteroaryl, wherein the substituted means being substituted by one or more groups selected from the group consisting of R', -SR', -SOR', -SO₂R', -SO₂NR'R", -NR'SO₂R" and -P(= O)R'R"; with the proviso that the C₆-C₁₄ aryl or 5-14-membered heteroaryl contains at least one substituent selected from -SR', -SOR', -SO₂R', -SO₂NR'R", -NR'SO₂R", or -P(= O)R'R"; R' and R" are the same or different, and are each independently selected from the substituted or unsubstituted group consisting of hydrogen, deuterium, halogen, nitro, hydroxyl, cyano, ester group, amino, amido, C₁-C₆ alkyl, C₃-C₈ cycloalkyl, C₄-C₁₀ cycloalkenyl, 4-8 membered heterocyclyl, C₆-C₁₄ aryl and 5-14 membered heteroaryl; or when R' and R" are attached to the same N atom, R' and R" together with the N atom to which they are attached form a substituted or unsubstituted 4-8-membered heterocyclyl;
R³ is selected from the substituted or unsubstituted group consisting of hydrogen, deuterium, C₁-C₁₈ alkyl, deuterated C₁-C₁₈ alkyl, halogenated C₁-C₁₈ alkyl, C₃-C₂₀ cycloalkyl, C₁-C₁₈ alkoxy, deuterated C₁-C₁₈ alkoxy, halogenated C₁-C₁₈ alkoxy, halogen, nitro, hydroxyl, cyano, ester group, amino, amido, sulfonamido, carbamido, 4-20-membered heterocyclyl, C₆-C₁₄ aryl and 5-14-membered heteroaryl;
L is selected from the group consisting of bond, -C(O)- and C₁-C₃ alkylene;
R⁴ is selected from the substituted or unsubstituted group consisting of hydrogen, deuterium, C₁-C₁₈ alkyl, deuterated C₁-C₁₈ alkyl, halogenated C₁-C₁₈ alkyl, C₃-C₂₀ cycloalkyl, C₁-C₁₈ alkoxy, deuterated C₁-C₁₈ alkoxy, halogenated C₁-C₁₈ alkoxy, amino, hydroxy, 4-20 membered heterocyclyl, C₆-C₁₄ aryl and 5-14 membered heteroaryl;
R⁵ is selected from the substituted or unsubstituted group consisting of hydrogen, deuterium, C₁-C₁₈ alkyl, deuterated C₁-C₁₈ alkyl, halogenated C₁-C₁₈ alkyl, C₃-C₂₀ cycloalkyl, C₁-C₁₈ alkoxy, deuterated C₁-C₁₈ alkoxy, halogenated C₁-C₁₈ alkoxy, halogen, nitro, hydroxyl, cyano, ester group, amino, amido, sulfonamido, carbamido, 4-20-membered heterocyclyl, C₆-C₁₄ aryl and 5-14-membered heteroaryl;
R⁶ is selected from the substituted or unsubstituted group consisting of hydrogen, deuterium, C₁-C₁₈ alkyl, deuterated C₁-C₁₈ alkyl, halogenated C₁-C₁₈ alkyl, C₃-C₂₀ cycloalkyl, C₁-C₁₈ alkoxy, deuterated C₁-C₁₈ alkoxy, halogenated C₁-C₁₈ alkoxy, 4-20 membered heterocyclyl, C₆-C₁₄ aryl and 5-14 membered heteroaryl;
R⁸ is independently selected from the substituted or unsubstituted group consisting of hydrogen, deuterium, C₁-C₁₈ alkyl, deuterated C₁-C₁₈ alkyl, halogenated C₁-C₁₈ alkyl, C₃-C₂₀ cycloalkyl, C₁- C₁₈ alkoxy, deuterated C₁-C₁₈ alkoxy, halogenated C₁-C₁₈ alkoxy, amino, hydroxyl, 4-20 membered heterocyclyl, C₆-C₁₄ aryl and 5-14 membered heteroaryl;
wherein, unless otherwise stated, the "substituted" means being substituted by one or more substituents selected from the group consisting of hydrogen, deuterium, C₁-C₁₈ alkyl, deuterated C₁-C₁₈ alkyl, halogenated C₁-C₁₈ alkyl, C₃-C₂₀ cycloalkyl, C₁-C₁₈ alkoxy, deuterated C₁-C₁₈ alkoxy, halogenated C₁-C₁₈ alkoxy, C₆-C₁₄ aryl, 5-14-membered heteroaryl, 4-20-membered heterocyclyl, halogen, nitro, hydroxyl, cyano, ester group, amino, NR_{b}C(=O)ORₑ, OC(=O)Rₑ, OC(=O)NR_{b}Rₑ, amido, sulfonamido and carbamido; R_{b} and R_{c} can be independently hydrogen, deuterium, C1-C6 alkyl, C3-C8 cycloalkyl, 4-8-membered heterocyclyl, 5-14-membered heteroaryl or C6-C14 aryl, or R_{b} and Rₑ together with the N atom can form 4-8-membered heterocyclyl; Rₑ can be independently hydrogen, C1-C6 alkyl, C3-C8 cycloalkyl, C2-C6 alkenyl, C3-C6 cycloalkenyl, C2-C6 alkynyl, 4-8-membered heterocyclyl, 5-14-membered heteroaryl or C6-C14 aryl;
with the proviso that
when B is N, R¹ is selected from the group consisting of **-C(O)C(R*^{A}*)** 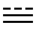 **C(R*^{B}*)*ₚ*** and **-S(O)₂C(R*^{A}*)** 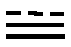 **C(R*^{B}*)*ₚ*,** wherein p is 2;
when B is CH or CR⁵, R¹ is selected from the group consisting of **-NR⁶C(O)C(R*^{A}*)** 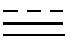 **C(R*^{B}*)*ₚ*** and **-NR⁶S(O)₂C(R*^{A}*)** 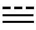 **C(R*^{B}*)*ₚ*** wherein p is 2;
when V is C(Cl), R² is not selected from: 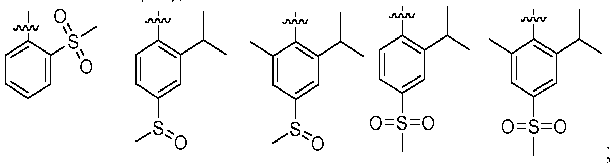 and L is not selected from bond; and
R⁴ is not selected from:

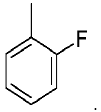

2. The compound of formula (I), or stereoisomer, tautomer, crystal form, pharmaceutically acceptable salt, hydrate, solvate or prodrug thereof of claim 1, wherein it has the structure shown in formula (III): 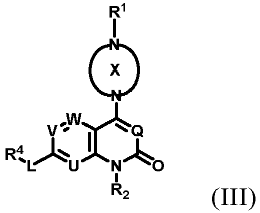 R¹, R², R⁴, X, L, U, V, W and Q are as defined in claim 1.

3. The compound of formula (I), or stereoisomer, tautomer, crystal form, pharmaceutically acceptable salt, hydrate, solvate or prodrug thereof of claim 1, wherein it has the structure shown in formula (IV): 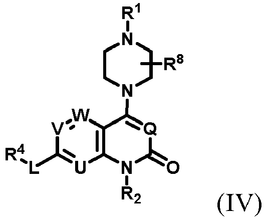 wherein:
R¹, R², R⁴, R⁸, L, U, V, W and Q are as defined in claim 1.

4. The compound of formula (I), or stereoisomer, tautomer, crystal form, pharmaceutically acceptable salt, hydrate, solvate or prodrug thereof of claim 1, wherein it has the structure shown in formula (V): 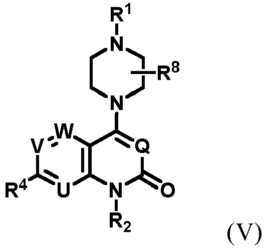 wherein:
R¹, R², R⁴, R⁸, U, V, W and Q are as defined in claim 1.

5. The compound of formula (I), or stereoisomer, tautomer, crystal form, pharmaceutically acceptable salt, hydrate, solvate or prodrug thereof of claim 1, wherein it has the structure shown in formula (VI): 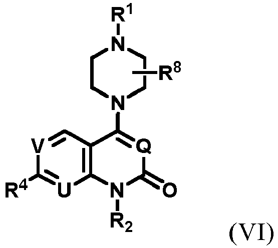 wherein:
R¹, R², R⁴, R⁸, U, V and Q are as defined in claim 1.

6. The compound of formula (I), or stereoisomer, tautomer, crystal form, pharmaceutically acceptable salt, hydrate, solvate or prodrug thereof of claim 1, wherein it has the structure shown in formula (VII): 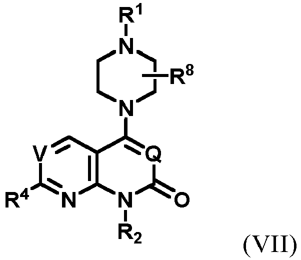 wherein:
R¹, R², R⁴, R⁸, V and Q are as defined in claim 1.

7. The compound of formula (I), or stereoisomer, tautomer, crystal form, pharmaceutically acceptable salt, hydrate, solvate or prodrug thereof of any one of claims 1-6, wherein it has the structure shown in formula (VIII): 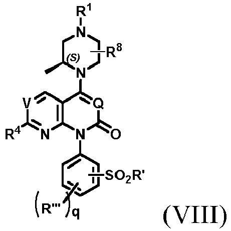 wherein,
R‴ is selected from the substituted or unsubstituted group consisting of deuterium, halogen, nitro, hydroxyl, cyano, ester group, amino, amido, C₁-C₆ alkyl, C₃-C₈ cycloalkyl , C₄-C₁₀ cycloalkenyl, 4-8 membered heterocyclyl, C₆-C₁₄ aryl , and 5-14 membered heteroaryl, wherein, the substituted means being substituted by one or more substituents selected from the group consisting of deuterium, halogen, nitro, hydroxyl, cyano, ester group, amino, amido, C₁-C₆ alkyl, C₃-C₈ cycloalkyl, C₄-C₁₀ cycloalkenyl, 4-8-membered heterocyclyl, C₆-C₁₄ aryl and 5-14 membered heteroaryl;
q is selected from 1, 2, 3 or 4;
R¹, R⁴, R⁸, R', V and Q are as defined in claim 1.

8. The compound of formula (I), or stereoisomer, tautomer, crystal form, pharmaceutically acceptable salt, hydrate, solvate or prodrug thereof of claim 7, wherein it has the structure shown in formula (IX): 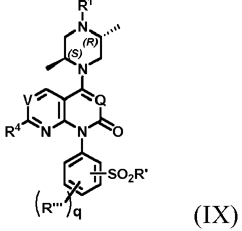 wherein,
R¹ is selected from 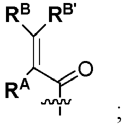 wherein, R^{A} is selected from H, D, halogen or cyano; R^{B} and R^{B'} are the same or different, and are each independently selected from H, D, halogen, cyano, substituted or unsubstituted C₁-C₃ alkyl; wherein, the substituted means being substituted by one or more substituents selected from the group consisting of D, halogen, cyano, C₁-C₃ alkyl, C₃-C₆ cycloalkyl, 4-6-membered heterocyclyl and NR^{IV}R^{V}; R^{IV} and R^{V} are the same or different, and are each independently selected from H, C₁-C₃ alkyl, C₃-C₆ cycloalkyl or 4-6-membered heterocyclyl; or R^{IV}, R^{V} and adjacent N cyclize together to form 4-6-membered heterocyclyl;
R⁴, R', V, Q, R‴, and q are as defined in claim 7.

9. The compound of formula (I), or stereoisomer, tautomer, crystal form, pharmaceutically acceptable salt, hydrate, solvate or prodrug thereof of any one of claims 1-8, wherein it has the structure shown in formula (X) or (XI): 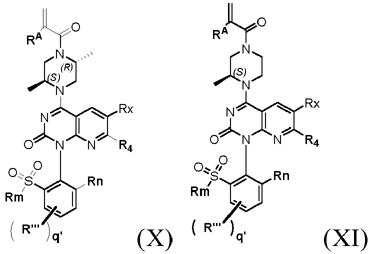 wherein,
R⁴ is selected from substituted or unsubstituted C6-C14 aryl or 5-10 membered heteroaryl, wherein the substituted means being substituted by one or more (such as 2, 3, 4 or 5) substituents selected from the group consisting of deuterium, halogen, ester group, cyano, NR_{b}C(=O)ORₑ, OC (= O)Rₑ, OC (= O)NR_{b}Rₑ, amino, C₁-C₆ alkyl, halogenated C₁-C₆ alkyl, and hydroxyl; R_{b} and Rₑ can be independently hydrogen, deuterium, C₁-C₆ alkyl, C₃-C₈ cycloalkyl, 4-8 membered heterocyclyl, 5-14 membered heteroaryl or C₆-C₁₄ aryl, or R_{b} and Rₑ together with the N atom can form 4-8 membered heterocyclyl; Rₑ can be independently hydrogen, C₁-C₆ alkyl, C₃-C₈ cycloalkyl, C₂-C₆ alkenyl, C₃-C₆ cycloalkenyl, C₂-C₆ alkynyl, 4-8 membered heterocyclyl, 5-14-membered heteroaryl or C6-C14 aryl;
Rm is selected from the substituted or unsubstituted group consisting of amino, C₁-C₆ alkyl, C₃-C₆ cycloalkyl and 4-6 membered heterocyclyl, wherein the substituted means being substituted by one or more substituents selected from the group consisting of deuterium, halogen, nitro, hydroxyl, cyano, ester group, amino, amido, C₁-C₃ alkyl, C₃-C₆ cycloalkyl and 4-6 membered heterocyclyl;
Rn is selected from the substituted or unsubstituted group consisting of amino, C₁-C₆ alkyl, C₁-C₆ alkoxy, C₃-C₆ cycloalkyl , -O-C₃-C₆ cycloalkyl, C₁-C₆ alkyl C₃-C₆ cycloalkyl, -O-C₁-C₆alkyl C₃-C₆ cycloalkyl and 4-6 membered heterocyclyl, wherein the substituted means being substituted by one or more substituents selected from the group consisting of deuterium, halogen, nitro, hydroxyl, cyano, ester group, amino, amido, C₁-C₃ alkyl, C₁-C₃ haloalkyl, C₃-C₆ cycloalkyl and 4-6 membered heterocyclyl;
Rx is selected from F or Cl;
R^{A} is selected from H, D, or halogen;
q' is selected from 0, 1, 2 or 3;
R‴ is selected from the substituted or unsubstituted group consisting of deuterium, halogen, nitro, hydroxyl, cyano, ester group, amino, amido, C₁-C₆ alkyl, C₃-C₈ cycloalkyl , C₄-C₁₀ cycloalkenyl, 4-8 membered heterocyclyl, C₆-C₁₄ aryl , 5-14 membered heteroaryl, wherein, the substituted means being substituted by one or more substituents selected from the group consisting of deuterium, halogen, nitro, hydroxyl, cyano, ester group, amino, amido, C₁-C₆ alkyl, C₃-C₈ cycloalkyl, C₄-C₁₀ cycloalkenyl, 4-8-membered heterocyclyl, C₆-C₁₄ aryl and 5-14 membered heteroaryl.

10. The compound of formula (I), or stereoisomer, tautomer, crystal form, pharmaceutically acceptable salt, hydrate, solvate or prodrug thereof of claim 1, wherein the compound is selected from the group consisting of

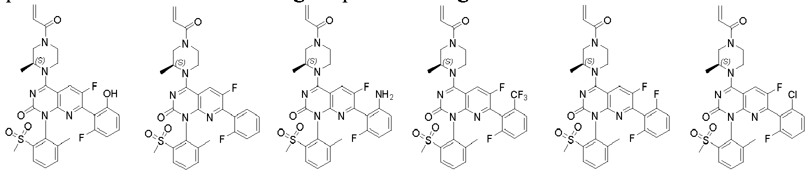

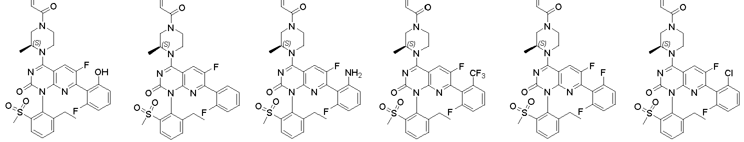

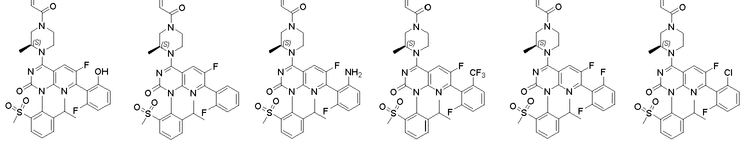

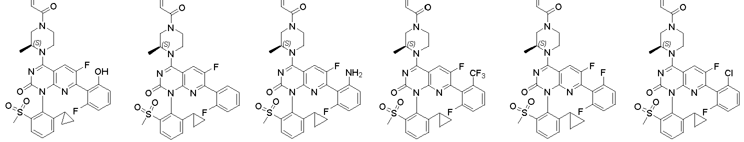

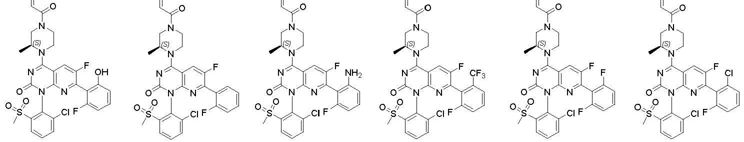

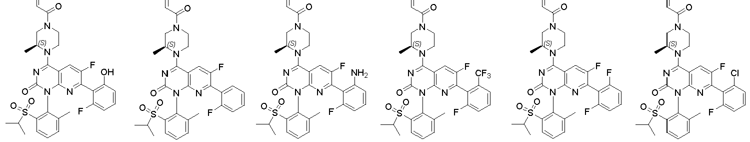

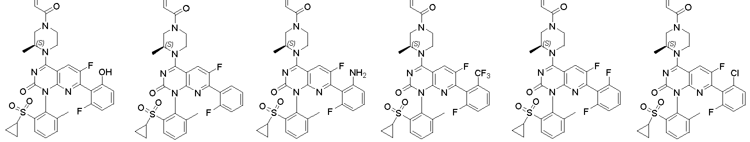

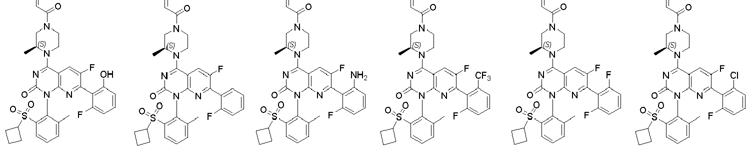

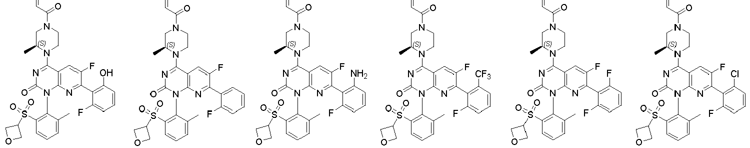

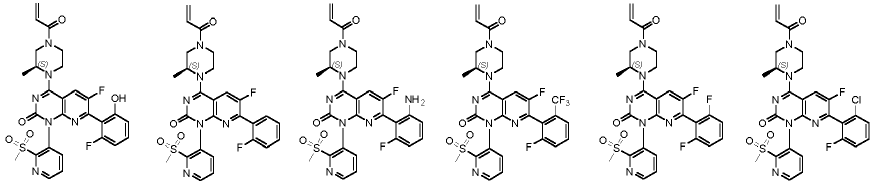

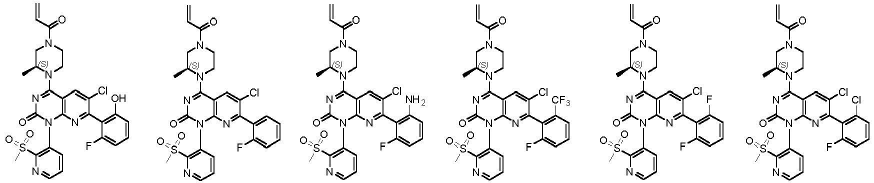

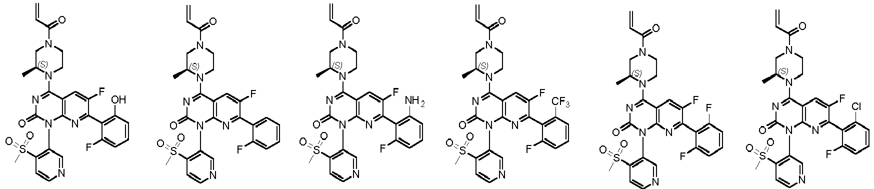

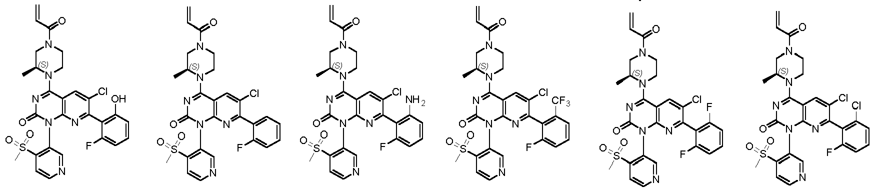

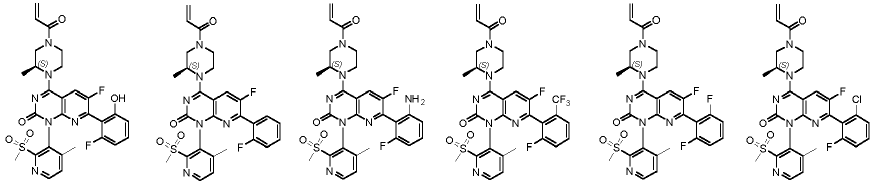

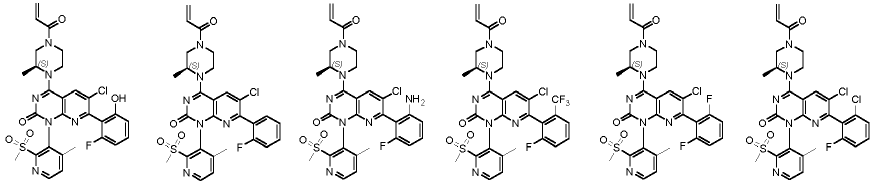

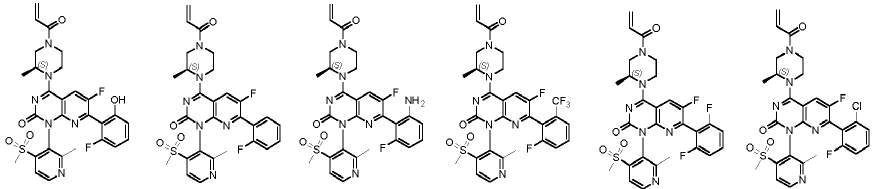

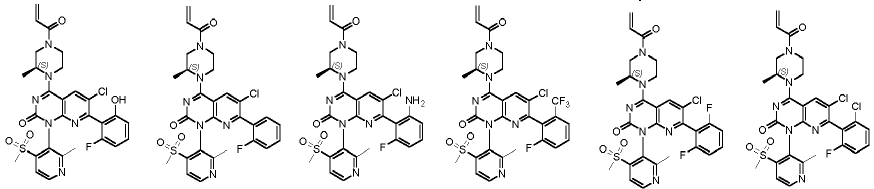

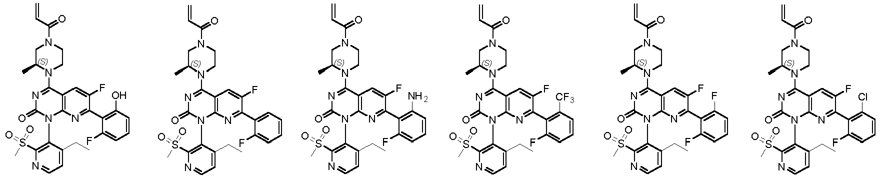

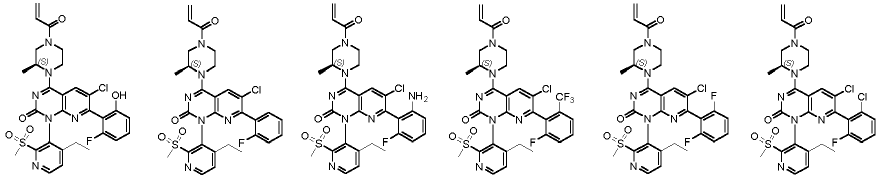

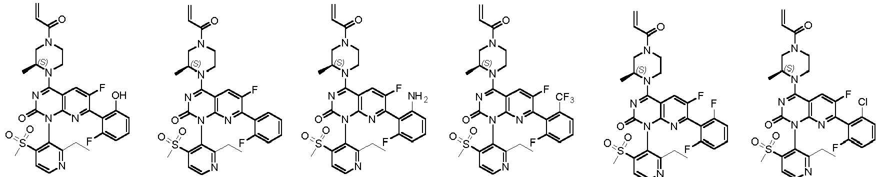

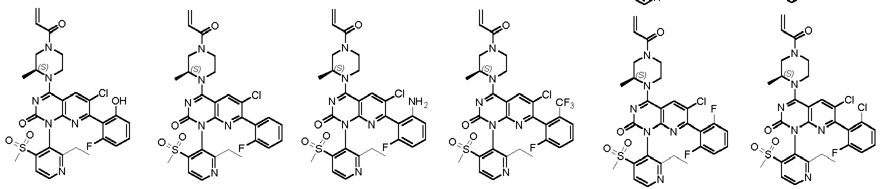

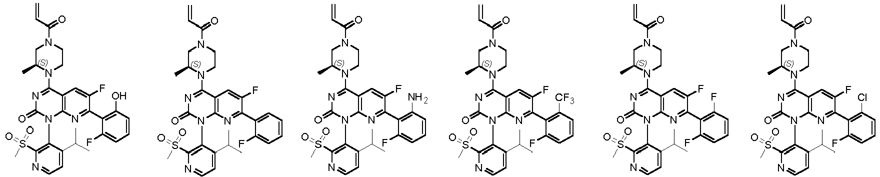

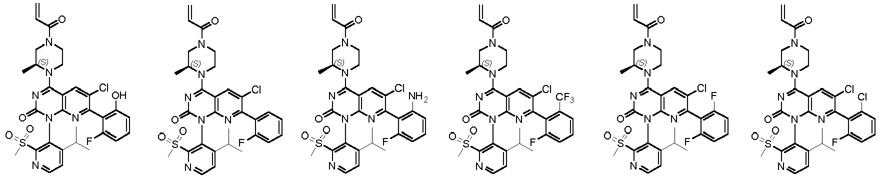

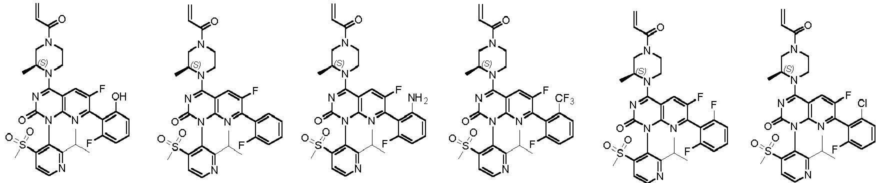

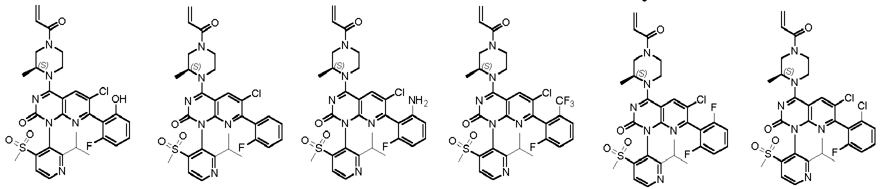

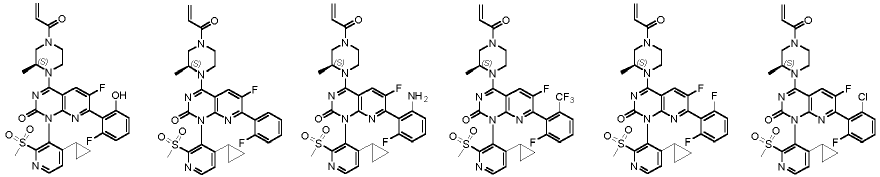

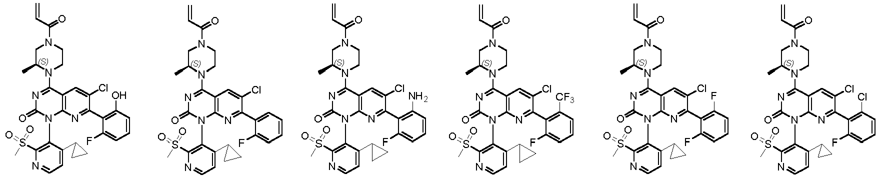

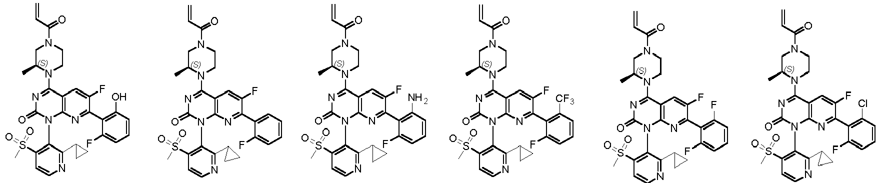

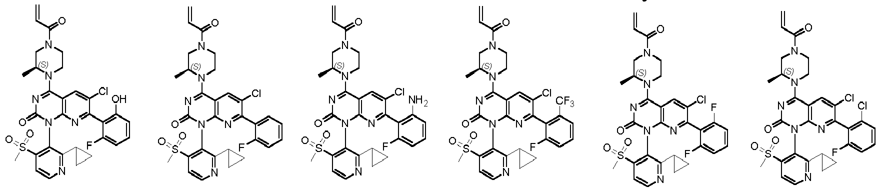

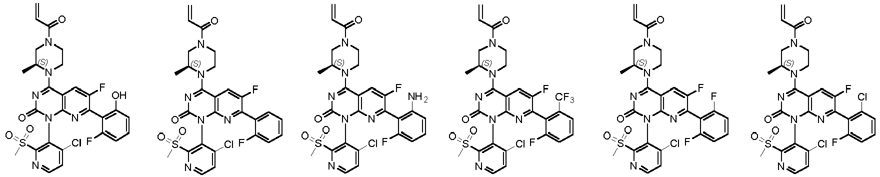

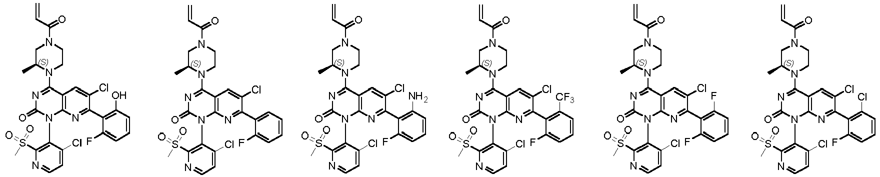

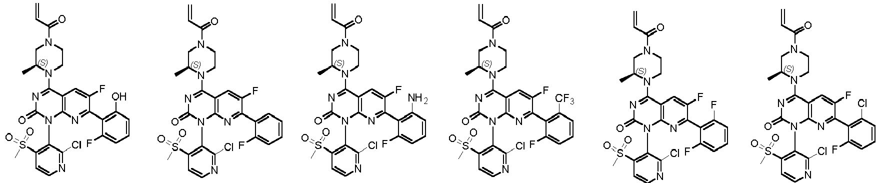

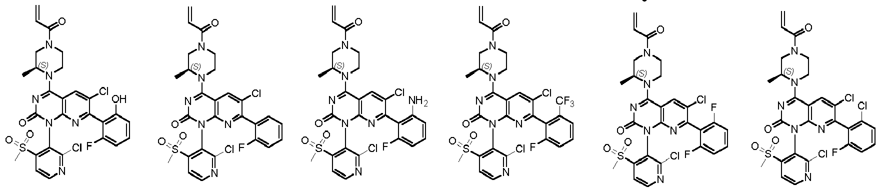

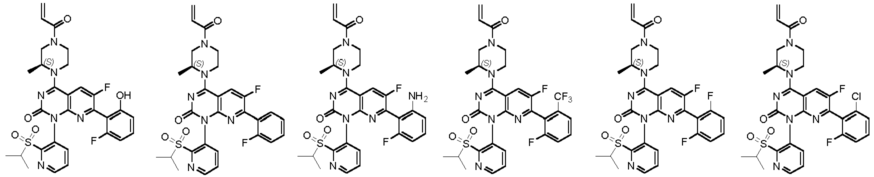

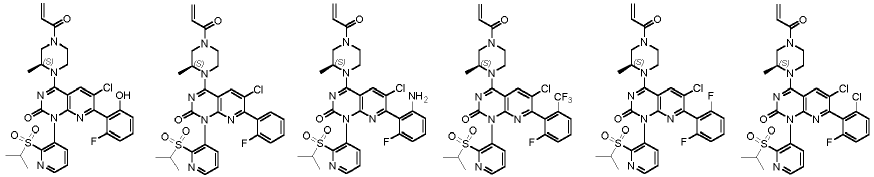

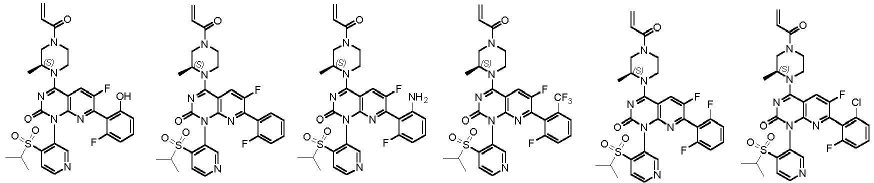

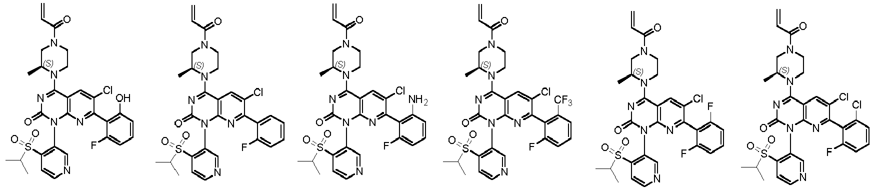

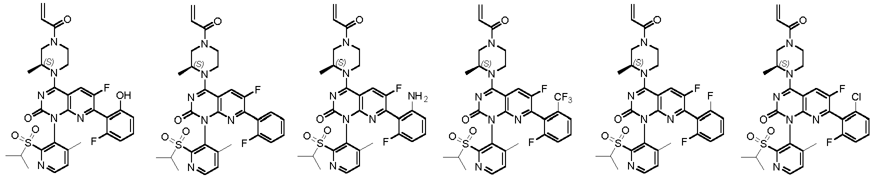

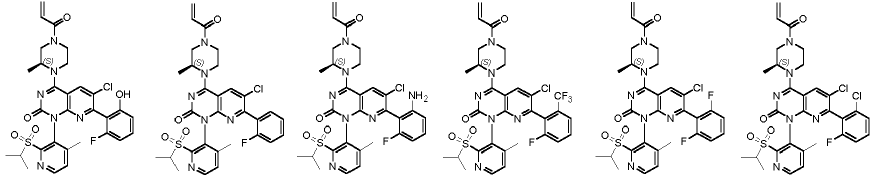

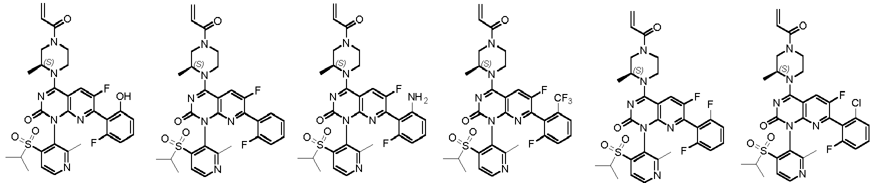

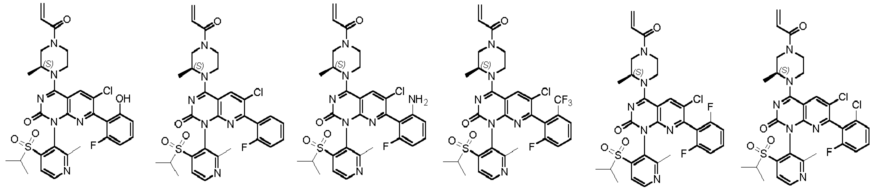

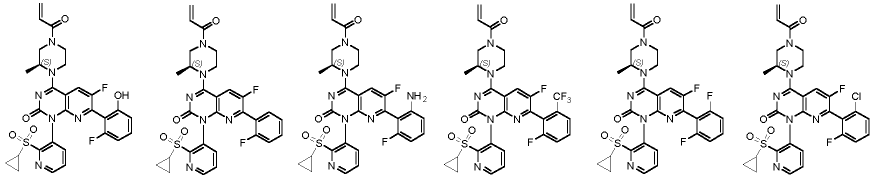

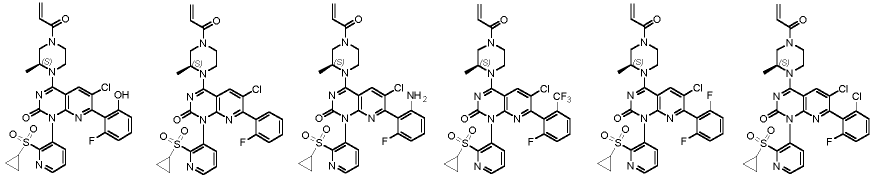

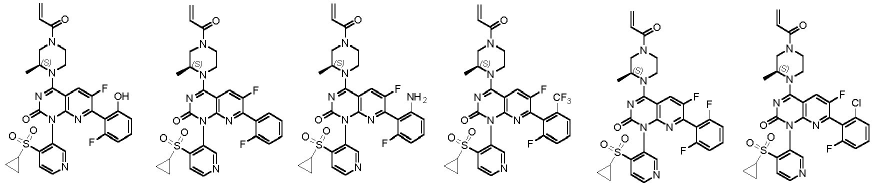

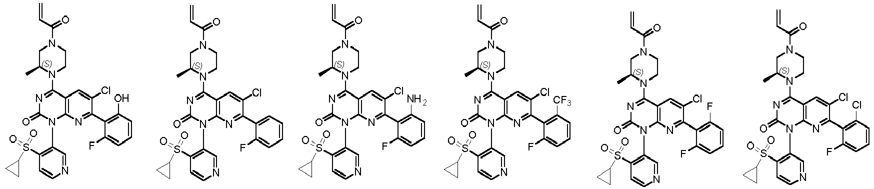

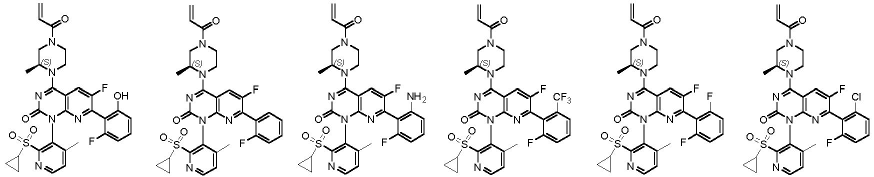

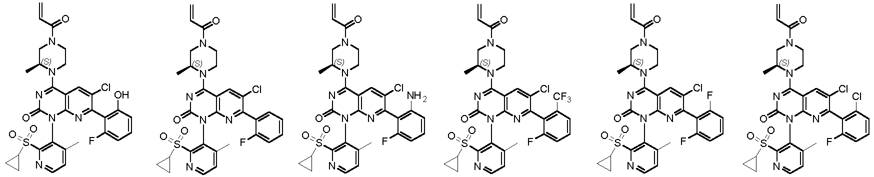

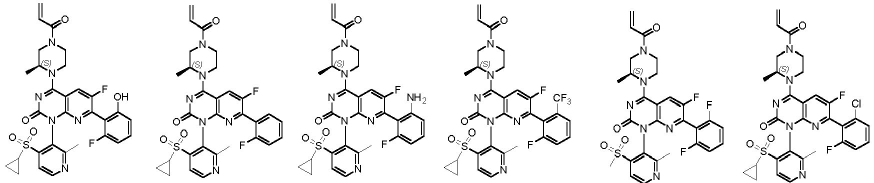

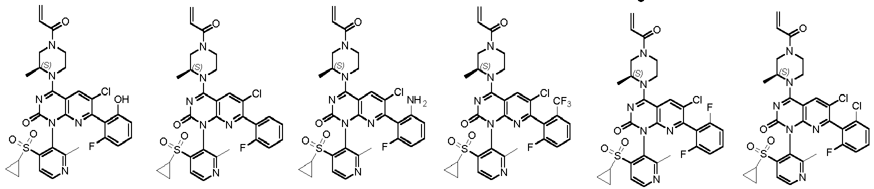

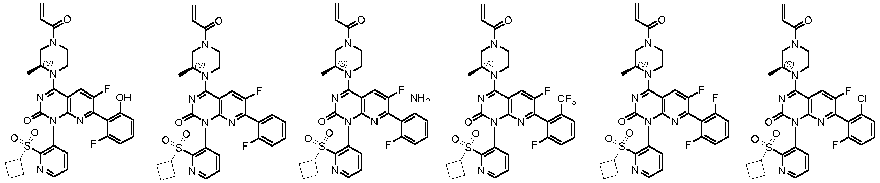

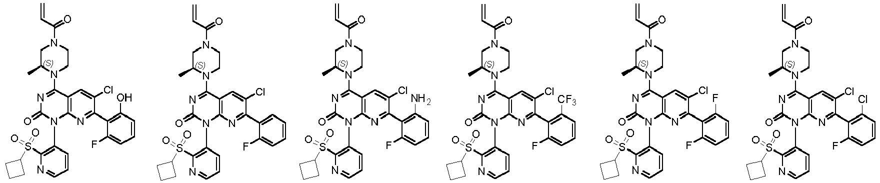

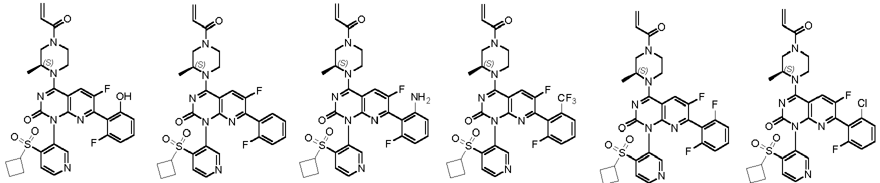

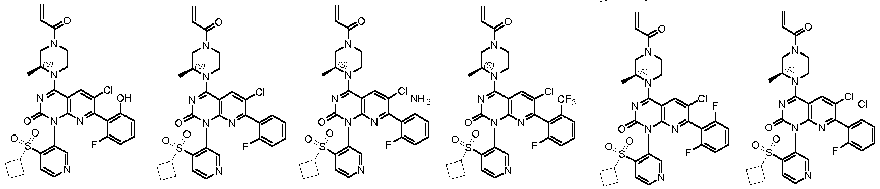

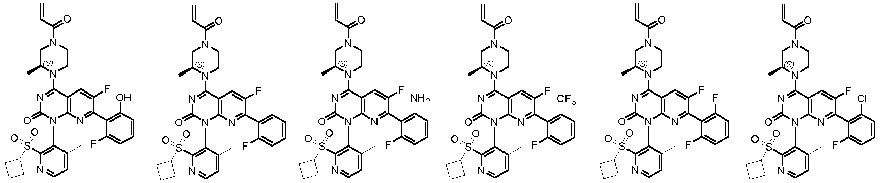

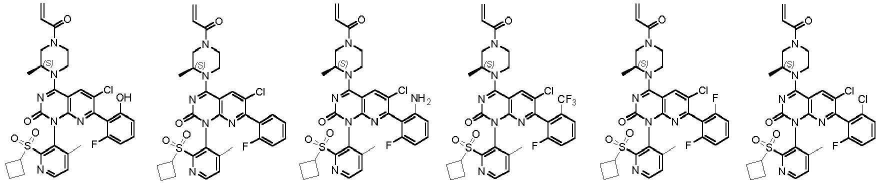

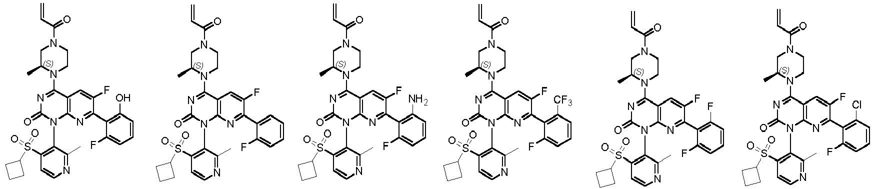

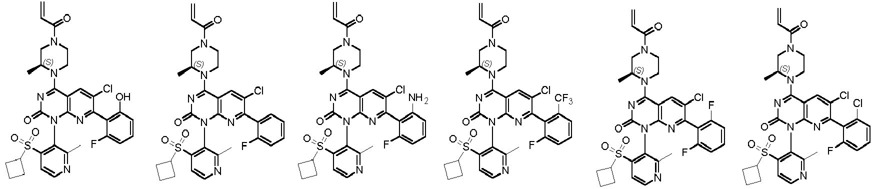

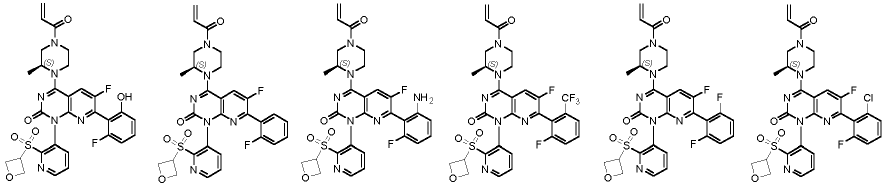

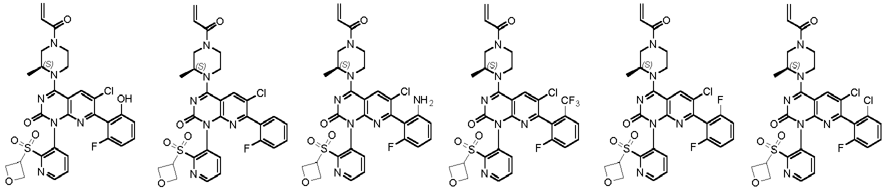

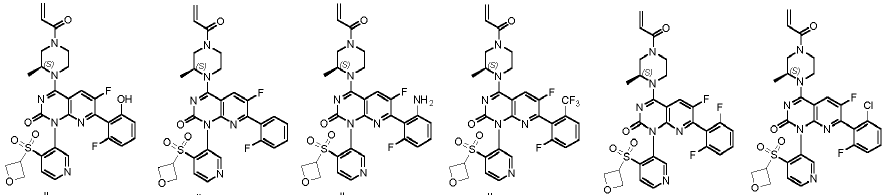

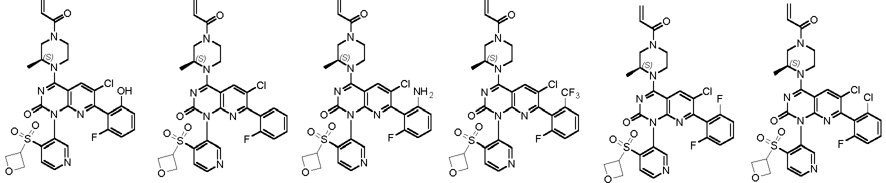

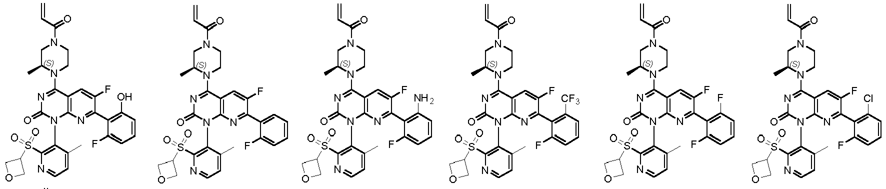

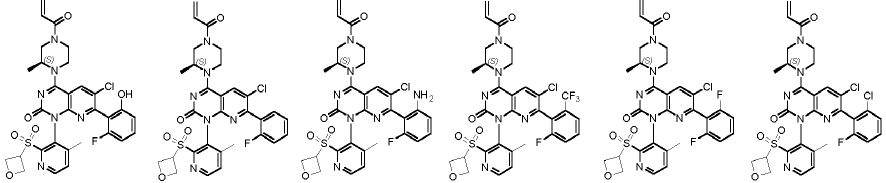

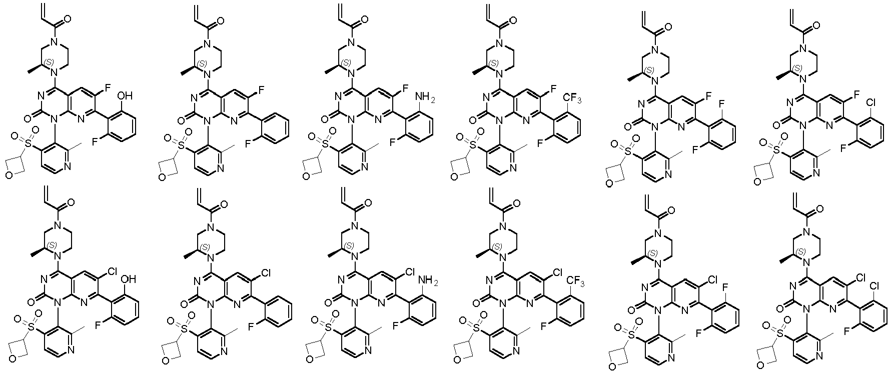

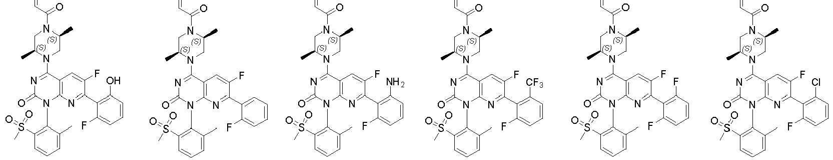

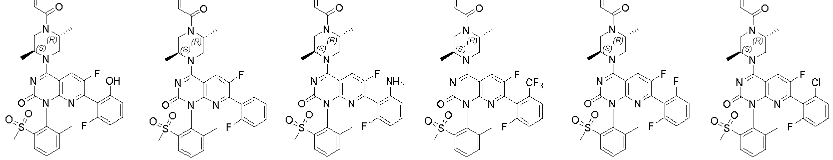

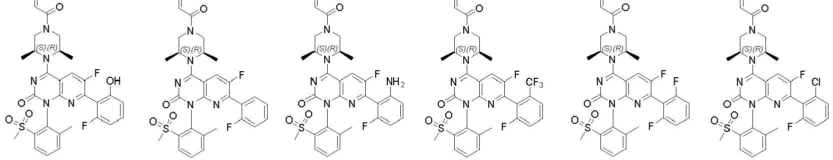

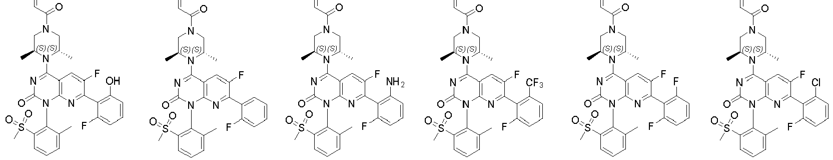

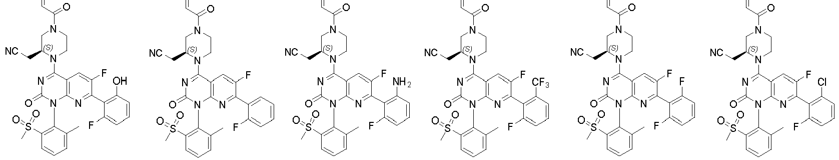

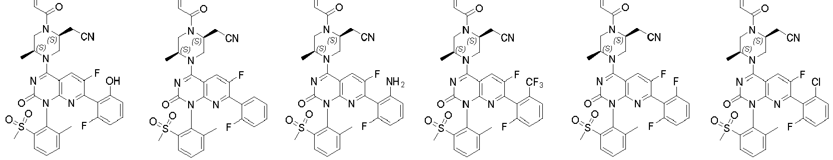

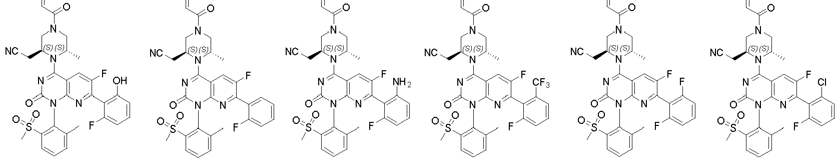

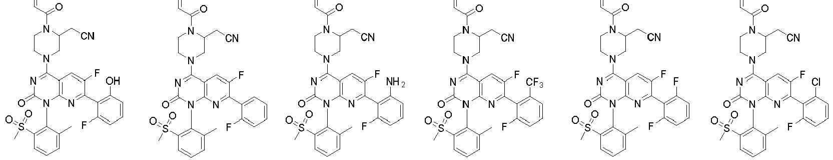

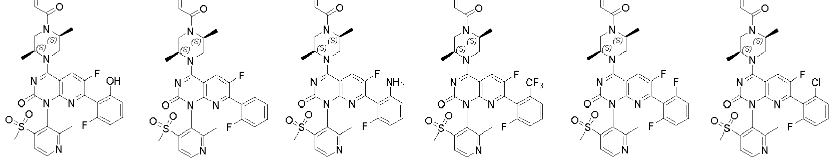

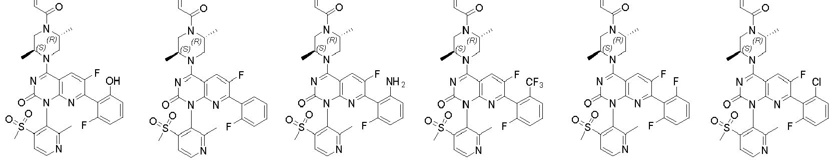

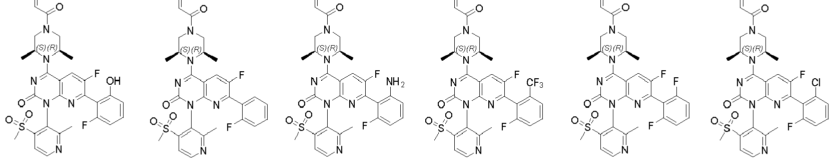

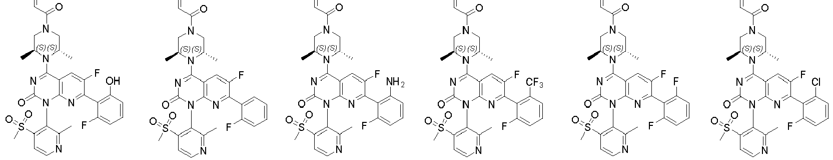

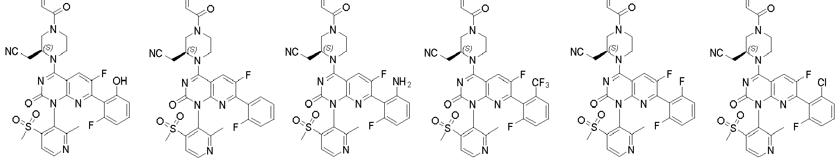

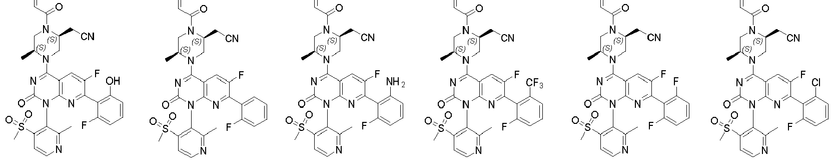

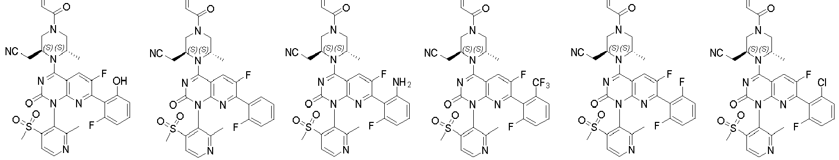

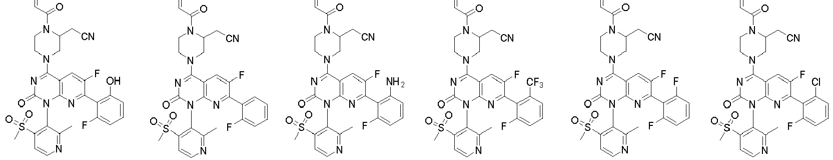

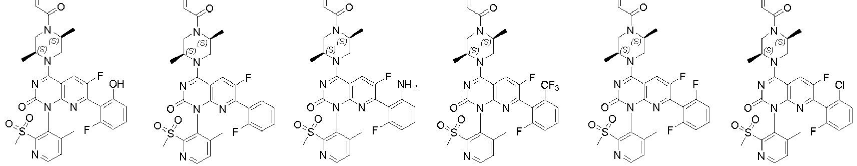

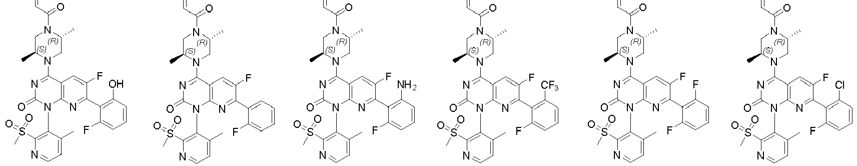

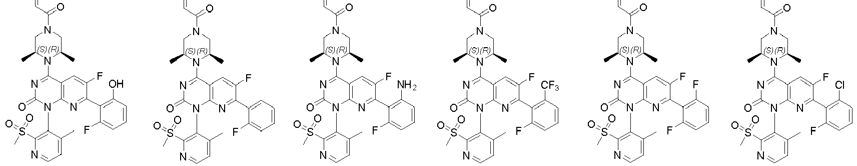

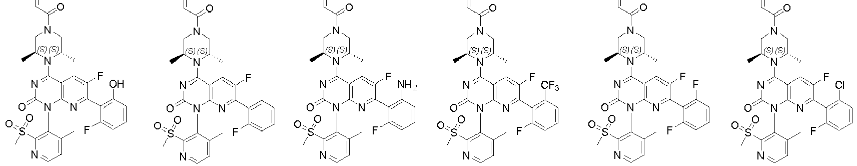

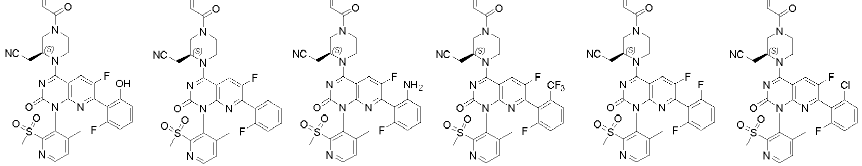

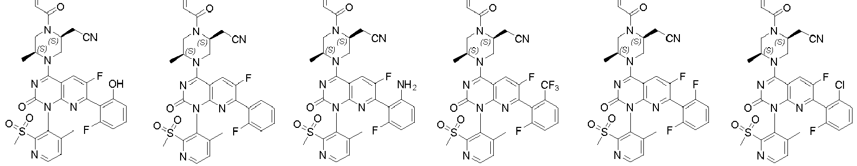

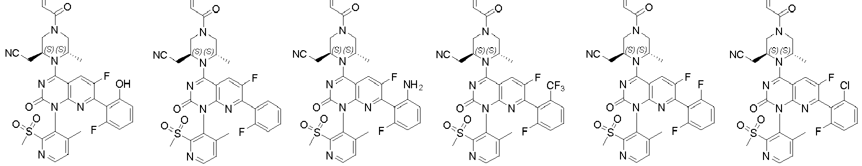

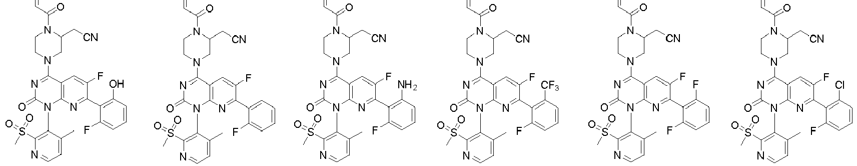

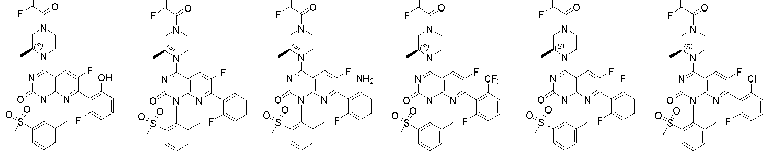

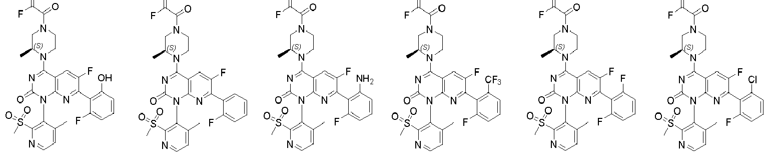

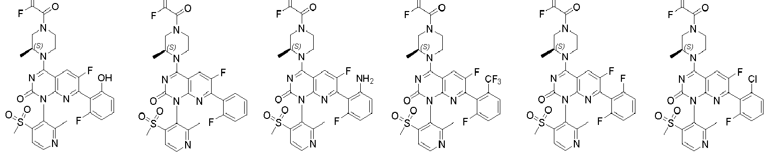

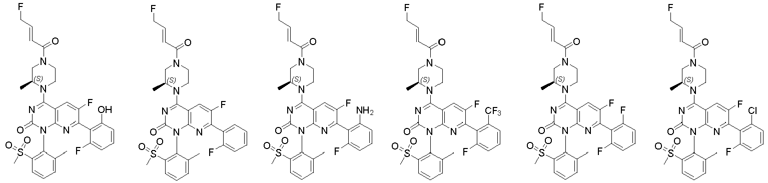

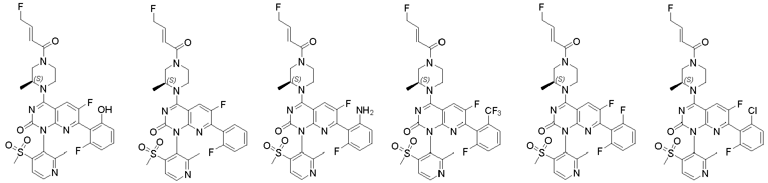

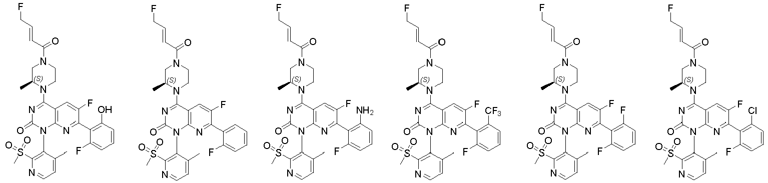

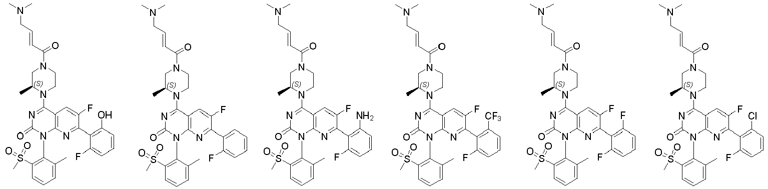

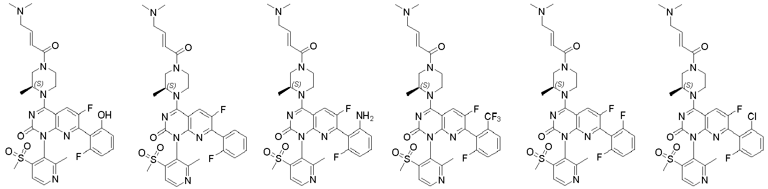

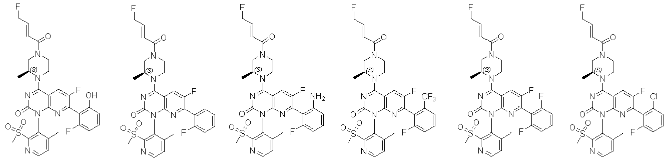

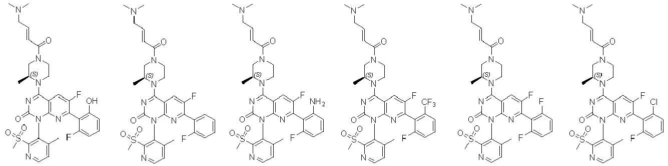

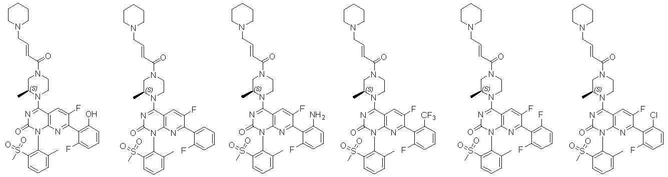

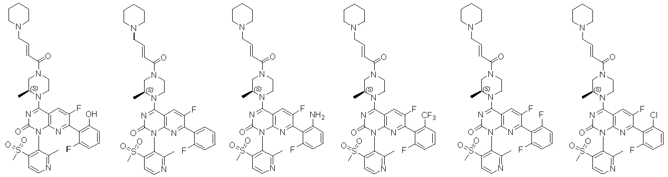

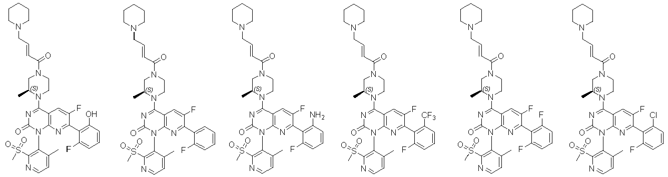

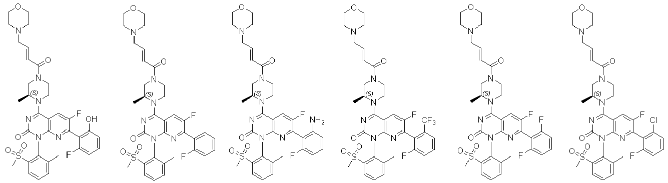

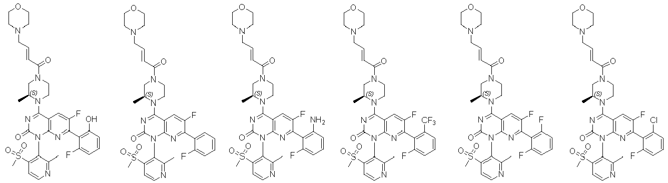

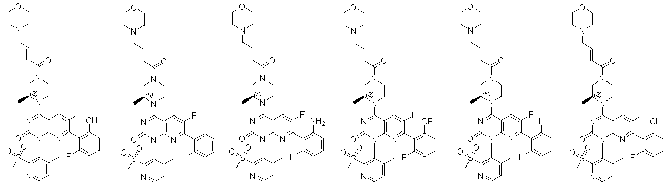

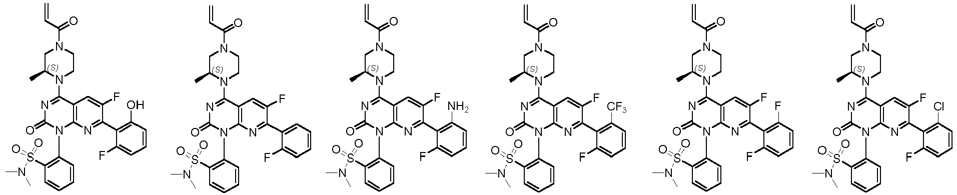

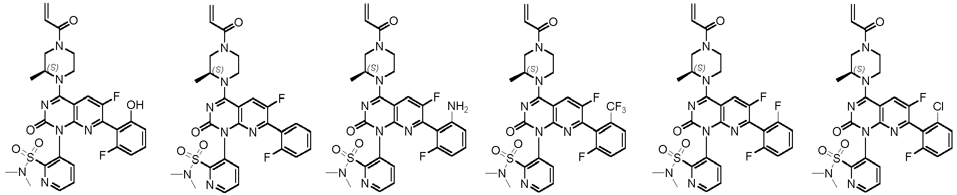

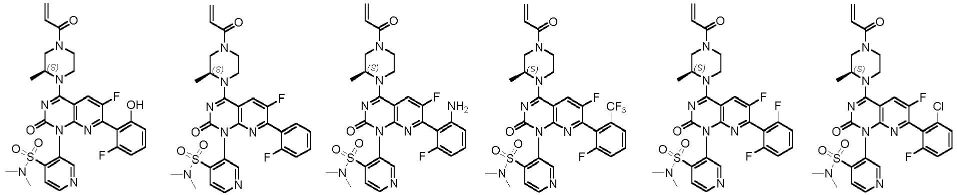

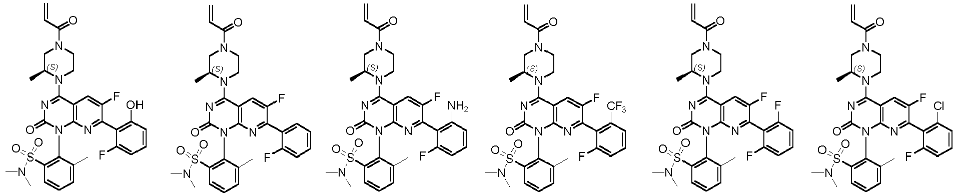

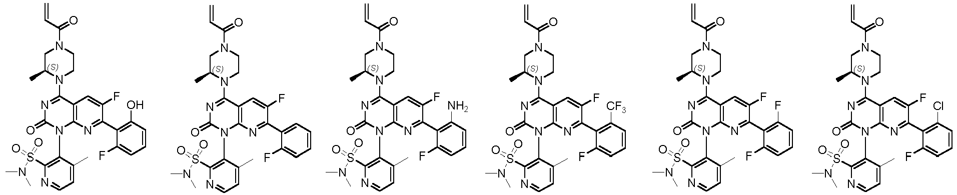

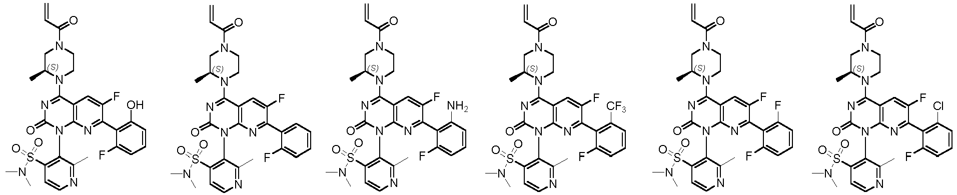

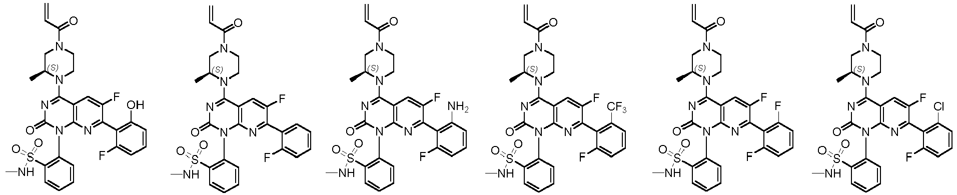

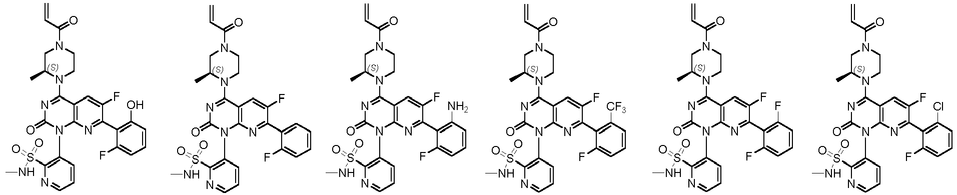

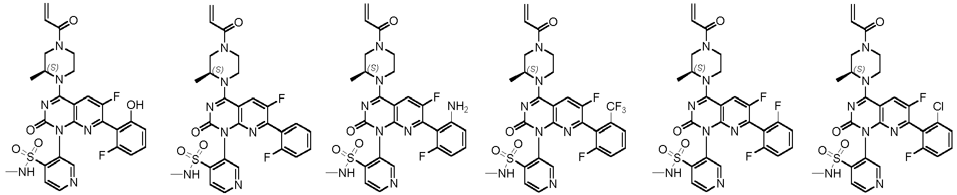

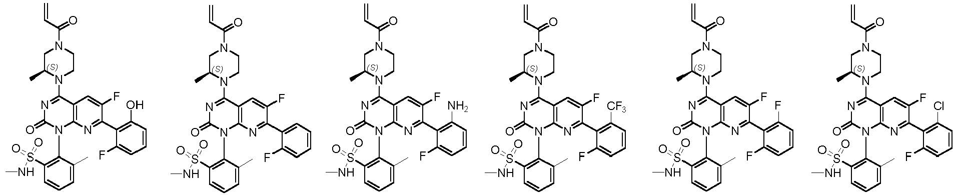

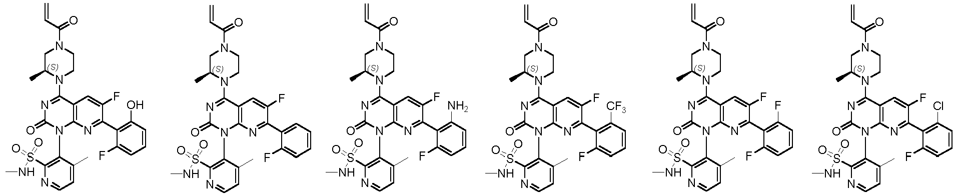

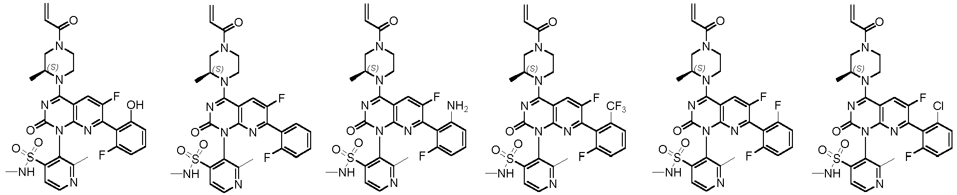

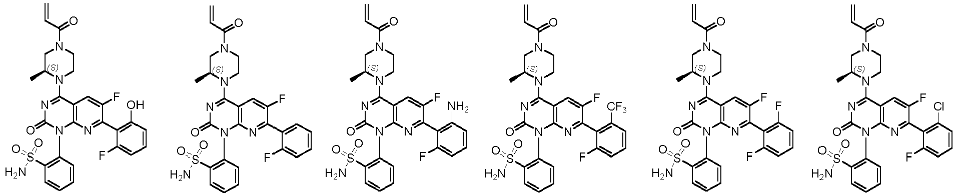

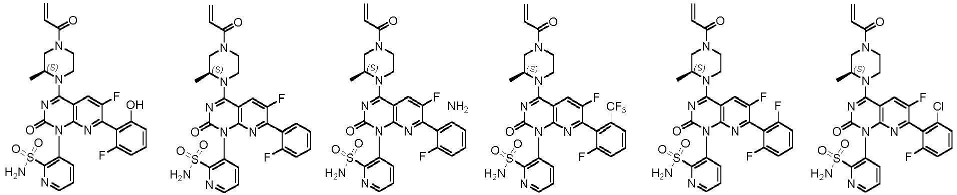

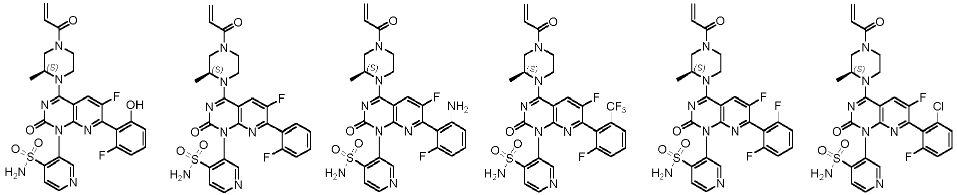

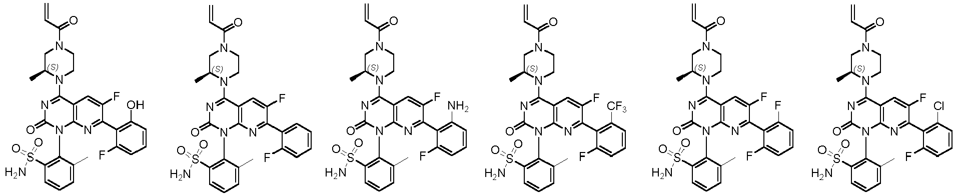

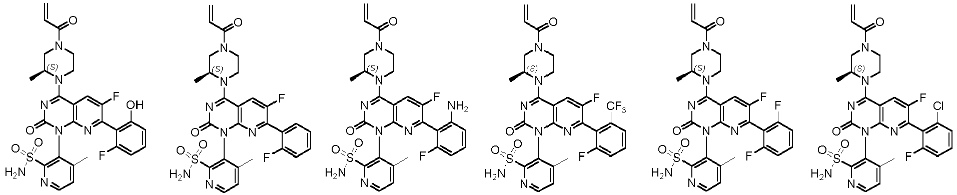

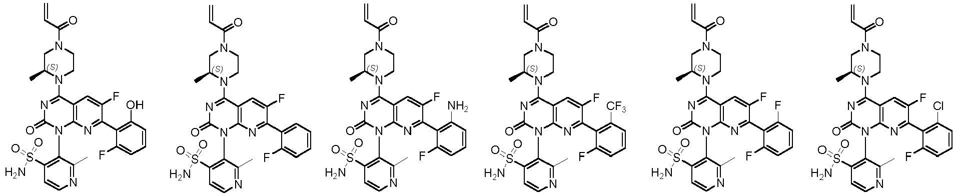

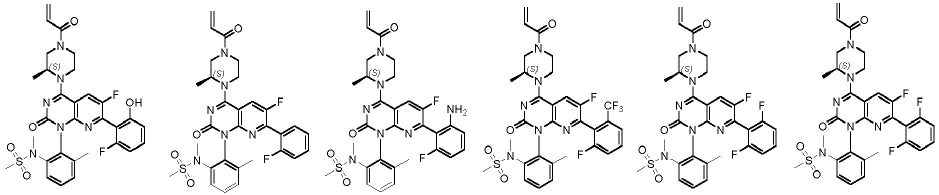

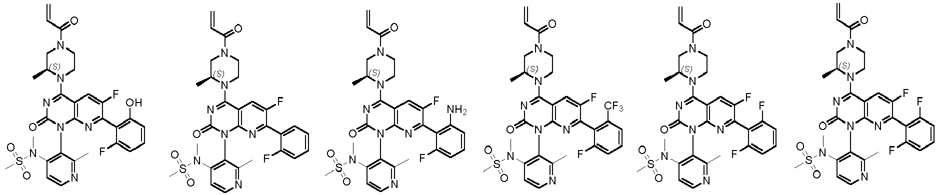

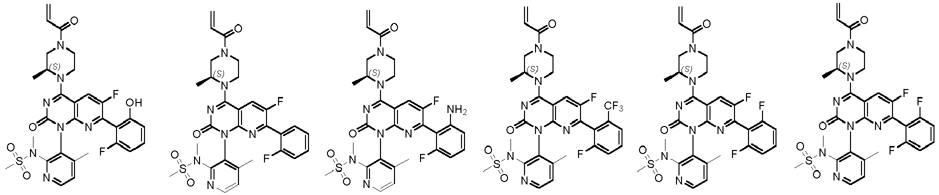

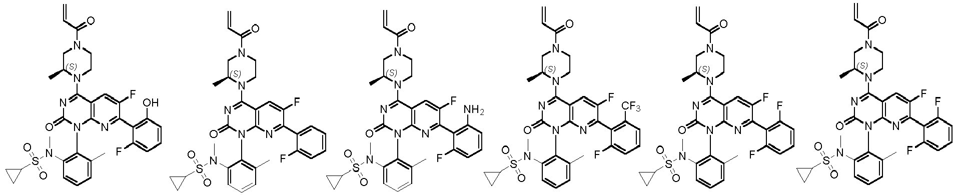

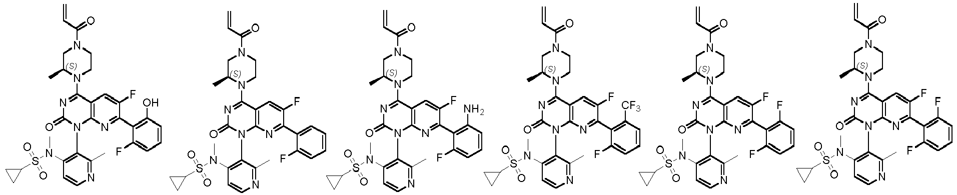

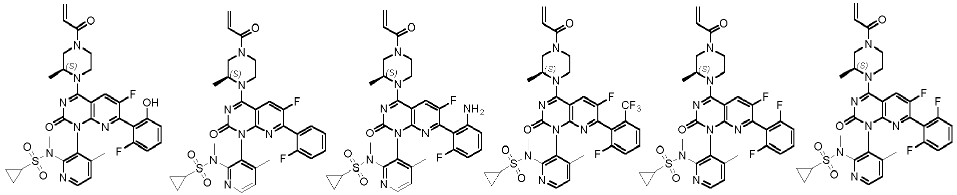

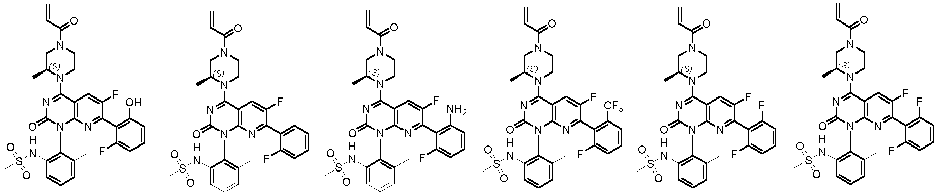

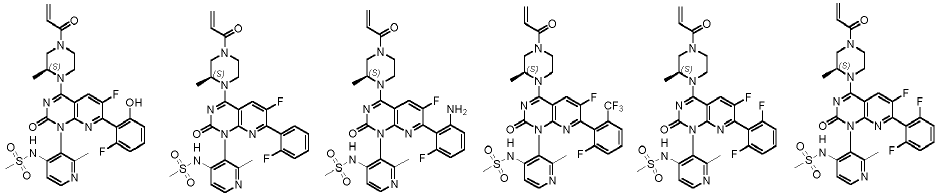

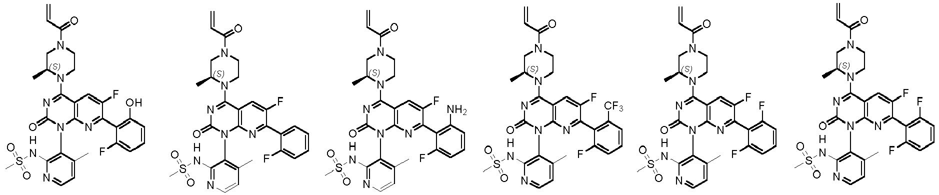

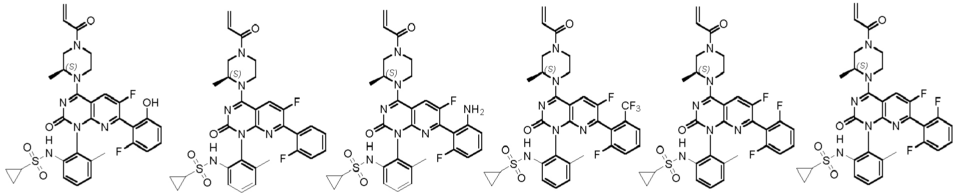

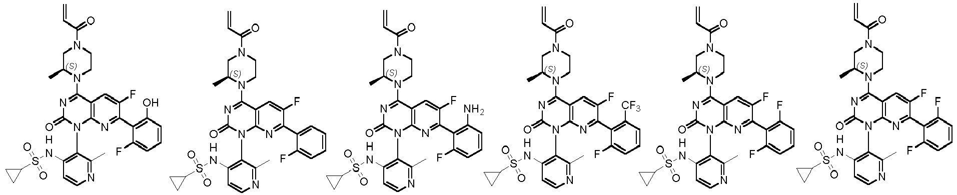

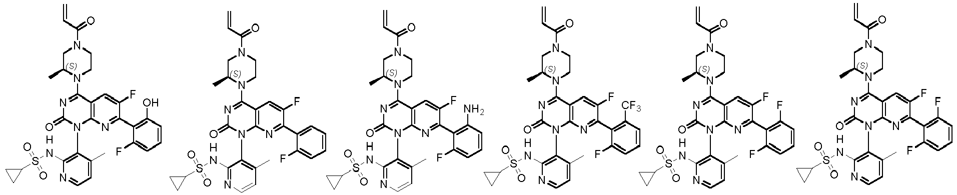

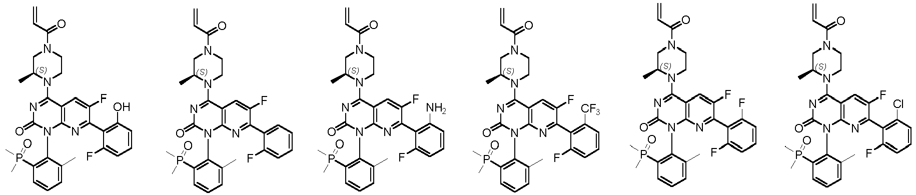

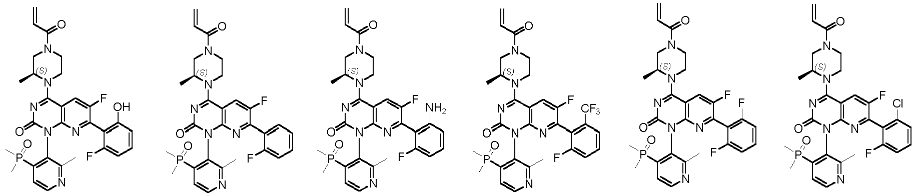

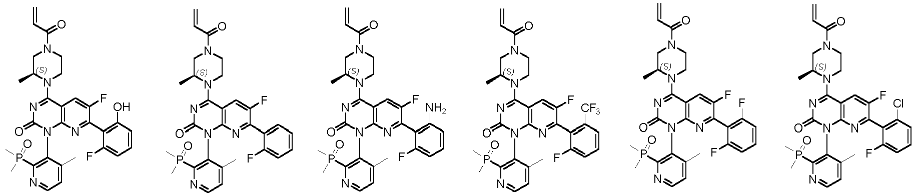

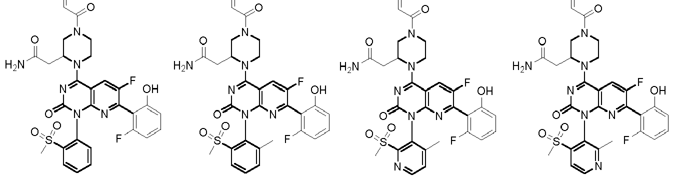

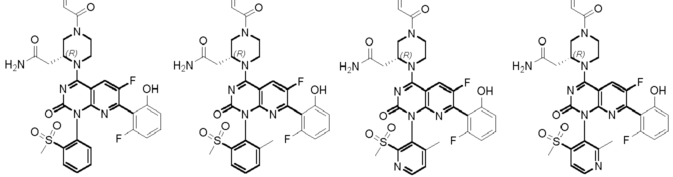

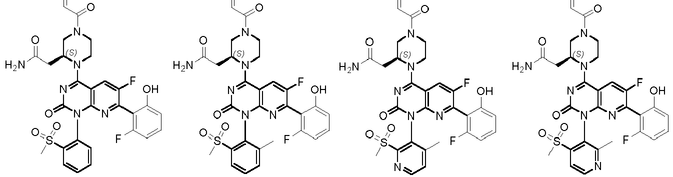

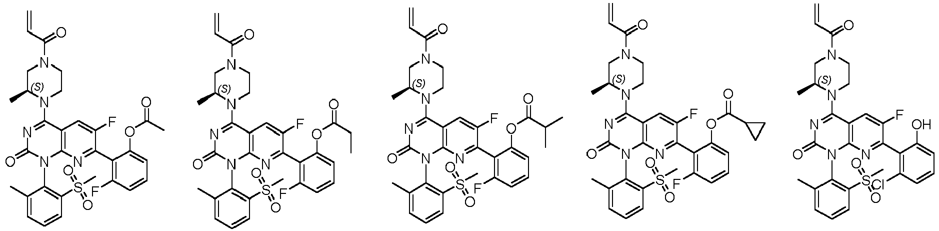

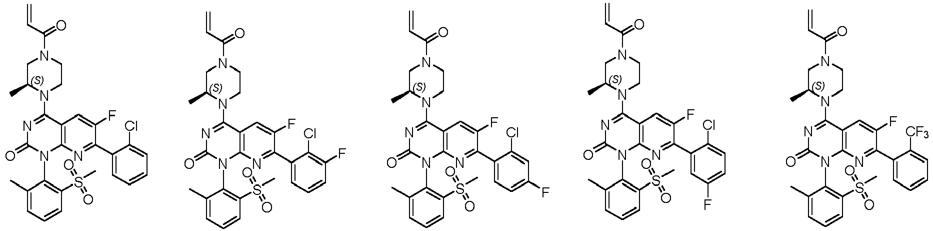

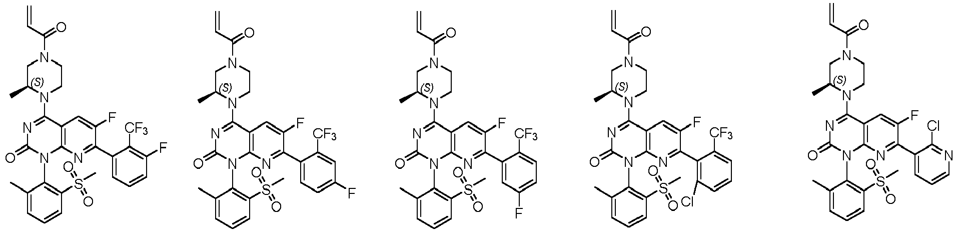

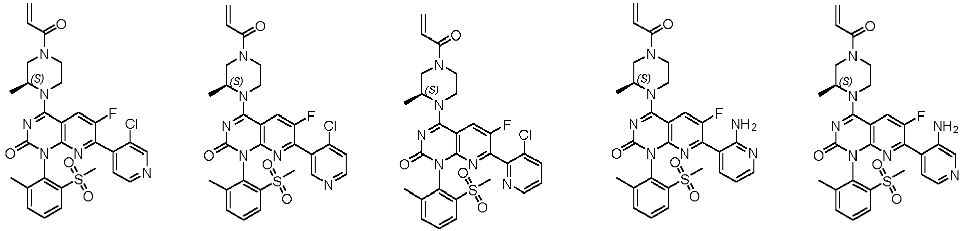

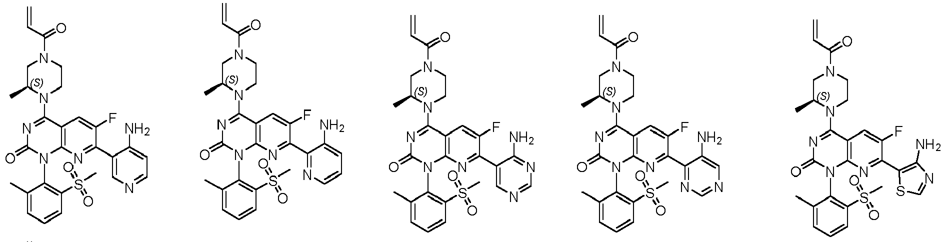

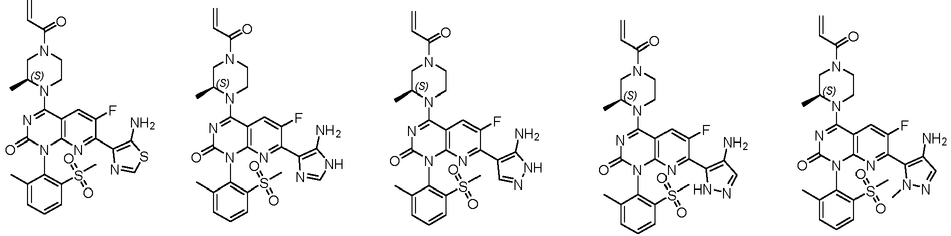

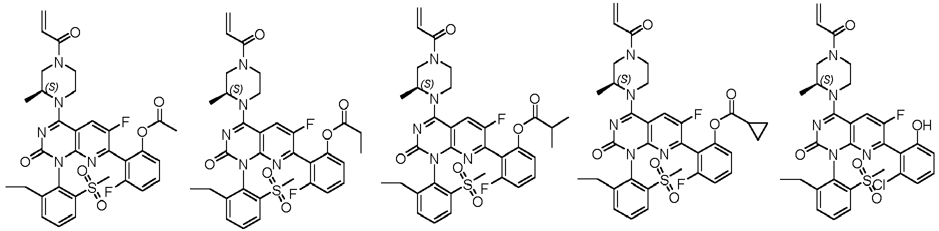

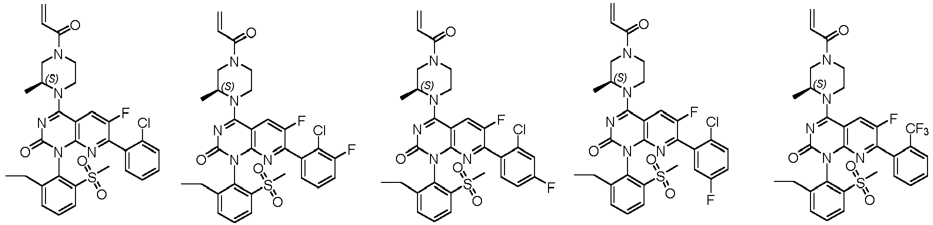

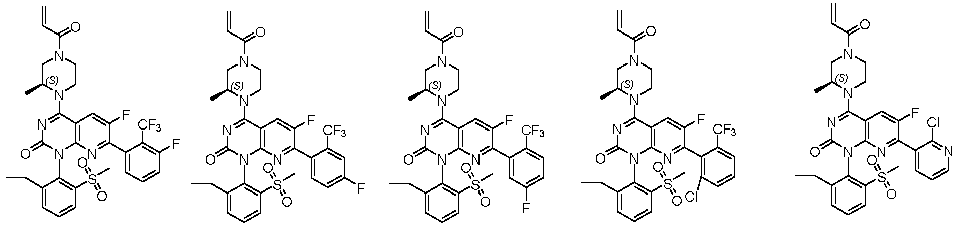

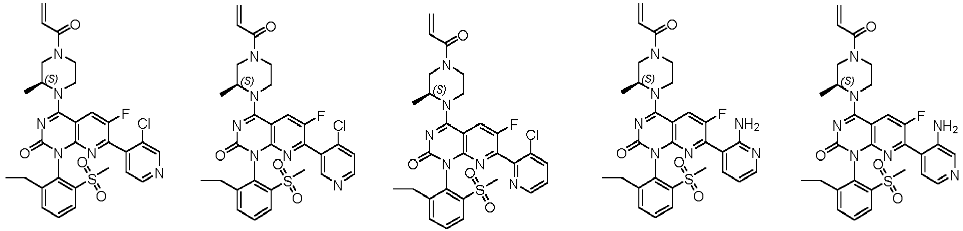

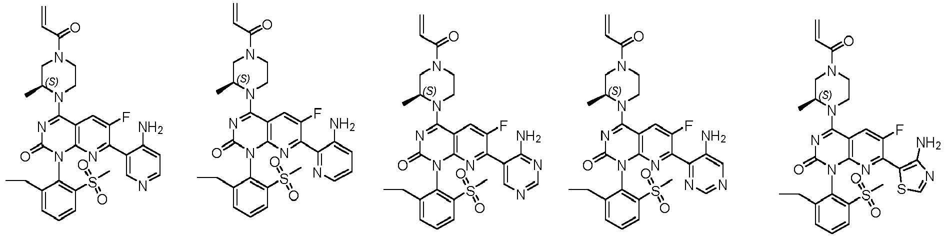

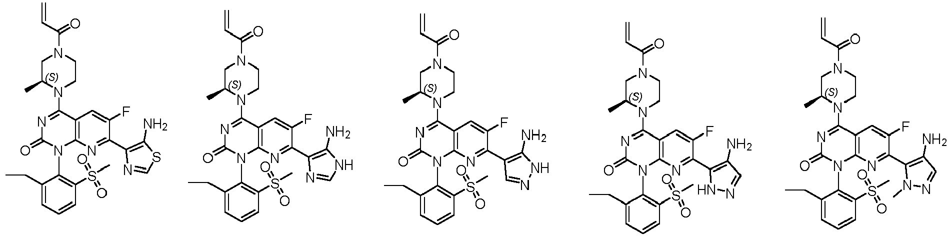

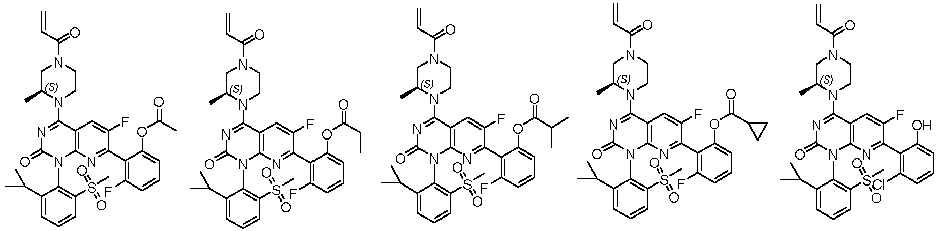

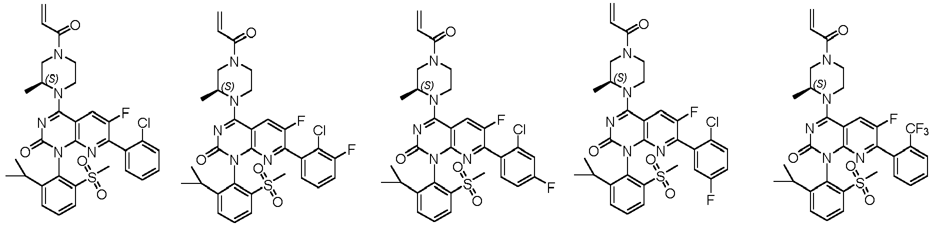

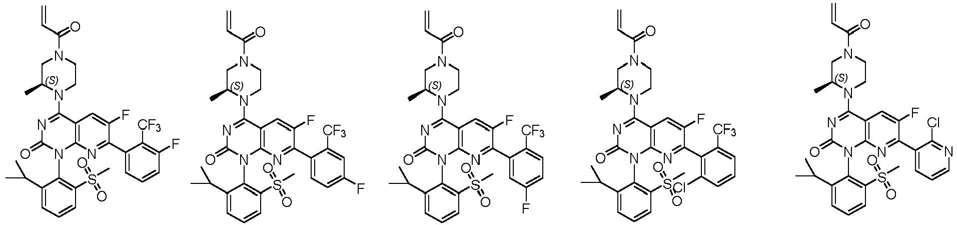

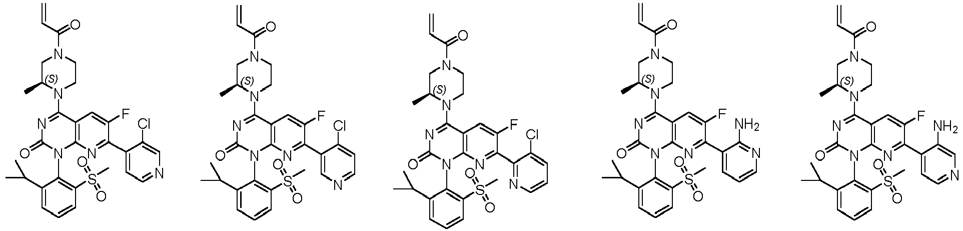

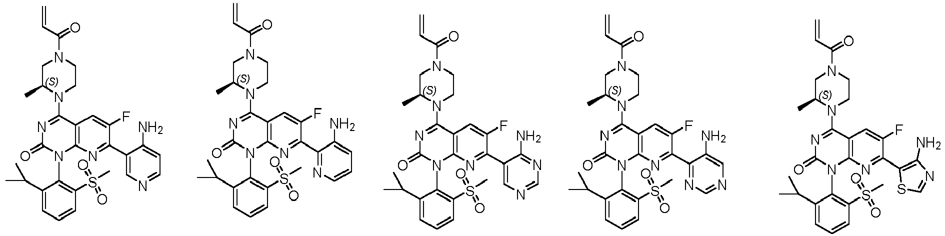

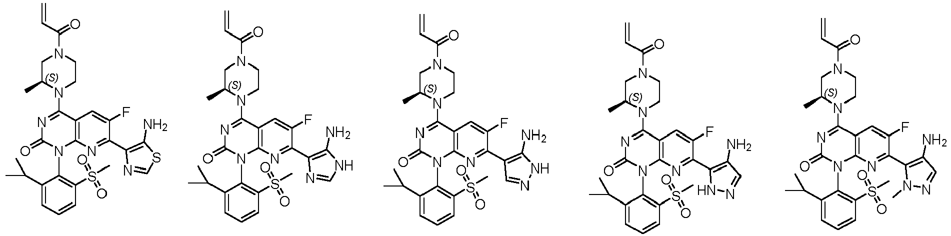

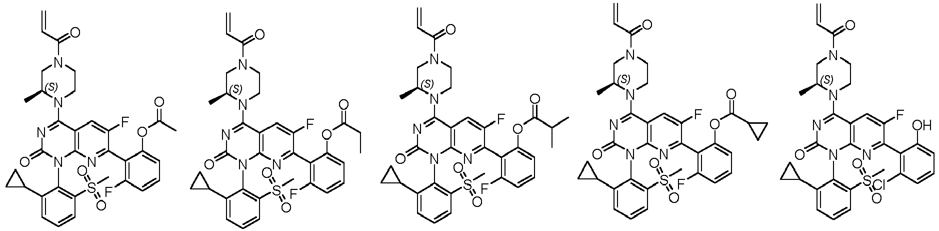

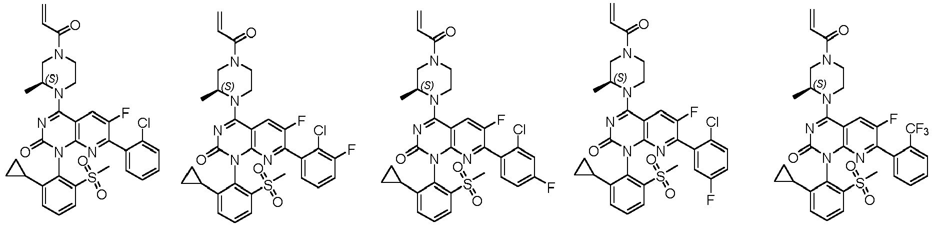

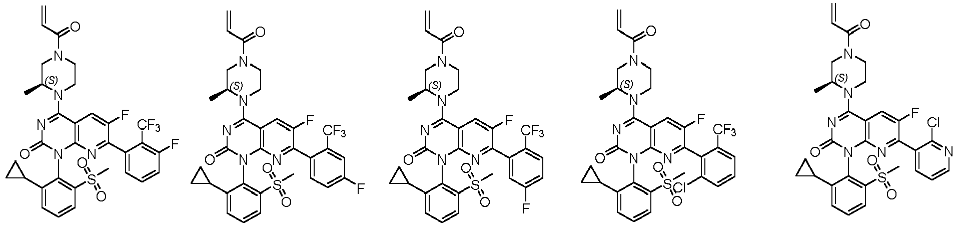

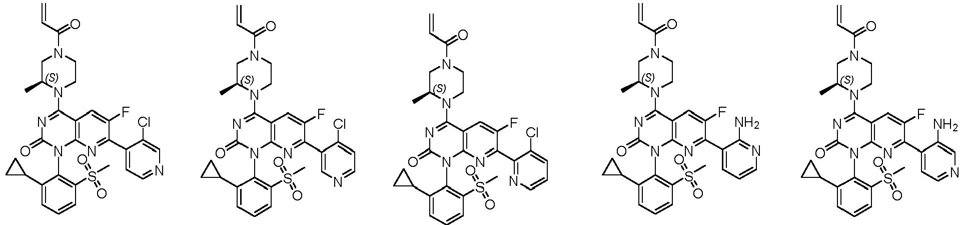

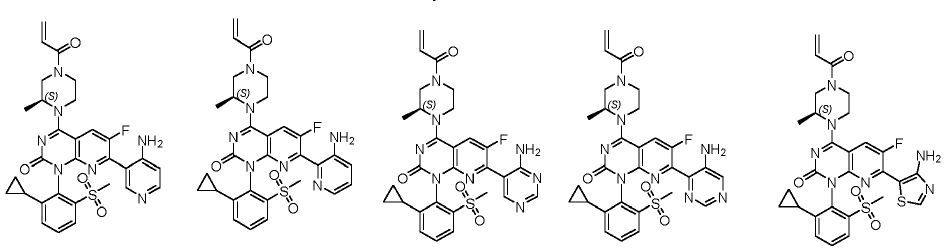

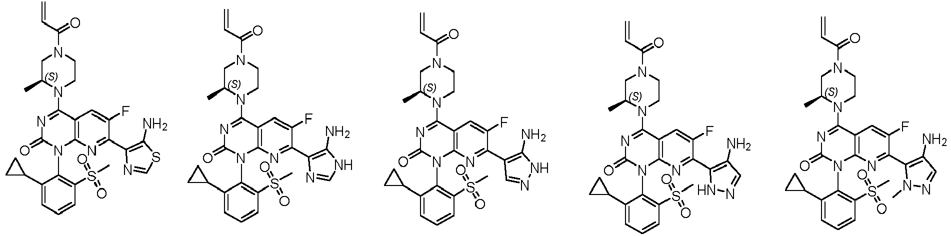

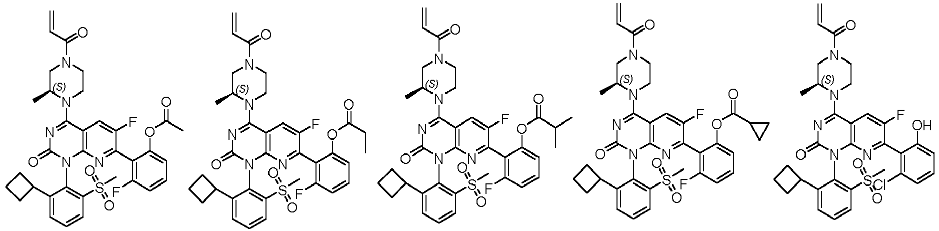

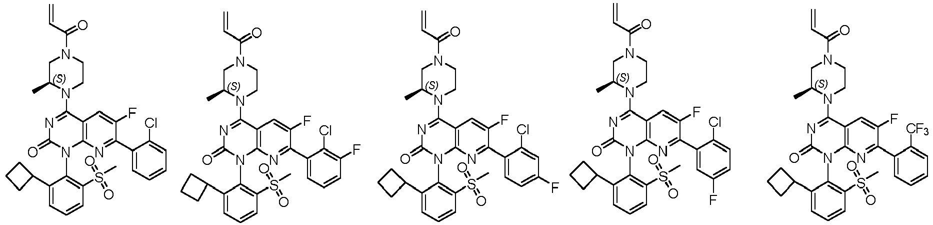

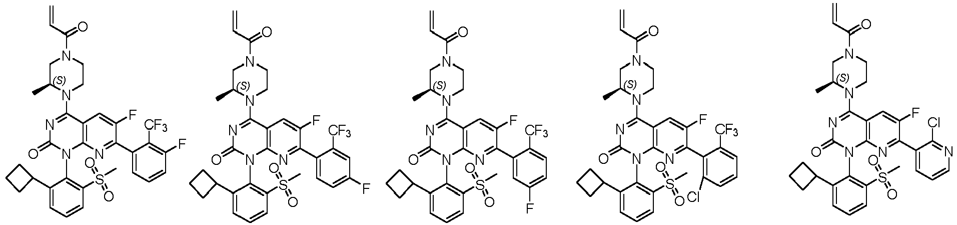

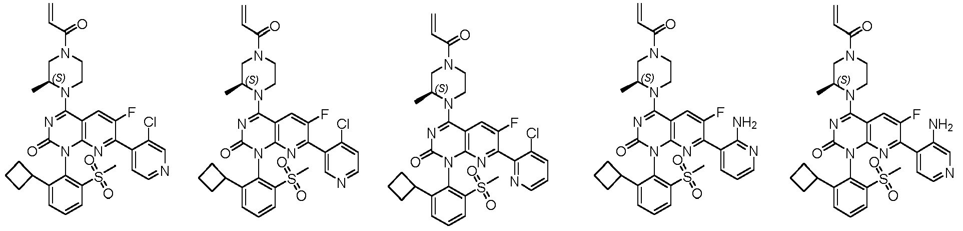

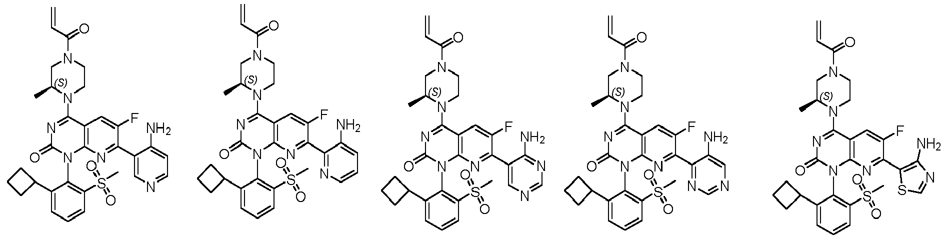

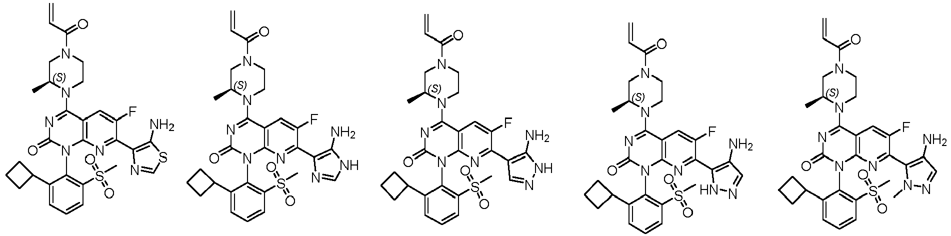

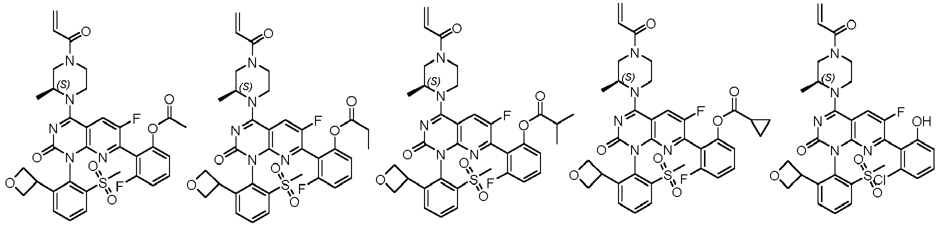

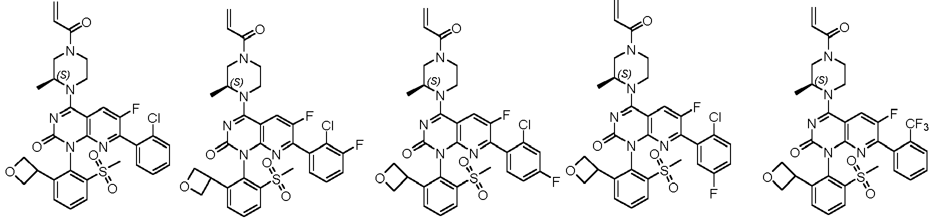

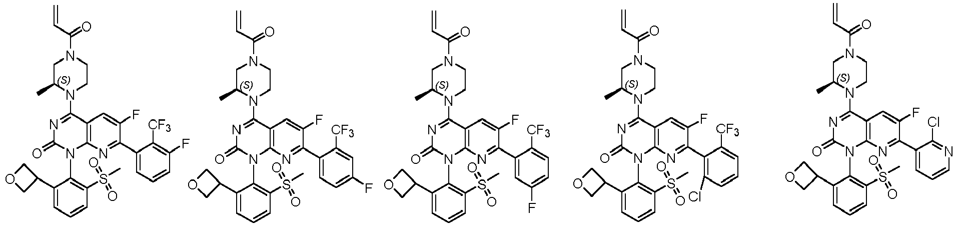

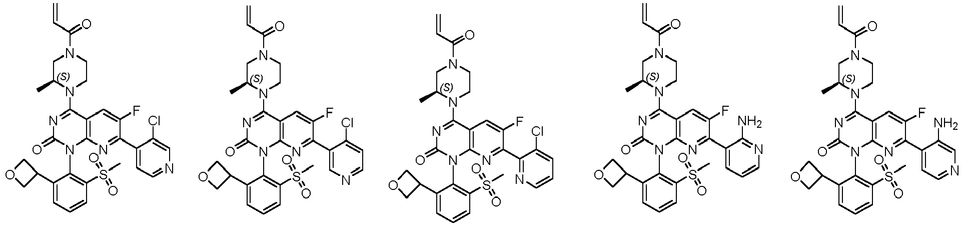

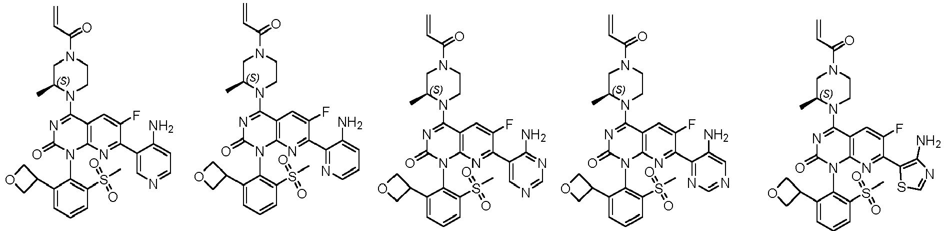

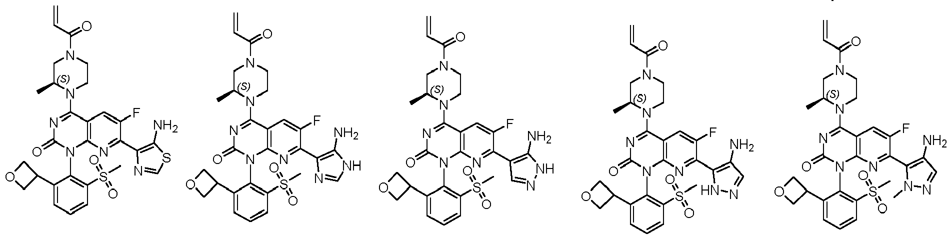

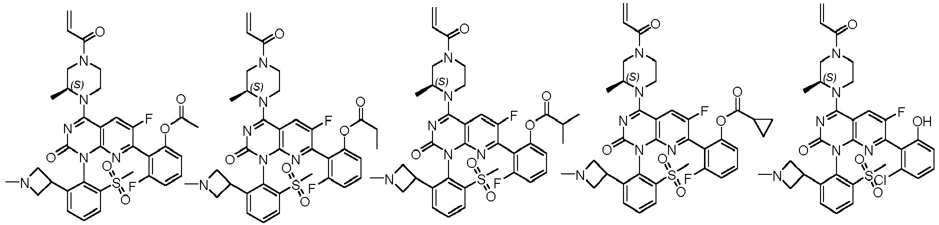

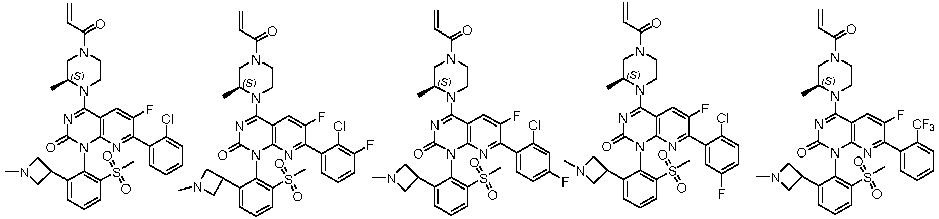

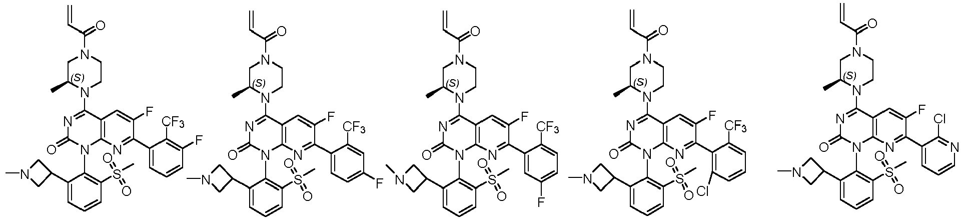

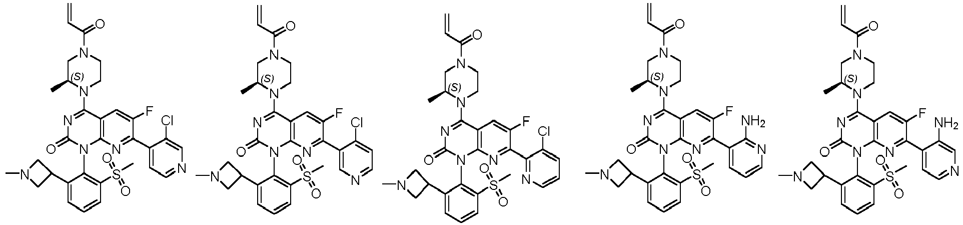

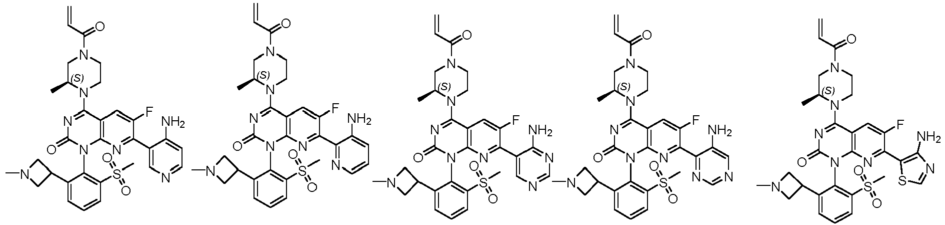

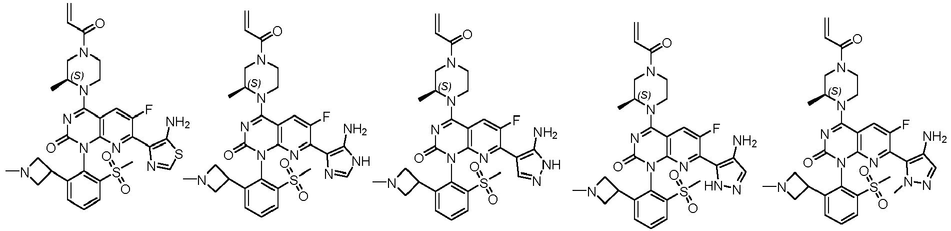

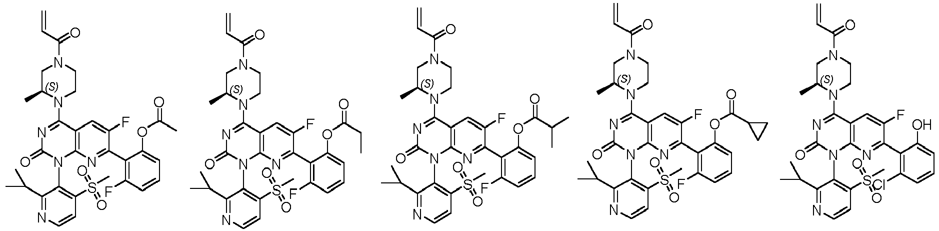

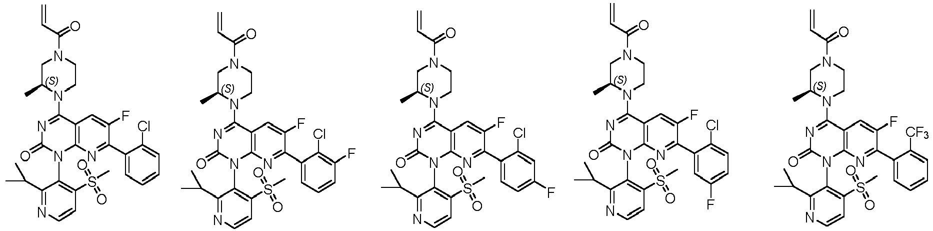

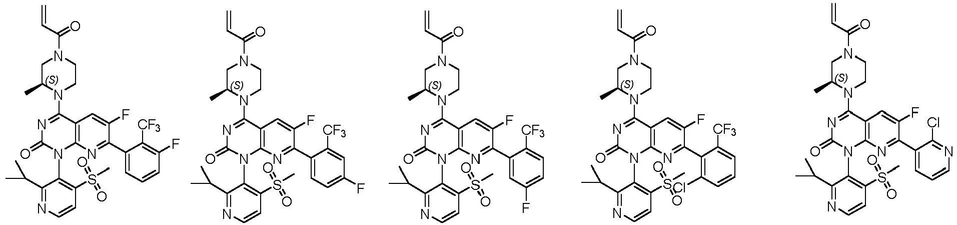

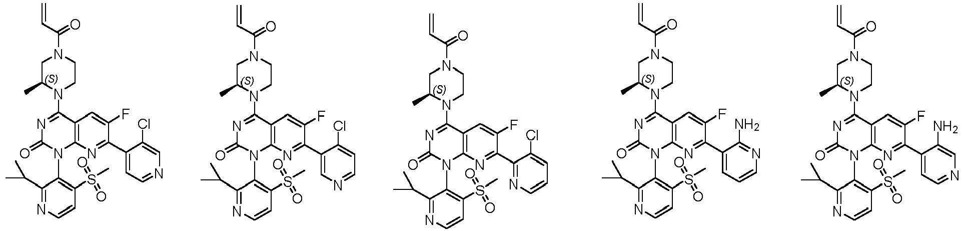

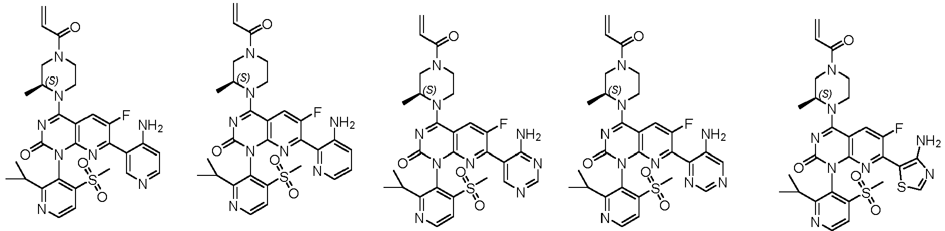

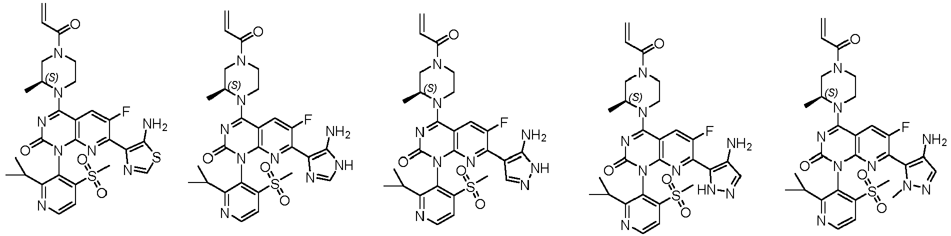

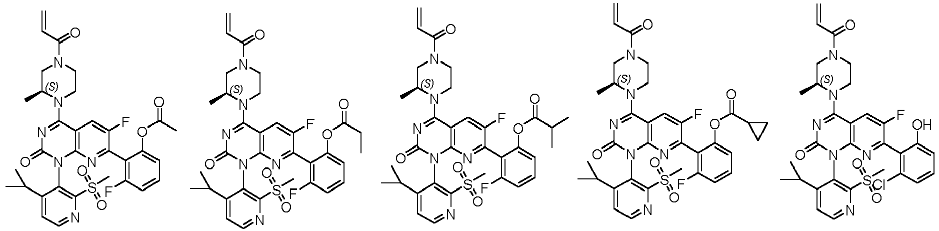

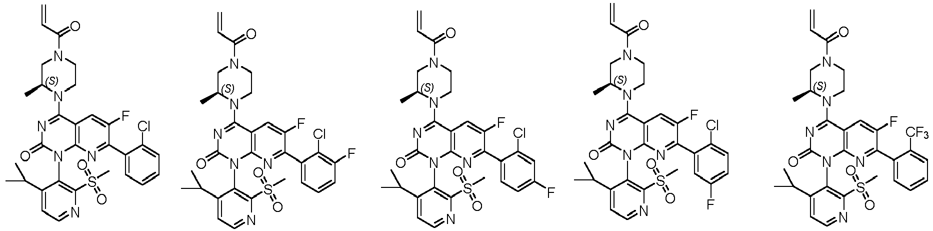

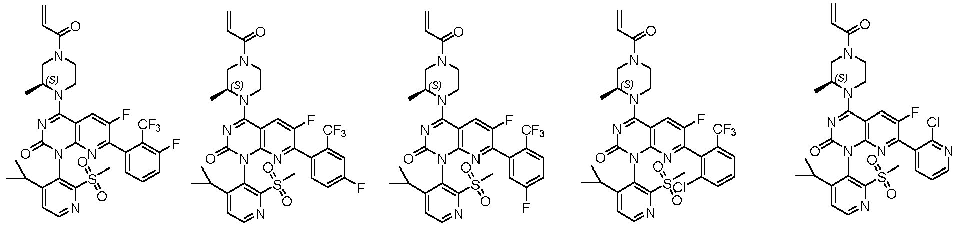

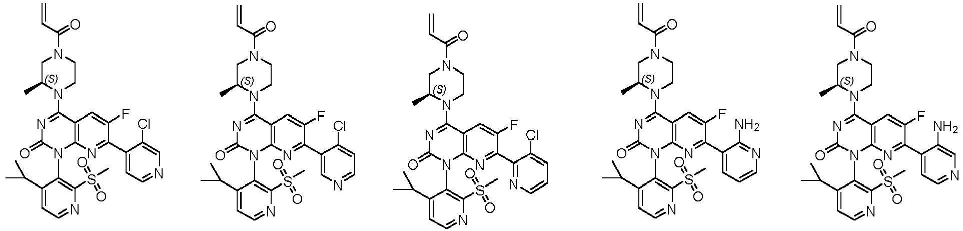

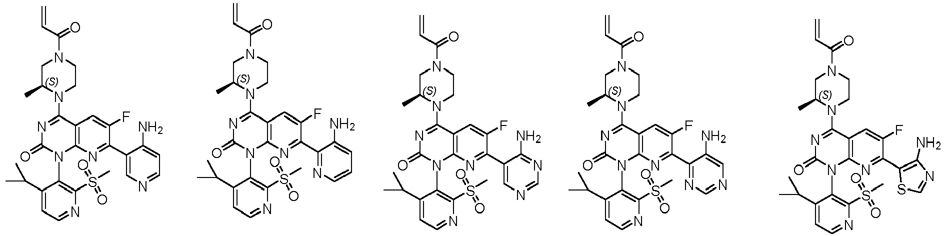

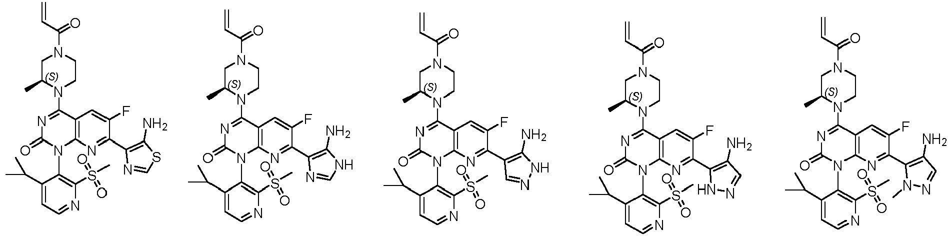

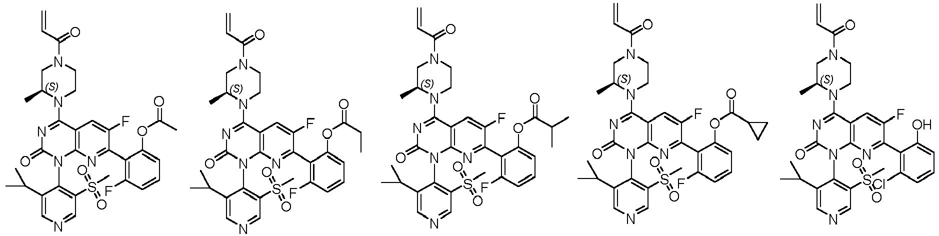

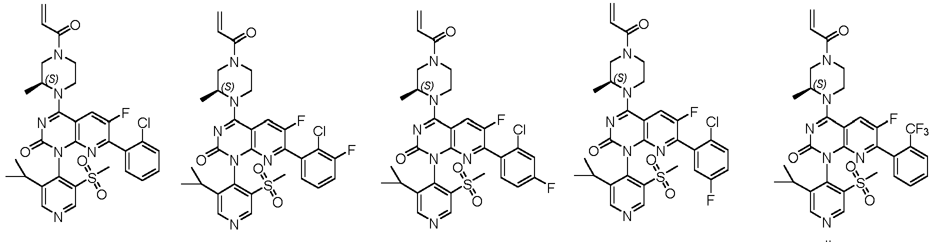

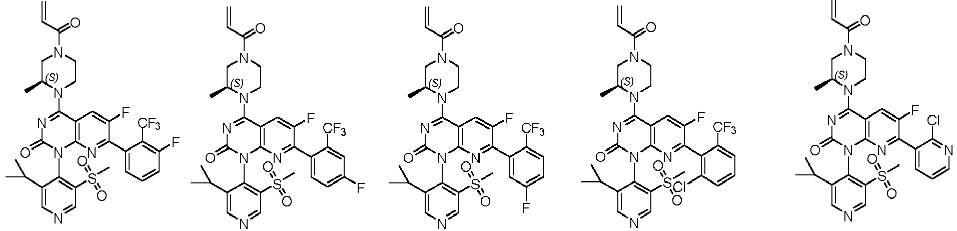

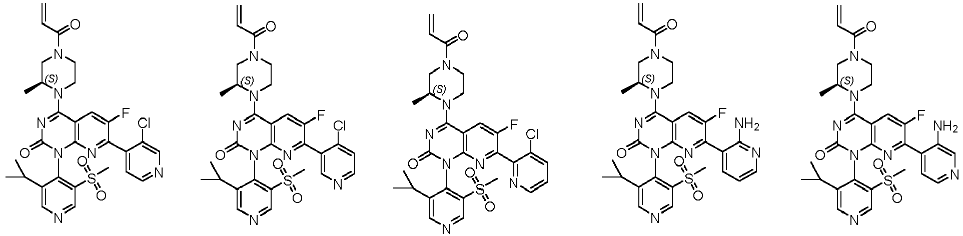

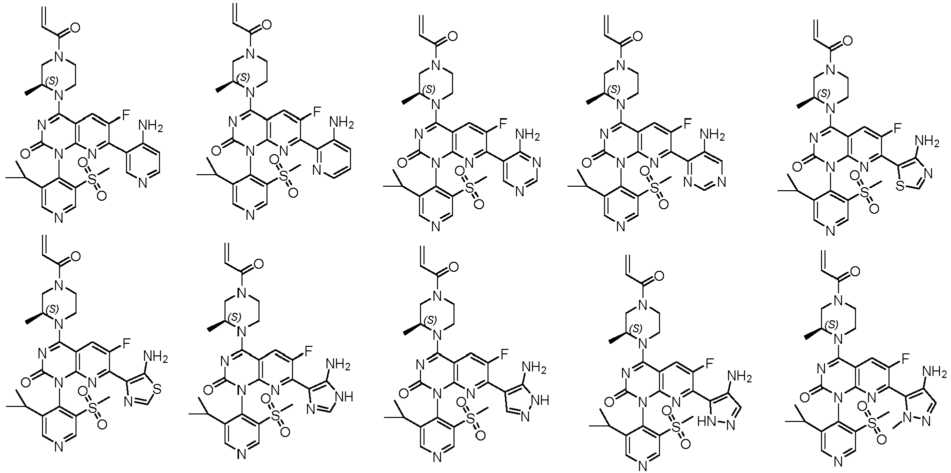

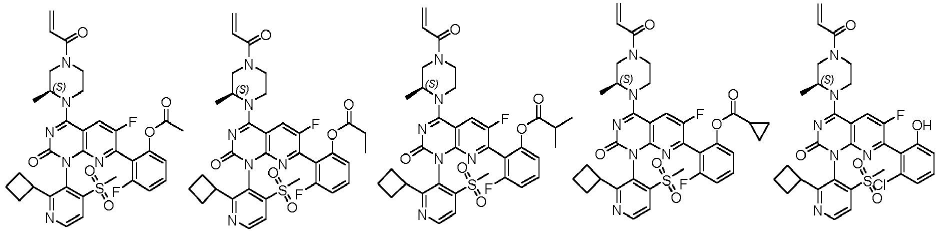

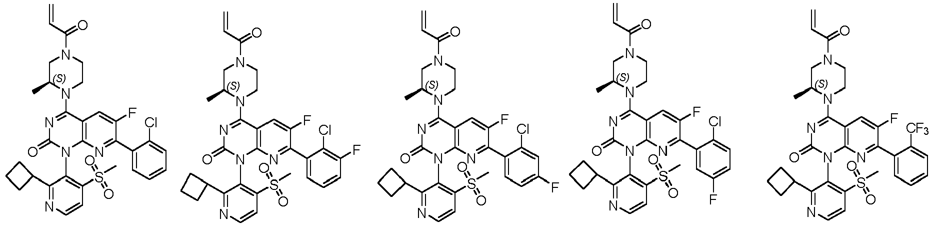

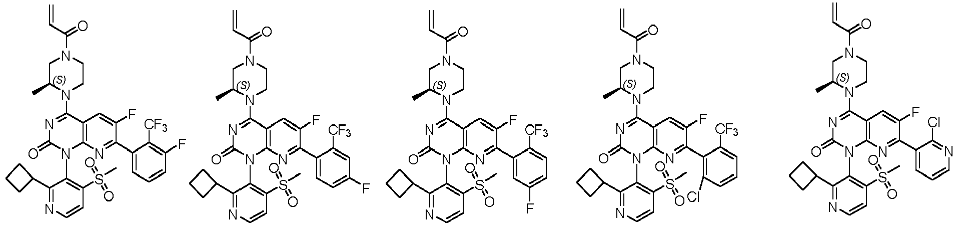

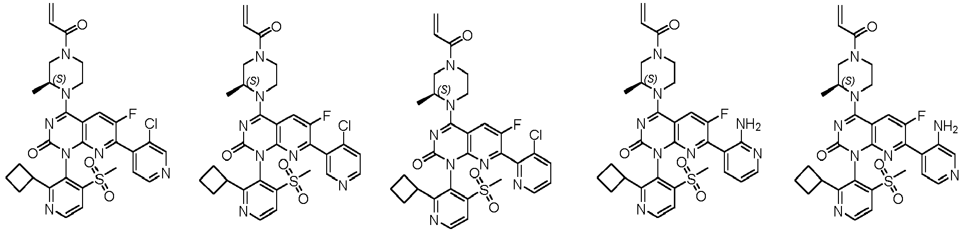

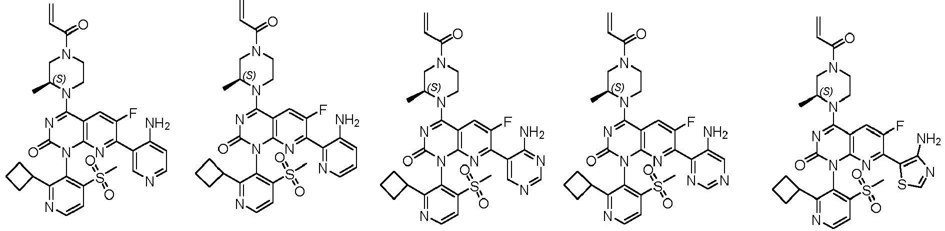

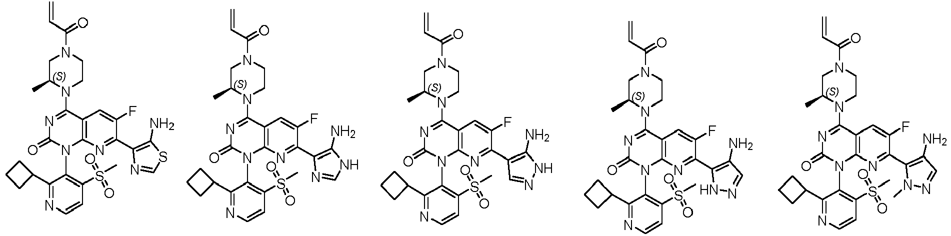

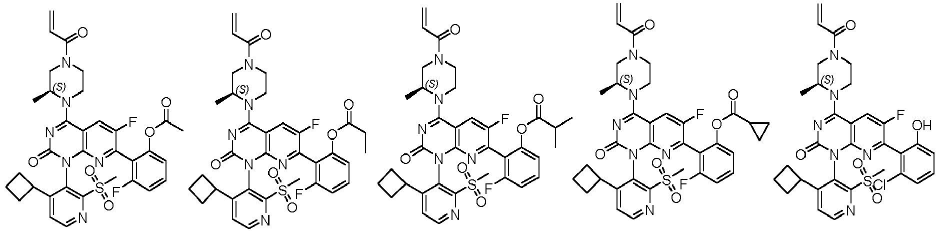

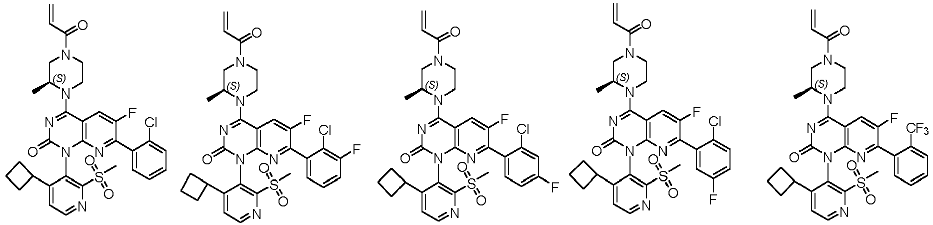

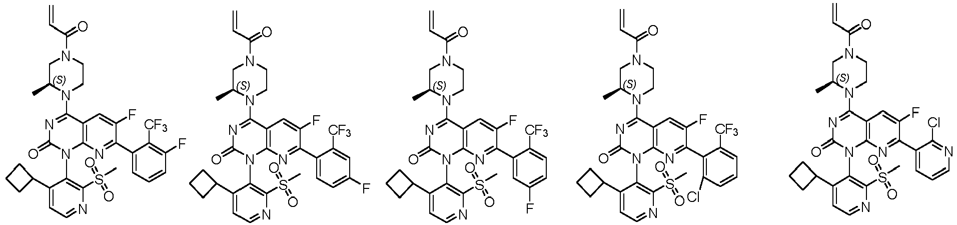

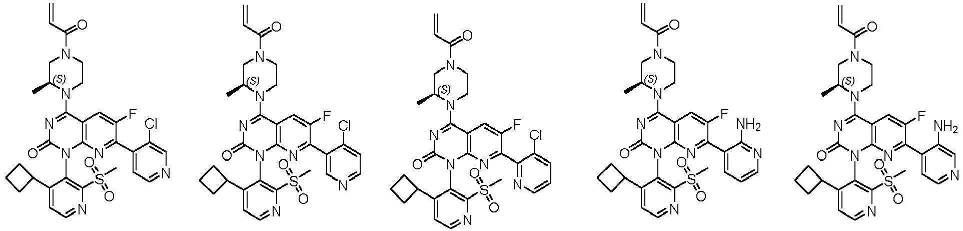

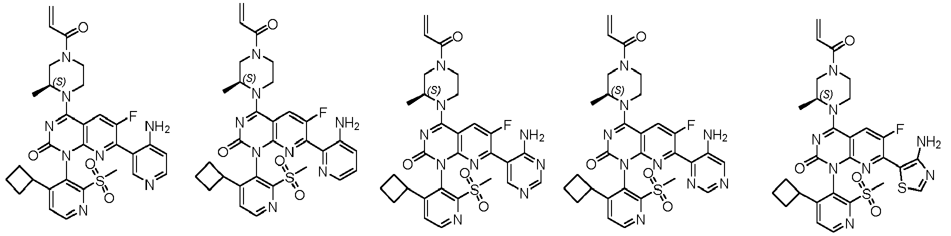

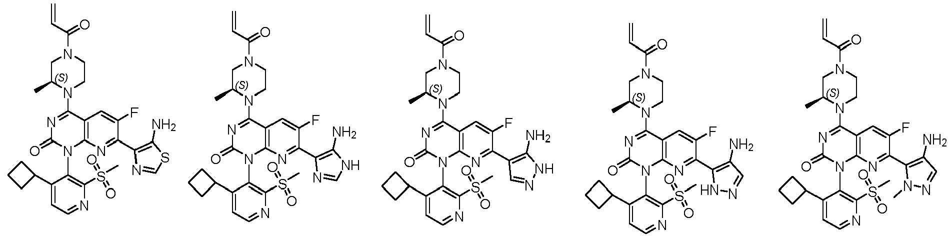

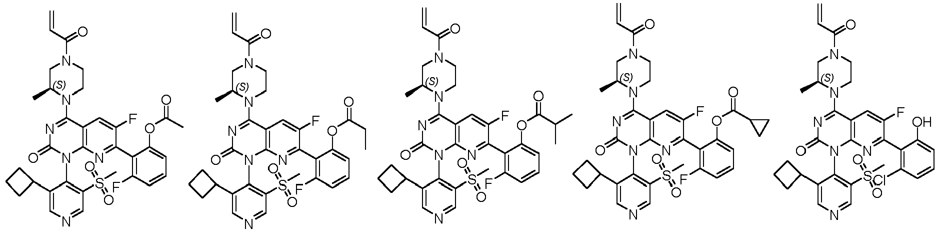

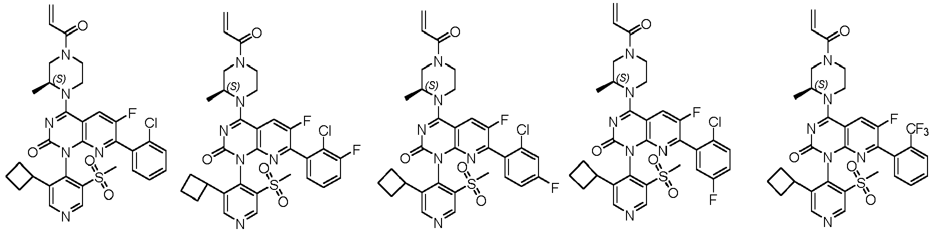

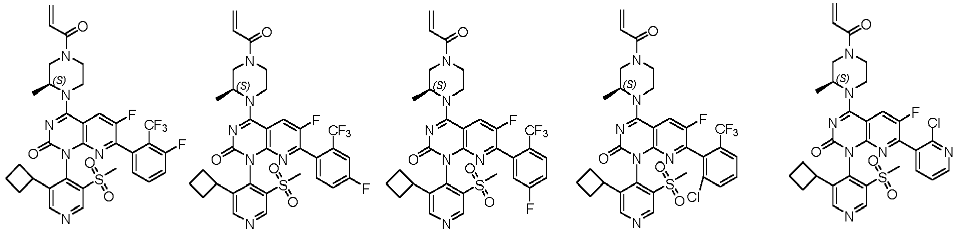

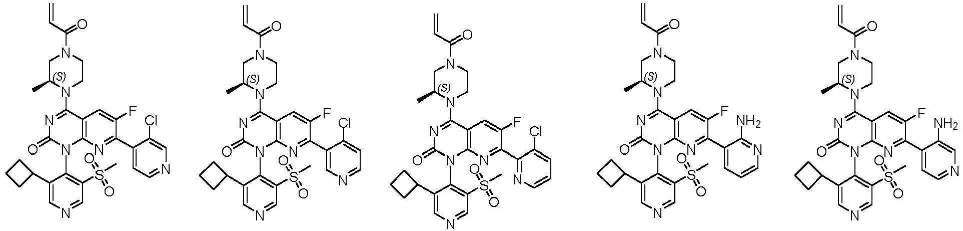

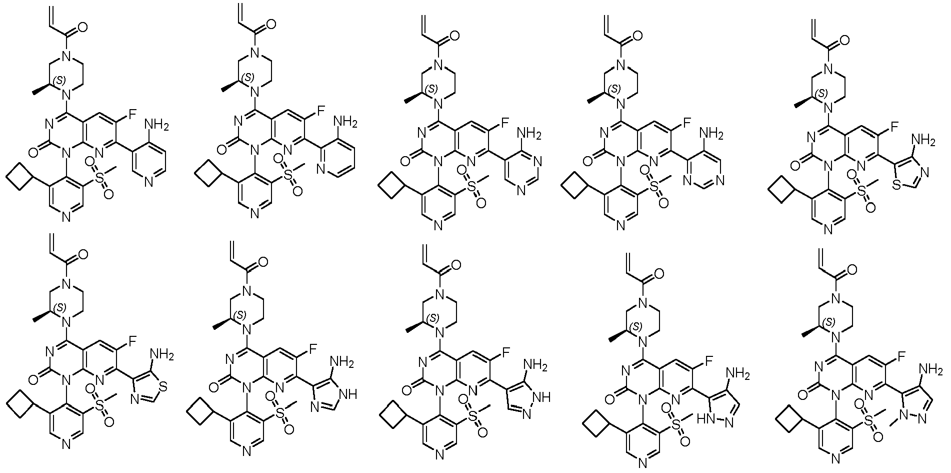

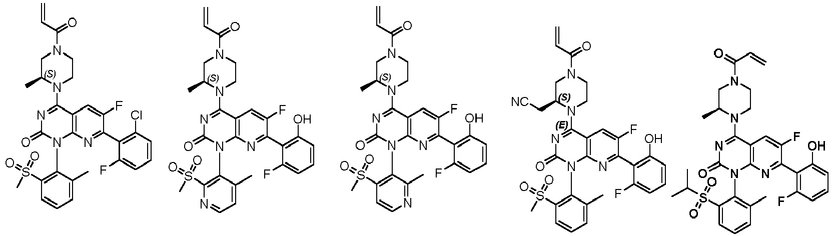

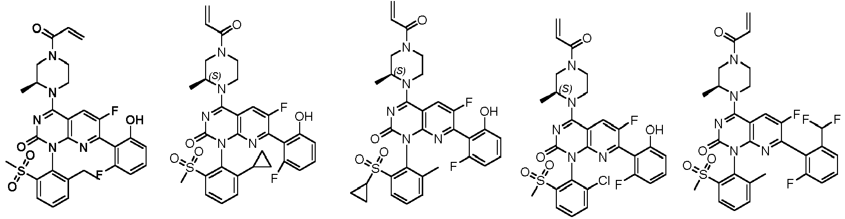

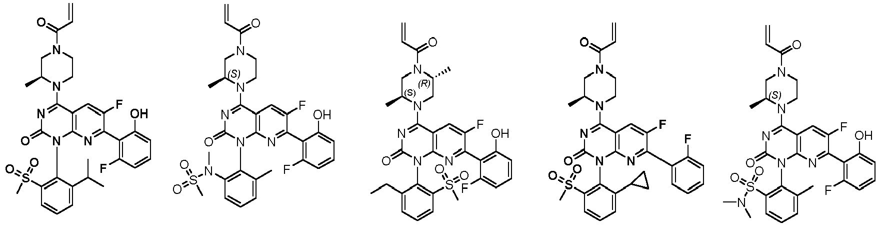

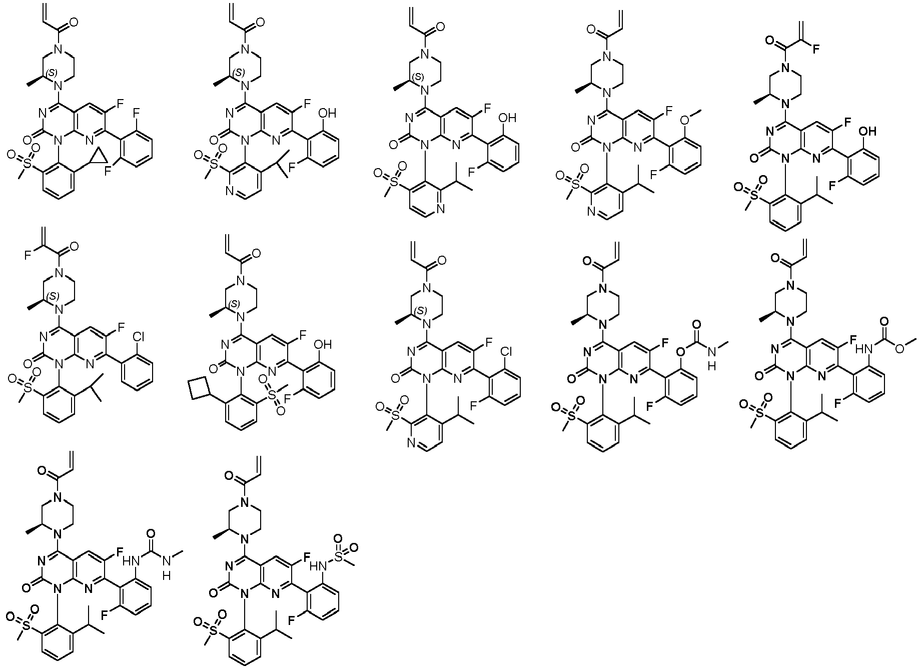

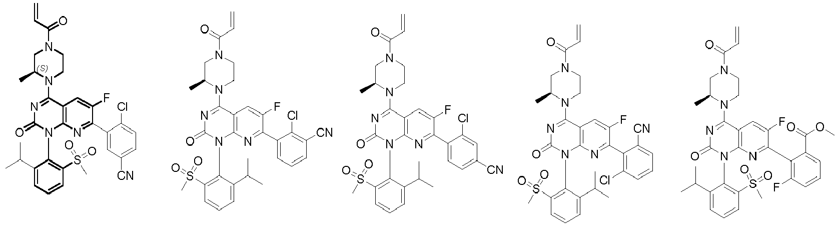

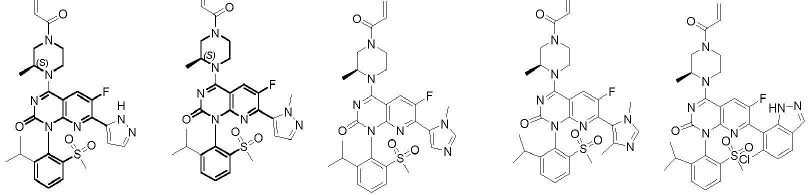

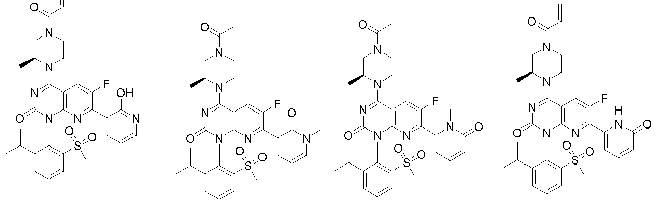

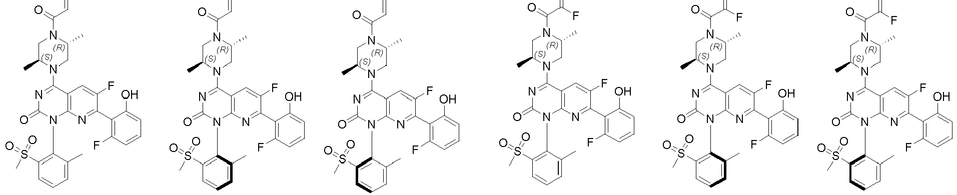

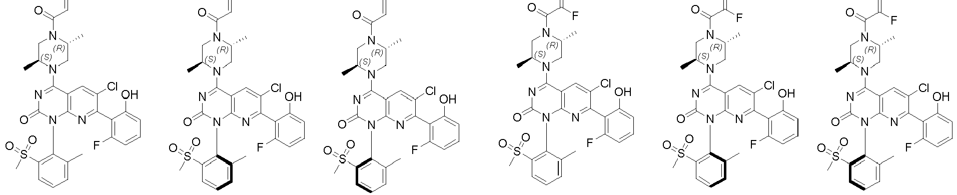

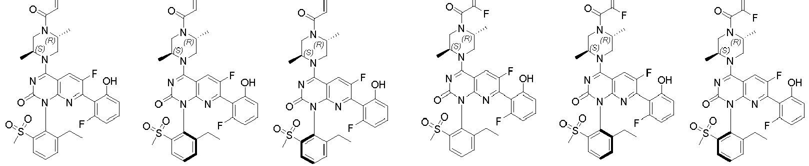

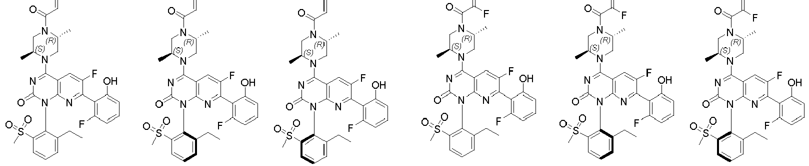

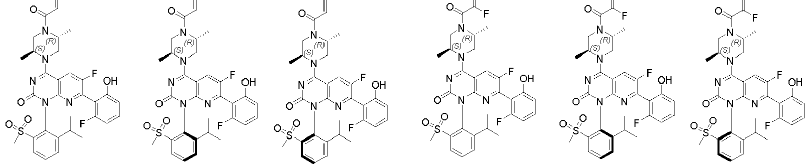

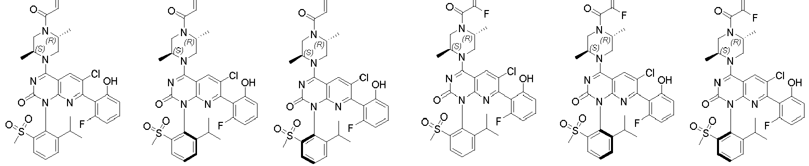

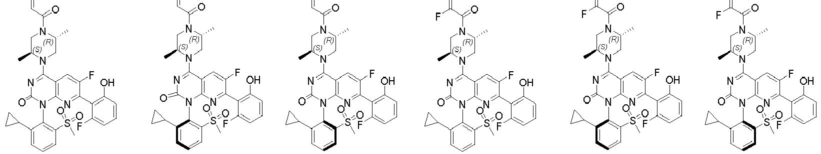

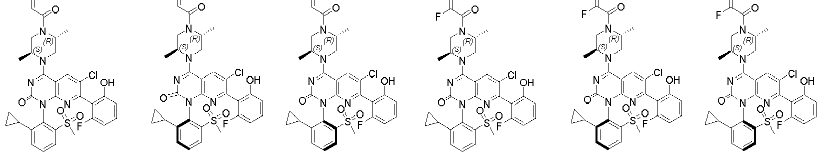

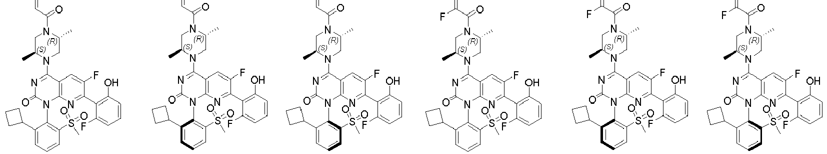

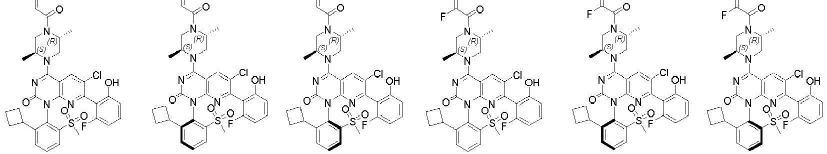

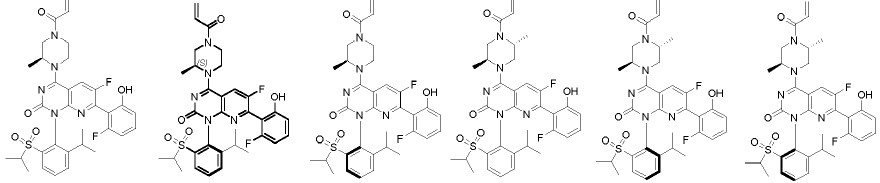

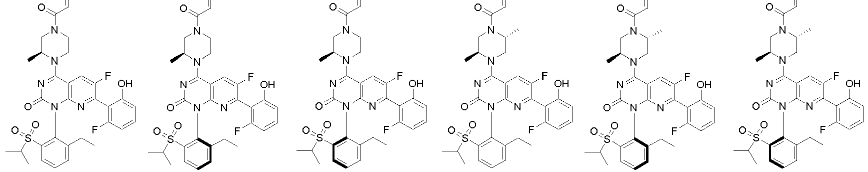

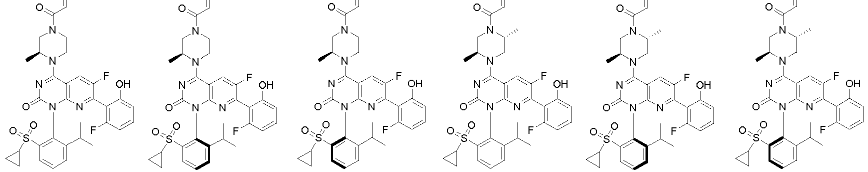

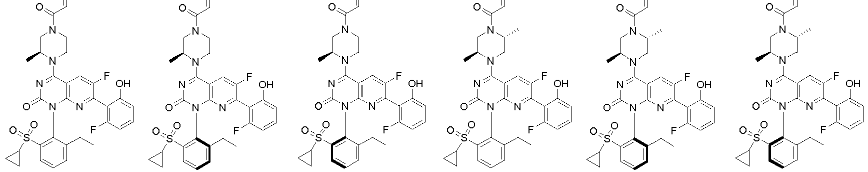

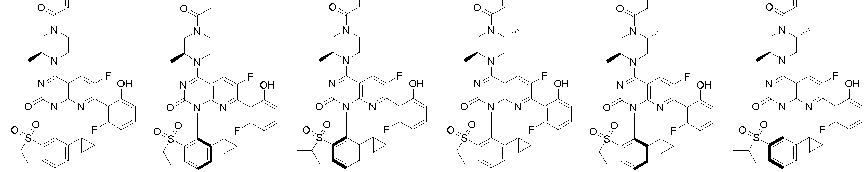

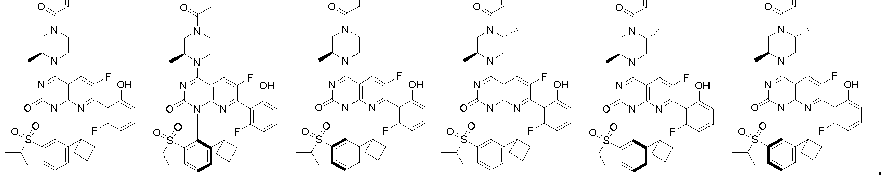

11. Preparation method of the compound of formula (I), or stereoisomer, tautomer, crystal form, pharmaceutically acceptable salt, hydrate, solvate or prodrug thereof comprising the steps: 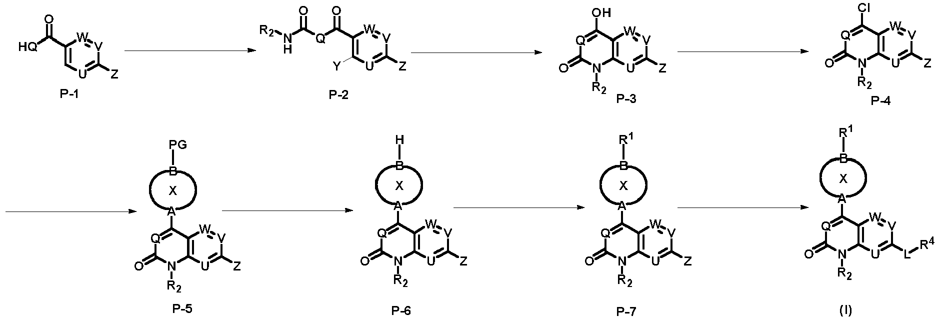
(i) in an inert solvent, the compound of formula P-1 first reacts with oxalyl chloride, and then reacts with the amino compound R₂-NH₂ to obtain the compound of formula P-2;
(ii) in an inert solvent, under the action of a first base, the compound of formula P-2 undergoes a ring closing reaction to obtain the compound of formula P-3;
(iii) in an inert solvent, under the action of a second base, the compound of formula P-3 reacts with phosphorus oxychloride to obtain the compound of formula P-4;
(iv) in an inert solvent, in the presence of a base, the compound of formula P-4 reacts with 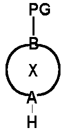 through coupling or substitution reaction to obtain the compound of formula P-5;
(v) in an inert solvent, in the presence of an acid, the compound of formula P-5 is deprotected to obtain the compound of formula P-6;
(vi) in an inert solvent, in the presence of a base, the compound of formula P-6 reacts with R₁E through coupling, substitution or acylation reaction to obtain the compound of formula P-7;
(vii) in an inert solvent, in the presence of base and catalyst, the compound of formula P-7 reacts with R₄-L-E₁ through coupling, substitution or acylation reaction to obtain the compound of formula (I);
wherein,
E is selected from halogen, OH, OCOR¹ or OCO(ⁱBu);
E₁ is selected from -BH₂, -B(OH)₂, 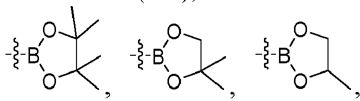 -Sn(Bu)₃, or -ZnBr;
PG is an amino protection group, and the protection group is selected from the group consisting of Boc, Bn, Cbz and Fmoc;
Y and Z are leaving groups, and the leaving groups are selected from the group consisting of halogen and OTf;
the first base is selected from the group consisting of KHMDS, NaHMDS, LiHMDS, NaH, NaOMe, NaOEt, and ^{t}BuONa;
the second base is selected from the group consisting of TEA, DIPEA, DMAP and N,N-dimethylaniline;
R¹, R², R⁴, L, A, B, X, U, V, W and Q are as defined in claim 1.

12. A pharmaceutical composition comprises one or more of the compounds of formula (I), or stereoisomer, tautomer, crystal form, pharmaceutically acceptable salt, hydrate, solvate or prodrug thereof of claim 1; and pharmaceutically acceptable carrier.

13. A use of the compound of formula (I), or stereoisomer, tautomer, crystal form, pharmaceutically acceptable salt, hydrate, solvate or prodrug thereof of claim 1, or the pharmaceutical composition of claim 12 for preparing a medicant for preventing and/or treating the disease related to the activity or expression of KRAS ^{G12C}.
